# EUROPEAN PATENT APPLICATION

(11) **EP 4 778 938 A2**
(43) Date of publication of application: **22.07.2026**
(21) Application number: 26162620.4
(22) Date of filing: 04.06.2020
(51) Int. Cl.: C07K 16/00

(54) **RODENTS HAVING A LIMITED LAMBDA LIGHT CHAIN REPERTOIRE EXPRESSED FROM THE KAPPA LOCUS AND USES THEREOF**

(30) Priority: 05.06.2019 US 201962857712 P
(62) Divisional of application: 20750000.0
(71) Applicant: Regeneron Pharmaceuticals, Inc., Tarrytown, NY 10591 (US)
(72) Inventor: MCWHIRTER, John, New York, 10591 (US); HANSEN, Johanna, New York, 10591 (US); BABB, Robert, New York, 10591 (US); GUO, Chunguang, New York, 10591 (US); MACDONALD, Lynn, New York, 10591 (US); MURPHY, Andrew J., New York, 10591 (US)
(74) Representative: J A Kemp LLP

(57) **Abstract**

The present disclosure provides, among other things, genetically modified non-human animals whose germline genome comprises an engineered endogenous immunoglobulin K light chain locus comprising a single rearranged human immunoglobulin λ light chain variable region operably linked to a non-human Cλ gene segment, where the single rearranged human immunoglobulin λ light chain variable region comprises a human Vλ gene segment and a human Jλ gene segment. All immunoglobulin λ light chains expressed by B cells of the genetically modified non-human animal include human immunoglobulin λ light chain variable domains expressed from the single rearranged human immunoglobulin λ light chain variable region or a somatically hypermutated version thereof. Such animals, tissues from such animals, and cells from such animals represent an effective platform for producing antibodies, e.g., bispecific antibodies.

## Description

### CROSS REFERENCE TO RELATED APPLICATIONS

This application claims priority to United States Provisional Application No. 62/857,712, filed June 5, 2019, which is incorporated herein by reference.

### SEQUENCE LISTING

The instant application contains a Sequence Listing, which has been submitted electronically in ASCII format and is hereby incorporated by reference in its entirety. The ASCII copy, created May 20, 2020, is named 2010794-2050_SL.txt, and is 47,576 bytes in size.

### BACKGROUND

Human antibodies are the most rapidly growing class of therapeutics. Of the technologies that are currently used for their production, the development of genetically engineered animals (e.g., rodents) engineered with genetic material encoding human antibodies, in whole or in part, has revolutionized the field of human therapeutic monoclonal antibodies for the treatment of various diseases. Still, development of improved *in vivo* systems for generating human monoclonal antibodies that maximize human antibody repertoires in host genetically engineered animals is needed.

### SUMMARY

The present disclosure provides genetically modified rodents. In some embodiments, a genetically modified rodent as provided is a rat or a mouse. In some embodiments, all endogenous sequences are rat or mouse sequences. For example, in some embodiments, a genetically modified rodent is a rat and all endogenous sequences are rat sequences. In some embodiments, a genetically modified rodent is a mouse and all endogenous sequences are mouse sequences.

In some embodiments, the present disclosure provides a breeding colony of genetically modified rodents provided herein comprising a first genetically modified rodent, a second genetically modified rodent, and a third genetically modified rodent, where the first, second, and third genetically modified rodent are each a genetically modified rodent as described herein. In some embodiments, a third genetically modified rodent is the progeny of a first genetically modified rodent and a second genetically modified rodent.

A genetically modified rodent as provided has a germline genome comprising a limited human λ light chain variable region repertoire. In some embodiments, a limited human λ light chain variable region repertoire can comprise one or two unrearranged human Vλ gene segments and one or more unrearranged human Jλ gene segments. In some embodiments, a limited human λ light chain variable region repertoire can comprise two unrearranged human Vλ gene segments and four unrearranged human Jλ gene segments. In some embodiments, a limited human λ light chain variable region repertoire can comprise two unrearranged human Vλ gene segments and five unrearranged human Jλ gene segments. In some embodiments, a limited human λ light chain variable region repertoire can comprise a single rearranged human immunoglobulin λ light chain variable region, comprising a single rearranged human immunoglobulin λ light chain variable region that comprises a human Vλ gene segment and a human Jλ gene segment.

In some embodiments, all immunoglobulin λ light chains expressed by B cells of a genetically modified rodent as provided include human immunoglobulin λ light chain variable domains expressed from a limited human λ light chain variable region repertoire. In some embodiments, all immunoglobulin λ light chains expressed by B cells of a genetically modified rodent as provided include human immunoglobulin λ light chain variable domains expressed from a single rearranged human immunoglobulin λ light chain variable region or a somatically hypermutated version thereof. In some embodiments, all immunoglobulin light chains expressed by B cells of a genetically modified rodent provided herein include human immunoglobulin λ light chain variable domains expressed from the single rearranged human immunoglobulin λ light chain variable region or a somatically hypermutated version thereof. In some embodiments, all heavy chains expressed by B cells of the genetically modified rodent include human immunoglobulin heavy chain variable domains and rodent immunoglobulin heavy chain constant domains.

In some embodiments, a genetically modified rodent as provided comprises an engineered endogenous rodent immunoglobulin light chain locus comprising a limited human λ light chain variable region repertoire. In some embodiments, a genetically modified rodent as provided comprises an engineered endogenous rodent immunoglobulin λ light chain locus comprising a limited human λ light chain variable region repertoire. In some embodiments, a genetically modified rodent as provided comprises an engineered endogenous rodent immunoglobulin κ light chain locus comprising a limited human λ light chain variable region repertoire. In some embodiments, the germline genome of the genetically modified rodent is homozygous for the engineered endogenous immunoglobulin light chain locus (e.g., engineered endogenous immunoglobulin λ or κ light chain locus). In some embodiments, the germline genome of the genetically modified rodent is heterozygous for the engineered endogenous immunoglobulin light chain locus (e.g., engineered endogenous immunoglobulin λ or κ light chain locus).

In some embodiments, a germline genome of a genetically modified rodent comprises an engineered endogenous immunoglobulin κ locus comprising two alleles. In some embodiments, a first allele comprises a limited human λ light chain variable region repertoire and a second allele comprises a limited human κ light chain variable region repertoire. In some embodiments, a germline genome of a genetically modified rodent, and thus the rodent cell and the rodent tissue, as described herein, comprises a first engineered endogenous immunoglobulin κ light chain locus allele comprising a single rearranged human immunoglobulin λ light chain variable region operably linked to a rodent Cλ gene segment, wherein the single rearranged human immunoglobulin λ light chain variable region comprises a human Vλ gene segment and a human Jλ gene segment. In some embodiments, the genetically modified rodent, and thus the rodent cell or the rodent tissue, as described herein, comprises a second engineered endogenous immunoglobulin κ light chain locus allele comprising a single rearranged human immunoglobulin κ light chain variable region operably linked to a rodent Cκ gene segment, wherein the single rearranged human immunoglobulin κ light chain variable region comprises a human Vκ gene segment and a human Jκ gene segment. In some embodiments, such a non-human animal or non-human tissue can express a λ light chain from the first engineered endogenous immunoglobulin κ light chain locus allele and a κ light chain from the second engineered endogenous immunoglobulin κ light chain locus allele. In some embodiments, the single rearranged human immunoglobulin κ light chain variable region comprises Vκ3-20 or Vκ1-39 and the single rearranged human immunoglobulin λ light chain variable region comprises Vλ1-51 or Vλ2-14. In one embodiment, the single rearranged human immunoglobulin κ light chain variable region is Vκ3-20/Jκ1, and the single rearranged human immunoglobulin λ light chain variable region is Vλ1-51/Jλ2 or Vλ2-14/Jλ2.

In some embodiments, a genetically modified rodent as provided comprises a limited human λ light chain variable region repertoire operably linked to a light chain constant region gene segment. In some embodiments, a genetically modified rodent as provided comprises a limited human λ light chain variable region repertoire operably linked to a Cκ gene segment. In some embodiments, a genetically modified rodent as provided comprises a limited human λ light chain variable region repertoire operably linked to a Cλ gene segment.

In some embodiments, a genetically modified rodent as provided comprises an engineered endogenous immunoglobulin κ light chain locus comprising a single rearranged human immunoglobulin λ light chain variable region operably linked to a rodent Cλ gene segment, wherein the single rearranged human immunoglobulin λ light chain variable region comprises a human Vλ gene segment and a human Jλ gene segment.

In some embodiments, a human Vλ gene segment is selected from a group consisting of: Vλ4-69, Vλ8-61, Vλ4-60, Vλ6-57, Vλ10-54, Vλ5-52, Vλ1-51, Vλ9-49, Vλ1-47, Vλ7-46, Vλ5-45, Vλ1-44, Vλ7-43, Vλ1-40, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, a human Vλ gene segment is selected from a group consisting of: Vλ5-52, Vλ1-51, Vλ9-49, Vλ1-47, Vλ7-46, Vλ5-45, Vλ1-44, Vλ7-43, Vλ1-40, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, a human Vλ gene segment is selected from a group consisting of: Vλ1-51, Vλ5-45, Vλ1-44, Vλ1-40, Vλ3-21, and Vλ2-14. In some embodiments, a human Vλ gene segment is Vλ1-51 or Vλ2-14. In some embodiments, a human Jλ gene segment is selected from a group consisting of: Jλ1, Jλ2, Jλ3, Jλ6, and Jλ7. In some embodiments, a human Jλ gene segment is selected from a group consisting of: Jλ1, Jλ2, Jλ3, and Jλ7. In some embodiments, a human Jλ gene segment is Jλ2.

In some embodiments, a genetically modified rodent as provided lacks a rodent Cκ gene at an engineered endogenous immunoglobulin κ light chain locus.

In some embodiments, a genetically modified rodent as provided has a germline genome comprising an engineered endogenous immunoglobulin heavy chain locus. In some embodiments, an engineered endogenous immunoglobulin heavy chain locus comprises one or more unrearranged human V_{H} gene segments, one or more unrearranged human D_{H} gene segments, and one or more unrearranged human J_{H} gene segments. In some embodiments, one or more unrearranged human V_{H} gene segments, one or more unrearranged human D_{H} gene segments, and one or more unrearranged human J_{H} gene segments are operably linked to one or more rodent immunoglobulin heavy chain constant region genes.

In some embodiments, a genetically modified rodent as provided has a germline genome comprising an engineered endogenous immunoglobulin heavy chain locus comprising one or more unrearranged human V_{H} gene segments, one or more unrearranged human D_{H} gene segments, and one or more unrearranged human J_{H} gene segments operably linked to one or more rodent immunoglobulin heavy chain constant region genes. In some embodiments, a genetically modified rodent as provided has a germline genome that is homozygous for the engineered endogenous immunoglobulin heavy chain locus.

In some embodiments, a genetically modified rodent as provided has a germline genome comprising one or more unrearranged human V_{H} gene segments, one or more unrearranged human D_{H} gene segments, and one or more unrearranged human J_{H} gene segments in place of one or more endogenous V_{H} gene segments, one or more endogenous D_{H} gene segments, one or more endogenous J_{H} gene segments, or a combination thereof. In some embodiments, a genetically modified rodent as provided has a germline genome comprising one or more unrearranged human V_{H} gene segments, one or more unrearranged human D_{H} gene segments, and one or more unrearranged human J_{H} gene segments that replace one or more endogenous V_{H} gene segments, one or more endogenous D_{H} gene segments, and one or more endogenous J_{H} gene segments, respectively.

In some embodiments, one or more unrearranged human V_{H} gene segments comprise V_{H}3-74, V_{H}3-73, V_{H}3-72, V_{H}2-70, V_{H}1-69, V_{H}3-66, V_{H}3-64, V_{H}4-61, V_{H}4-59, V_{H}1-58, V_{H}3-53, V_{H}5-51, V_{H}3-49, V_{H}3-48, V_{H}1-46, V_{H}1-45, V_{H}3-43, V_{H}4-39, V_{H}4-34, V_{H}3-33, V_{H}4-31, V_{H}3-30, V_{H}4-28, V_{H}2-26,V_{H}1-24, V_{H}3-23, V_{H}3-21, V_{H}3-20, V_{H}1-18, V_{H}3-15, V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2, V_{H}6-1, or any combination thereof. In some embodiments one or more unrearranged human D_{H} gene segments comprise D_{H}1-1, D_{H}2-2, D_{H}3-3, D_{H}4-4, D_{H}5-5, D_{H}6-6, D_{H}1-7, D_{H}2-8, D_{H}3-9, D_{H}3-10, D_{H}5-12, D_{H}6-13, D_{H}2-15, D_{H}3-16, D_{H}4-17, D_{H}6-19, D_{H}1-20, D_{H}2-21, D_{H}3-22, D_{H}6-25, D_{H}1-26, D_{H}7-27, or any combination thereof. In some embodiments, one or more unrearranged human J_{H} gene segments comprise J_{H}1, J_{H}2, J_{H}3, J_{H}4, J_{H}5, J_{H}6, or any combination thereof.

In some embodiments, (i) one or more unrearranged human V_{H} gene segments comprise V_{H}3-74, V_{H}3-73, V_{H}3-72, V_{H}2-70, V_{H}1-69, V_{H}3-66, V_{H}3-64, V_{H}4-61, V_{H}4-59, V_{H}1-58, V_{H}3-53, V_{H}5-51, V_{H}3-49, V_{H}3-48, V_{H}1-46, V_{H}1-45, V_{H}3-43, V_{H}4-39, V_{H}4-34, V_{H}3-33, V_{H}4-31, V_{H}3-30, V_{H}4-28, V_{H}2-26, V_{H}1-24, V_{H}3-23, V_{H}3-21, V_{H}3-20, V_{H}1-18, V_{H}3-15, V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2, V_{H}6-1, or any combination thereof, (ii) one or more unrearranged human D_{H} gene segments comprise D_{H}1-1, D_{H}2-2, D_{H}3-3, D_{H}4-4, D_{H}5-5, D_{H}6-6, D_{H}1-7, D_{H}2-8, D_{H}3-9, D_{H}3-10, D_{H}5-12, D_{H}6-13, D_{H}2-15, D_{H}3-16, D_{H}4-17, D_{H}6-19, D_{H}1-20, D_{H}2-21, D_{H}3-22, D_{H}6-25, D_{H}1-26, D_{H}7-27, or any combination thereof, and (iii) one or more unrearranged human J_{H} gene segments comprise J_{H}1, J_{H}2, J_{H}3, J_{H}4, J_{H}5, J_{H}6, or any combination thereof.

In some embodiments, a genetically modified rodent as provided has a germline genome comprising one or more rodent immunoglobulin heavy chain constant region genes. In some embodiments, one or more rodent immunoglobulin heavy chain constant region genes are one or more endogenous rodent immunoglobulin heavy chain constant region genes.

In some embodiments, a genetically modified rodent as provided has a germline genome comprising an engineered endogenous immunoglobulin heavy chain locus lacking a functional endogenous rodent Adam6 gene. In some embodiments, a genetically modified rodent as provided has a germline genome comprising one or more nucleotide sequences encoding one or more rodent ADAM6 polypeptides, functional orthologs, functional homologs, or functional fragments thereof. In some embodiments, a genetically modified rodent as provided expresses one or more rodent ADAM6 polypeptides, functional orthologs, functional homologs, or functional fragments thereof. In some embodiments, a genetically modified rodent as provided has a germline genome comprising one or more nucleotide sequences encoding one or more rodent ADAM6 polypeptides, functional orthologs, functional homologs, or functional fragments thereof that are included on the same chromosome as the engineered endogenous immunoglobulin heavy chain locus. In some embodiments, a genetically modified rodent as provided has a germline genome comprising an engineered endogenous immunoglobulin heavy chain locus comprisinig one or more nucleotide sequences encoding one or more rodent ADAM6 polypeptides, functional orthologs, functional homologs, or functional fragments thereof. In some embodiments, a genetically modified rodent as provided has a germline genome comprising one or more nucleotide sequences encoding one or more rodent ADAM6 polypeptides, functional orthologs, functional homologs, or functional fragments thereof in place of a human Adam6 pseudogene. In some embodiments, a genetically modified rodent as provided has a germline genome comprising one or more nucleotide sequences encoding one or more rodent ADAM6 polypeptides, functional orthologs, functional homologs, or functional fragments thereof that replace a human Adam6 pseudogene.

In some embodiments, a genetically modified rodent as provided has a germline genome comprising one or more human V_{H} gene segments comprising a first and a second human V_{H} gene segment, and one or more nucleotide sequences encoding one or more rodent ADAM6 polypeptides, functional orthologs, functional homologs, or functional fragments thereof between the first human V_{H} gene segment and the second human V_{H} gene segment. In some embodiments, a first human V_{H} gene segment is V_{H}1-2 and a second human V_{H} gene segment is V_{H}6-1.

In some embodiments, one or more nucleotide sequences encoding one or more rodent ADAM6 polypeptides, functional orthologs, functional homologs, or functional fragments thereof are between a human V_{H} gene segment and a human D_{H} gene segment.

In some embodiments, a rodent Cλ gene has a sequence that is at least 80%, at least 85%, at least 90%, at least 95%, at least 98% or at least 99% identical to a mouse Cλ1. In some embodiments, a rodent Cλ gene has a sequence that is at least 80%, at least 85%, at least 90%, at least 95%, at least 98% or at least 99% identical to a mouse Cλ2. In some embodiments, a rodent Cλ gene has a sequence that is at least 80%, at least 85%, at least 90%, at least 95%, at least 98% or at least 99% identical to a mouse Cλ3 gene.

In some embodiments, a rodent Cλ gene is or comprises a mouse Cλ gene. In some embodiments, a rodent Cλ gene is or comprises a mouse Cλ1 gene.

In some embodiments, a rodent Cλ gene has a sequence that is at least 80%, at least 85%, at least 90%, at least 95%, at least 98% or at least 99% identical to a rat Cλ1. In some embodiments, a rodent Cλ gene has a sequence that is at least 80%, at least 85%, at least 90%, at least 95%, at least 98% or at least 99% identical to a rat Cλ2. In some embodiments, a rodent Cλ gene has a sequence that is at least 80%, at least 85%, at least 90%, at least 95%, at least 98% or at least 99% identical to a rat Cλ3. In some embodiments, a rodent Cλ gene has a sequence that is at least 80%, at least 85%, at least 90%, at least 95%, at least 98% or at least 99% identical to a rat Cλ4 gene.

In some embodiments, a rodent Cλ gene is or comprises a rat Cλ gene.

In some embodiments, a single rearranged human immunoglobulin λ light chain variable region is in place of one or more rodent Vκ gene segments, one or more rodent Jκ gene segments, or any combination thereof. In some embodiments, a single rearranged human immunoglobulin λ light chain variable region replaces one or more rodent Vκ gene segments, one or more rodent Jκ gene segments, or any combination thereof.

In some embodiments, a genetically modified mouse provided herein comprises a functional endogenous immunoglobulin λ light chain locus. In some embodiments, a genetically modified mouse provided herein comprises an inactivated endogenous immunoglobulin λ light chain locus. In some embodiments, an endogenous immunoglobulin λ light chain locus is inactivated by deleting or inverting all or a portion of an endogenous immunoglobulin λ light chain locus. In some embodiments, endogenous Vλ gene segments, endogenous Jλ gene segments, and endogenous Cλ genes are deleted in whole or in part.

In some embodiments, a genetically modified mouse provided herein does not detectably express endogenous immunoglobulin κ light chain variable domains.

The present disclosure provides rodent embryos comprising genetic modifications as described herein. In some embodiments, a rodent embryo has a genome comprising an engineered endogenous immunoglobulin κ light chain locus comprising a single rearranged human immunoglobulin λ light chain variable region operably linked to a rodent Cλ gene segment, where the single rearranged human immunoglobulin λ light chain variable region comprises a human Vλ gene segment and a human Jλ gene segment. In some embodiments, the genome of a rodent embryo is homozygous for the engineered endogenous immunoglobulin κ light chain locus. In some embodiments, a human Vλ gene segment is selected from a group consisting of: Vλ1-51, Vλ5-45, Vλ1-44, Vλ1-40, Vλ3-21, and Vλ2-14. In some embodiments, a human Jλ gene segment is selected from a group consisting of: Jλ1, Jλ2, Jλ3, Jλ6, and Jλ7. In some embodiments, a human Vλ gene segment is Vλ1-51 or Vλ2-14. In some embodiments, a human Jλ gene segment is Jλ2.

In some embodiments, a rodent embryo is provided whose genome comprises an engineered endogenous immunoglobulin heavy chain locus comprising one or more unrearranged human V_{H} gene segments, one or more unrearranged human D_{H} gene segments, and one or more unrearranged human J_{H} gene segments operably linked to one or more endogenous immunoglobulin heavy chain constant region genes. In some embodiments, the genome of a rodent embryo is homozygous for the engineered endogenous immunoglobulin heavy chain locus.

The present disclosure provides B cells of a genetically modified rodent described herein. In some embodiments, a B cell of a genetically modified rodent described herein comprises a single rearranged human immunoglobulin λ light chain variable region of the engineered endogenous κ light chain locus or a somatically hypermutated version thereof.

In some embodiments, the present disclosure provides a B cell of a genetically modified rodent as described herein, comprising a single rearranged human immunoglobulin λ light chain variable region of the engineered endogenous κ light chain locus or somatically hypermutated version thereof. In some embodiments, a B cell of the genetically modified rodent as described herein comprises a rearranged human immunoglobulin heavy chain variable region derived from a human V_{H} gene segment of the one or more unrearranged human V_{H} gene segments, a human D_{H} gene segment of the one or more unrearranged human D_{H} gene segments, and a human J_{H} gene segment of the one or more unrearranged human J_{H} gene segments in the engineered endogenous heavy chain locus.

The present disclosure provides hybridomas generated from a B cell of a genetically modified rodent as described herein.

The present disclosure provides a population of B cells from a single, genetically modified rodent as described herein. In some embodiments, all antibodies expressed by the population of B cells include human immunoglobulin λ light chain variable domains expressed from a single rearranged human immunoglobulin λ light chain variable region or a somatically hypermutated version thereof. In some embodiments, antibodies expressed by the population of B cells include a plurality of human immunoglobulin heavy chain variable domains expressed from at least two different rearranged human immunoglobulin heavy chain variable regions or somatically hypermutated version thereof.

The present disclosure provides stem cells, such as embryonic stem (ES) cells comprising genetic modifications as described herein. In some embodiments, a stem cell (e.g., ES cell) comprises an engineered endogenous immunoglobulin κ light chain locus comprising a single rearranged human immunoglobulin λ light chain variable region operably linked to a rodent Cλ gene segment. In some embodiments, a genome of a stem cell (e.g., ES cell) is homozygous for the engineered endogenous immunoglobulin κ light chain locus. In some embodiments, a human Vλ gene segment is selected from a group consisting of: Vλ4-69, Vλ8-61, Vλ4-60, Vλ6-57, Vλ10-54, Vλ5-52, Vλ1-51, Vλ9-49, Vλ1-47, Vλ7-46, Vλ5-45, Vλ1-44, Vλ7-43, Vλ1-40, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, a human Vλ gene segment is selected from a group consisting of: Vλ5-52, Vλ1-51, Vλ9-49, Vλ1-47, Vλ7-46, Vλ5-45, Vλ1-44, Vλ7-43, Vλ1-40, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, a human Vλ gene segment is selected from a group consisting of: Vλ1-51, Vλ5-45, Vλ1-44, Vλ1-40, Vλ3-21, and Vλ2-14. In some embodiments, a human Jλ gene segment is selected from a group consisting of: Jλ1, Jλ2, Jλ3, Jλ6, and Jλ7. In some embodiments, a human Jλ gene segment is selected from a group consisting of: Jλ1, Jλ2, Jλ3, and Jλ7. In some embodiments, a human Vλ gene segment is Vλ1-51 or Vλ2-14. In some embodiments, a human Jλ gene segment is Jλ2.

In some embodiments, a stem cell (e.g., ES cell) comprises an engineered endogenous immunoglobulin heavy chain locus comprising one or more unrearranged human V_{H} gene segments, one or more unrearranged human D_{H} gene segments, and one or more unrearranged human J_{H} gene segments operably linked to one or more endogenous immunoglobulin heavy chain constant region genes.

A mammalian cell expressing an antibody is provided by the present disclosure. In some embodiments, an antibody expressed by a mammalian cell comprises a heavy chain comprising a human immunoglobulin heavy chain variable domain and a light chain comprising a human immunoglobulin λ light chain variable domain, where the human immunoglobulin heavy chain variable domain, the human immunoglobulin λ light chain variable domain, or both were identified from, were isolated from, or are identical to a genetically modified rodent described herein.

In some embodiments, an antibody prepared by a method comprising the steps of (a) exposing a genetically modified rodent described herein to an antigen of interest; (b) maintaining the genetically modified rodent under conditions sufficient for the genetically modified rodent to produce an immune response to the antigen of interest; and (c) recovering from the genetically modified rodent: (i) an antibody that binds the antigen of interest, (ii) a nucleotide that encodes a human light variable domain, a human heavy chain variable domain, a light chain, or a heavy chain of an antibody that binds the antigen of interest, or (iii) a cell that expresses an antibody that binds the antigen of interest, where an antibody of (c) includes human heavy chain variable and human λ light chain variable domains. In some embodiments, an antibody is a bispecific antibody.

In some embodiments, a method of making an antibody comprises the steps of (a) exposing a genetically modified rodent as described herein to an antigen; (b) allowing the genetically modified rodent to develop an immune response to the antigen; and (c) isolating an antibody specific to the antigen, a B cell expressing an antibody specific to the antigen, or one or more nucleotide sequences encoding an antibody specific to the antigen from the genetically modified rodent. In some embodiments, an antibody is a bispecific antibody.

In some embodiments, a method of making an antibody comprises the steps of: (a) expressing in a mammalian cell said antibody comprising two human immunoglobulin λ light chains and two human immunoglobulin heavy chains, where each human immunoglobulin λ light chain includes a human immunoglobulin λ light chain variable domain and each human immunoglobulin heavy chain includes a human immunoglobulin heavy chain variable domain, where the amino acid sequence of at least one of the human immunoglobulin heavy chain variable domains, at least one of the λ light chain variable domains, or a combination thereof was identified from or was isolated from, a genetically modified rodent as described herein; and (b) obtaining the antibody. In some embodiments, an antibody is a bispecific antibody.

In some embodiments, a method of making a bispecific antibody is provided, comprising: (a) contacting a first genetically modified rodent as described herein with a first epitope of a first antigen, (b) contacting a second genetically modified rodent as described herein with a second epitope of a second antigen, (c) isolating a B cell that expresses a first antibody specific for the first epitope of the first antigen from the first genetically modified rodent and determining a first human immunoglobulin heavy chain variable domain of the first antibody; (d) isolating a B cell that expresses a second antibody specific for the second epitope of the second antigen from the second genetically modified rodent and determining a second human immunoglobulin heavy chain variable domain of the second antibody; (e) operably linking a nucleotide sequence encoding the first human immunoglobulin heavy chain variable domain to a nucleotide sequence encoding a first human immunoglobulin constant domain to produce a first nucleotide sequence encoding a first human heavy chain; (f) operably linking a nucleotide sequence encoding the second human immunoglobulin heavy chain variable domain to a nucleotide sequence encoding a second human immunoglobulin constant domain to produce a second nucleotide sequence encoding a second human heavy chain; (g) expressing in a mammalian cell: (i) the first nucleotide sequence; (ii) the second nucleotide sequence; and (iii) a third nucleotide sequence comprising the single rearranged human immunoglobulin λ light chain variable region or a somatically hypermutated version thereof operably linked to a human immunoglobulin λ light chain constant region.

In some embodiments, a method of making a bispecific antibody is provided herein, which method comprises (a) expressing in a mammalian cell: (i) a first nucleotide sequence comprising a first human immunoglobulin heavy chain variable region operably linked to a first human immunoglobulin constant region; (ii) a second nucleotide sequence comprising a second human immunoglobulin heavy chain variable region operably linked to a second human immunoglobulin constant region; and (iii) a third nucleotide sequence comprising human immunoglobulin λ light chain variable region operably linked to a human immunoglobulin λ light chain constant region. In some embodiments, a first human immunoglobulin heavy chain variable region encodes a first human heavy chain variable domain identified from, isolated from, or identical to a first antibody in a first genetically modified rodent as described herein that had been immunized with a first epitope of a first antigen, where the first antibody specifically binds the first epitope of the first antigen. In some embodiments, a second human immunoglobulin heavy chain variable region encodes a second human heavy chain variable domain identified from, isolated from, or identical to a second antibody in a second genetically modified rodent as described herein that had been immunized with a second epitope of a second antigen, where the second antibody specifically binds the second epitope of the second antigen. In some embodiments, a human immunoglobulin λ light chain variable region of a third nucleotide is a single rearranged human immunoglobulin λ light chain variable region or a somatically hypermutated version thereof.

In some embodiments, a first genetically modified rodent and a second genetically modified rodent are the same genetically modified rodent. In some embodiments, a first genetically modified rodent and a second genetically modified rodent are different genetically modified rodents.

In some embodiments, a first antigen and a second antigen are the same antigen, and a first epitope and a second epitope are different epitopes. In some embodiments, a first antigen and a second antigen are different antigens.

In some embodments, a method for making a human immunoglobulin heavy chain comprises the steps of (a) exposing a genetically modified rodent as described herein to an antigen of interest; (b) obtaining a human immunoglobulin heavy chain variable domain sequence of an antibody that specifically binds the antigen and that was generated by the genetically modified rodent; and (c) operably linking the human immunoglobulin heavy variable domain sequence to a human immunoglobulin heavy chain constant domain sequence to form a human immunoglobulin heavy chain. In some embodiments, a human immunoglobulin heavy chain generated by the method of this paragraph is provided.

In some embodiments, a method for making a human immunoglobulin heavy chain variable domain comprises the steps of (a) exposing a genetically modified rodent as described herein to an antigen of interest; and (b) obtaining a human immunoglobulin heavy chain variable domain sequence of an antibody that specifically binds the antigen and that was generated by the genetically modified rodent. In some embodiments, a human immunoglobulin heavy chain variable domain generated by this method of this paragraph is provided.

In some embodiments, a method of making a collection of human immunoglobulin heavy chain variable domains comprises the steps of (a) exposing a genetically modified rodent as described herein to an antigen of interest, and (b) isolating the collection of human immunoglobulin heavy chain variable domains from the genetically modified rodent. In some embodiments, a collection of human immunoglobulin heavy chain variable domains each bind to a human immunoglobulin λ light chain variable domain expressed from the single rearranged human immunoglobulin λ light chain variable region or a somatically hypermutated version thereof, where the human λ light chain variable domain, paired with any one of the human immunoglobulin heavy chain variable domains in the collection, binds an antigen.

In some embodiments, a method for making a human immunoglobulin λ light chain comprises the steps of: (a) exposing a genetically modified rodent as described herein to an antigen of interest; (b) obtaining a human immunoglobulin λ light chain variable domain sequence of an antibody that specifically binds the antigen and that was generated by the genetically modified rodent; and (c) operably linking the human immunoglobulin λ light variable domain sequence to a human immunoglobulin λ light chain constant domain sequence to form a human immunoglobulin λ light chain. In some embodiments, a human immunoglobulin λ light chain generated by the method of this paragraph is provided.

In some embodiments, a method for generating a human immunoglobulin λ light chain variable domain comprises the steps of: (a) exposing a genetically modified rodent described herein to an antigen of interest; and (b) obtaining a human immunoglobulin λ light chain variable domain sequence of an antibody that specifically binds the antigen and that was generated by the genetically modified rodent. In some embodimetns, a human immunoglobulin λ light chain variable domain generated by the method of this paragraph is provided.

In some embodiments, a method for making a nucleotide sequence encoding a human immunoglobulin heavy chain comprises the steps of (a) exposing a genetically modified rodent as described herein to an antigen of interest; (b) obtaining a human immunoglobulin heavy chain variable region encoding a human immunoglobulin heavy chain variable domain sequence of an antibody that specifically binds the antigen and that was generated by the genetically modified rodent; and (c) operably linking the human immunoglobulin heavy variable region to a human immunoglobulin heavy chain constant region sequence to form a nucleotide sequence encoding a human immunoglobulin heavy chain. In some embodiments, a nucleotide sequence encoding a human immunoglobulin heavy chain generated by the method of this paragraph is provided.

In some embodiments, a method for making a nucleotide sequence comprising a human immunoglobulin heavy chain variable region comprises the steps of: (a) exposing a genetically modified rodent as described herein to an antigen of interest; and (b) obtaining a human immunoglobulin heavy chain variable region encoding a human immunoglobulin heavy chain variable domain sequence of an antibody that specifically binds the antigen and that was generated by the genetically modified rodent. In some embodiments, a nucleotide sequence comprising a human immunoglobulin heavy chain variable region generated by the method of this paragraph is provided.

In some embodiments, a method for making a nucleotide sequence encoding a human immunoglobulin λ light chain comprises the steps of: (a) exposing a genetically modified rodent as described herein to an antigen of interest; (b) obtaining a human immunoglobulin λ light chain variable region encoding a human immunoglobulin λ light chain variable domain sequence of an antibody that specifically binds the antigen and that was generated by the genetically modified rodent; and (c) operably linking the human immunoglobulin λ light variable region to a human immunoglobulin λ light chain constant region sequence to form a nucleotide sequence encoding a human immunoglobulin λ light chain. In some embodiments, a nucleotide sequence encoding a human immunoglobulin λ light chain generated by the method of this paragraph is provided.

In some embodiments, a method for making a nucleotide sequence comprising a human immunoglobulin λ light chain variable region comprises the steps of: (a) exposing a genetically modified rodent as described herein to an antigen of interest; and (b) obtaining a human immunoglobulin λ light chain variable region encoding a human immunoglobulin λ light chain variable domain sequence of an antibody that specifically binds the antigen and that was generated by the genetically modified rodent. In some embodiments, a nucleotide sequence comprising a human immunoglobulin λ light chain variable region generated by the method of this paragraph is provided.

In some embodiments, a targeting vector is provided. In some embodiments, a targeting vector comprises (i) a 5' homology arm comprising a nucleotide sequence corresponding to a 5' target sequence in an endogenous rodent κ light chain locus; (ii) a single rearranged human immunoglobulin λ light chain variable region comprising a Vλ gene segment and a Jλ gene segment; (iii) a rodent Cλ gene segment; and (iv) a 3' homology arm comprising a nucleotide sequence corresponding to a 3' target sequence in the endogenous rodent κ light chain locus. In some embodiments, a human Vλ gene segment is selected from a group consisting of: Vλ4-69, Vλ8-61, Vλ4-60, Vλ6-57, Vλ10-54, Vλ5-52, Vλ1-51, Vλ9-49, Vλ1-47, Vλ7-46, Vλ5-45, Vλ1-44, Vλ7-43, Vλ1-40, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, a human Vλ gene segment is selected from a group consisting of: Vλ5-52, Vλ1-51, Vλ9-49, Vλ1-47, Vλ7-46, Vλ5-45, Vλ1-44, Vλ7-43, Vλ1-40, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, a human Vλ gene segment is selected from a group consisting of: Vλ1-51, Vλ5-45, Vλ1-44, Vλ1-40, Vλ3-21, and Vλ2-14. In some embodiments, a human Vλ gene segment is Vλ1-51 or Vλ2-14. In some embodiments, a human Jλ gene segment is selected from a group consisting of: Jλ1, Jλ2, Jλ3, Jλ6, and Jλ7. In some embodiments, a human Jλ gene segment is selected from a group consisting of: Jλ1, Jλ2, Jλ3, and Jλ7. In some embodiments, a human Jλ gene segment is Jλ2.

In some embodiments, a method of making a genetically modified rodent described herein comprises the steps of: (a) introducing a single rearranged human immunoglobulin λ light chain variable region comprising a human Vλ gene segment and a human Jλ gene segment into an engineered endogenous immunoglobulin κ light chain locus in the genome of a rodent ES cell; and (b) generating a rodent using the rodent ES cell generated in step (a).

In some embodiments, a method of making a genetically modified rodent described herein comprises the steps of: (a) introducing a single rearranged human immunoglobulin λ light chain variable region operably linked to a rodent Cλ gene segment into an engineered endogenous immunoglobulin κ light chain locus in the genome of a rodent ES cell, wherein the single rearranged human immunoglobulin λ light chain variable region comprises a human Vλ gene segment and a human Jλ gene segment; and (b) generating a rodent using the rodent ES cell generated in step (a). In some embodiments, the genome of a rodent ES cell comprises one or more unrearranged human V_{H} gene segments, one or more unrearranged human D_{H} gene segments, and one or more unrearranged human J_{H} gene segments operably linked to one or more endogenous immunoglobulin heavy chain constant region genes at an engineered endogenous immunoglobulin heavy chain locus. In some embodiments, the genome of a rodent ES cell further comprises one or more nucleotide sequences encoding one or more rodent ADAM6 polypeptides, functional orthologs, functional homologs, or functional fragments thereof.

In some embodiments, a method of making a genetically modified rodent ES cell comprises the step of introducing a single rearranged human immunoglobulin λ light chain variable region comprising a human Vλ gene segment and a human Jλ gene segment into an engineered endogenous immunoglobulin κ light chain locus in the genome of a rodent ES cell. In some embodiments, a method of making a genetically modified rodent ES cell comprises the step of introducing a single rearranged human immunoglobulin λ light chain variable region operably linked to a rodent Cλ gene segment into an engineered endogenous immunoglobulin κ light chain locus in the genome of a rodent ES cell, wherein the single rearranged human immunoglobulin λ light chain variable region comprises a human Vλ gene segment and a human Jλ gene segment. In some embodiments, a genome of a rodent ES cell comprises one or more unrearranged human V_{H} gene segments, one or more unrearranged human D_{H} gene segments, and one or more unrearranged human J_{H} gene segments operably linked to one or more endogenous immunoglobulin heavy chain constant region genes at an engineered endogenous immunoglobulin heavy chain locus. In some embodiments, the genome of a rodent ES cell further comprises one or more nucleotide sequences encoding one or more rodent ADAM6 polypeptides, functional orthologs, functional homologs, or functional fragments thereof.

In some embodiments, a method of making a genetically modified rodent as described herien, comprises the step of (a) engineering an endogenous immunoglobulin κ light chain locus in the germline genome of the rodent to comprise a single rearranged human immunoglobulin λ light chain variable region operably linked to a rodent Cλ gene segment, where the single rearranged human immunoglobulin λ light chain variable region comprises a human Vλ gene segment and a human Jλ gene segment, so that all immunoglobulin λ light chains expressed by B cells of the genetically modified rodent include human immunoglobulin λ light chain variable domains expressed from the single rearranged human immunoglobulin λ light chain variable region or a somatically hypermutated version thereof. In some embodiments, a method further comprises the step of (b) engineering an endogenous immunoglobulin heavy chain locus in the germline genome of the rodent to comprise one or more unrearranged human V_{H} gene segments, one or more unrearranged human D_{H} gene segments, and one or more unrearranged human J_{H} gene segments operably linked to one or more rodent immunoglobulin heavy chain constant region genes, so that all heavy chains expressed by B cells of the genetically modified rodent include a human immunoglobulin heavy chain variable domains and a rodent immunoglobulin heavy chain constant domains. In some embodiments, step (b) comprises engineering the endogenous immunoglobulin heavy chain locus to further comprise one or more nucleotide sequences encoding one or more rodent ADAM6 polypeptides, functional orthologs, functional homologs, or functional fragments thereof. In some embodiments, step (a) and/or step (b) are carried out in a rodent ES cell.

In some embodiments, a human Vλ gene segment is selected from a group consisting of: Vλ4-69, Vλ8-61, Vλ4-60, Vλ6-57, Vλ10-54, Vλ5-52, Vλ1-51, Vλ9-49, Vλ1-47, Vλ7-46, Vλ5-45, Vλ1-44, Vλ7-43, Vλ1-40, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, a human Vλ gene segment is selected from a group consisting of: Vλ5-52, Vλ1-51, Vλ9-49, Vλ1-47, Vλ7-46, Vλ5-45, Vλ1-44, Vλ7-43, Vλ1-40, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, a human Vλ gene segment is selected from a group consisting of: Vλ1-51, Vλ5-45, Vλ1-44, Vλ1-40, Vλ3-21, and Vλ2-14. In some embodiments, a human Vλ gene segment is Vλ1-51 or Vλ2-14. In some embodiments, a human Jλ gene segment is selected from a group consisting of: Jλ1, Jλ2, Jλ3, Jλ6, and Jλ7. In some embodiments, a human Jλ gene segment is selected from a group consisting of: Jλ1, Jλ2, Jλ3, and Jλ7. In some embodiments, a human Jλ gene segment is Jλ2.

In some embodiments, the endogenous immunoglobulin κ light chain locus lacks a rodent Cκ gene.

In some embodimetns, one or more unrearranged human V_{H} gene segments, one or more unrearranged human D_{H} gene segments, and one or more unrearranged human J_{H} gene segments are in place of one or more endogenous V_{H} gene segments, one or more endogneous D_{H} gene segments, one or more endogenous J_{H} gene segments, or a combination thereof. In some embodiments, one or more unrearranged human V_{H} gene segments, one or more unrearranged human D_{H} gene segments, and one or more unrearranged human J_{H} gene segments replace one or more endogenous V_{H} gene segments, one or more endogenous D_{H} gene segments, and one or more endogenous J_{H} gene segments, respectively.

In some embodiments, one or more unrearranged human V_{H} gene segments comprise V_{H}3-74, V_{H}3-73, V_{H}3-72, V_{H}2-70, V_{H}1-69, V_{H}3-66, V_{H}3-64, V_{H}4-61, V_{H}4-59, V_{H}1-58, V_{H}3-53, V_{H}5-51, V_{H}3-49, V_{H}3-48, V_{H}1-46, V_{H}1-45, V_{H}3-43, V_{H}4-39, V_{H}4-34, V_{H}3-33, V_{H}4-31, V_{H}3-30, V_{H}4-28, V_{H}2-26, V_{H}1-24, V_{H}3-23, V_{H}3-21, V_{H}3-20, V_{H}1-18, V_{H}3-15, V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2, V_{H}6-1, or any combination thereof. In some embodiments, one or more unrearranged human D_{H} gene segments comprise D_{H}1-1, D_{H}2-2, D_{H}3-3, D_{H}4-4, D_{H}5-5, D_{H}6-6, D_{H}1-7, D_{H}2-8, D_{H}3-9, D_{H}3-10, D_{H}5-12, D_{H}6-13, D_{H}2-15, D_{H}3-16, D_{H}4-17, D_{H}6-19, D_{H}1-20, D_{H}2-21, D_{H}3-22, D_{H}6-25, D_{H}1-26, D_{H}7-27, or any combination thereof. In some embodiments, one or more unrearranged human J_{H} gene segments comprise J_{H}1, J_{H}2, J_{H}3, J_{H}4, J_{H}5, J_{H}6, or any combination thereof.

In some embodiments, one or more rodent immunoglobulin heavy chain constant region genes are one or more endogenous rodent immunoglobulin heavy chain constant region genes.

In some embodiments, an endogenous immunoglobulin heavy chain locus lacks a functional endogenous rodent Adam6 gene. In some embodiments, the germline genome of a genetically modified rodent comprises one or more nucleotide sequences encoding one or more rodent ADAM6 polypeptides, functional orthologs, functional homologs, or functional fragments thereof. In some embodiments, one or more rodent ADAM6 polypeptides, functional orthologs, functional homologs, or functional fragments thereof are expressed by the genetically modified rodent. In some embodiments, one or more nucleotide sequences encoding one or more rodent ADAM6 polypeptides, functional orthologs, functional homologs, or functional fragments thereof are included on the same chromosome as the engineered endogenous immunoglobulin heavy chain locus. In some embodiments, an engineered endogenous immunoglobulin heavy chain locus comprises the one or more nucleotide sequences encoding one or more rodent ADAM6 polypeptides, functional orthologs, functional homologs, or functional fragments thereof. In some embodiments, one or more nucleotide sequences encoding one or more rodent ADAM6 polypeptides, functional orthologs, functional homologs, or functional fragments thereof are in place of a human Adam6 pseudogene. In some embodiments, one or more nucleotide sequences encoding one or more rodent ADAM6 polypeptides, functional orthologs, functional homologs, or functional fragments thereof replace a human Adam6 pseudogene.

In some embodiments, one or more human V_{H} gene segments comprise a first and a second human V_{H} gene segment, and the one or more nucleotide sequences encoding one or more rodent ADAM6 polypeptides, functional orthologs, functional homologs, or functional fragments thereof are between the first human V_{H} gene segment and the second human V_{H} gene segment. In some embodiments, a first human V_{H} gene segment is V_{H}1-2 and a second human V_{H} gene segment is V_{H}6-1.

In some embodiments, one or more nucleotide sequences encoding one or more rodent ADAM6 polypeptides, functional orthologs, functional homologs, or functional fragments thereof are between a human V_{H} gene segment and a human D_{H} gene segment.

In some embodiments, a rodent Cλ gene has a sequence that is at least 80%, at least 85%, at least 90%, at least 95%, at least 98% or at least 99% identical to a mouse Cλ1. In some embodiments, a rodent Cλ gene has a sequence that is at least 80%, at least 85%, at least 90%, at least 95%, at least 98% or at least 99% identical to a mouse Cλ2. In some embodiments, a rodent Cλ gene has a sequence that is at least 80%, at least 85%, at least 90%, at least 95%, at least 98% or at least 99% identical to a mouse Cλ3 gene.

In some embodiments, a rodent Cλ gene is or comprises a mouse Cλ gene. In some embodiments, a rodent Cλ gene is or comprises a mouse Cλ1 gene.

In some embodiments, a rodent Cλ gene has a sequence that is at least 80%, at least 85%, at least 90%, at least 95%, at least 98% or at least 99% identical to a rat Cλ1. In some embodiments, a rodent Cλ gene has a sequence that is at least 80%, at least 85%, at least 90%, at least 95%, at least 98% or at least 99% identical to a rat Cλ2. In some embodiments, a rodent Cλ gene has a sequence that is at least 80%, at least 85%, at least 90%, at least 95%, at least 98% or at least 99% identical to a rat Cλ3. In some embodiments, a rodent Cλ gene has a sequence that is at least 80%, at least 85%, at least 90%, at least 95%, at least 98% or at least 99% identical to a rat Cλ4 gene.

In some embodiments, a rodent Cλ gene is or comprises a rat Cλ gene.

In some embodiments, a single rearranged human immunoglobulin λ light chain variable region is in place of one or more rodent Vκ gene segments, one or more rodent Jκ gene segments, or any combination thereof. In some embodiments, a single rearranged human immunoglobulin λ light chain variable region replaces one or more rodent Vκ gene segments, one or more rodent Jκ gene segments, or any combination thereof. In some embodiments, a germline genome of the genetically modified rodent described herein further comprises an inactivated endogenous immunoglobulin λ light chain locus. In some embodiments, an endogenous immunoglobulin λ light chain locus is inactivated by deleting or inverting all or a portion of an endogenous immunoglobulin λ light chain locus. In some embodiments, endogenous Vλ gene segments, endogenous Jλ gene segments, and endogenous Cλ genes are deleted in whole or in part.

### BRIEF DESCRIPTION OF THE DRAWING

The Drawing included herein, which is composed of the following Figures, is for illustration purposes only and not for limitation.

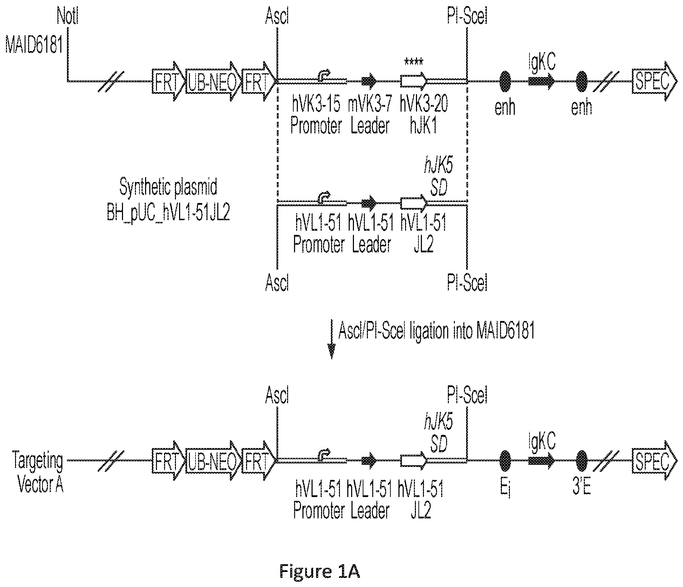

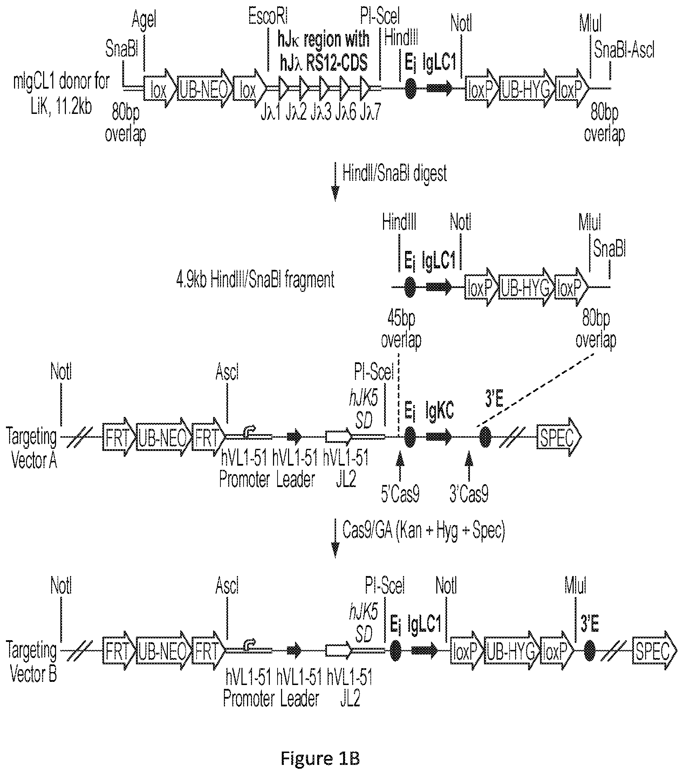

**Figure 1A** **and** **Figure 1B** show illustrations of an exemplary embodiment, not to scale, of a strategy for constructing a targeting vector (described in Examples 1 and 4) used in generating an embodiment of a non-human animal according to the present disclosure. Unless labeling in the diagram suggests otherwise (e.g., as for selection cassettes, loxP sites, etc.), filled shapes and single lines represent mouse sequences, and empty shapes and double lines represent human sequences. "FRT" indicates a Frt recombinase system site; "UB-NEO" indicates a neomycin resistant gene with a UB promoter; "enh" indicates an enhancer; "Ei" indicates an intronic enhancer; "3'E" indicates a 3' enhancer; "SPEC" indicates spectinomycin resistance gene; "SD" indicates a splice donor site; "CDS" indicates a coding sequence; "lox" indicates a lox2372 site; "loxP" indicates a lox P site; and "UB-HYG" indicates a hygromycin resistant gene with a UB promoter.

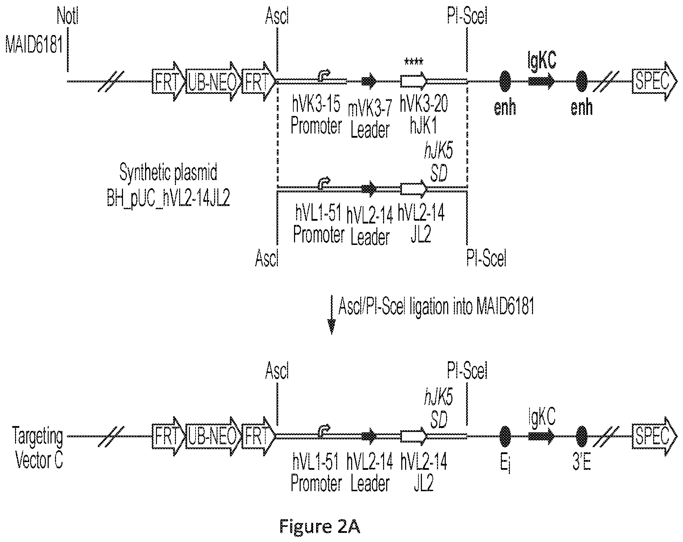

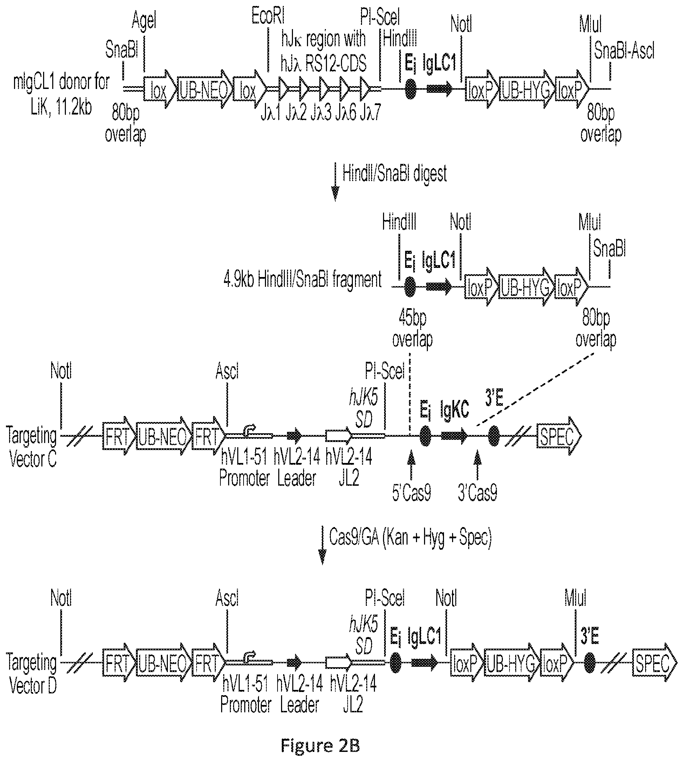

**Figure 2A** **and** **Figure 2B** show illustrations of an exemplary embodiment, not to scale, of a strategy for constructing a targeting vector (described in Examples 7 and 10) used in generating an embodiment of a non-human animal according to the present disclosure. Unless labeling in the diagram suggests otherwise (e.g., as for selection cassettes, loxP sites, etc.), filled shapes and single lines represent mouse sequences, and empty shapes and double lines represent human sequences. "FRT" indicates a Frt recombinase system site; "UB-NEO" indicates a neomycin resistant gene with a UB promoter; "enh" indicates an enhancer; "Ei" indicates an intronic enhancer; "3'E" indicates a 3' enhancer; "SPEC" indicates spectinomycin resistance gene; "SD" indicates a splice donor site; "CDS" indicates a coding sequence; "lox" indicates a lox2372 site; "loxP" indicates a lox P site; and "UB-HYG" indicates a hygromycin resistant gene with a UB promoter.

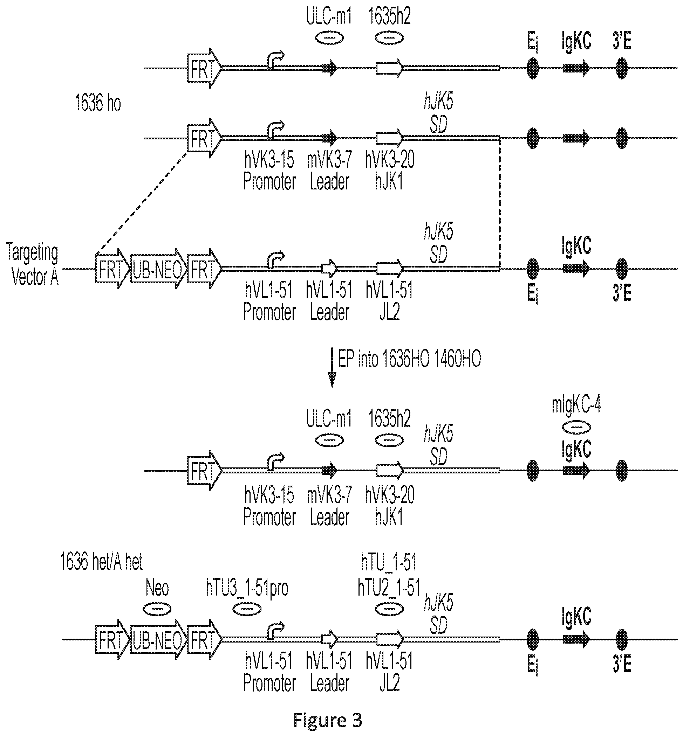

**Figure 3** shows an illustration, not to scale, of the insertion of Targeting Vector A (as described in Example 1) into an engineered Igκ light chain locus of a rodent embryonic stem (ES) cell clone (as described in Example 2), which ES cell clone was used in generating an embodiment of the rodent according to the present disclosure. Included in the illustration are the approximate positions of various probes (indicated by circled dash marks) used for confirming that embryonic stem (ES) cell clones are positive for certain exemplary sequences. Unless labeling in the diagram suggests otherwise (e.g., as for selection cassettes, loxP sites, etc.), filled shapes and single lines represent mouse sequences, and empty shapes and double lines represent human sequences. "FRT" indicates a Frt recombinase system site; "UB-NEO" indicates a neomycin resistant gene with a UB promoter; "Ei" indicates an intronic enhancer; "3'E" indicates a 3' enhancer; and "SD" indicates a splice donor site. "Het" indicates a heterozygous mouse, "ho" indicates a homozygous mouse.

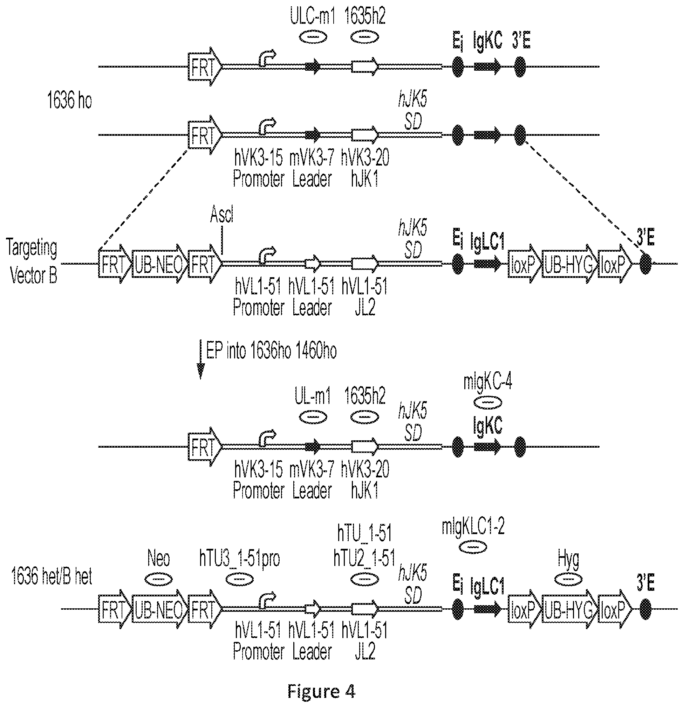

**Figure 4** shows an illustration, not to scale, of the insertion of Targeting Vector B (as described in Example 4) into an engineered Igκ light chain locus of a rodent embryonic stem (ES) cell clone (as described in Example 5), which ES cell clone was used in generating an embodiment of the rodent according to the present disclosure. Included in the illustration are the approximate positions of various probes (indicated by circled dash marks) used for confirming that embryonic stem (ES) cell clones are positive for certain exemplary sequences. Unless labeling in the diagram suggests otherwise (e.g., as for selection cassettes, loxP sites, etc.), filled shapes and single lines represent mouse sequences, and empty shapes and double lines represent human sequences. "FRT" indicates a Frt recombinase system site; "UB-NEO" indicates a neomycin resistant gene with a UB promoter; "Ei" indicates an intronic enhancer; "3'E" indicates a 3' enhancer; "SD" indicates a splice donor site; loxP" indicates a lox P site; and "UB-HYG" indicates a hygromycin resistant gene with a UB promoter. "Het" indicates a heterozygous mouse, "ho" indicates a homozygous mouse.

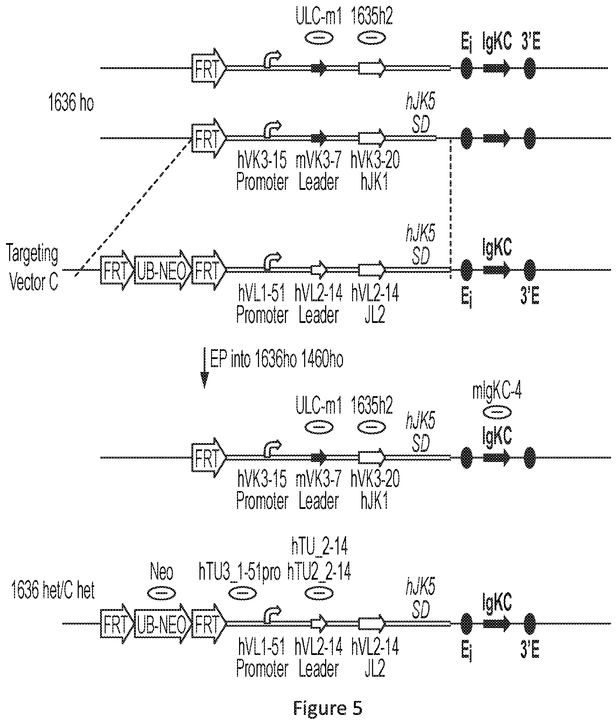

**Figure 5** shows an illustration, not to scale, of the insertion of Targeting Vector C (as described in Example 7) into an engineered Igκ light chain locus of a rodent embryonic stem (ES) cell clone (as described in Example 8), which ES cell clone was used in generating an embodiment of the rodent according to the present disclosure. Included in the illustration are the approximate positions of various probes (indicated by circled dash marks) used for confirming that embryonic stem (ES) cell clones are positive for certain exemplary sequences. Unless labeling in the diagram suggests otherwise (e.g., as for selection cassettes, loxP sites, etc.), filled shapes and single lines represent mouse sequences, and empty shapes and double lines represent human sequences. "FRT" indicates a Frt recombinase system site; "UB-NEO" indicates a neomycin resistant gene with a UB promoter; "Ei" indicates an intronic enhancer; "3'E" indicates a 3' enhancer; and "SD" indicates a splice donor site. "Het" indicates a heterozygous mouse, "ho" indicates a homozygous mouse.

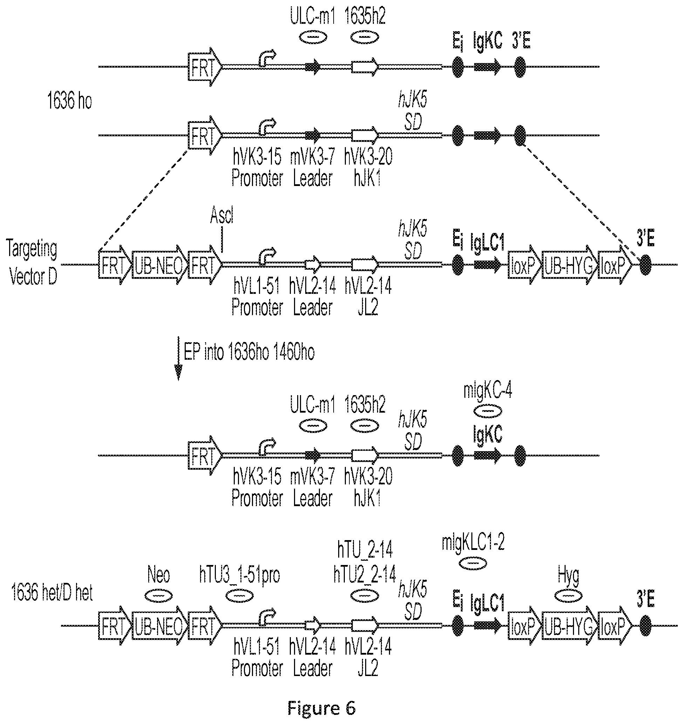

**Figure 6** shows an illustration, not to scale, of the insertion of Targeting Vector D (as described in Example 10) into an engineered Igκ light chain locus of a rodent embryonic stem (ES) cell clone (as described in Example 11), which ES cell clone was used in generating an embodiment of the rodent according to the present disclosure. Included in the illustration are the approximate positions of various probes (indicated by circled dash marks) used for confirming that embryonic stem (ES) cell clones are positive for certain exemplary sequences. Unless labeling in the diagram suggests otherwise (e.g., as for selection cassettes, loxP sites, etc.), filled shapes and single lines represent mouse sequences, and empty shapes and double lines represent human sequences. "FRT" indicates a Frt recombinase system site; "UB-NEO" indicates a neomycin resistant gene with a UB promoter; "Ei" indicates an intronic enhancer; "3'E" indicates a 3' enhancer; "SD" indicates a splice donor site; "loxP" indicates a lox P site; and "UB-HYG" indicates a hygromycin resistant gene with a UB promoter. "Het" indicates a heterozygous mouse, "ho" indicates a homozygous mouse.

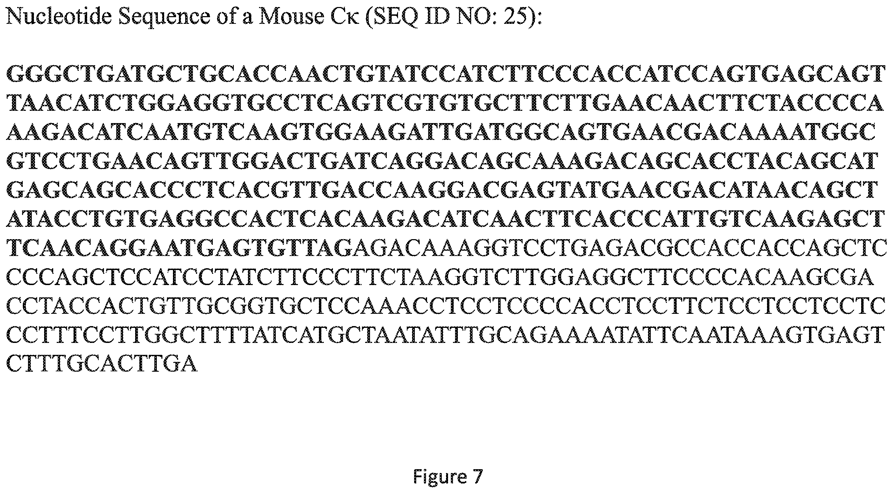

**Figure 7** shows a nucleotide sequence of a mouse Cκ (SEQ ID NO: 25). The coding sequence is denoted by bold letters, and the 3' untranslated region is not bolded.

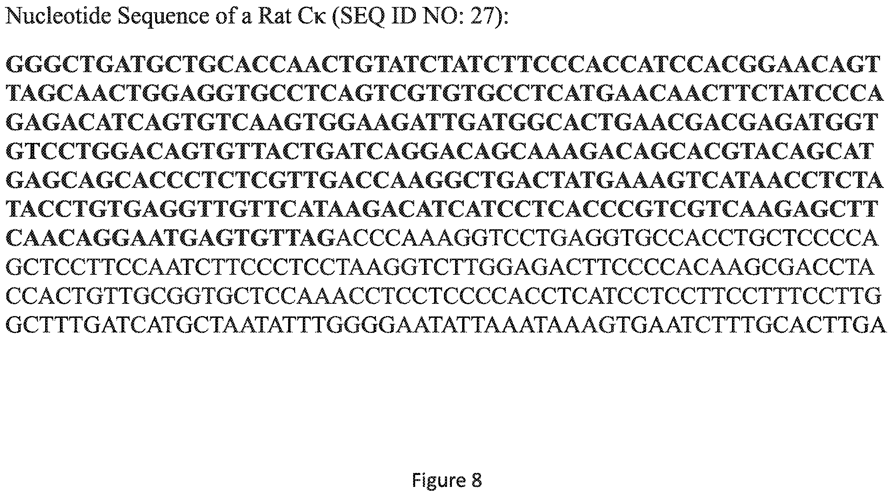

**Figure 8** shows a nucleotide sequence of a rat Cκ (SEQ ID NO: 27). The coding sequence is denoted by bold letters, and the 3' untranslated region is not bolded.

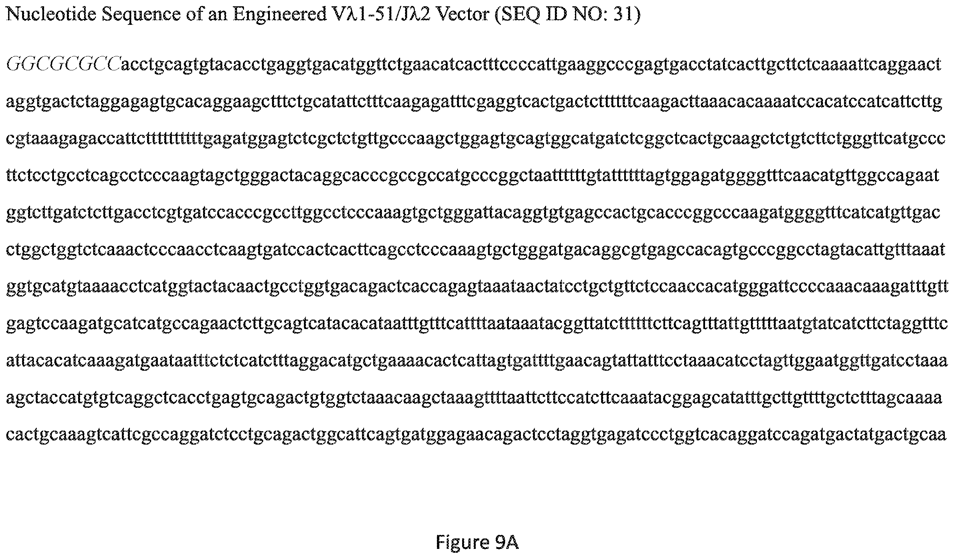

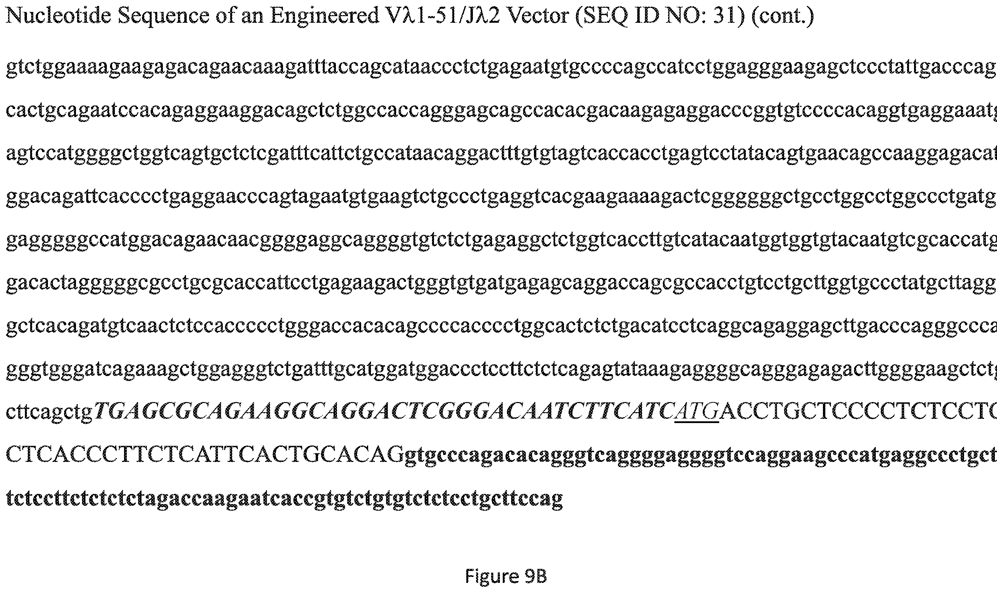

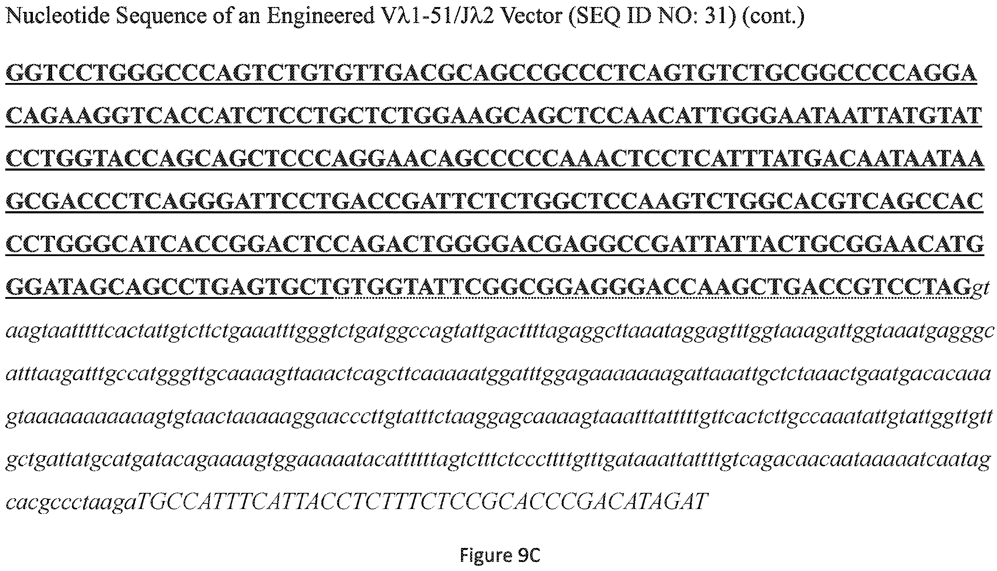

**Figures 9A-9C** show a nucleotide sequence of an engineered Vλ1-51/Jλ2 vector (SEQ ID NO: 31). Restriction enzyme site sequences - AscI (5' end of sequence) and PI-SceI (3' end of sequence) - are denoted in uppercase, italicized letters; a Vλ1-51 promoter sequence is denoted by lowercase (not bold) letters; a Vλ1 51 5' UTR sequence is denoted by uppercase, italicized, and bold letters; a rearranged Vλ1-51/Jλ2 sequence, which includes: a Vλ1-51, exon 1 sequence is denoted by uppercase (not bold) letters, where the Vλ1-51, exon 1 start codon is further italicized and underlined; a Vλ1-51, intron 1 sequence is denoted by lowercase, bold letters; a Vλ1-51, exon 2 sequence denoted by uppercase, bold, and underlined (solid underline) letters, and a a Jλ2 sequence denoted by uppercase, bold and underlined (dashed underline) letters; and a human Jκ5-Cκ intron sequence is denoted by lowercase, italicized letters.

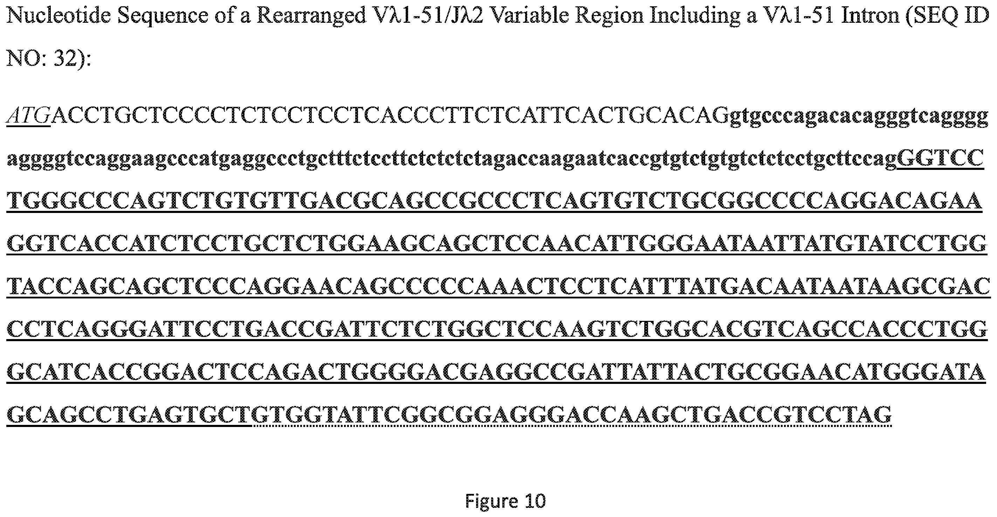

**Figure 10** shows a nucleotide sequence of a rearranged Vλ1-51/Jλ2 variable region including a Vλ1-51 intron (SEQ ID NO: 32). A Vλ1-51, exon 1 sequence is denoted by uppercase letters, where the Vλ1-51, exon 1 start codon is further italicized and underlined; a Vλ1-51, intron 1 sequence is denoted by lowercase, bold letters; a Vλ1-51, exon 2 sequence denoted by uppercase, bold, and underlined (solid underline) letters; and a Jλ2 sequence denoted by uppercase, bold and underlined (dashed underline) letters.

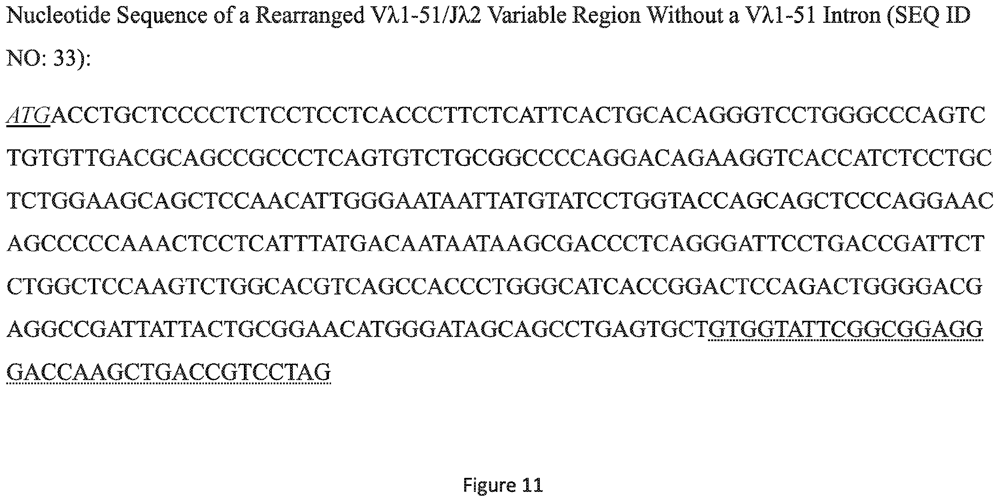

**Figure 11** shows a nucleotide sequence of a rearranged Vλ1-51/Jλ2 variable region without a Vλ1-51 intron (SEQ ID NO: 33). A Vλ1-51 coding sequence is denoted by uppercase letters, where the Vλ1-51 start codon is further italicized and underlined; and a Jλ2 sequence is denoted by uppercase letters with a dashed underline.

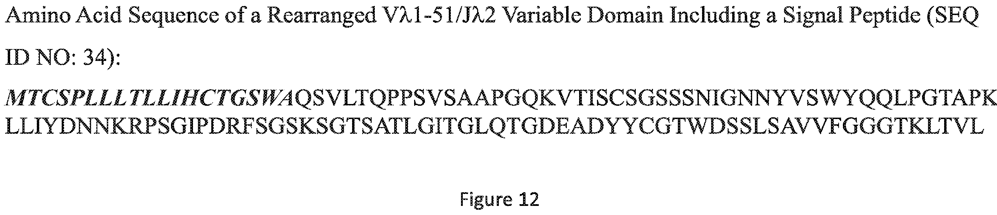

**Figure 12** shows an amino acid sequence of a rearranged Vλ1-51/Jλ2 variable domain including a signal peptide (SEQ ID NO: 34). The italicized, bold text indicates the sequence of a signal peptide.

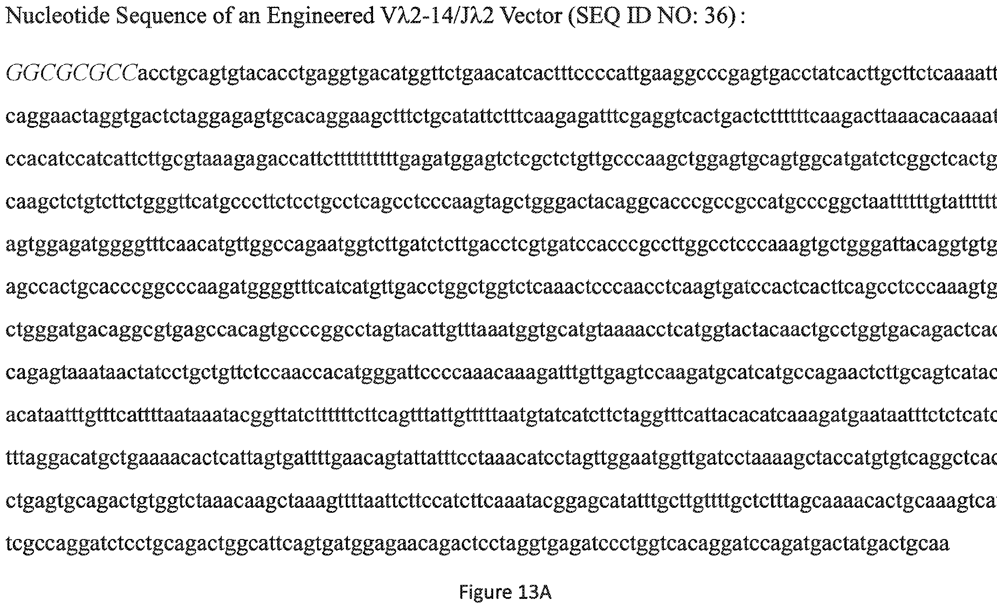

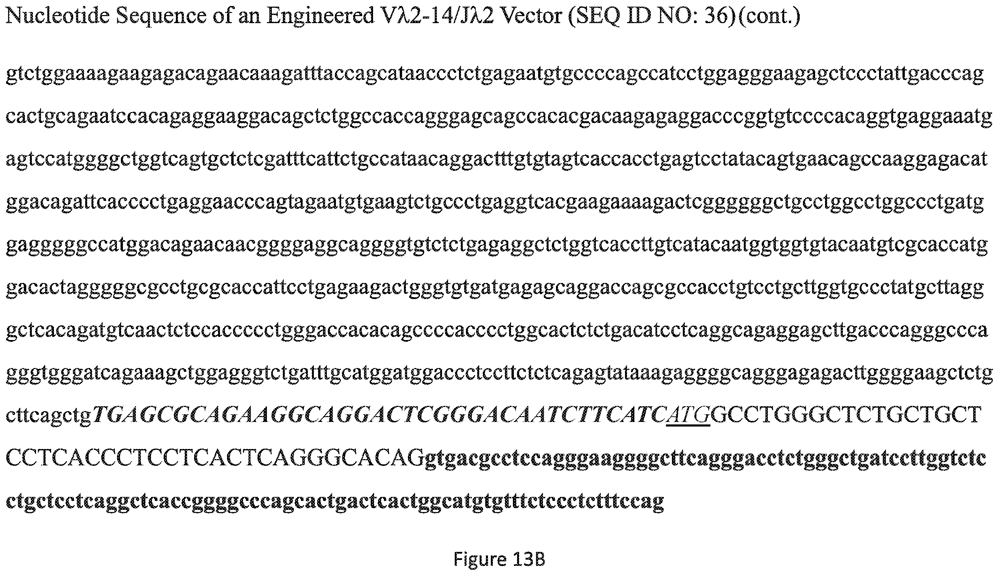

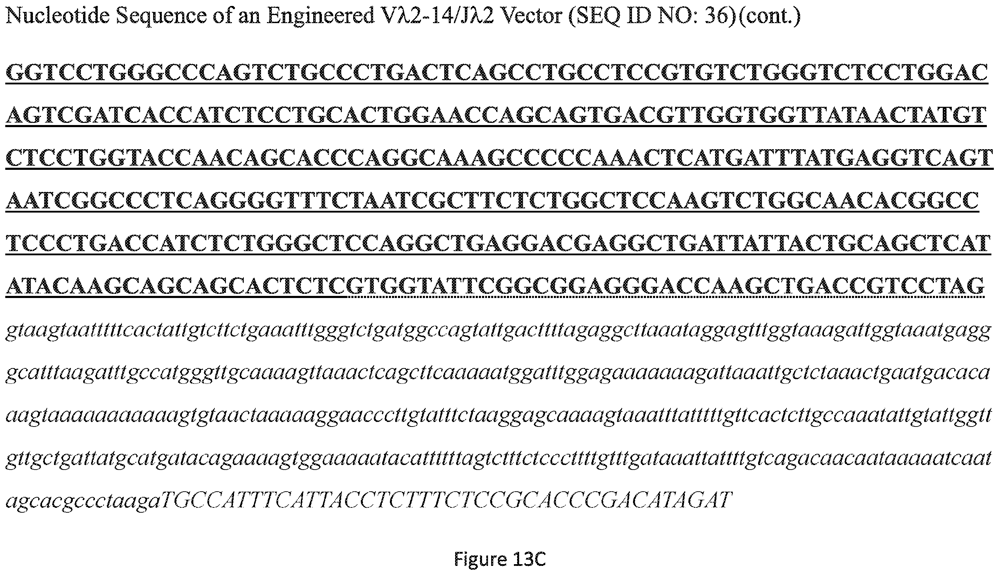

**Figures 13A-13C** show a nucleotide sequence of an engineered Vλ2-14/Jλ2 vector (SEQ ID NO: 36). Restriction enzyme site sequences - AscI (5' end of sequence) and PI-SceI (3' end of sequence) - are denoted in uppercase, italicized letters; a Vλ1-51 promoter sequence is denoted by lowercase (not bold) letters; a Vλ1 51 5' UTR sequence is denoted by uppercase, italicized, and bold letters; a rearranged Vλ2-14/Jλ2 sequence, which includes: a Vλ2-14, exon 1 sequence is denoted by uppercase, (not bold) letters, where the Vλ2-14, exon 1 start codon is further italicized and underlined; a Vλ2-14, intron 1 sequence is denoted by lowercase, bold letters; a Vλ2-14, exon 2 sequence denoted by uppercase, bold, and underlined (solid underline) letters, and a Jλ2 sequence denoted by uppercase, bold and underlined (dashed underline) letters; and a human Jκ5-Cκ intron sequence is denoted by lowercase, italicized letters.

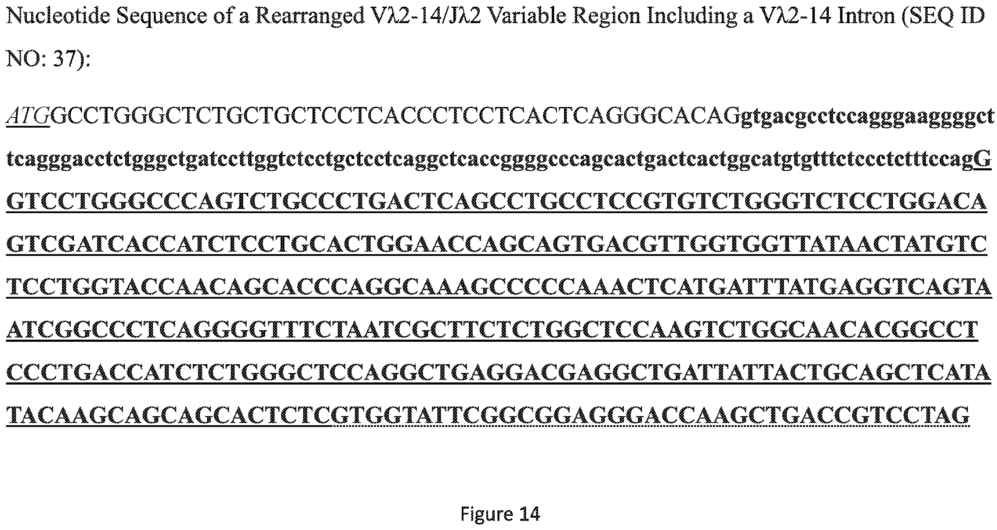

**Figure 14** shows a nucleotide sequence of a rearranged Vλ2-14/Jλ2 variable region including a Vλ2-14 intron (SEQ ID NO: 37). A Vλ2-14, exon 1 sequence is denoted by uppercase letters, where the Vλ2-14, exon 1 start codon is further italicized and underlined; a Vλ2-14, intron 1 sequence is denoted by lowercase, bold letters; a Vλ1-51, exon 2 sequence denoted by uppercase, bold, and underlined (solid underline) letters; and a Jλ2 sequence denoted by uppercase, bold and underlined (dashed underline) letters.

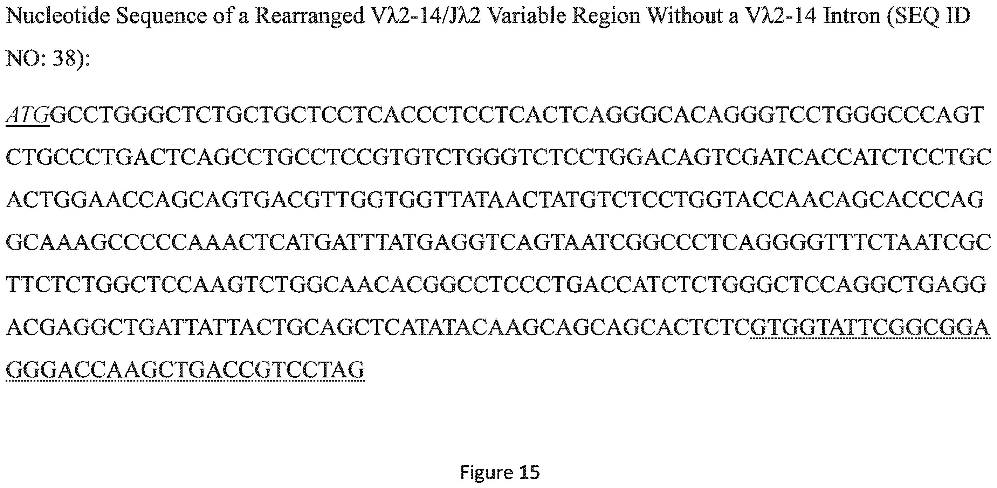

**Figure 15** shows a nucleotide sequence of a rearranged Vλ2-14/Jλ2 variable region without a Vλ2-14 intron (SEQ ID NO: 38). A Vλ2-14 sequence is denoted by uppercase letters, where the Vλ2-14 start codon is further italicized and underlined; and a Jλ2 sequence is denoted by uppercase letters with a dashed underline.

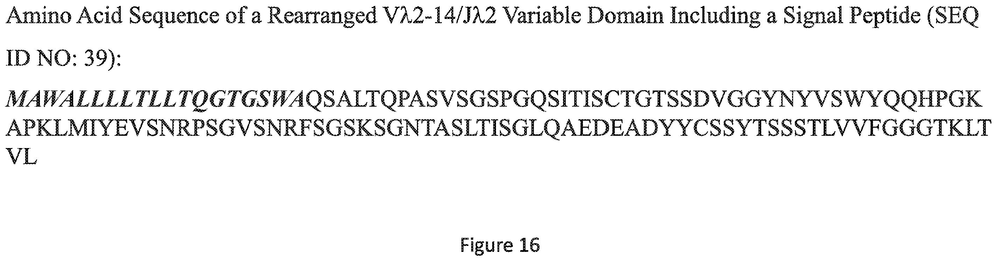

**Figure 16** shows an amino acid sequence of a rearranged Vλ2-14/Jλ2 variable domain including a signal peptide (SEQ ID NO: 39). The italicized, bold text indicates the sequence of a signal peptide.

### BRIEF DESCRIPTION OF SELECTED SEQUENCES IN THE SEQUENCE LISTING

Representative nucleotide and amino acid sequences of various human Vλ and Jλ gene segments, which can be utilized in some embodiments of a non-human animal as described herein, are available from the International Immunogenetics Information System website, www.imgt.org, or in LeFranc, M-P., The Immunoglobulin FactsBook, Academic Press, May, 23, 2001 (referred to herein as "LeFranc 2001").

The following are representative nucleotide and amino acid sequences of various mouse, rat, or human lambda constant regions or domains, which can be utilized in some embodiments of a non-human animal as described herein.

**TABLE 5: Nucleotide Sequence of Certain Mouse Cλ Gene Segments**

| **Gene Segment** | **SEQ ID NO** | **Sequence** |
|---|---|---|
| Cλ1 | 1 | GCCAGCCCAAGTCTTCGCCATCAGTCACCCTGTTTCCACC TTCCTCTGAAGAGCTCGAGACTAACAAGGCCACACTGGT GTGTACGATCACTGATTTCTACCCAGGTGTGGTGACAGT GGACTGGAAGGTAGATGGTACCCCTGTCACTCAGGGTAT GGAGACAACCCAGCCTTCCAAACAGAGCAACAACAAGT ACATGGCTAGCAGCTACCTGACCCTGACAGCAAGAGCAT GGGAAAGGCATAGCAGTTACAGCTGCCAGGTCACTCATG AAGGTCACACTGTGGAGAAGAGTTTGTCCCGTGCTGACT GTTCC |
| Cλ2 | 2 | GTCAGCCCAAGTCCACTCCCACTCTCACCGTGTTTCCACC TTCCTCTGAGGAGCTCAAGGAAAACAAAGCCACACTGGT GTGTCTGATTTCCAACTTTTCCCCGAGTGGTGTGACAGTG GCCTGGAAGGCAAATGGTACACCTATCACCCAGGGTGTG GACACTTCAAATCCCACCAAAGAGGGCAACAAGTTCATG GCCAGCAGCTTCCTACATTTGACATCGGACCAGTGGAGA TCTCACAACAGTTTTACCTGTCAAGTTACACATGAAGGG GACACTGTGGAGAAGAGTCTGTCTCCTGCAGAATGTCTC |
| Cλ3 | 3 | GTCAGCCCAAGTCCACTCCCACACTCACCATGTTTCCAC CTTCCCCTGAGGAGCTCCAGGAAAACAAAGCCACACTCG TGTGTCTGATTTCCAATTTTTCCCCAAGTGGTGTGACAGT GGCCTGGAAGGCAAATGGTACACCTATCACCCAGGGTGT GGACACTTCAAATCCCACCAAAGAGGACAACAAGTACA TGGCCAGCAGCTTCTTACATTTGACATCGGACCAGTGGA GATCTCACAACAGTTTTACCTGCCAAGTTACACATGAAG GGGACACTGTGGAGAAGAGTCTGTCTCCTGCAGAATGTC TC |

**TABLE 6: Amino Acid Sequence of Certain Mouse Cλ Gene Segments**

| **Gene Segment** | **SEQ ID NO** | **Sequence** |
|---|---|---|
| Cλ1 | 4 | GQPKSSPSVTLFPPSSEELETNKATLVCTITDFYPGVVTVDW KVDGTPVTQGMETTQPSKQSNNKYMASSYLTLTARAWER HSSYSCQVTHEGHTVEKSLSRADCS |
| Cλ2 | 5 | GQPKSTPTLTVFPPSSEELKENKATLVCLISNFSPSGVTVAW KANGTPITQGVDTSNPTKEGNKFMASSFLHLTSDQWRSHN SFTCQVTHEGDTVEKSLSPAECL |
| Cλ3 | 6 | GQPKSTPTLTMFPPSPEELQENKATLVCLISNFSPSGVTVAW KANGTPITQGVDTSNPTKEDNKYMASSFLHLTSDQWRSHN SFTCQVTHEGDTVEKSLSPAECL |

**TABLE 7: Nucleotide Sequence of Certain Rat Cλ Gene Segments**

| **Gene Segment** | **SEQ ID NO** | **Sequence** |
|---|---|---|
| Cλ1 | 7 | GTCAGCCCAAGTCCACTCCCACACTCACAGTATTTCCAC CTTCAACTGAGGAGCTCCAGGGAAACAAAGCCACACTG GTGTGTCTGATTTCTGATTTCTACCCGAGTGATGTGGAAG TGGCCTGGAAGGCAAATGGTGCACCTATCTCCCAGGGTG TGGACACTGCAAATCCCACCAAACAGGGCAACAAATAC ATCGCCAGCAGCTTCTTACGTTTGACAGCAGAACAGTGG AGATCTCGCAACAGTTTTACCTGCCAAGTTACACATGAA GGGAACACTGTGGAGAAGAGTCTGTCTCCTGCAGAATGT GTC |
| Cλ2 | 8 | ACCAACCCAAGGCTACGCCCTCAGTCACCCTGTTCCCAC CTTCCTCTGAAGAGCTCAAGACTGACAAGGCTACACTGG TGTGTATGGTGACAGATTTCTACCCTGGTGTTATGACAGT GGTCTGGAAGGCAGATGGTACCCCTATCACTCAGGGTGT GGAGACTACCCAGCCTTTCAAACAGAACAACAAGTACAT GGCTACCAGCTACCTGCTTTTGACAGCAAAAGCATGGGA GACTCATAGCAATTACAGCTGCCAGGTCACTCACGAAGA GAACACTGTGGAGAAGAGTTTGTCCCGTGCTGAGTGTTC C |
| Cλ3 | 9 | GTCAGCCCAAGTCCACTCCCACACTCACAGTATTTCCAC CTTCAACTGAGGAGCTCCAGGGAAACAAAGCCACACTG GTGTGTCTGATTTCTGATTTCTACCCGAGTGATGTGGAAG TGGCCTGGAAGGCAAATGGTGCACCTATCTCCCAGGGTG TGGACACTGCAAATCCCACCAAACAGGGCAACAAATAC ATCGCCAGCAGCTTCTTACGTTTGACAGCAGAACAGTGG AGATCTCGCAACAGTTTTACCTGCCAAGTTACACATGAA GGGAACACTGTGGAAAAGAGTCTGTCTCCTGCAGAGTGT GTC |
| Cλ4 | 10 | ACCAACCCAAGGCTACGCCCTCAGTCACCCTGTTCCCAC CTTCCTCTGAAGAGCTCAAGACTGACAAGGCTACACTGG TGTGTATGGTGACAGATTTCTACCCTGGTGTTATGACAGT GGTCTGGAAGGCAGATGGTACCCCTATCACTCAGGGTGT GGAGACTACCCAGCCTTTCAAACAGAACAACAAGTACAT GGCTACCAGCTACCTGCTTTTGACAGCAAAAGCATGGGA GACTCATAGCAATTACAGCTGCCAGGTCACTCACGAAGA GAACACTGTGGAGAAGAGTTTGTCCCGTGCTGAGTGTTC C |

**TABLE 8: Amino Acid Sequence of Certain Rat Cλ Gene Segments**

| **Gene Segment** | **SEQ ID NO** | **Sequence** |
|---|---|---|
| Cλ1 | 11 | GQPKSTPTLTVFPPSTEELQGNKATLVCLISDFYPSDVEVA WKANGAPISQGVDTANPTKQGNKYIASSFLRLTAEQWRSR NSFTCQVTHEGNTVEKSLSPAECV |
| Cλ2 | 12 | DQPKATPSVTLFPPSSEELKTDKATLVCMVTDFYPGVMTV VWKADGTPITQGVETTQPFKQNNKYMATSYLLLTAKAWE THSNYSCQVTHEENTVEKSLSRAECS |
| Cλ3 | 13 | GQPKSTPTLTVFPPSTEELQGNKATLVCLISDFYPSDVEVA WKANGAPISQGVDTANPTKQGNKYIASSFLRLTAEQWRSR NSFTCQVTHEGNTVEKSLSPAECV |
| Cλ4 | 14 | DQPKATPSVTLFPPSSEELKTDKATLVCMVTDFYPGVMTV VWKADGTPITQGVETTQPFKQNNKYMATSYLLLTAKAWE THSNYSCQVTHEENTVEKSLSRAECS |

**TABLE 9: Nucleotide Sequence of Certain Human Cλ Gene Segments**

| **Gene Segment** | **SEQ ID NO** | **Sequence** |
|---|---|---|
| Cλ1 | 15 | CCCAAGGCCAACCCCACGGTCACTCTGTTCCCGCCCTCC TCTGAGGAGCTCCAAGCCAACAAGGCCACACTAGTGTGT CTGATCAGTGACTTCTACCCGGGAGCTGTGACAGTGGCT TGGAAGGCAGATGGCAGCCCCGTCAAGGCGGGAGTGGA GACGACCAAACCCTCCAAACAGAGCAACAACAAGTACG CGGCCAGCAGCTACCTGAGCCTGACGCCCGAGCAGTGG AAGTCCCACAGAAGCTACAGCTGCCAGGTCACGCATGA AGGGAGCACCGTGGAGAAGACAGTGGCCCCTACAGAAT GTTCATAG |
| Cλ2 | 16 | GTCAGCCCAAGGCTGCCCCCTCGGTCACTCTGTTCCCGC CCTCCTCTGAGGAGCTTCAAGCCAACAAGGCCACACTGG TGTGTCTCATAAGTGACTTCTACCCGGGAGCCGTGACAG TGGCTTGGAAAGCAGATAGCAGCCCCGTCAAGGCGGGA GTGGAGACCACCACACCCTCCAAACAAAGCAACAACAA GTACGCGGCCAGCAGCTATCTGAGCCTGACGCCTGAGCA GTGGAAGTCCCACAGAAGCTACAGCTGCCAGGTCACGC ATGAAGGGAGCACCGTGGAGAAGACAGTGGCCCCTACA GAATGTTCA |
| Cλ3 | 17 | CCCAAGGCTGCCCCCTCGGTCACTCTGTTCCCACCCTCCT CTGAGGAGCTTCAAGCCAACAAGGCCACACTGGTGTGTC TCATAAGTGACTTCTACCCGGGAGCCGTGACAGTTGCCT GGAAGGCAGATAGCAGCCCCGTCAAGGCGGGGGTGGAG ACCACCACACCCTCCAAACAAAGCAACAACAAGTACGC GGCCAGCAGCTACCTGAGCCTGACGCCTGAGCAGTGGA AGTCCCACAAAAGCTACAGCTGCCAGGTCACGCATGAA GGGAGCACCGTGGAGAAGACAGTTGCCCCTACGGAATG TTCATAG |
| Cλ6 | 18 | GGTCAGCCCAAGGCTGCCCCATCGGTCACTCTGTTCCCG CCCTCCTCTGAGGAGCTTCAAGCCAACAAGGCCACACTG GTGTGCCTGATCAGTGACTTCTACCCGGGAGCTGTGAAA GTGGCCTGGAAGGCAGATGGCAGCCCCGTCAACACGGG AGTGGAGACCACCACACCCTCCAAACAGAGCAACAACA AGTACGCGGCCAGCAGCTACCTGAGCCTGACGCCTGAGC AGTGGAAGTCCCACAGAAGCTACAGCTGCCAGGTCACG CATGAAGGGAGCACCGTGGAGAAGACAGTGGCCCCTGC AGAATGTTCATAG |
| Cλ7 | 19 | GTCAGCCCAAGGCTGCCCCCTCGGTCACTCTGTTCCCAC CCTCCTCTGAGGAGCTTCAAGCCAACAAGGCCACACTGG TGTGTCTCGTAAGTGACTTCTACCCGGGAGCCGTGACAG TGGCCTGGAAGGCAGATGGCAGCCCCGTCAAGGTGGGA GTGGAGACCACCAAACCCTCCAAACAAAGCAACAACAA GTATGCGGCCAGCAGCTACCTGAGCCTGACGCCCGAGCA GTGGAAGTCCCACAGAAGCTACAGCTGCCGGGTCACGC ATGAAGGGAGCACCGTGGAGAAGACAGTGGCCCCTGCA GAATGCTCT |

**TABLE 10: Amino Acid Sequence of Certain Human Cλ Gene Segments**

| **Gene Segment** | **SEQ ID NO** | **Sequence** |
|---|---|---|
| Cλ1 | 20 | PKANPTVTLFPPSSEELQANKATLVCLISDFYPGAVTVAWK ADGSPVKAGVETTKPSKQSNNKYAASSYLSLTPEQWKSHR SYSCQVTHEGSTVEKTVAPTECS |
| Cλ2 | 21 | QPKAAPSVTLFPPSSEELQANKATLVCLISDFYPGAVTVAW KADSSPVKAGVETTTPSKQSNNKYAASSYLSLTPEQWKSH RSYSCQVTHEGSTVEKTVAPTECS |
| Cλ3 | 22 | PKAAPSVTLFPPSSEELQANKATLVCLISDFYPGAVTVAWK ADSSPVKAGVETTTPSKQSNNKYAASSYLSLTPEQWKSHK SYSCQVTHEGSTVEKTVAPTECS |
| Cλ6 | 23 | QPKAAPSVTLFPPSSEELQANKATLVCLISDFYPGAVKVAW KADGSPVNTGVETTTPSKQSNNKYAASSYLSLTPEQWKSH RSYSCQVTHEGSTVEKTVAPAECS |
| Cλ7 | 24 | QPKAAPSVTLFPPSSEELQANKATLVCLVSDFYPGAVTVA WKADGSPVKVGVETTKPSKQSNNKYAASSYLSLTPEQWKS HRSYSCRVTHEGSTVEKTVAPAECS |

The following are representative nucleotide and amino acid sequences of mouse, rat, or human kappa constant regions or domains, which can be utilized in some embodiments of a non-human animal as described herein.

Nucleotide Sequence of a Mouse Cκ (SEQ ID NO: 25) (as reproduced in Figure 7):

GGGCTGATGCTGCACCAACTGTATCCATCTTCCCACCATCCAGTGAGC
AGTTAACATCTGGAGGTGCCTCAGTCGTGTGCTTCTTGAACAACTTCTACCCCAAAG
ACATCAATGTCAAGTGGAAGATTGATGGCAGTGAACGACAAAATGGCGTCCTGAAC
AGTTGGACTGATCAGGACAGCAAAGACAGCACCTACAGCATGAGCAGCACCCTCAC
GTTGACCAAGGACGAGTATGAACGACATAACAGCTATACCTGTGAGGCCACTCACA
AGACATCAACTTCACCCATTGTCAAGAGCTTCAACAGGAATGAGTGTTAGAGACAA
AGGTCCTGAGACGCCACCACCAGCTCCCCAGCTCCATCCTATCTTCCCTTCTAAGGT
CTTGGAGGCTTCCCCACAAGCGACCTACCACTGTTGCGGTGCTCCAAACCTCCTCCC
CACCTCCTTCTCCTCCTCCTCCCTTTCCTTGGCTTTTATCATGCTAATATTTGCAGAAA
ATATTCAATAAAGTGAGTCTTTGCACTTGA

With regard to SEQ ID NO: 25, the following applies:
- The coding sequence is denoted by bold letters; and
- The 3' untranslated region is not bolded.

Amino Acid Sequence of a Mouse Cκ (SEQ ID NO: 26):

ADAAPTVSIFPPSSEQLTSGGASVVCFLNNFYPKDINVKWKIDGSERQNGV
LNSWTDQDSKDSTYSMSSTLTLTKDEYERHNSYTCEATHKTSTSPIVKSFNRNEC

Nucleotide Sequence of a Rat Cκ (SEQ ID NO: 27) (as reproduced in Figure 8):

GGGCTGATGCTGCACCAACTGTATCTATCTTCCCACCATCCACGGAAC
AGTTAGCAACTGGAGGTGCCTCAGTCGTGTGCCTCATGAACAACTTCTATCCCAGAG

ACATCAGTGTCAAGTGGAAGATTGATGGCACTGAACGACGAGATGGTGTCCTGGAC
AGTGTTACTGATCAGGACAGCAAAGACAGCACGTACAGCATGAGCAGCACCCTCTC
GTTGACCAAGGCTGACTATGAAAGTCATAACCTCTATACCTGTGAGGTTGTTCATAA
GACATCATCCTCACCCGTCGTCAAGAGCTTCAACAGGAATGAGTGTTAGACCCAAA
GGTCCTGAGGTGCCACCTGCTCCCCAGCTCCTTCCAATCTTCCCTCCTAAGGTCTTGG
AGACTTCCCCACAAGCGACCTACCACTGTTGCGGTGCTCCAAACCTCCTCCCCACCT
CATCCTCCTTCCTTTCCTTGGCTTTGATCATGCTAATATTTGGGGAATATTAAATAAA
GTGAATCTTTGCACTTGA

With regard to SEQ ID NO: 27, the following applies:
- The coding sequence is denoted by bold letters; and
- The 3' untranslated region is not bolded.

Amino Acid Sequence of a Rat Cκ (SEQ ID NO: 28):

ADAAPTVSIFPPSTEQLATGGASVVCLMNNFYPRDISVKWKIDGTERRDG
VLDSVTDQDSKDSTYSMSSTLSLTKADYESHNLYTCEVVHKTSSSPVVKSFNRNEC

Nucleotide Sequence of a Human Cκ (SEQ ID NO: 29):

GAACTGTGGCTGCACCATCTGTCTTCATCTTCCCGCCATCTGATGAGCA
GTTGAAATCTGGAACTGCCTCTGTTGTGTGCCTGCTGAATAACTTCTATCCCAGAGA
GGCCAAAGTACAGTGGAAGGTGGATAACGCCCTCCAATCGGGTAACTCCCAGGAGA
GTGTCACAGAGCAGGACAGCAAGGACAGCACCTACAGCCTCAGCAGCACCCTGACG
CTGAGCAAAGCAGACTACGAGAAACACAAAGTCTACGCCTGCGAAGTCACCCATCA
GGGCCTGAGCTCGCCCGTCACAAAGAGCTTCAACAGGGGAGAGTGT

Amino Acid Sequence of a Human Cκ (SEQ ID NO: 30):

TVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRGEC

Nucleotide Sequence of an Engineered Vλ1-51/Jλ2 Vector(SEQ ID NO: 31) (as shown schematically inFigure 1A and reproduced in Figures 9A-9C):

GGCGCGCCacctgcagtgtacacctgaggtgacatggttctgaacatcactttccccattgaaggcccgagtg
acctatcacttgcttctcaaaattcaggaactaggtgactctaggagagtgcacaggaagctttctgcatattctttcaagagatttcgaggtca

ctgactcttttttcaagacttaaacacaaaatccacatccatcattcttgcgtaaagagaccattcttttttttttgagatggagtctcgctctgttgc
ccaagctggagtgcagtggcatgatctcggctcactgcaagctctgtcttctgggttcatgcccttctcctgcctcagcctcccaagtagctgg
gactacaggcacccgccgccatgcccggctaattttttgtattttttagtggagatggggtttcaacatgttggccagaatggtcttgatctcttg
acctcgtgatccacccgccttggcctcccaaagtgctgggattacaggtgtgagccactgcacccggcccaagatggggtttcatcatgttg
acctggctggtctcaaactcccaacctcaagtgatccactcacttcagcctcccaaagtgctgggatgacaggcgtgagccacagtgcccg
gcctagtacattgtttaaatggtgcatgtaaaacctcatggtactacaactgcctggtgacagactcaccagagtaaataactatcctgctgttct
ccaaccacatgggattccccaaacaaagatttgttgagtccaagatgcatcatgccagaactcttgcagtcatacacataatttgtttcattttaa
taaatacggttatcttttttcttcagtttattgtttttaatgtatcatcttctaggtttcattacacatcaaagatgaataatttctctcatctttaggacatg
ctgaaaacactcattagtgattttgaacagtattatttcctaaacatcctagttggaatggttgatcctaaaagctaccatgtgtcaggctcacctg
agtgcagactgtggtctaaacaagctaaagttttaattcttccatcttcaaatacggagcatatttgcttgttttgctctttagcaaaacactgcaaa
gtcattcgccaggatctcctgcagactggcattcagtgatggagaacagactcctaggtgagatccctggtcacaggatccagatgactatg
actgcaagtctggaaaagaagagacagaacaaagatttaccagcataaccctctgagaatgtgccccagccatcctggagggaagagctc
cctattgacccagcactgcagaatccacagaggaaggacagctctggccaccagggagcagccacacgacaagagaggacccggtgtc
cccacaggtgaggaaatgagtccatggggctggtcagtgctctcgatttcattctgccataacaggactttgtgtagtcaccacctgagtccta
tacagtgaacagccaaggagacatggacagattcacccctgaggaacccagtagaatgtgaagtctgccctgaggtcacgaagaaaaga
ctcggggggctgcctggcctggccctgatggagggggccatggacagaacaacggggaggcaggggtgtctctgagaggctctggtca
ccttgtcatacaatggtggtgtacaatgtcgcaccatggacactagggggcgcctgcgcaccattcctgagaagactgggtgtgatgagag
caggaccagcgccacctgtcctgcttggtgccctatgcttagggctcacagatgtcaactctccaccccctgggaccacacagccccaccc
ctggcactctctgacatcctcaggcagaggagcttgacccagggcccagggtgggatcagaaagctggagggtctgatttgcatggatgg
accctccttctctcagagtataaagaggggcagggagagacttggggaagctctgcttcagctgTGAGCGCAGAAGGCAGG
ACTCGGGACAATCTTCATCATGACCTGCTCCCCTCTCCTCCTCACCCTTCTCATTCACT
GCACAGgtgcccagacacagggtcaggggaggggtccaggaagcccatgaggccctgctttctccttctctctctagaccaag
aatcaccgtgtctgtgtctctcctgcttccagGGTCCTGGGCCCAGTCTGTGTTGACGCAGCCGCC
CTCAGTGTCTGCGGCCCCAGGACAGAAGGTCACCATCTCCTGCTCTGGAAGCA
GCTCCAACATTGGGAATAATTATGTATCCTGGTACCAGCAGCTCCCAGGAACA
GCCCCCAAACTCCTCATTTATGACAATAATAAGCGACCCTCAGGGATTCCTGAC
CGATTCTCTGGCTCCAAGTCTGGCACGTCAGCCACCCTGGGCATCACCGGACT
CCAGACTGGGGACGAGGCCGATTATTACTGCGGAACATGGGATAGCAGCCTGA
GTGCTGTGGTATTCGGCGGAGGGACCAAGCTGACCGTCCTAGgtaagtaatttttcactattg
tcttctgaaatttgggtctgatggccagtattgacttttagaggcttaaataggagtttggtaaagattggtaaatgagggcatttaagatttg
ccatgggttgcaaaagttaaactcagcttcaaaaatggatttggagaaaaaaagattaaattgctctaaactgaatgacacaaagtaa

aaaaaaaaagtgtaactaaaaaggaacccttgtatttctaaggagcaaaagtaaatttatttttgttcactcttgccaaatattgtattggtt
gttgctgattatgcatgatacagaaaagtggaaaaatacattttttagtctttctcccttttgtttgataaattattttgtcagacaacaataaa
aatcaatagcacgccctaagaTGCCATTTCATTACCTCTTTCTCCGCACCCGACATAGAT

With regard to SEQ ID NO: 31, the following applies:
- Restriction enzyme site sequences - AscI (5' end of sequence) and PI-SceI (3' end of sequence) - are denoted in uppercase, italicized letters;
- A Vλ1-51 promoter sequence is denoted by lowercase (not bold) letters;
- A Vλ1-51 5' UTR sequence is denoted by uppercase, italicized, and bold letters;
- A rearranged Vλ1-51/Jλ2 sequence, which includes:
   ∘ A Vλ1-51, exon 1 sequence is denoted by uppercase (not bold) letters, where the Vλ1-51, exon 1 start codon is further italicized and underlined;
   ∘ A Vλ1-51, intron 1 sequence is denoted by lowercase, bold letters;
   ∘ A Vλ1-51, exon 2 sequence denoted by uppercase, bold, and underlined (solid underline) letters, and
   ∘ A a Jλ2 sequence denoted by uppercase, bold and underlined (dashed underline) letters; and
- A human Jκ5-Cκ intron sequence is denoted by lowercase, italicized letters.

Nucleotide Sequence of a Rearranged Vλ1-51/Jλ2 Variable Region Including a Vλ1-51 Intron (SEQ ID NO: 32) (as reproduced in Figure 10):

ATGACCTGCTCCCCTCTCCTCCTCACCCTTCTCATTCACTGCACAGgtgcc
cagacacagggtcaggggaggggtccaggaagcccatgaggccctgctttctccttctctctctagaccaagaatcaccgtgtctgtgtctc
tcctgcttccagGGTCCTGGGCCCAGTCTGTGTTGACGCAGCCGCCCTCAGTGTCTGCGGCC
CCAGGACAGAAGGTCACCATCTCCTGCTCTGGAAGCAGCTCCAACATTGGGAATAA
TTATGTATCCTGGTACCAGCAGCTCCCAGGAACAGCCCCCAAACTCCTCATTTATGA
CAATAATAAGCGACCCTCAGGGATTCCTGACCGATTCTCTGGCTCCAAGTCTGGCAC
GTCAGCCACCCTGGGCATCACCGGACTCCAGACTGGGGACGAGGCCGATTATTACT
GCGGAACATGGGATAGCAGCCTGAGTGCTGTGGTATTCGGCGGAGGGACCAAGCTG
ACCGTCCTAG

With regard to SEQ ID NO: 32, the following applies:
- A Vλ1-51, exon 1 sequence is denoted by uppercase letters, where the Vλ1-51, exon 1 start codon is further italicized and underlined;
- A Vλ1-51, intron 1 sequence is denoted by lowercase, bold letters;
- A Vλ1-51, exon 2 sequence denoted by uppercase, bold, and underlined (solid underline) letters; and
- A Jλ2 sequence denoted by uppercase, bold and underlined (dashed underline) letters.

Nucleotide Sequence of a Rearranged Vλ1-51/Jλ2 Variable Region Without a Vλ1-51 Intron (SEQ ID NO: 33) (as reproduced in Figure 11):

ATGACCTGCTCCCCTCTCCTCCTCACCCTTCTCATTCACTGCACAGGGT
CCTGGGCCCAGTCTGTGTTGACGCAGCCGCCCTCAGTGTCTGCGGCCCCAGGACAGA
AGGTCACCATCTCCTGCTCTGGAAGCAGCTCCAACATTGGGAATAATTATGTATCCT
GGTACCAGCAGCTCCCAGGAACAGCCCCCAAACTCCTCATTTATGACAATAATAAG
CGACCCTCAGGGATTCCTGACCGATTCTCTGGCTCCAAGTCTGGCACGTCAGCCACC
CTGGGCATCACCGGACTCCAGACTGGGGACGAGGCCGATTATTACTGCGGAACATG
GGATAGCAGCCTGAGTGCTGTGGTATTCGGCGGAGGGACCAAGCTGACCGTCCTAG

With regard to SEQ ID NO: 33, the following applies:
- A Vλ1-51 coding sequence is denoted by uppercase letters, where the Vλ1-51 start codon is further italicized and underlined; and
- A Jλ2 sequence is denoted by uppercase letters with a dashed underline.

Amino Acid Sequence of a Rearranged Vλ1-51/Jλ2 Variable Domain Including a Signal Peptide (SEQ ID NO: 34) (as reproduced in Figure 12):

MTCSPLLLTLLIHCTGSWAQSVLTQPPSVSAAPGQKVTISCSGSSSNIGNN
YVSWYQQLPGTAPKLLIYDNNKRPSGIPDRFSGSKSGTSATLGITGLQTGDEADYYCGT
WDSSLSAVVFGGGTKLTVL

With regard to SEQ ID NO: 34, the italicized, bold text indicates the sequence of a signal peptide.

Amino Acid Sequence of a Rearranged Vλ1-51/Jλ2 Variable Domain Without a Signal Peptide (SEQ ID NO: 35):

QSVLTQPPSVSAAPGQKVTISCSGSSSNIGNNYVSWYQQLPGTAPKLLIYD
NNKRPSGIPDRFSGSKSGTSATLGITGLQTGDEADYYCGTWDSSLSAVVFGGGTKLTVL

Nucleotide Sequence of an Engineered Vλ2-14/Jλ2 Vector (SEQ ID NO: 36) (as shown schematically in Figure 2A and reproduced in Figures 13A-13C):

GGCGCGCCacctgcagtgtacacctgaggtgacatggttctgaacatcactttccccattgaaggcccgagtgacctatcacttgcttc
tcaaaattcaggaactaggtgactctaggagagtgcacaggaagctttctgcatattctttcaagagatttcgaggtcactgactcttttttcaag
acttaaacacaaaatccacatccatcattcttgcgtaaagagaccattcttttttttttgagatggagtctcgctctgttgcccaagctggagtgca
gtggcatgatctcggctcactgcaagctctgtcttctgggttcatgcccttctcctgcctcagcctcccaagtagctgggactacaggcaccc
gccgccatgcccggctaattttttgtattttttagtggagatggggtttcaacatgttggccagaatggtcttgatctcttgacctcgtgatccacc
cgccttggcctcccaaagtgctgggattacaggtgtgagccactgcacccggcccaagatggggtttcatcatgttgacctggctggtctca
aactcccaacctcaagtgatccactcacttcagcctcccaaagtgctgggatgacaggcgtgagccacagtgcccggcctagtacattgttt
aaatggtgcatgtaaaacctcatggtactacaactgcctggtgacagactcaccagagtaaataactatcctgctgttctccaaccacatggg
attccccaaacaaagatttgttgagtccaagatgcatcatgccagaactcttgcagtcatacacataatttgtttcattttaataaatacggttatct
tttttcttcagtttattgtttttaatgtatcatcttctaggtttcattacacatcaaagatgaataatttctctcatctttaggacatgctgaaaacactcat
tagtgattttgaacagtattatttcctaaacatcctagttggaatggttgatcctaaaagctaccatgtgtcaggctcacctgagtgcagactgtg
gtctaaacaagctaaagttttaattcttccatcttcaaatacggagcatatttgcttgttttgctctttagcaaaacactgcaaagtcattcgccagg
atctcctgcagactggcattcagtgatggagaacagactcctaggtgagatccctggtcacaggatccagatgactatgactgcaagtctgg
aaaagaagagacagaacaaagatttaccagcataaccctctgagaatgtgccccagccatcctggagggaagagctccctattgacccag
cactgcagaatccacagaggaaggacagctctggccaccagggagcagccacacgacaagagaggacccggtgtccccacaggtgag
gaaatgagtccatggggctggtcagtgctctcgatttcattctgccataacaggactttgtgtagtcaccacctgagtcctatacagtgaacag
ccaaggagacatggacagattcacccctgaggaacccagtagaatgtgaagtctgccctgaggtcacgaagaaaagactcggggggctg
cctggcctggccctgatggagggggccatggacagaacaacggggaggcaggggtgtctctgagaggctctggtcaccttgtcatacaat
ggtggtgtacaatgtcgcaccatggacactagggggcgcctgcgcaccattcctgagaagactgggtgtgatgagagcaggaccagcgc
cacctgtcctgcttggtgccctatgcttagggctcacagatgtcaactctccaccccctgggaccacacagccccacccctggcactctctga
catcctcaggcagaggagcttgacccagggcccagggtgggatcagaaagctggagggtctgatttgcatggatggaccctccttctctca
gagtataaagaggggcagggagagacttggggaagctctgcttcagctgTGAGCGCAGAAGGCAGGACTCGGGA
CAATCTTCATCATGGCCTGGGCTCTGCTGCTCCTCACCCTCCTCACTCAGGGCACAGgt
gacgcctccagggaaggggcttcagggacctctgggctgatccttggtctcctgctcctcaggctcaccggggcccagcactgactc
actggcatgtgtttctccctctttccagGGTCCTGGGCCCAGTCTGCCCTGACTCAGCCTGCCTC
CGTGTCTGGGTCTCCTGGACAGTCGATCACCATCTCCTGCACTGGAACCAGCA

GTGACGTTGGTGGTTATAACTATGTCTCCTGGTACCAACAGCACCCAGGCAAA
GCCCCCAAACTCATGATTTATGAGGTCAGTAATCGGCCCTCAGGGGTTTCTAAT
CGCTTCTCTGGCTCCAAGTCTGGCAACACGGCCTCCCTGACCATCTCTGGGCTC
CAGGCTGAGGACGAGGCTGATTATTACTGCAGCTCATATACAAGCAGCAGCAC
TCTCGTGGTATTCGGCGGAGGGACCAAGCTGACCGTCCTAGgtaagtaatttttcactattgtc
ttctgaaatttgggtctgatggccagtattgacttttagaggcttaaataggagtttggtaaagattggtaaatgagggcatttaagatttgc
catgggttgcaaaagttaaactcagcttcaaaaatggatttggagaaaaaaagattaaattgctctaaactgaatgacacaaagtaaa
aaaaaaaagtgtaactaaaaaggaacccttgtatttctaaggagcaaaagtaaatttatttttgttcactcttgccaaatattgtattggttg
ttgctgattatgcatgatacagaaaagtggaaaaatacattttttagtctttctcccttttgtttgataaattattttgtcagacaacaataaaa
atcaatagcacgccctaagaTGCCATTTCATTACCTCTTTCTCCGCACCCGACATAGAT

With regard to SEQ ID NO: 36, the following applies:
- Restriction enzyme site sequences - AscI (5' end of sequence) and PI-SceI (3' end of sequence) - are denoted in uppercase, italicized letters;
- A Vλ1-51 promoter sequence is denoted by lowercase (not bold) letters;
- A Vλ1-51 5' UTR sequence is denoted by uppercase, italicized, and bold letters;
- A rearranged Vλ2-14/Jλ2 sequence, which includes:
   ∘ A Vλ2-14, exon 1 sequence is denoted by uppercase, (not bold) letters, where the Vλ2-14, exon 1 start codon is further italicized and underlined;
   ∘ A Vλ2-14, intron 1 sequence is denoted by lowercase, bold letters;
   ∘ A Vλ2-14, exon 2 sequence denoted by uppercase, bold, and underlined (solid underline) letters, and
   ∘ A Jλ2 sequence denoted by uppercase, bold and underlined (dashed underline) letters; and
   ∘ A human Jκ5-Cκ intron sequence is denoted by lowercase, italicized letters.

Nucleotide Sequence of a Rearranged Vλ2-14/Jλ2 Variable Region Including a Vλ2-14 Intron (SEQ ID NO: 37) (as reproduced in Figure 14):

ATGGCCTGGGCTCTGCTGCTCCTCACCCTCCTCACTCAGGGCACAGgtgacgcctccagggaag
gggcttcagggacctctgggctgatccttggtctcctgctcctcaggctcaccggggcccagcactgactcactggcatgtgtttctccctctt
tccagGGTCCTGGGCCCAGTCTGCCCTGACTCAGCCTGCCTCCGTGTCTGGGTCTCCTG
GACAGTCGATCACCATCTCCTGCACTGGAACCAGCAGTGACGTTGGTGGTTATAACT

ATGTCTCCTGGTACCAACAGCACCCAGGCAAAGCCCCCAAACTCATGATTTATGAGG
TCAGTAATCGGCCCTCAGGGGTTTCTAATCGCTTCTCTGGCTCCAAGTCTGGCAACA
CGGCCTCCCTGACCATCTCTGGGCTCCAGGCTGAGGACGAGGCTGATTATTACTGCA
GCTCATATACAAGCAGCAGCACTCTCGTGGTATTCGGCGGAGGGACCAAGCTGACC
GTCCTAG

With regard to SEQ ID NO: 37, the following applies:
- A Vλ2-14, exon 1 sequence is denoted by uppercase letters, where the Vλ2-14, exon 1 start codon is further italicized and underlined;
- A Vλ2-14, intron 1 sequence is denoted by lowercase, bold letters;
- A Vλ1-51, exon 2 sequence denoted by uppercase, bold, and underlined (solid underline) letters; and
- A Jλ2 sequence denoted by uppercase, bold and underlined (dashed underline) letters.

Nucleotide Sequence of a Rearranged Vλ2-14/Jλ2 Variable Region Without a Vλ2-14 Intron (SEQ ID NO: 38) (as reproduced in Figure 15):

ATGGCCTGGGCTCTGCTGCTCCTCACCCTCCTCACTCAGGGCACAGGGT
CCTGGGCCCAGTCTGCCCTGACTCAGCCTGCCTCCGTGTCTGGGTCTCCTGGACAGT
CGATCACCATCTCCTGCACTGGAACCAGCAGTGACGTTGGTGGTTATAACTATGTCT
CCTGGTACCAACAGCACCCAGGCAAAGCCCCCAAACTCATGATTTATGAGGTCAGT
AATCGGCCCTCAGGGGTTTCTAATCGCTTCTCTGGCTCCAAGTCTGGCAACACGGCC
TCCCTGACCATCTCTGGGCTCCAGGCTGAGGACGAGGCTGATTATTACTGCAGCTCA
TATACAAGCAGCAGCACTCTCGTGGTATTCGGCGGAGGGACCAAGCTGACCGTCCT
AG

With regard to SEQ ID NO: 38, the following applies:
- A Vλ2-14 sequence is denoted by uppercase letters, where the Vλ2-14 start codon is further italicized and underlined; and
- A Jλ2 sequence is denoted by uppercase letters with a dashed underline.

Amino Acid Sequence of a Rearranged Vλ2-14/Jλ2 Variable Domain Including a Signal Peptide (SEQ ID NO: 39) (as reproduced in Figure 16):

MAWALLLLTLLTQGTGSWAQSALTQPASVSGSPGQSITISCTGTSSDVGGY
NYVSWYQQHPGKAPKLMIYEVSNRPSGVSNRFSGSKSGNTASLTISGLQAEDEADYYCS
SYTSSSTLVVFGGGTKLTVL

With regard to SEQ ID NO: 39, the italicized, bold text indicates the sequence of a signal peptide.

Amino Acid Sequence of a Rearranged Vλ2-14/Jλ2 Variable Domain Without a Signal Peptide (SEQ ID NO: 40):

QSALTQPASVSGSPGQSITISCTGTSSDVGGYNYVSWYQQHPGKAPKLMI
YEVSNRPSGVSNRFSGSKSGNTASLTISGLQAEDEADYYCSSYTSSSTLVVFGGGTKLTV
L

### DEFINITIONS

The scope of the present invention is defined by the claims appended hereto and is not limited by certain embodiments described herein. Those skilled in the art, reading the present specification, will be aware of various modifications that may be equivalent to such described embodiments, or otherwise within the scope of the claims. In general, terms used herein are in accordance with their understood meaning in the art, unless clearly indicated otherwise. Explicit definitions of certain terms are provided below; meanings of these and other terms in particular instances throughout this specification will be clear to those skilled in the art from context. Additional definitions for the following and other terms are set forth throughout the specification. Patent and non-patent literature references cited within this specification, or relevant portions thereof, are incorporated herein by reference in their entireties.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

The articles "a" and "an," as used herein, should be understood to include the plural referents unless clearly indicated to the contrary. Claims or descriptions that include "or" between one or more members of a group are considered satisfied if one, more than one, or all of the group members are present in, employed in, or otherwise relevant to a given product or process unless indicated to the contrary or otherwise evident from the context. In some embodiments, exactly one member of a group is present in, employed in, or otherwise relevant to a given product or process. In some embodiments, more than one, or all group members are present in, employed in, or otherwise relevant to a given product or process. It is to be understood that the invention encompasses all variations, combinations, and permutations in which one or more limitations, elements, clauses, descriptive terms, etc., from one or more of the listed claims is introduced into another claim dependent on the same base claim (or, as relevant, any other claim) unless otherwise indicated or unless it would be evident to one of ordinary skill in the art that a contradiction or inconsistency would arise. Where elements are presented as lists (e.g., in Markush group or similar format), it is to be understood that each subgroup of the elements is also disclosed, and any element(s) can be removed from the group. It should be understood that, in general, where embodiments or aspects are referred to as "comprising" particular elements, features, etc., certain embodiments or aspects "consist," or "consist essentially of," such elements, features, etc. For purposes of simplicity, those embodiments have not in every case been specifically set forth in so many words herein. It should also be understood that any embodiment or aspect can be explicitly excluded from the claims, regardless of whether the specific exclusion is recited in the specification.

***Administration:*** as used herein, includes the administration of a composition (e.g., antigen or antibody) to a subject or system (e.g., to a cell, organ, tissue, organism, or relevant component or set of components thereof). The skilled artisan will appreciate that route of administration may vary depending, for example, on the subject or system to which the composition is being administered, the nature of the composition, the purpose of the administration, etc. For example, in certain embodiments, administration to an animal subject (e.g., to a human or a rodent) may be bronchial (including by bronchial instillation), buccal, enteral, interdermal, intra-arterial, intradermal, intragastric, intramedullary, intramuscular, intranasal, intraperitoneal, intrathecal, intravenous, intraventricular, mucosal, nasal, oral, rectal, subcutaneous, sublingual, topical, tracheal (including by intratracheal instillation), transdermal, vaginal and/or vitreal. In some embodiments, administration may involve intermittent dosing. In some embodiments, administration may involve continuous dosing (e.g., perfusion) for at least a selected period of time.

***Antigen binding protein:*** as used herein, refers to any protein or polypeptide that specifically binds to at least one antigen of interest. Antigen binding proteins include, but are not limited to, antibodies, heavy chains, light chains (e.g., λ or κ light chains), heavy chain variable domains, light chain variable domains (e.g., λ or κ light chain variable domains), and single chain variable fragments (ScFv). In some embodiments, antigen binding proteins can be multispecific and specifically bind to two or more epitopes or antigens.

***Approximately:*** as applied to one or more values of interest, includes to a value that is similar to a stated reference value. In certain embodiments, the term *"approximately"* or *"about"* refers to a range of values that fall within ± 10% (greater than or less than) of the stated reference value unless otherwise stated or otherwise evident from the context (except where such number would exceed 100% of a possible value).

***Biologically active:*** as used herein, refers to a characteristic of any agent that has activity in a biological system, *in vitro* or *in vivo* (e.g., in an organism). For instance, an agent that, when present in an organism, has a biological effect within that organism is considered to be biologically active. In particular embodiments, where a protein or polypeptide is biologically active, a portion of that protein or polypeptide that shares at least one biological activity of the protein or polypeptide is typically referred to as a *"biologically active"* portion.

***Comparable:*** as used herein, refers to two or more agents, entities, situations, sets of conditions, etc. that may not be identical to one another but that are sufficiently similar to permit comparison there between so that conclusions may reasonably be drawn based on differences or similarities observed. Persons of ordinary skill in the art will understand, in context, what degree of identity is required in any given circumstance for two or more such agents, entities, situations, sets of conditions, etc. to be considered comparable.

***Conservative:*** as used herein, refers to instances when describing a conservative amino acid substitution, including a substitution of an amino acid residue by another amino acid residue having a side chain R group with similar chemical properties (e.g., charge or hydrophobicity). In general, a conservative amino acid substitution will not substantially change the functional properties of interest of a protein, for example, the ability of a receptor to bind to a ligand. Examples of groups of amino acids that have side chains with similar chemical properties include: aliphatic side chains such as glycine (Gly, G), alanine (Ala, A), valine (Val, V), leucine (Leu, L), and isoleucine (Ile, I); aliphatic-hydroxyl side chains such as serine (Ser, S) and threonine (Thr, T); amide-containing side chains such as asparagine (Asn, N) and glutamine (Gln, Q); aromatic side chains such as phenylalanine (Phe, F), tyrosine (Tyr, Y), and tryptophan (Trp, W); basic side chains such as lysine (Lys, K), arginine (Arg, R), and histidine (His, H); acidic side chains such as aspartic acid (Asp, D) and glutamic acid (Glu, E); and sulfur-containing side chains such as cysteine (Cys, C) and methionine (Met, M). Conservative amino acids substitution groups include, for example, valine/leucine/isoleucine (Val/Leu/Ile, V/L/I), phenylalanine/tyrosine (Phe/Tyr, F/Y), lysine/arginine (Lys/Arg, K/R), alanine/valine (Ala/Val, A/V), glutamate/aspartate (Glu/Asp, E/D), and asparagine/glutamine (Asn/Gln, N/Q). In some embodiments, a conservative amino acid substitution can be a substitution of any native residue in a protein with alanine, as used in, for example, alanine scanning mutagenesis. In some embodiments, a conservative substitution is made that has a positive value in the PAM250 log-likelihood matrix disclosed in Gonnet, G.H. et al., 1992, Science 256:1443-1445, which is incorporated herein by reference in its entirety. In some embodiments, a substitution is a moderately conservative substitution wherein the substitution has a nonnegative value in the PAM250 log-likelihood matrix.

***Control:*** as used herein, refers to the art-understood meaning of a *"control"* being a standard against which results are compared. Typically, controls are used to augment integrity in experiments by isolating variables in order to make a conclusion about such variables. In some embodiments, a control is a reaction or assay that is performed simultaneously with a test reaction or assay to provide a comparator. A *"control"* also includes a *"control animal."* A *"control animal"* may have a modification as described herein, a modification that is different as described herein, or no modification (i.e., a wild-type animal). In one experiment, a *"test"* parameter (e.g., a variable being tested) is applied. In a second experiment, the *"control,"* the variable being tested is not applied. In some embodiments, a control is a historical control (i.e., of a test or assay performed previously, or an amount or result that is previously known). In some embodiments, a control is or comprises a printed or otherwise saved record. A control may be a positive control or a negative control.

***Disruption:*** as used herein, refers to the result of a homologous recombination event with a DNA molecule (e.g., with an endogenous homologous sequence such as a gene or gene locus). In some embodiments, a disruption may achieve or represent an insertion, deletion, substitution, replacement, missense mutation, or a frame-shift of a DNA sequence(s), or any combination thereof. Insertions may include the insertion of entire genes or gene fragments, e.g., exons, which may be of an origin other than the endogenous sequence (e.g., a heterologous sequence). In some embodiments, a disruption may increase expression and/or activity of a gene or gene product (e.g., of a polypeptide encoded by a gene). In some embodiments, a disruption may decrease expression and/or activity of a gene or gene product. In some embodiments, a disruption may alter sequence of a gene or an encoded gene product (e.g., an encoded polypeptide). In some embodiments, a disruption may truncate or fragment a gene or an encoded gene product (e.g., an encoded polypeptide). In some embodiments, a disruption may extend a gene or an encoded gene product. In some such embodiments, a disruption may achieve assembly of a fusion polypeptide. In some embodiments, a disruption may affect level, but not activity, of a gene or gene product. In some embodiments, a disruption may affect activity, but not level, of a gene or gene product. In some embodiments, a disruption may have no significant effect on level of a gene or gene product. In some embodiments, a disruption may have no significant effect on activity of a gene or gene product. In some embodiments, a disruption may have no significant effect on either level or activity of a gene or gene product.

***Determining, measuring, evaluating, assessing, assaying*** and ***analyzing**:* are used interchangeably herein to refer to any form of measurement, and include determining if an element is present or not. These terms include both quantitative and/or qualitative determinations. Assaying may be relative or absolute. *"Assaying for the presence of"* can be determining the amount of something present and/or determining whether or not it is present or absent.

***Endogenous promoter:*** as used herein, refers to a promoter that is naturally associated, e.g., in a wild-type organism, with an endogenous gene.

***Engineered:*** as used herein refers, in general, to the aspect of having been manipulated by the hand of man. For example, in some embodiments, a polynucleotide may be considered to be *"engineered"* when two or more sequences that are not linked together in that order in nature are manipulated by the hand of man to be directly linked to one another in the engineered polynucleotide. In some embodiments, an engineered polynucleotide may comprise a regulatory sequence that is found in nature in operative association with a first coding sequence but not in operative association with a second coding sequence, is linked by the hand of man so that it is operatively associated with the second coding sequence. Alternatively, or additionally, in some embodiments, first and second nucleic acid sequences that each encode polypeptide elements or domains that in nature are not linked to one another may be linked to one another in a single engineered polynucleotide. Comparably, in some embodiments, a cell or organism may be considered to be *"engineered"* if it has been manipulated so that its genetic information is altered (e.g., new genetic material not previously present has been introduced, or previously present genetic material has been altered or removed). As is common practice and is understood by persons of skill in the art, progeny of an engineered polynucleotide or cell are typically still referred to as *"engineered"* even though the actual manipulation was performed on a prior entity. Furthermore, as will be appreciated by persons of skill in the art, a variety of methodologies are available through which *"engineering"* as described herein may be achieved. For example, in some embodiments, *"engineering"* may involve selection or design (e.g., of nucleic acid sequences, polypeptide sequences, cells, tissues, and/or organisms) through use of computer systems programmed to perform analysis or comparison, or otherwise to analyze, recommend, and/or select sequences, alterations, etc.). Alternatively, or additionally, in some embodiments, *"engineering"* may involve use of *in vitro* chemical synthesis methodologies and/or recombinant nucleic acid technologies such as, for example, nucleic acid amplification (e.g., via the polymerase chain reaction) hybridization, mutation, transformation, transfection, etc., and/or any of a variety of controlled mating methodologies. As will be appreciated by those skilled in the art, a variety of established such techniques (e.g., for recombinant DNA, oligonucleotide synthesis, and tissue culture and transformation (e.g., electroporation, lipofection, etc.)) are well known in the art and described in various general and more specific references that are cited and/or discussed throughout the present specification. See e.g., Sambrook et al., Molecular Cloning: A Laboratory Manual 2nd ed., Cold Spring Harbor Laboratory Press, Cold Spring Harbor, N.Y., 1989 and Principles of Gene Manipulation: An Introduction to Genetic Manipulation, 5th Ed., ed. By Old, R.W. and S.B. Primrose, Blackwell Science, Inc., 1994, incorporated herein by reference in their entireties.

***Functional:*** as used herein, refers to a form or fragment of an entity (e.g., a gene or gene segment) that exhibits a particular property (e.g., forms part of a coding sequence) and/or activity. For example, in the context of immunoglobulins, variable regions are encoded by unique gene segments (i.e., V, D and/or J) that are assembled (or recombined) to form functional coding sequences. When present in the genome, gene segments are organized in clusters, although variations do occur. A *"functional"* gene segment is a gene segment represented in an expressed sequence (i.e., a variable region) for which the corresponding genomic DNA has been isolated (i.e., cloned) and identified by sequence. Some immunoglobulin gene segment sequences contain open reading frames and are considered functional although not represented in an expressed repertoire, while other immunoglobulin gene segment sequences contain mutations (e.g., point mutations, insertions, deletions, etc.) resulting in a stop codon and/or truncated sequence which subsequently render(s) such gene segment sequences unable to perform the property/ies and/or activity/ies associated with a non-mutated sequence(s). Such sequences are not represented in expressed sequences and, therefore, categorized as pseudogenes.

***Gene:*** as used herein, refers to a DNA sequence in a chromosome that codes for a product (e.g., an RNA product and/or a polypeptide product). In some embodiments, a gene includes coding sequence (i.e., sequence that encodes a particular product). In some embodiments, a gene includes non-coding sequence. In some particular embodiments, a gene may include both coding (e.g., exonic) and non-coding (e.g., intronic) sequence. In some embodiments, a gene may include one or more regulatory sequences (e.g., promoters, enhancers, etc.) and/or intron sequences that, for example, may control or impact one or more aspects of gene expression (e.g., cell-type-specific expression, inducible expression, etc.). For the purpose of clarity, we note that, as used in the present disclosure, the term *"gene"* generally refers to a portion of a nucleic acid that encodes a polypeptide or fragment thereof; the term may optionally encompass regulatory sequences, as will be clear from context to those of ordinary skill in the art. This definition is not intended to exclude application of the term *"gene"* to non-protein-coding expression units but rather to clarify that, in most cases, the term as used in this document refers to a polypeptide-coding nucleic acid.

***Genetically modified non-human animal or genetically engineered non-human animal:*** are used interchangeably herein and refer to any non-naturally occurring non-human animal (e.g., a rodent, e.g., a rat or a mouse) in which one or more of the cells of the non-human animal contain heterologous nucleic acid and/or gene encoding a polypeptide of interest, in whole or in part. For example, in some embodiments, a *"genetically modified non-human animal"* or *"genetically engineered non-human animal"* refers to non-human animal that contains a transgene or transgene construct as described herein. In some embodiments, a heterologous nucleic acid and/or gene is introduced into the cell, directly or indirectly by introduction into a precursor cell, by way of deliberate genetic manipulation, such as by microinjection or by infection with a recombinant virus. The term genetic manipulation does not include classic breeding techniques, but rather is directed to introduction of recombinant DNA molecule(s). This molecule may be integrated within a chromosome. The phrases *"genetically modified non-human animal"* or *"genetically engineered non-human animal"* refers to animals that are heterozygous or homozygous for a heterologous nucleic acid and/or gene, and/or animals that have single or multi-copies of a heterologous nucleic acid and/or gene.

***Germline Configuration:*** as used herein, refers to an arrangement of sequences (e.g., gene segments) as found in an endogenous germline genome of a wild-type animal (e.g., mouse, rat, or human). Examples of germline configurations of immunoglobulin gene segments can be found, e.g., in LeFranc, M-P., The Immunoglobulin FactsBook, Academic Press, May, 23, 2001 (referred to herein as "LeFranc 2001"):
An exemplary configuration of human heavy chain variable region gene segments and human heavy chain constant region genes can be found at p. 47 of LeFranc 2001;
An exemplary configuration of human λ light chain variable region gene segments and human λ light chain constant region genes can be found at p. 61 of LeFranc 2001;
An exemplary configuration of human κ light chain variable region gene segments and human κ light chain constant region genes can be found at p. 53 of LeFranc 2001;
An exemplary configuration of mouse heavy chain variable region gene segments and mouse heavy chain constant region genes can be found at Lucas, J. et al., Chapter 1: The Structure and Regulation of the Immunoglobulin Loci, Molecular Biology of B Cells, 2nd Edition,
Academic Press, 2015 (Lucas);
An exemplary configuration of mouse λ light chain variable region gene segments and mouse λ light chain constant region genes can be found at LeFranc, M-P et al., Chapter 4: Immunoglobulin Lambda (IGL) Genes of Human and Mouse, Molecular Biology of B Cells, 1st Edition, Academic Press, 2004 (LeFranc 2004);
An exemplary configuration of mouse κ light chain variable region gene segments and mouse κ light chain constant region genes can be found at Christele, M-J, et al., Nomenclature and
Overview of the Mouse (*Mus musculus* and *Mus* sp.) Immunoglobulin Kappa (IGK) Genes,
Exp Clin Immunogenet 2001, 18:255-279 (Christele);
Each of the cited sections of LeFranc 2001, Lucas, LeFranc 2004, and Christele are incorporated herein by reference.

***Germline Genome:*** as used herein, refers to the genome found in a germ cell (e.g., a gamete, e.g., a sperm or egg) used in the formation of an animal. A germline genome is a source of genomic DNA for cells in an animal. As such, an animal (e.g., a mouse or rat) having a modification in its germline genome is considered to have the modification in the genomic DNA of all of its cells.

***Germline Sequence:*** as used herein, refers to a DNA sequence as found in an endogenous germline genome of a wild-type animal (e.g., mouse, rat, or human), or an RNA or amino acid sequence encoded by a DNA sequence as found in an endogenous germline genome of an animal (e.g., mouse, rat, or human). Representative germline sequences of immunoglobulin gene segments can be found, e.g., in LeFranc 2001:
Representative germline nucleotide sequences of human V_{H} gene segments and representative germline amino acid sequences of human V_{H} gene segments, which can be utilized in some embodiments as described herein, can be found pages 107-234 of LeFranc 2001;
Representative germline nucleotide sequences of human D gene segments and representative germline amino acid sequences of human D gene segments, which can be utilized in some embodiments as described herein, can be found pages 98-100 of LeFranc 2001;
Representative germline nucleotide sequences of human J_{H} gene segments and representative germline amino acid sequences of human J_{H} gene segments, which can be utilized in some embodiments as described herein, can be found page 104 of LeFranc 2001;
Representative germline nucleotide sequences of human Vλ gene segments and representative germline amino acid sequences of human Vλ gene segments, which can be utilized in some embodiments of a non-human animal as described herein, can be found pages 350-428 of LeFranc 2001; and
Representative germline nucleotide sequences of human Jλ gene segments and representative germline amino acid sequences of human Jλ gene segments, which can be utilized in some embodiments of a non-human animal as described herein, can be found pages 346 of LeFranc 2001.
Each of the cited sections of LeFranc 2001 are incorporated herein by reference.

***Heterologous:*** as used herein, refers to an agent or entity from a different source. For example, when used in reference to a polypeptide, gene, or gene product present in a particular cell or organism, the term clarifies that the relevant polypeptide, gene, or gene product: 1) was engineered by the hand of man; 2) was introduced into the cell or organism (or a precursor thereof) through the hand of man (e.g., via genetic engineering); and/or 3) is not naturally produced by or present in the relevant cell or organism (e.g., the relevant cell type or organism type). *"Heterologous"* also includes a polypeptide, gene or gene product that is normally present in a particular native cell or organism, but has been altered or modified, for example, by mutation or placement under the control of non-naturally associated and, in some embodiments, non-endogenous regulatory elements (e.g., a promoter).

***Host cell:*** as used herein, refers to a cell into which a nucleic acid or protein has been introduced. Persons of skill upon reading this disclosure will understand that such a term refers not only to the particular subject cell, but also is used to refer to the progeny of such a cell. Because certain modifications may occur in succeeding generations due to either mutation or environmental influences, such progeny may not, in fact, be identical to the parent cell, but are still included within the scope of the phrase *"host cell."* In some embodiments, a host cell is or comprises a prokaryotic or eukaryotic cell. In general, a host cell is any cell that is suitable for receiving and/or producing a heterologous nucleic acid or protein, regardless of the Kingdom of life to which the cell is designated. Exemplary cells include those of prokaryotes and eukaryotes (single-cell or multiple-cell), bacterial cells (e.g., strains of *Escherichia coli, Bacillus spp., Streptomyces spp.,* etc.), mycobacteria cells, fungal cells, yeast cells (e.g., *Saccharomyces cerevisiae, Schizosaccharomyces pombe, Pichia pastoris, Pichia methanolica,* etc.), plant cells, insect cells (e.g., SF-9, SF-21, baculovirus-infected insect cells, Trichoplusia ni, etc.), non-human animal cells, human cells, or cell fusions such as, for example, hybridomas or quadromas. In some embodiments, a cell is a human, monkey, ape, hamster, rat, or mouse cell. In some embodiments, a cell is eukaryotic and is selected from the following cells: Chinese Hamster Ovarian (CHO) (e.g., CHO K1, DXB-11 CHO, Veggie-CHO), COS (e.g., COS-7), retinal cell, Vero, CV1, kidney (e.g., HEK293, 293 EBNA, MSR 293, MDCK, HaK, BHK), HeLa, HepG2, WI38, MRC 5, Colo205, HB 8065, HL-60, (e.g., BHK21), Jurkat, Daudi, A431 (epidermal), CV-1, U937, 3T3, L cell, C127 cell, SP2/0, NS-0, MMT 060562, Sertoli cell, BRL 3A cell, HT1080 cell, myeloma cell, tumor cell, and a cell line derived from an aforementioned cell. In some embodiments, a cell comprises one or more viral genes, e.g., a retinal cell that expresses a viral gene (e.g., a PER.C6^{®} cell). In some embodiments, a host cell is or comprises an isolated cell. In some embodiments, a host cell is part of a tissue. In some embodiments, a host cell is part of an organism.

***Identity:*** as used herein in connection with a comparison of sequences, refers to identity as determined by a number of different algorithms known in the art that can be used to measure nucleotide and/or amino acid sequence identity. In some embodiments, identities as described herein are determined using a ClustalW v. 1.83 (slow) alignment employing an open gap penalty of 10.0, an extend gap penalty of 0.1, and using a Gonnet similarity matrix (MACVECTOR^{™} 10.0.2, MacVector Inc., 2008).

***In place of*** as used herein, refers to a positional substitution in which a first nucleic acid sequence is located at the position of a second nucleic acid sequence in a chromosome (e.g., where the second nucleic acid sequence was previously (e.g., originally) located in a chromosome, e.g., at the endogenous locus of the second nucleic acid sequence). The phrase "in place of" does not require that the second nucleic acid sequence be removed from, e.g., a locus or chromosome. In some embodiments, the second nucleic acid sequence and the first nucleic acid sequence are comparable to one another in that, for example, the first and second sequences are homologous to one another, contain corresponding elements (e.g., protein-coding elements, regulatory elements, etc.), and/or have similar or identical sequences. In some embodiments, a first and/or second nucleic acid sequence includes one or more of a promoter, an enhancer, a splice donor site, a splice acceptor site, an intron, an exon, an untranslated region (UTR); in some embodiments, a first and/or second nucleic acid sequence includes one or more coding sequences. In some embodiments, a first nucleic acid sequence is a homolog or variant (e.g., mutant) of the second nucleic acid sequence. In some embodiments, a first nucleic acid sequence is an ortholog or homolog of the second sequence. In some embodiments, a first nucleic acid sequence is or comprises a human nucleic acid sequence. In some embodiments, including where the first nucleic acid sequence is or comprises a human nucleic acid sequence, the second nucleic acid sequence is or comprises a rodent sequence (e.g., a mouse or rat sequence). In some embodiments, including where the first nucleic acid sequence is or comprises a human nucleic acid sequence, the second nucleic acid sequence is or comprises a human sequence. In some embodiments, a first nucleic acid sequence is a variant or mutant (i.e., a sequence that contains one or more sequence differences, e.g., substitutions, as compared to the second sequence) of the second sequence. The nucleic acid sequence so placed may include one or more regulatory sequences that are part of source nucleic acid sequence used to obtain the sequence so placed (e.g., promoters, enhancers, 5'- or 3'-untranslated regions, etc.). For example, in various embodiments, a first nucleic acid sequence is a substitution of an endogenous sequence with a heterologous sequence that results in the production of a gene product from the nucleic acid sequence so placed (comprising the heterologous sequence), but not expression of the endogenous sequence; a first nucleic acid sequence is of an endogenous genomic sequence with a nucleic acid sequence that encodes a polypeptide that has a similar function as a polypeptide encoded by the endogenous sequence (e.g., the endogenous genomic sequence encodes a non-human variable region polypeptide, in whole or in part, and the DNA fragment encodes one or more human variable region polypeptides, in whole or in part). In various embodiments, a human immunoglobulin gene segment or fragment thereof is in place of an endogenous non-human immunoglobulin gene segment or fragment.

***In vitro:*** as used herein refers to events that occur in an artificial environment, e.g., in a test tube or reaction vessel, in cell culture, etc., rather than within a multi-cellular organism.

***In vivo:*** as used herein refers to events that occur within a multi-cellular organism, such as a human and/or a non-human animal. In the context of cell-based systems, the term may be used to refer to events that occur within a living cell (as opposed to, for example, *in vitro* systems).

***Isolated:*** as used herein, refers to a substance and/or entity that has been (1) separated from at least some of the components with which it was associated when initially produced (whether in nature and/or in an experimental setting), and/or (2) designed, produced, prepared, and/or manufactured by the hand of man. Isolated substances and/or entities may be separated from about 10%, about 20%, about 30%, about 40%, about 50%, about 60%, about 70%, about 80%, about 90%, about 91%, about 92%, about 93%, about 94%, about 95%, about 96%, about 97%, about 98%, about 99%, or more than about 99% of the other components with which they were initially associated. In some embodiments, isolated agents are separated from 10% to 100%, 15%-100%, 20%-100%, 25%-100%, 30%-100%, 35%-100%, 40%-100%, 45%-100%, 50%-100%, 55%-100%, 60%-100%, 65%-100%, 70%-100%, 75%-100%, 80%-100%, 85%-100%, 90%-100%, 95%-100%, 96%-100%, 97%-100%, 98%-100%, or 99%-100% of the other components with which they were initially associated. In some embodiments, isolated agents are separated from 10% to 100%, 10%-99%, 10%-98%, 10%-97%, 10%-96%, 10%-95%, 10%-90%, 10%-85%, 10%-80%, 10%-75%, 10%-70%, 10%-65%, 10%-60%, 10%-55%, 10%-50%, 10%-45%, 10%-40%, 10%-35%, 10%-30%, 10%-25%, 10%-20%, or 10%-15% of the other components with which they were initially associated. In some embodiments, isolated agents are separated from 11% to 99%, 12%-98%, 13%-97%, 14%-96%, 15%-95%, 20%-90%, 25%-85%, 30%-80%, 35%-75%, 40%-70%, 45%-65%, 50%-60%, or 55%-60% of the other components with which they were initially associated. In some embodiments, isolated agents are about 80%, about 85%, about 90%, about 91%, about 92%, about 93%, about 94%, about 95%, about 96%, about 97%, about 98%, about 99%, or more than about 99% pure. In some embodiments, isolated agents are 80%-99%, 85%-99%, 90%-99%, 95%-99%, 96%-99%, 97%-99%, or 98%-99% pure. In some embodiments, isolated agents are 80%-99%, 80%-98%, 80%-97%, 80%-96%, 80%-95%, 80%-90%, or 80%-85% pure. In some embodiments, isolated agents are 85%-98%, 90%-97%, or 95%-96% pure. In some embodiments, a substance is *"pure"* if it is substantially free of other components. In some embodiments, as will be understood by those skilled in the art, a substance may still be considered *"isolated"* or even *"pure",* after having been combined with certain other components such as, for example, one or more carriers or excipients (e.g., buffer, solvent, water, etc.); in such embodiments, percent isolation or purity of the substance is calculated without including such carriers or excipients. To give but one example, in some embodiments, a biological polymer such as a polypeptide or polynucleotide that occurs in nature is considered to be *"isolated"* when: a) by virtue of its origin or source of derivation is not associated with some or all of the components that accompany it in its native state in nature; b) it is substantially free of other polypeptides or nucleic acids of the same species from the species that produces it in nature; or c) is expressed by or is otherwise in association with components from a cell or other expression system that is not of the species that produces it in nature. Thus, for instance, in some embodiments, a polypeptide that is chemically synthesized, or is synthesized in a cellular system different from that which produces it in nature, is considered to be an *"isolated"* polypeptide. Alternatively, or additionally, in some embodiments, a polypeptide that has been subjected to one or more purification techniques may be considered to be an *"isolated"* polypeptide to the extent that it has been separated from other components: a) with which it is associated in nature; and/or b) with which it was associated when initially produced.

***Locus*** or ***loci:*** as used herein, refers to a location(s) of a gene, DNA sequence, polypeptide-encoding sequence, or position on a chromosome of the genome of an organism. For example, an *"immunoglobulin locus"* may refer to the location of an immunoglobulin gene segment (e.g., V, D, J or C), immunoglobulin gene segment DNA sequence, immunoglobulin gene segment-encoding sequence, or immunoglobulin gene segment position on a chromosome of the genome of an organism that has been identified as to where such a sequence resides. An *"immunoglobulin locus"* may comprise a regulatory element of an immunoglobulin gene segment, including, but not limited to, an enhancer, a promoter, 5' and/or 3' regulatory sequence or region, or a combination thereof. An *"immunoglobulin locus"* may comprise intergenic DNA, e.g., DNA that normally resides or appears between gene segments in a wild-type locus. Persons of ordinary skill in the art will appreciate that chromosomes may, in some embodiments, contain hundreds or even thousands of genes and demonstrate physical co-localization of similar genetic loci when comparing between different species. Such genetic loci can be described as having shared synteny.

***Naturally appears:*** as used herein in reference to a biological element (e.g., a nucleic acid sequence) means that the biological element can be found in a specified context and/or location, absent engineering (e.g., genetic engineering), in a cell or organism (e.g., an animal). In other words, a sequence that naturally appears in a specified context and/or location is not in the specified context and/or location as the result of engineering (e.g., genetic engineering). For example, a sequence that naturally appears adjacent to a human Jκ1 gene segment in an endogenous human immunoglobulin kappa light chain locus is a sequence that can be found adjacent to a human Jκ1 gene segment in an endogenous human immunoglobulin kappa light chain locus, absent genetic engineering, in a human. In some embodiments, a sequence can be obtained, derived, and/or isolated from where it naturally appears in a cell or organism. In some embodiments, a cell or organism is not a direct source of a sequence that naturally appears in the cell or organism. For example, a corresponding sequence in a cell or organism could be identified and then produced or replicated by mechanisms known in the art.

***Non-human animal:*** as used herein, refers to any vertebrate organism that is not a human. In some embodiments, a non-human animal is a cyclostome, a bony fish, a cartilaginous fish (e.g., a shark or a ray), an amphibian, a reptile, a mammal, and a bird. In some embodiments, a non-human animal is a mammal. In some embodiments, a non-human mammal is a primate, a goat, a sheep, a pig, a dog, a cow, or a rodent. In some embodiments, a non-human animal is a rodent such as a rat or a mouse.

***Nucleic acid:*** as used herein, refers to any compound and/or substance that is or can be incorporated into an oligonucleotide chain. In some embodiments, a *"nucleic acid"* is a compound and/or substance that is or can be incorporated into an oligonucleotide chain via a phosphodiester linkage. As will be clear from context, in some embodiments, *"nucleic acid"* refers to individual nucleic acid residues (e.g., nucleotides and/or nucleosides); in some embodiments, *"nucleic acid"* refers to an oligonucleotide chain comprising individual nucleic acid residues. In some embodiments, a "nucleic acid" is or comprises RNA; in some embodiments, a *"nucleic acid"* is or comprises DNA. In some embodiments, a *"nucleic acid"* is, comprises, or consists of one or more natural nucleic acid residues. In some embodiments, a *"nucleic acid'* is, comprises, or consists of one or more nucleic acid analogs. In some embodiments, a nucleic acid analog differs from a *"nucleic acid"* in that it does not utilize a phosphodiester backbone. For example, in some embodiments, a *"nucleic acid"* is, comprises, or consists of one or more *"peptide nucleic acids",* which are known in the art and have peptide bonds instead of phosphodiester bonds in the backbone. Alternatively, or additionally, in some embodiments, a *"nucleic acid"* has one or more phosphorothioate and/or 5'-N-phosphoramidite linkages rather than phosphodiester bonds. In some embodiments, a *"nucleic acid"* is, comprises, or consists of one or more natural nucleosides (e.g., adenosine, thymidine, guanosine, cytidine, uridine, deoxyadenosine, deoxythymidine, deoxyguanosine, and deoxycytidine). In some embodiments, a *"nucleic acid"* is, comprises, or consists of one or more nucleoside analogs (e.g., 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, 2-thiocytidine, methylated bases, intercalated bases, and combinations thereof). In some embodiments, a *"nucleic acid"* comprises one or more modified sugars (e.g., 2'-fluororibose, ribose, 2'-deoxyribose, arabinose, and hexose) as compared with those in natural nucleic acids. In some embodiments, a *"nucleic acid"* has a nucleotide sequence that encodes a functional gene product such as an RNA or polypeptide. In some embodiments, a *"nucleic acid'* includes one or more introns. In some embodiments, a *"nucleic acid"* includes one or more exons. In some embodiments, a *"nucleic acid'* is prepared by one or more of isolation from a natural source, enzymatic synthesis by polymerization based on a complementary template (*in vivo* or *in vitro*), reproduction in a recombinant cell or system, and chemical synthesis. In some embodiments, a *"nucleic acid"* is at least, e.g., but not limited to, 3, 4, 5, 6, 7, 8, 9, 10, 15, 20, 25, 30, 35, 40, 45, 50, 55, 60, 65, 70, 75, 80, 85, 90, 95, 100, 110, 120, 130, 140, 150, 160, 170, 180, 190, 20, 225, 250, 275, 300, 325, 350, 375, 400, 425, 450, 475, 500, 600, 700, 800, 900, 1000, 1500, 2000, 2500, 3000, 3500, 4000, 4500, 5000 or more residues long. In some embodiments, a *"nucleic acid"* is single stranded; in some embodiments, a *"nucleic acid"* is double stranded. In some embodiments, a *"nucleic acid"* has a nucleotide sequence comprising at least one element that encodes, or is the complement of a sequence that encodes, a polypeptide. In some embodiments, a *"nucleic acid"* has enzymatic activity.

***Operably linked:*** as used herein, refers to a juxtaposition of components, where the components described are in a relationship permitting them to function in their intended manner (e.g., when the components are present in the proper tissue, cell type, cellular activity, etc.). For example, one or more V_{H} gene segments, one or more D gene segments, and one or more J_{H} gene segments are "operably linked" to a heavy chain constant region if the V_{H}, D, and J_{H} gene segments can be spliced to the heavy chain constant region at the proper time in B cell development, regardless of whether such splicing occurs in, e.g., a cell outside the immune system (e.g., a germ cell). A control sequence *"operably linked"* to a coding sequence is ligated in such a way that expression of the coding sequence is achieved under conditions compatible with the control sequences. *"Operably linked"* sequences include both expression control sequences that are contiguous with a gene of interest and expression control sequences that act in trans or at a distance to control a gene of interest (or sequence of interest). The term *"expression control sequence"* includes polynucleotide sequences, which are necessary to affect the expression and processing of coding sequences to which they are ligated. *"Expression control sequences"* include: appropriate transcription initiation, termination, promoter and enhancer sequences; efficient RNA processing signals such as splicing and polyadenylation signals; sequences that stabilize cytoplasmic mRNA; sequences that enhance translation efficiency (i.e., Kozak consensus sequence); sequences that enhance polypeptide stability; and when desired, sequences that enhance polypeptide secretion. The nature of such control sequences differs depending upon the host organism. For example, in prokaryotes, such control sequences generally include promoter, ribosomal binding site and transcription termination sequence, while in eukaryotes typically such control sequences include promoters and transcription termination sequence. The term *"control sequences"* is intended to include components whose presence is essential for expression and processing, and can also include additional components whose presence is advantageous, for example, leader sequences and fusion partner sequences.

***Polypeptide:*** as used herein, refers to any polymeric chain of amino acids. In some embodiments, a polypeptide has an amino acid sequence that occurs in nature. In some embodiments, a polypeptide has an amino acid sequence that does not occur in nature. In some embodiments, a polypeptide has an amino acid sequence that contains portions that occur in nature separately from one another (i.e., from two or more different organisms, for example, human and non-human portions). In some embodiments, a polypeptide has an amino acid sequence that is engineered in that it is designed and/or produced through action of the hand of man. In some embodiments, a polypeptide has an amino acid sequence encoded by a sequence that does not occur in nature (e.g., a sequence that is engineered in that it is designed and/or produced through action of the hand of man to encode said polypeptide).

***Rearranged:*** as used herein, describes a DNA sequence that includes two or more immunoglobulin gene segments joined (directly or indirectly) together, such that the joined gene segments together have a DNA sequence that encodes a variable region of an immunoglobulin. The two or more immunoglobulin gene segments of a rearranged DNA sequence are no longer associated with functioning recombination signal sequences (RSS), and as such cannot undergo further rearrangement. Those of skill in the art will recognize that, while two or more immunoglobulin gene segments of a rearranged DNA sequence may not be able to rearrange further, it does not mean that other immunoglobulin gene segments within the same locus cannot undergo, e.g., secondary rearrangement. Those of skill in the art will appreciate that rearranged gene segments (e.g., in a rearranged immunoglobulin variable region) can be joined together via a natural VDJ recombination process. Those of skill in the art will also appreciate that rearranged gene segments (e.g., in a rearranged immunoglobulin variable region) can be engineered to be joined together, e.g., by joining the gene segments using standard recombinant techniques. Rearranged immunoglobulin variable regions typically include two or more joined immunoglobulin gene segments. For example, a rearranged immunoglobulin λ light chain variable region can include a Vλ gene segment joined with a Jλ gene segment. A rearranged immunoglobulin heavy chain variable region can include a V_{H} gene segment, a D gene segment, a J_{H} gene segment that are joined. Those of skill in the art will also appreciate that all or substantially all intergenic sequence is generally removed between immunoglobulin gene segments in a rearranged immunoglobulin variable regions. Those of skill in the art will further appreciate that a rearranged sequence can include, among other things, introns in the gene segments.

***Recombinant:*** as used herein, refers to molecules (e.g., DNA, RNA, or polypeptides) formed by laboratory methods of genetic recombination (e.g., cloning) to bring together genetic material from multiple sources (e.g., organisms, tissues, cells, genomes, or portions of a genome). In some embodiments, recombinant polypeptides are designed, engineered, prepared, expressed, created or isolated by recombinant means, such as polypeptides expressed using a recombinant expression vector transfected into a host cell, polypeptides isolated from a recombinant, combinatorial human polypeptide library (Hoogenboom, H. R., 1997, TIB Tech. 15:62-70; Azzazy, H. and W.E. Highsmith, 2002, Clin. Biochem. 35:425-45; Gavilondo, J. V. and J.W. Larrick, 2002, BioTechniques 29:128-45; Hoogenboom H., and P. Chames, 2000, Immunol. Today 21:371-8, incorporated herein by reference in their entireties), antibodies isolated from an animal (e.g., a mouse) that has been genetically engineered to include human immunoglobulin genes (see e.g., Taylor, L. D. et al., 1992, Nucl. Acids Res. 20:6287-95; Kellermann, S-A. and L.L. Green, 2002, Curr. Opin. Biotechnol. 13:593-7; Little, M. et al., 2000, Immunol. Today 21:364-70; Osborn, M.J. et al., 2013, J. Immunol. 190:1481-90; Lee, E-C. et al., 2014, Nat. Biotech. 32(4):356-63; Macdonald, L.E. et al., 2014, Proc. Natl. Acad. Sci. U.S.A. 111(14):5147-52; Murphy, A.J. et al., 2014, Proc. Natl. Acad. Sci. U.S.A. 111(14):5153-8, each of which is incorporated herein by reference in its entirety) or polypeptides prepared, expressed, created or isolated by any other means that involves splicing selected sequence elements to one another. In some embodiments, one or more of such selected sequence elements is found in nature. In some embodiments, one or more of such selected sequence elements is designed *in silico.* In some embodiments, one or more such selected sequence elements result from mutagenesis (e.g., *in vivo* or *in vitro*) of a known sequence element, e.g., from a natural or synthetic (e.g., man-made) source. For example, in some embodiments, a recombinant polypeptide is comprised of sequences found in the genome of a source organism of interest (e.g., human, mouse, etc.). In some embodiments, a recombinant polypeptide has an amino acid sequence that resulted from mutagenesis (e.g., *in vitro* or *in vivo,* for example, in a non-human animal), so that the amino acid sequences of the recombinant polypeptides are sequences that, while originating from and related to polypeptides sequences, may not naturally exist within the genome of a non-human animal *in vivo.*

***Reference:*** as used herein, refers to a standard or control agent, animal, cohort, individual, population, sample, sequence or value against which an agent, animal, cohort, individual, population, sample, sequence or value of interest is compared. In some embodiments, a reference agent, animal, cohort, individual, population, sample, sequence or value is tested and/or determined substantially simultaneously with the testing or determination of an agent, animal, cohort, individual, population, sample, sequence or value of interest. In some embodiments, a reference agent, animal, cohort, individual, population, sample, sequence or value is a historical reference, optionally embodied in a tangible medium. In some embodiments, a reference may refer to a control. A *"reference"* also includes a *"reference animal." A "reference animal"* may have a modification as described herein, a modification that is different as described herein or no modification (i.e., a wild-type animal). Typically, as would be understood by persons of skill in the art, a reference agent, animal, cohort, individual, population, sample, sequence or value is determined or characterized under conditions comparable to those utilized to determine or characterize an agent, animal (e.g., a mammal), cohort, individual, population, sample, sequence or value of interest.

***Replacement:*** as used herein, refers to a process through which a *"replaced"* nucleic acid sequence (e.g., a gene) found in a host locus (e.g., in a genome) is removed from that locus, and a different, *"replacement"* nucleic acid is located in its place. In some embodiments, the replaced nucleic acid sequence and the replacement nucleic acid sequences are comparable to one another in that, for example, they are homologous to one another, contain corresponding elements (e.g., protein-coding elements, regulatory elements, etc.), and/or have similar or identical sequences. In some embodiments, a replaced nucleic acid sequence includes one or more of a promoter, an enhancer, a splice donor site, a splice acceptor site, an intron, an exon, an untranslated region (UTR); in some embodiments, a replacement nucleic acid sequence includes one or more coding sequences. In some embodiments, a replacement nucleic acid sequence is a homolog or variant (e.g., mutant) of the replaced nucleic acid sequence. In some embodiments, a replacement nucleic acid sequence is an ortholog or homolog of the replaced sequence. In some embodiments, a replacement nucleic acid sequence is or comprises a human nucleic acid sequence. In some embodiments, including where the replacement nucleic acid sequence is or comprises a human nucleic acid sequence, the replaced nucleic acid sequence is or comprises a rodent sequence (e.g., a mouse or rat sequence). In some embodiments, including where the replacement nucleic acid sequence is or comprises a human nucleic acid sequence, the replaced nucleic acid sequence is or comprises a human sequence. In some embodiments, a replacement nucleic acid sequence is a variant or mutant (i.e., a sequence that contains one or more sequence differences, e.g., substitutions, as compared to the replaced sequence) of the replaced sequence. The nucleic acid sequence so placed may include one or more regulatory sequences that are part of source nucleic acid sequence used to obtain the sequence so placed (e.g., promoters, enhancers, 5'- or 3'-untranslated regions, etc.). For example, in various embodiments, a replacement is a substitution of an endogenous sequence with a heterologous sequence that results in the production of a gene product from the nucleic acid sequence so placed (comprising the heterologous sequence), but not expression of the endogenous sequence; a replacement is of an endogenous genomic sequence with a nucleic acid sequence that encodes a polypeptide that has a similar function as a polypeptide encoded by the endogenous sequence (e.g., the endogenous genomic sequence encodes a non-human variable region polypeptide, in whole or in part, and the DNA fragment encodes one or more human variable region polypeptides, in whole or in part). In various embodiments, an endogenous non-human immunoglobulin gene segment or fragment thereof is replaced with a human immunoglobulin gene segment or fragment thereof.

***Substantially:*** as used herein, refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term *"substantially"* is therefore used herein to capture the potential lack of completeness inherent in many biological and chemical phenomena.

***Substantial similarity:*** as used herein, refers to a comparison between amino acid or nucleic acid sequences. As will be appreciated by those of ordinary skill in the art, two sequences are generally considered to be *"substantially similar"* if they contain similar residues (e.g., amino acids or nucleotides) in corresponding positions. As is understood in the art, while similar residues may be identical residues (see also ***Substantial Identity,*** below), similar residues may also be non-identical residues with appropriately comparable structural and/or functional characteristics. For example, as is well known by those of ordinary skill in the art, certain amino acids are typically classified as *"hydrophobic"* or *"hydrophilic"* amino acids, and/or as having *"polar"* or *"non-polar"* side chains. Substitution of one amino acid for another of the same type may often be considered a *"conservative"* substitution. Typical amino acid categorizations are summarized in the table below.

| | | | | | |
|---|---|---|---|---|---|
| Alanine | Ala | A | Nonpolar | Neutral | 1.8 |
| Arginine | Arg | R | Polar | Positive | -4.5 |
| Asparagine | Asn | N | Polar | Neutral | -3.5 |
| Aspartic acid | Asp | D | Polar | Negative | -3.5 |
| Cysteine | Cys | C | Nonpolar | Neutral | 2.5 |
| Glutamic acid | Glu | E | Polar | Negative | -3.5 |
| Glutamine | Gln | Q | Polar | Neutral | -3.5 |
| Glycine | Gly | G | Nonpolar | Neutral | -0.4 |
| Histidine | His | H | Polar | Positive | -3.2 |
| Isoleucine | Ile | I | Nonpolar | Neutral | 4.5 |
| Leucine | Leu | L | Nonpolar | Neutral | 3.8 |
| Lysine | Lys | K | Polar | Positive | -3.9 |
| Methionine | Met | M | Nonpolar | Neutral | 1.9 |
| Phenylalanine | Phe | F | Nonpolar | Neutral | 2.8 |
| Proline | Pro | P | Nonpolar | Neutral | -1.6 |
| Serine | Ser | S | Polar | Neutral | -0.8 |
| Threonine | Thr | T | Polar | Neutral | -0.7 |
| Tryptophan | Trp | W | Nonpolar | Neutral | -0.9 |
| Tyrosine | Tyr | Y | Polar | Neutral | -1.3 |
| Valine | Val | V | Nonpolar | Neutral | 4.2 |

| Ambiguous Amino Acids | 3-Letter | 1-Letter |
|---|---|---|
| Asparagine or aspartic acid | Asx | B |
| Glutamine or glutamic acid | Glx | Z |
| Leucine or Isoleucine | Xle | J |
| Unspecified or unknown amino acid | Xaa | X |

As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, S. F. et al., 1990, J. Mol. Biol., 215(3): 403-10; Altschul, S.F. et al., 1996, Meth. Enzymol. 266:460-80; Altschul, S.F. et al., 1997, Nucleic Acids Res., 25:3389-402; Baxevanis, A.D. and B.F.F. Ouellette (eds.) Bioinformatics: A Practical Guide to the Analysis of Genes and Proteins, Wiley, 1998; and Misener et al. (eds.) Bioinformatics Methods and Protocols, Methods in Molecular Biology, Vol. 132, Humana Press, 1998, incorporated herein by reference in their entireties. In addition to identifying similar sequences, the programs mentioned above typically provide an indication of the degree of similarity. In some embodiments, two sequences are considered to be substantially similar if at least, e.g., but not limited to, 80%, 85%, 90%, 91%, 92%, 93%, 94%, 95%, 96%, 97%, 98%, 99% or more of their corresponding residues are similar (e.g., identical or include a conservative substitution) over a relevant stretch of residues. In some embodiments, the relevant stretch is a complete sequence (e.g. a sequence of a gene, a gene segment, a sequence encoding a domain, a polypeptide, or a domain). In some embodiments, the relevant stretch is at least 9, 10, 11, 12, 13, 14, 15, 16, 17 or more residues. In some embodiments, the relevant stretch is at least 10, 15, 20, 25, 30, 35, 40, 45, 50, or more residues. In some embodiments, the relevant stretch includes contiguous residues along a complete sequence. In some embodiments, the relevant stretch includes discontinuous residues along a complete sequence, for example, noncontiguous residues brought together by the folded conformation of a polypeptide or a portion thereof.

***Substantial identity:*** as used herein, refers to a comparison between amino acid or nucleic acid sequences. As will be appreciated by those of ordinary skill in the art, two sequences are generally considered to be *"substantially identical"* if they contain identical residues (e.g., amino acids or nucleotides) in corresponding positions. As is well-known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, S. F. et al., 1990, J. Mol. Biol., 215(3): 403-10; Altschul, S.F. et al., 1996, Meth. Enzymol. 266:460-80; Altschul, S.F. et al., 1997, Nucleic Acids Res., 25:3389-402; Baxevanis, A.D. and B.F.F. Ouellette (eds.) Bioinformatics: A Practical Guide to the Analysis of Genes and Proteins, Wiley, 1998; and Misener et al. (eds.) Bioinformatics Methods and Protocols, Methods in Molecular Biology, Vol. 132, Humana Press, 1998, each of which is incorporated herein by reference in its entirety. In addition to identifying identical sequences, the programs mentioned above typically provide an indication of the degree of identity. In some embodiments, two sequences are considered to be substantially identical if at least 80%, 85%, 90%, 91%, 92%, 93%, 94%, 95%, 96%, 97%, 98%, 99% or more of their corresponding residues are identical over a relevant stretch of residues. In some embodiments, a relevant stretch of residues is a complete sequence. In some embodiments, a relevant stretch of residues is, e.g., but not limited to, at least 10, 15, 20, 25, 30, 35, 40, 45, 50, or more residues.

***Targeting construct*** or ***targeting vector:*** as used herein, refers to a polynucleotide molecule that comprises a targeting region. A targeting region comprises a sequence that is identical or substantially identical to a sequence in a target cell, tissue or animal and provides for integration of the targeting construct into a position within the genome of the cell, tissue or animal via homologous recombination. Targeting regions that target using site-specific recombinase recognition sites (e.g., *lox*P or Frt sites) are also included and described herein. In some embodiments, a targeting construct as described herein further comprises a nucleic acid sequence or gene of particular interest, a selectable marker, control and/or regulatory sequences, and other nucleic acid sequences that allow for recombination mediated through exogenous addition of proteins that aid in or facilitate recombination involving such sequences. In some embodiments, a targeting construct as described herein further comprises a gene of interest in whole or in part, wherein the gene of interest is a heterologous gene that encodes a polypeptide, in whole or in part, that has a similar function as a protein encoded by an endogenous sequence. In some embodiments, a targeting construct as described herein further comprises a humanized gene of interest, in whole or in part, wherein the humanized gene of interest encodes a polypeptide, in whole or in part, that has a similar function as a polypeptide encoded by an endogenous sequence. In some embodiments, a targeting construct (or targeting vector) may comprise a nucleic acid sequence manipulated by the hand of man. For example, in some embodiments, a targeting construct (or targeting vector) may be constructed to contain an engineered or recombinant polynucleotide that contains two or more sequences that are not linked together in that order in nature yet manipulated by the hand of man to be directly linked to one another in the engineered or recombinant polynucleotide.

***Transgene*** or ***transgene construct:*** as used herein, refers to a nucleic acid sequence (encoding e.g., a polypeptide of interest, in whole or in part) that has been introduced into a cell by the hand of man such as by the methods described herein. A transgene could be partly or entirely heterologous, i.e., foreign, to the genetically engineered animal or cell into which it is introduced. A transgene can include one or more transcriptional regulatory sequences and any other nucleic acid, such as introns or promoters, which may be necessary for expression of a selected nucleic acid sequence.

***Unrearranged:*** as used herein, describes a DNA sequence that includes two or more immunoglobulin gene segments that have not undergone a recombination event or otherwise been joined, and therefore, include intergenic sequence(s) between them. Those of skill in the art will appreciate that unrearranged V gene segments and J gene segments can be associated with an intact recombination signal sequence (RSS). Unrearranged D gene segments can be flanked by two intact recombination signal sequences (RSSs). Those of skill in the art will further appreciate that unrearranged gene segments can include, among other things, introns.

***Vector:*** as used herein, refers to a nucleic acid molecule capable of transporting another nucleic acid to which it is associated. In some embodiment, vectors are capable of extra-chromosomal replication and/or expression of nucleic acids to which they are linked in a host cell such as a eukaryotic and/or prokaryotic cell. Vectors capable of directing the expression of operably linked genes are referred to herein as *"expression vectors."*

***Wild-type:*** as used herein, refers to an entity having a structure and/or activity as found in nature in a *"normal"* (as contrasted with mutant, diseased, altered, engineered, etc.) state or context. Those of ordinary skill in the art will appreciate that wild-type genes and polypeptides often exist in multiple different forms (e.g., alleles).

### DETAILED DESCRIPTION

The present disclosure provides the insight that endogenous antibody development mechanisms in non-human animals, including immunoglobulin chain pairing and affinity maturation, can be exploited to generate antigen-specific, high-affinity antibodies including exogenous human immunoglobulin sequences. Such animals can generate a normal and robust immune response, which can be utilized to make, e.g., human antibody therapeutics. The present disclosure recognizes that genetically modified non-human animals provide an effective and efficient platform for generating antibodies including human variable domains, including human heavy chain, κ light chain, and λ light chain domains. The present disclosure further recognizes that genetically modified non-human animals are able to successfully utilize human heavy chain, κ light chain, and λ light chain variable region gene segments to generate affinity-matured human heavy chain, κ light chain, and λ light chain variable domains.

The present disclosure recognizes that the production of antibodies including human λ light chain variable domains in non-human animals has previously presented challenges, as expression of λ light chains in certain non-human animals is low. For example, mice utilize κ light chains significantly more than λ light chains (i.e., at a κ:λ ratio of ~95:5). The present disclosure further recognizes that the production of antibodies including a universal light chain (e.g., a light chain capable of binding to a plurality of heavy chains) by limiting the light chain variable region repertoire of a non-human animal can be challenging as it eliminates some of the most powerful diversity-generating mechanisms for the generation of high-affinity antibodies, e.g., combinatorial diversity, junctional diversity, and secondary rearrangement. It also significantly minimizes diversity-generating effects that would otherwise result from "mix and match" heavy and light chain pairing. In view of these recognitions, a need remains in the art for platforms and methods for producing human λ light chain variable regions in non-human animals from a limited human λ light chain variable regions.

The present disclosure provides the insight that non-human animals (e.g., rodents, e.g., rats or mice) including a limited human λ light chain variable region repertoire at a κ light chain locus are able to effectively generate high-affinity, antigen-specific antibodies. This result is unexpected, as a limited human λ light chain variable region repertoire in a non-human animal forces the non-human animals to utilize λ light chain variable region sequences. The use of λ light chain variable region sequences is contrary to the natural preference of certain non-human animals. As mentioned above, use of a limited human λ light chain variable region repertoire in a non-human animal also eliminates many natural mechanisms used to generate high-affinity, antigen-specific antibodies.

The present disclosure provides genetically modified non-human animals (e.g., rodents, e.g., rats or mice) that express human immunoglobulin λ light chain variable domains, where the non-human animal has a limited human λ light chain variable region repertoire (comprising one or two human Vλ gene segments). In some embodiments, the present disclosure provides a genetically modified non-human animal that expresses human immunoglobulin λ light chain variable domains, where the non-human animal has a limited human λ light chain variable repertoire, and human immunoglobulin heavy chain variable domains. A biological system for generating human λ light chain variable domain, expressed from a limited human λ light chain variable region repertoire, that associates with a diverse repertoire of affinity-matured human heavy chain variable domains is also provided. Methods for making an antigen binding protein comprising a human immunoglobulin variable domain (e.g., an antibody, a heavy chain, a light chain (e.g., a λ light chain), a heavy chain variable domain, a light chain variable domain (e.g., a λ light chain variable domain), a single chain variable fragment (ScFv)) are provided. In some embodiments, a method comprises immunizing a non-human animal described herein with an antigen of interest. In some embodiments, a method comprises employing an immunoglobulin variable region gene sequence of a non-human animal (e.g., a rodent, e.g. a rat or a mouse) described herein in an antigen binding protein (e.g., a binding protein that specifically binds an antigen of interest). Methods include methods for making human immunoglobulin heavy and/or λ light chain variable domains suitable for use in making multi-specific antigen-binding proteins.

Genetically engineered non-human animals (e.g., rodents, e.g., rats or mice) are provided that express a limited repertoire of human λ light chain variable domains from a limited repertoire of human λ light chain variable region gene segments. In some embodiments, a non-human animal described herein is genetically engineered to include a single rearranged human λ light chain variable region sequence (a Vλ/Jλ sequence). In some embodiments, a non-human animal described herein is genetically engineered to include only one or two human unrerranged λ light chain variable region gene segments. In some embodiments, a non-human animal described herein is genetically engineered to include only one or two unrearranged human Vλ gene segments and one or more unrearranged human Jλ gene segments. In certain embodiments, a non-human animal described herein includes four or five unrearranged human Jλ gene segments. Rearranged human λ light chain variable domains expressed by a non-human animal described herein are capable of pairing with a plurality of affinity-matured human heavy chains expressed by such a non-human animal, where the plurality of heavy chain variable regions are capable of specifically binding different epitopes. In some embodiments, a non-human animal as described herein expresses and selects suitable affinity-matured human immunoglobulin heavy chain variable domains derived from a repertoire of unrearranged human heavy chain variable region gene segments, where the affinity-matured human heavy chain variable domains associate and express with a human λ light chain variable domain derived from the limited human immunoglobulin λ light chain variable region gene repertoire of the non-human animal. The present disclosure provides the insight that human λ light chain variable domains and human heavy chain variable domains (along with the encoding human λ light chain variable regions and human heavy chain variable regions) can be utilized in the production of multi-specific antibodies, particularly bispecific antibodies.

### Antibody repertoires in non-human animals

Immunoglobulins (also called antibodies) are large (~150 kD), Y-shaped glycoproteins that are produced by B cells of a host immune system to neutralize pathogens (e.g., viruses, bacteria, etc.). Each immunoglobulin (Ig) is composed of two identical heavy chains and two identical light chains, each of which has two structural components: a variable domain and a constant domain. The heavy and light chain variable regions differ in antibodies produced by different B cells, but are the same for all antibodies produced by a single B cell or B cell clone. The heavy and light chain variable regions of each antibody together comprise the antigen-binding region (or antigen-binding site). Immunoglobulins can exist in different varieties that are referred to as isotypes or classes based on the heavy chain constant regions (or domains) that they contain. The heavy chain constant region is identical in all antibodies of the same isotype, but differs in antibodies of different isotypes. The table below summarizes the nine antibody isotypes in mouse and human.

| Mouse | Human |
|---|---|
| IgM | IgM |
| IgD | IgD |
| IgG1 | IgG1 |
| IgG2a | IgG2 |
| IgG2b | IgG3 |
| IgG2c | IgG4 |
| IgG3 | IgE |
| IgE | IgA1 |
| IgA | IgA2 |

Additional isotypes have been identified in other species. Isotypes confer specialized biological properties on the antibody due to the different structural characteristics among the different isotypes and are found in different locations (cells, tissues, etc.) within an animal body. Initially, B cells produce IgM and IgD with identical antigen-binding regions. Upon activation, B cells switch to different isotypes by a process referred to as class switching, which involves a change of the constant region of the antibody produced by the B cell while the variable regions remain the same, thereby preserving antigen specificity of the original antibody (B cell).

Two separate loci (Igκ and Igλ) contain the gene segments that, upon rearrangement, encode the light chains of antibodies, and exhibit both allelic and isotypic exclusion. The expression ratios of κ⁺ to λ⁺ B cells vary among species. For example, humans demonstrate a ratio of about 60:40 (κ:λ). In mice and rats, a ratio of 95:5 (κ:λ) is observed. Interestingly, the κ:λ ratio observed in cats (5:95) is opposite of mice and rats. Several studies have been conducted to elucidate the possible reasons behind these observed ratios, and both the complexity of the locus (i.e., number of gene segments, in particular, V gene segments) and the efficiency of gene segment rearrangement have been proposed as rationale. The human immunoglobulin λ light chain locus extends over 1,000 kb and contains approximately 70 Vλ gene segments (29 to 33 functional) and seven Jλ-Cλ gene segment pairs (four to five functional) organized into three clusters (see, e.g., Figure 1 of U.S. Patent No. 9,006,511, which is incorporated herein by reference in its entirety). The majority of the observed Vλ regions in the expressed antibody repertoire are encoded by gene segments contained within the most proximal cluster (referred to as cluster A). The mouse immunoglobulin λ light chain locus is strikingly different than the human locus and, depending on the strain, contains only a few Vλ and Jλ gene segments organized in two distinct gene clusters (see, e.g., Figure 2 of U.S. Patent No. 9,006,511, which is incorporated herein by reference in its entirety).

Development of therapeutic antibodies for the treatment of various human diseases has largely been centered on the creation of engineered non-human animal lines, in particular, engineered rodent lines, harboring varying amounts of genetic material in their genomes corresponding to human immunoglobulin genes (reviewed in, e.g., Brüggemann, M. et al., 2015, Arch. Immunol. Ther. Exp. 63:101-8, which is incorporated herein by reference in its entirety). Initial efforts in creating such genetically engineered rodent lines focused on integration of portions of human immunoglobulin loci that could, by themselves, support recombination of gene segments and production of heavy and/or light chains that were entirely human while having endogenous immunoglobulin loci inactivated (see e.g., Brüggemann, M. et al., 1989, Proc. Nat. Acad. Sci. U.S.A. 86:67-09-13; Brüggemann, M. et al., 1991, Eur. J. Immunol. 21:1323-6; Taylor, L.D. et al., 1992, Nucl. Acids Res. 20:6287-6295; Davies, N.P. et al., 1993, Biotechnol. 11:911-4; Green, L.L. et al., 1994, Nat. Genet. 7:13-21; Lonberg, N. et al., 1994, Nature 368:856-9; Taylor, L.D. et al., 1994, Int. Immunol. 6:579-91; Wagner, S.D. et al., 1994, Eur. J. Immunol. 24:2672-81; Fishwild, D.M. et al., 1996, Nat. Biotechnol. 14:845-51; Wagner, S.D. et al., 1996, Genomics 35:405-14; Mendez, M.J. et al., 1997, Nat. Genet. 15:146-56; Green, L.L. et al., 1998, J. Exp. Med. 188:483-95; Xian, J. et al., 1998, Transgenics 2:333-43; Little, M. et al., 2000, Immunol. Today 21:364-70; Kellermann, S.A. and L.L. Green, 2002, Cur. Opin. Biotechnol. 13:593-7, each of which is incorporated by reference in their entirety). In particular, some efforts have included integration of human immunoglobulin λ light chain sequences (see, e.g., U.S. Patent Application Publication Nos. 2002/0088016 A1, 2003/0217373 A1 and 2011/0236378 A1; U.S. Patent Nos. 6,998,514 and 7,435,871; Nicholson, I.C. et al., 1999, J. Immunol. 163:6898-906; Popov, A.V et al., 1999, J. Exp. Med. 189(10):1611-19, each of which is incorporated herein by reference in its entirety). Such efforts have focused on the random integration of yeast artificial chromosomes containing human Vλ, Jλ and Cλ sequences thereby creating mouse strains that express fully human immunoglobulin λ light chains (i.e., human Vλ and Cλ domains). More recent efforts have employed similar strategies using constructs that also contain human Vλ, Jλ and Cλ sequences (Osborn, M.J. et al., 2013, J. Immunol. 190:1481-90; Lee, E-C. et al., 2014, Nat. Biotech. 32(4):356-63, each of which is incorporated herein by reference in its entirety).

Yet other efforts have included the specific insertion of human Vλ and Jλ gene segments into endogenous rodent immunoglobulin light chain loci (κ and λ) so that said human Vλ and Jλ gene segments are operably linked to endogenous immunoglobulin light chain constant region genes (see, e.g., U.S. Patent Nos. 9,006,511, 9,012,717, 9,029,628, 9,035,128, 9,066,502, 9,150,662 and 9,163,092; all of which are incorporated herein by reference in their entireties). In some embodiments of such animals, all of the human Vλ gene segments from clusters A and B and either one or four human Jλ gene segments were inserted into endogenous immunoglobulin κ and immunoglobulin λ light chain loci. Several different human Vλ and Jλ gene segments demonstrated proper rearrangement at both engineered rodent immunoglobulin light chain loci to form functional light chains expressed in the rodent antibody repertoire, which light chains included human Vλ domains in the context of either endogenous Cκ and Cλ regions (see, e.g., Table 7 and Figures 11-13 of U.S. Patent No. 9,006,511, which is incorporated herein by reference in its entirety). In particular, mice having engineered immunoglobulin κ light chain loci harboring human Vλ and Jλ gene segments demonstrated a human lambda to endogenous lambda ratio (as measured by IgCκ to IgCλ ratio) of about 1:1 in the splenic compartment (see, e.g., Table 4 of U.S. Patent No. 9,006,511, which is incorporated herein by reference in its entirety). Indeed, both engineered mouse strains (i.e., engineered immunoglobulin κ or engineered immunoglobulin λ light chain loci) demonstrated that human Vλ domains could be expressed from endogenous immunoglobulin light chain loci in rodents, which normally display a large bias in light chain expression (see above). The present disclosure provides the recognition that alternate engineered immunoglobulin light chain locus structures can be produced to maximize expression of human λ light chain variable domains from a limited human λ light variable region repertoire. Such alternate engineered immunoglobulin light chain locus structures provide the capacity for unique antibody repertoires resulting from their design.

The present disclosure provides a non-human animal whose germline genome contains an engineered endogenous immunoglobulin κ light chain locus comprising a single rearranged human immunoglobulin λ light chain variable region operably linked to a rodent Cλ gene segment. A single rearranged human immunoglobulin λ light chain variable region comprises a human Vλ gene segment and a human Jλ gene segment. In some embodiments, all immunoglobulin λ light chains expressed by B cells of the genetically modified rodent include human immunoglobulin λ light chain variable domains expressed from the single rearranged human immunoglobulin λ light chain variable region or a somatically hypermutated version thereof. In some embodiments, an engineered endogenous immunoglobulin κ light chain locus comprises a single rearranged human immunoglobulin λ light chain variable region operably linked to a non-human or human immunoglobulin λ or immunoglobulin κ light chain constant region gene. In some embodiments, expression of such light chains can be achieved by insertion of said single rearranged human immunoglobulin λ light chain variable region into an endogenous immunoglobulin κ light chain locus (or allele). In some embodiments, provided non-human animals are engineered so that expression of endogenous immunoglobulin λ light chain variable regions is inactivated (e.g., by gene deletion). In some embodiments, provided non-human animals are engineered so that expression of endogenous immunoglobulin κ light chain variable regions is inactivated (e.g., by insertion, replacement or substitution).

### Universal Light Chain

Prior efforts to make useful multispecific antigen-binding proteins, *e.g.*, bispecific antibodies, have been hindered by a variety of problems that frequently share a common paradigm: *in vitro* selection or manipulation of sequences to rationally engineer, or to engineer through trial-and-error, a suitable format for pairing a heterodimeric bispecific human immunoglobulin. Unfortunately, most if not all of the *in vitro* engineering approaches provide largely *ad hoc* fixes that are suitable, if at all, for individual molecules.

*In vivo* methods for employing complex organisms to select appropriate pairings that are capable of leading to human therapeutics have been developed (see, e.g., U.S. Patent No. 10,143,186, which is incorporated by reference in its entirety). Prior non-human animals, however, have not been produced that are capable of generating a robust immune response that includes the production of high-affinity universal λ light chains having human immunoglobulin λ light chain variable domains at sufficient titer levels. Generally, native non-human sequences are frequently not a good source for human therapeutic sequences. For at least that reason, generating non-human heavy chain immunoglobulin variable domains that pair with a universal human light chain is of limited practical utility. More *in vitro* engineering efforts would be expended in a trial-and-error process to try to humanize the non-human heavy chain variable sequences, while hoping to retain epitope specificity and affinity and the ability to couple with the common human light chain, with uncertain outcome. At the end of such a process, the final product may maintain some of the specificity and affinity, and associate with the universal light chain, but ultimately immunogenicity in a human would likely remain a profound risk.

Therefore, a suitable non-human animal for making human therapeutics would include a suitably large repertoire of human heavy chain variable region gene segments in place of endogenous non-human heavy chain variable region gene segments. The human heavy chain variable region gene segments should be able to rearrange and splice to an endogenous non-human heavy chain constant region to form a reverse chimeric heavy chain (*i.e.,* a heavy chain comprising a human variable domain and a non-human constant domain). The heavy chain locus should be capable of undergoing class switching and somatic hypermutation so that a suitably large repertoire of heavy chain variable domains are available for the non-human animal to select one that can associate with human λ light chain variable domains encoded by a limited repertoire of human λ light chain variable regions.

A non-human animal that selects a universal light chain for a plurality of heavy chains has a practical utility. In various embodiments, antibodies that express in a non-human animal that can only express universal light chains will have heavy chains that can associate and express with an identical or substantially identical light chain. This is particularly useful in making bispecific antibodies. For example, such a non-human animal can be immunized with a first immunogen to generate a B cell that expresses an antibody that specifically binds a first epitope. The non-human animal (or a non-human animal including the same genetic modifications to its heavy and λ light chain loci) can be immunized with a second immunogen to generate a B cell that expresses an antibody that specifically binds the second epitope. Variable heavy chain regions can be cloned from the B cells and expressed with the same heavy chain constant region, and the same light chain, and expressed in a cell to make a bispecific antibody, where the light chain component of the bispecific antibody has been selected by a non-human animal to associate and express with the light chain component. Non-human animals that express universal κ light chains have been developed (see, e.g., U.S. Patent No. 10,143,186, which is incorporated by reference in its entirety). However, there remains a need for development of non-human animals that are able to express universal λ light chains, including human λ light chain variable domains.

The present disclosure provides engineered non-human animals (e.g., rodents, e.g., rats or mice) for generating immunoglobulin λ light chains that will suitably pair with a rather diverse family of heavy chains, including heavy chains whose variable regions depart from germline sequences, e.g., affinity matured or somatically hypermutated variable regions. In various embodiments, a non-human animal as described herein is engineered to express and pair human λ light chain variable domains with human heavy chain variable domains that comprise somatic mutations, thus enabling a route to high affinity binding proteins (e.g., antibodies) suitable for use as human therapeutics.

A genetically engineered non-human animal (e.g., a rodent, e.g., a rat or mouse) as described herein, through the long and complex process of antibody selection within an organism, makes biologically appropriate choices in pairing a diverse collection of human heavy chain variable domains with a limited number of light chain options. In order to achieve this, non-human animals are engineered to present a limited number of human λ light chain variable domain options in conjunction with a wide diversity of human heavy chain variable domain options. Upon challenge with an immunogen, a non-human animal described herein can maximize the number of solutions in its repertoire to develop an antibody to the immunogen, limited largely or solely by the number or light chain options in its repertoire. In various embodiments, this includes allowing a non-human animal to achieve suitable and compatible somatic mutations of the light chain variable domain that will nonetheless be compatible with a relatively large variety of human heavy chain variable domains, including, in particular, somatically hypermutated human heavy chain variable domains.

To achieve a limited repertoire of human λ light chain options, a non-human animal as described herein can be engineered to render nonfunctional or substantially nonfunctional its ability to make, or rearrange, native non-human λ and/or κ light chain variable domains. In some embodiments, this can be achieved, e.g., by deleting a non-human animal's λ and/or κ light chain variable region gene segments. In some embodiments, the endogenous non-human locus can then be modified with a suitable exogenous human λ light chain variable region sequence(s) of choice, operably linked to the endogenous non-human light chain constant domain. In some embodiments, exogenous human variable region gene segments are unrearranged (e.g., two Vλ gene segments and one or more Jλ gene segments) and can rearrange and splice to an endogenous non-human light chain constant region gene to form a rearranged reverse chimeric light chain gene (human variable, non-human constant). In some embodiments, exogenous human variable gene segments are rearranged (e.g., one Vλ gene segement and one Jλ gene segment) and can splice to an endogenous non-human light chain constant region gene to form a reverse chimeric light chain gene comprising a human variable region and a non-human constant region. In some embodiments, exogenous human variable gene segments are rearranged (e.g., one Vλ gene segement and one Jλ gene segment) and can splice to an exogenous human light chain constant region gene to form a humanized light chain gene comprising a human variable region and a human constant region. In various embodiments, the light chain variable region is capable of being somatically hypermutated. In various embodiments, appropriate enhancer(s) is retained in the non-human animal. Enhancers have been reported to maximize the ability of the light chain variable regions to acquire somatic mutations. For example, in modifying a κ locus of a non-human animal by replacing endogenous non-human animal κ variable region gene segments with human λ variable region gene segments, a non-human κ intronic enhancer and non-human κ 3' enhancer are functionally maintained, or undisrupted. Although embodiments in which enhancers are removed or disrupted are contemplated by this disclosure, such embodiments would be expected to reduce or eliminate somatic hypermutation. In such embodiments, somatic hypermutation of a light chain variable region is reduced relative to, e.g., a light chain variable region comprising one or more endogenous, non-human enhancers.

A genetically engineered non-human animal is provided that expresses a limited repertoire of reverse chimeric (human variable, non-human constant) light chains associated with a diversity of reverse chimeric (human variable, non-human constant) heavy chains. In various embodiments, the endogenous non-human κ light chain variable region gene segments are deleted and replaced with a single (or two) human λ light chain variable region gene segments, operably linked to the endogenous non-human κ constant region gene. In some embodiments, the non-human κ intronic enhancer and the non-human κ 3' enhancer are maintained. Without being bound to any one theory, enhancers can, among other things, maximize somatic hypermutation of the human λ light chain variable region gene segments. In various embodiments, a non-human animal also comprises a nonfunctional λ light chain locus, or a deletion thereof, or a deletion that renders the locus unable to make a λ light chain.

A genetically engineered non-human animal is provided that, in various embodiments, comprises a light chain variable region locus lacking an endogenous non-human light chain variable gene segment and comprising a human variable gene segment, in some embodiments a rearranged human immunoglobulin λ light chain variable region, operably linked to a non-human Cλ gene segment, wherein the locus is capable of undergoing somatic hypermutation, and wherein the locus expresses a light chain comprising the human immunoglobulin λ light chain variable region linked to a non-human Cλ gene segment. Thus, in various embodiments, the locus comprises a non-human κ 3' enhancer, which is correlated with a normal, or wild-type, level of somatic hypermutation.

In various embodiments, a genetically engineered non-human animal, when immunized with an antigen of interest, generates B cells that exhibit a diversity of rearrangements of human immunoglobulin heavy chain variable regions that express and function with one or with two rearranged light chains. In some embodiments, human λ light chain variable regions comprise somatic hypermutations. In some embodiments, the human λ light chain variable regions each comprise 1 to 5 somatic hypermutations. In various embodiments, light chains expressed by a non-human animal described herein are capable of associating and expressing with any heavy chain including a human immunoglobulin heavy chain variable region expressed in the non-human animal.

### Provided non-human animals, cells and tissues

Non-human animals are provided that express (e.g., whose B cells express) antibodies that contain light chains including a human λ light chain variable domain derived from (i) one or two unrearranged Vλ gene segments and one or more unrearranged Jλ gene segments, or (ii) a single rearranged human λ light chain variable region in the place of non-human immunoglobulin κ light chain variable region sequences at the endogenous non-human λ light chain locus in the germline genome of the non-human animal. It would be understood that, in various embodiments described herein, a genetically modified non-human animal is a rodent, such as a rat or a mouse, and non-human elements described herein (enhancers, constant regions, etc.) are rodent, such as rat or mouse elements. Suitable examples of non-human animals described herein include, but are not limited to, rodents, for example, rats or mice, in particular, mice.

The present disclosure provides improved *in vivo* systems for identifying and developing new antigen-binding proteins, antibodies, antibody components (e.g., antigen-binding portions and/or compositions or formats that include them), and/or antibody-based therapeutics that can be used, for example, in the treatment of a variety of diseases that affect humans. Further, the present disclosure encompasses the recognition that non-human animals (e.g., rodents, e.g. rats or mice) having engineered immunoglobulin loci, such as an engineered immunoglobulin κ light chain locus including a limited λ light chain variable region repertoire, are useful. In some embodiments, non-human animals described herein provide improved *in vivo* systems for development of antibodies and/or antibody-based therapeutics for administration to humans. In some embodiments, non-human animals described herein provide improved *in vivo* systems for development of antibodies and/or antibody-based therapeutics that contain human λ light chain variable domains characterized by improved and/or different performance (e.g., expression and/or representation in an antigen-specific antibody repertoire) as compared to antibodies and/or antibody-based therapeutics obtained from existing *in vivo* systems that contain human Vλ region sequences.

The present disclosure provides, among other things, a non-human animal (e.g., rodent, e.g., rat or mouse), non-human (e.g., rodent, e.g., rat or mouse) cell or non-human (e.g., rodent, e.g., rat or mouse) tissue having an endogenous immunoglobulin κ light chain locus that has been engineered to include a limited human λ light chain variable region repertoire. In some embodiments, sequences of a limited human λ light chain variable region repertoire are operably linked to a non-human light chain constant region. In some embodiments, a non-human light chain constant region is a rodent (e.g., mouse or rat) light chain constant region. In some embodiments, a non-human light chain constant region is a κ or λ light chain constant region. In some embodiments, sequences of a limited human λ light chain variable region repertoire are operably linked to a non-human (e.g., rodent, e.g., rat or mouse) Cκ. In some embodiments, sequences of a limited human λ light chain variable region repertoire are operably linked to a non-human (e.g., rodent, e.g., rat or mouse) Cλ (e.g., Cλ1). In some embodiments, a non-human λ light chain constant region (e.g., a mouse Cλ, e.g., a mouse Cλ1) is in place of an endogenous non-human Cκ.

The present disclosure provides, among other things, a non-human animal (e.g., rodent, e.g., rat or mouse), non-human (e.g., rodent, e.g., rat or mouse) cell or non-human (e.g., rodent, e.g., rat or mouse) tissue having an endogenous immunoglobulin κ light chain locus that has been engineered to include two unrearranged human Vλ gene segments and one or more unrearranged human Jλ gene segments. In some embodiments, the two unrearranged human Vλ gene segments are selected from the group consisting of Vλ4-69, Vλ8-61, Vλ4-60, Vλ6-57, Vλ10-54, Vλ5-52, Vλ1-51, Vλ9-49, Vλ1-47, Vλ7-46, Vλ5-45, Vλ1-44, Vλ7-43, Vλ1-40, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, the two unrearranged human Vλ gene segments are selected from the group consisting of Vλ5-52, Vλ1-51, Vλ9-49, Vλ1-47, Vλ7-46, Vλ5-45, Vλ1-44, Vλ7-43, Vλ1-40, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, the two unrearranged human Vλ gene segments are selected from the group consisting of Vλ1-51, Vλ5-45, Vλ1-44, Vλ1-40, Vλ3-21, and Vλ2-14. In some embodiments, the one or more unrearranged human Jλ gene segments are selected from the group consisting of Jλ1, Jλ2, Jλ3, Jλ6, and Jλ7. In some embodiments, the one or more unrearranged human Jλ gene segments include Jλ1, Jλ2, Jλ3, and Jλ7. In some embodiments, the one or more unrearranged human Jλ gene segments include Jλ1, Jλ2, Jλ3, Jλ6, and Jλ7. In some embodiments, the two unrearranged human Vλ gene segments and one or more unrearranged human Jλ gene segments are operably linked to a non-human light chain constant region. In some embodiments, a non-human light chain constant region is a rodent (e.g., mouse or rat) light chain constant region. In some embodiments, a non-human light chain constant region is a κ or λ light chain constant region. In some embodiments, the two unrearranged human Vλ gene segments and one or more unrearranged human Jλ gene segments are operably linked to a non-human (e.g., rodent, e.g., rat or mouse) Cκ. In some embodiments, the two unrearranged human Vλ gene segments and one or more unrearranged human Jλ gene segments are operably linked to a non-human (e.g., rodent, e.g., rat or mouse) Cλ (e.g., Cλ1). In some embodiments, a non-human λ light chain constant region (e.g., a rodent, e.g., rat or mouse, such as a mouse Cλ, e.g., a mouse Cλ1) is in place of an endogenous non-human (e.g., rodent, e.g., rat or mouse) Cκ.

The present disclosure provides, among other things, a non-human animal (e.g., rodent, e.g., rat or mouse), non-human (e.g., rodent, e.g., rat or mouse) cell or non-human (e.g., rodent, e.g., rat or mouse) tissue having an endogenous immunoglobulin κ light chain locus that has been engineered to include two unrearranged human Vλ gene segments and four unrearranged human Jλ gene segments operably linked to a non-human (e.g., rodent, e.g., rat or mouse) Cκ. In some embodiments, the two unrearranged human Vλ gene segments are selected from the group consisting of Vλ1-51, Vλ5-45, Vλ1-44, Vλ1-40, Vλ3-21, and Vλ2-14. In some embodiments, the four unrearranged human Jλ gene segments are Jλ1, Jλ2, Jλ3, and Jλ7.

The present disclosure provides, among other things, a non-human animal (e.g., rodent, e.g., rat or mouse), non-human (e.g., rodent, e.g., rat or mouse) cell or non-human (e.g., rodent, e.g., rat or mouse) tissue having an endogenous immunoglobulin κ light chain locus that has been engineered to include two unrearranged human Vλ gene segments and five unrearranged human Jλ gene segments operably linked to a non-human (e.g., rodent, e.g., rat or mouse) Cκ. In some embodiments, the two unrearranged human Vλ gene segments are selected from the group consisting of Vλ1-51, Vλ5-45, Vλ1-44, Vλ1-40, Vλ3-21, and Vλ2-14. In some embodiments, the five unrearranged human Jλ gene segments are Jλ1, Jλ2, Jλ3, Jλ6, and Jλ7.

The present disclosure provides, among other things, a non-human animal (e.g., rodent, e.g., rat or mouse), non-human (e.g., rodent, e.g., rat or mouse) cell or non-human (e.g., rodent, e.g., rat or mouse) tissue having an endogenous immunoglobulin κ light chain locus that has been engineered to include two unrearranged human Vλ gene segments and four unrearranged human Jλ gene segments operably linked to a non-human (e.g., rodent, e.g., rat or mouse) Cλ (e.g., Cλ1). In some embodiments, the two unrearranged human Vλ gene segments are selected from the group consisting of Vλ1-51, Vλ5-45, Vλ1-44, Vλ1-40, Vλ3-21, and Vλ2-14. In some embodiments, the four unrearranged human Jλ gene segments are Jλ1, Jλ2, Jλ3, and Jλ7.

The present disclosure provides, among other things, a non-human animal (e.g., rodent, e.g., rat or mouse), non-human (e.g., rodent, e.g., rat or mouse) cell or non-human (e.g., rodent, e.g., rat or mouse) tissue having an endogenous immunoglobulin κ light chain locus that has been engineered to include two unrearranged human Vλ gene segments and five unrearranged human Jλ gene segments operably linked to a non-human (e.g., rodent, e.g., rat or mouse) Cλ (e.g., Cλ1). In some embodiments, the two unrearranged human Vλ gene segments are selected from the group consisting of Vλ1-51, Vλ5-45, Vλ1-44, Vλ1-40, Vλ3-21, and Vλ2-14. In some embodiments, the five unrearranged human Jλ gene segments are Jλ1, Jλ2, Jλ3, Jλ6, and Jλ7.

The present disclosure provides, among other things, a non-human animal (e.g., rodent, e.g., rat or mouse), non-human (e.g., rodent, e.g., rat or mouse) cell or non-human (e.g., rodent, e.g., rat or mouse) tissue having an endogenous immunoglobulin κ light chain locus that has been engineered to include one unrearranged human Vλ gene segment and one or more unrearranged human Jλ gene segments. In some embodiments, the one unrearranged human Vλ gene segment is selected from the group consisting of Vλ4-69, Vλ8-61, Vλ4-60, Vλ6-57, Vλ10-54, Vλ5-52, Vλ1-51, Vλ9-49, Vλ1-47, Vλ7-46, Vλ5-45, Vλ1-44, Vλ7-43, Vλ1-40, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, the one unrearranged human Vλ gene segment is selected from the group consisting of Vλ5-52, Vλ1-51, Vλ9-49, Vλ1-47, Vλ7-46, Vλ5-45, Vλ1-44, Vλ7-43, Vλ1-40, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, the one unrearranged human Vλ gene segment is selected from the group consisting of Vλ1-51, Vλ5-45, Vλ1-44, Vλ1-40, Vλ3-21, and Vλ2-14. In some embodiments, the one or more unrearranged human Jλ gene segments are selected from the group consisting of Jλ1, Jλ2, Jλ3, Jλ6, and Jλ7. In some embodiments, the one or more unrearranged human Jλ gene segments include Jλ1, Jλ2, Jλ3, and Jλ7. In some embodiments, the one or more unrearranged human Jλ gene segments include Jλ1, Jλ2, Jλ3, Jλ6, and Jλ7. In some embodiments, the one unrearranged human Vλ gene segment and one or more unrearranged human Jλ gene segments are operably linked to a non-human light chain constant region. In some embodiments, a non-human light chain constant region is a rodent (e.g., mouse or rat) light chain constant region. In some embodiments, a non-human light chain constant region is a κ or λ light chain constant region. In some embodiments, one unrearranged human Vλ gene segment and one or more unrearranged human Jλ gene segments are operably linked to a non-human (e.g., rodent, e.g., rat or mouse) Cκ. In some embodiments, one unrearranged human Vλ gene segments and one or more unrearranged human Jλ gene segments are operably linked to a non-human (e.g., rodent, e.g., rat or mouse) Cλ (e.g., Cλ1). In some embodiments, a non-human λ light chain constant region (e.g., a rodent, e.g., rat or mouse, such as a mouse Cλ, e.g., a mouse Cλ1) is in place of an endogenous non-human (e.g., rodent, e.g., rat or mouse) Cx.

The present disclosure provides, among other things, a non-human animal (e.g., rodent, e.g., rat or mouse), non-human (e.g., rodent, e.g., rat or mouse) cell or non-human (e.g., rodent, e.g., rat or mouse) tissue having an endogenous immunoglobulin κ light chain locus that has been engineered to include one unrearranged human Vλ gene segments and four unrearranged human Jλ gene segments operably linked to a non-human (e.g., rodent, e.g., rat or mouse) Cκ. In some embodiments, the one unrearranged human Vλ gene segment is selected from the group consisting of Vλ1-51, Vλ5-45, Vλ1-44, Vλ1-40, Vλ3-21, and Vλ2-14. In some embodiments, the four unrearranged human Jλ gene segments are Jλ1, Jλ2, Jλ3, and Jλ7. In some embodiments, the one unrearranged human Vλ gene segment is Vλ1-51, and the four unrearranged human Jλ gene segments are Jλ1, Jλ2, Jλ3, and Jλ7. In some embodiments, the one unrearranged human Vλ gene segment is Vλ2-14, and the four unrearranged human Jλ gene segments are Jλ1, Jλ2, Jλ3, and Jλ7.

The present disclosure provides, among other things, a non-human animal (e.g., rodent, e.g., rat or mouse), non-human (e.g., rodent, e.g., rat or mouse) cell or non-human (e.g., rodent, e.g., rat or mouse) tissue having an endogenous immunoglobulin κ light chain locus that has been engineered to include one unrearranged human Vλ gene segment and five unrearranged human Jλ gene segments operably linked to a non-human (e.g., rodent, e.g., rat or mouse) Cκ. In some embodiments, the one unrearranged human Vλ gene segment is selected from the group consisting of Vλ1-51, Vλ5-45, Vλ1-44, Vλ1-40, Vλ3-21, and Vλ2-14. In some embodiments, the five unrearranged human Jλ gene segments are Jλ1, Jλ2, Jλ3, Jλ6, and Jλ7. In some embodiments, the one unrearranged human Vλ gene segment is Vλ1-51, and the five unrearranged human Jλ gene segments are Jλ1, Jλ2, Jλ3, Jλ6, and Jλ7. In some embodiments, the one unrearranged human Vλ gene segment is Vλ2-14, and the five unrearranged human Jλ gene segments are Jλ1, Jλ2, Jλ3, Jλ6, and Jλ7.

The present disclosure provides, among other things, a non-human animal (e.g., rodent, e.g., rat or mouse), non-human (e.g., rodent, e.g., rat or mouse) cell or non-human (e.g., rodent, e.g., rat or mouse) tissue having an endogenous immunoglobulin κ light chain locus that has been engineered to include one unrearranged human Vλ gene segments and four unrearranged human Jλ gene segments operably linked to a non-human (e.g., rodent, e.g., rat or mouse) Cλ. In some embodiments, the one unrearranged human Vλ gene segment is selected from the group consisting of Vλ1-51, Vλ5-45, Vλ1-44, Vλ1-40, Vλ3-21, and Vλ2-14. In some embodiments, the four unrearranged human Jλ gene segments are Jλ1, Jλ2, Jλ3, and Jλ7. In some embodiments, the one unrearranged human Vλ gene segment is Vλ1-51, and the four unrearranged human Jλ gene segments are Jλ1, Jλ2, Jλ3, and Jλ7. In some embodiments, the one unrearranged human Vλ gene segment is Vλ2-14, and the four unrearranged human Jλ gene segments are Jλ1, Jλ2, Jλ3, and Jλ7.

The present disclosure provides, among other things, a non-human animal (e.g., rodent, e.g., rat or mouse), non-human (e.g., rodent, e.g., rat or mouse) cell or non-human (e.g., rodent, e.g., rat or mouse) tissue having an endogenous immunoglobulin κ light chain locus that has been engineered to include one unrearranged human Vλ gene segment and five unrearranged human Jλ gene segments operably linked to a non-human (e.g., rodent, e.g., rat or mouse) Cλ. In some embodiments, the one unrearranged human Vλ gene segment is selected from the group consisting of Vλ1-51, Vλ5-45, Vλ1-44, Vλ1-40, Vλ3-21, and Vλ2-14. In some embodiments, the five unrearranged human Jλ gene segments are Jλ1, Jλ2, Jλ3, Jλ6, and Jλ7. In some embodiments, the one unrearranged human Vλ gene segment is Vλ1-51, and the five unrearranged human Jλ gene segments are Jλ1, Jλ2, Jλ3, Jλ6, and Jλ7. In some embodiments, the one unrearranged human Vλ gene segment is Vλ2-14, and the five unrearranged human Jλ gene segments are Jλ1, Jλ2, Jλ3, Jλ6, and Jλ7.

The present disclosure provides, among other things, a non-human animal (e.g., rodent, e.g., rat or mouse), non-human (e.g., rodent, e.g., rat or mouse) cell or non-human (e.g., rodent, e.g., rat or mouse) tissue having an endogenous immunoglobulin κ light chain locus that has been engineered to include a single rearranged human λ light chain variable region (V/J), which includes a human Vλ gene segment and a human Jλ gene segment. In some embodiments, a human Vλ gene segment of a single rearranged human λ light chain variable region is selected from the group consisting of Vλ4-69, Vλ8-61, Vλ4-60, Vλ6-57, Vλ10-54, Vλ5-52, Vλ1-51, Vλ9-49, Vλ1-47, Vλ7-46, Vλ5-45, Vλ1-44, Vλ7-43, Vλ1-40, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, a human Vλ gene segment of a single rearranged human λ light chain variable region is selected from the group consisting of Vλ5-52, Vλ1-51, Vλ9-49, Vλ1-47, Vλ7-46, Vλ5-45, Vλ1-44, Vλ7-43, Vλ1-40, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, a human Vλ gene segment of a single rearranged human λ light chain variable region is selected from the group consisting of Vλ1-51, Vλ5-45, Vλ1-44, Vλ1-40, Vλ3-21, and Vλ2-14. In some embodiments, a human Vλ gene segment of a single rearranged human λ light chain variable region is Vλ1-51. In some embodiments, a human Vλ gene segment of a single rearranged human λ light chain variable region is Vλ2-14. In some embodiments, a human Jλ gene segment of a single rearranged human λ light chain variable region is selected from the group consisting of Jλ1, Jλ2, Jλ3, Jλ6, and Jλ7. In some embodiments, a human Jλ gene segment of a single rearranged human λ light chain variable region is Jλ1. In some embodiments, a human Jλ gene segment of a single rearranged human λ light chain variable region is Jλ2. In some embodiments, a human Jλ gene segment of a single rearranged human λ light chain variable region is Jλ3. In some embodiments, a single rearranged human λ light chain variable region is operably linked to a non-human light chain constant region. In some embodiments, a non-human light chain constant region is a rodent (e.g., mouse or rat) light chain constant region. In some embodiments, a non-human light chain constant region is a κ or λ light chain constant region. In some embodiments, a single rearranged human λ light chain variable region is operably linked to a non-human (e.g., rodent, e.g., rat or mouse) Cκ. In some embodiments, a single rearranged human λ light chain variable region is operably linked to a non-human (e.g., rodent, e.g., rat or mouse) Cλ (e.g., Cλ1). In some embodiments, a non-human λ light chain constant region (e.g., a mouse Cλ, e.g., a mouse Cλ1) is in place of an endogenous non-human (e.g., rodent, e.g. rat or mouse) Cκ.

The present disclosure provides, among other things, a non-human animal (e.g., rodent, e.g., rat or mouse), non-human (e.g., rodent, e.g., rat or mouse) cell or non-human (e.g., rodent, e.g., rat or mouse) tissue having an endogenous immunoglobulin κ light chain locus that has been engineered to include a single rearranged human λ light chain variable region operably linked to a mouse Cκ, where the single rearranged human λ light chain variable region includes a human Vλ gene segment and a human Jλ gene segment. In some embodiments, the human Vλ gene segment is selected from the group consisting of Vλ1-51, Vλ5-45, Vλ1-44, Vλ1-40, Vλ3-21, and Vλ2-14. In some embodiments, the human Jλ gene segment is selected from the group consisting of Jλ1, Jλ2, Jλ3, and Jλ7. In some embodiments, the human Vλ gene segment is Vλ1-51 and the human Jλ gene segment is Jλ2. In some embodiments, the human Vλ gene segment is Vλ2-14 and the human Jλ gene segment is Jλ2.

The present disclosure provides, among other things, a non-human animal (e.g., rodent, e.g., rat or mouse), non-human (e.g., rodent, e.g., rat or mouse) cell or non-human (e.g., rodent, e.g., rat or mouse) tissue having an endogenous immunoglobulin κ light chain locus that has been engineered to include a single rearranged human λ light chain variable region operably linked to a non-human (e.g., rodent, e.g., rat or mouse) Cλ, where the single rearranged human λ light chain variable region includes a human Vλ gene segment and a human Jλ gene segment. In some embodiments, the human Vλ gene segment is selected from the group consisting of Vλ1-51, Vλ5-45, Vλ1-44, Vλ1-40, Vλ3-21, and Vλ2-14. In some embodiments, the human Jλ gene segment is selected from the group consisting of Jλ1, Jλ2, Jλ3, and Jλ7. In some embodiments, the human Vλ gene segment is Vλ1-51 and the human Jλ gene segment is Jλ2. In some embodiments, the human Vλ gene segment is Vλ2-14 and the human Jλ gene segment is Jλ2.

The present disclosure provides, among other things, a mouse, mouse cell or mouse tissue having an endogenous immunoglobulin κ light chain locus that has been engineered to include a single rearranged human λ light chain variable region operably linked to a mouse Cλ, where the single rearranged human λ light chain variable region includes a human Vλ gene segment and a human Jλ gene segment. In some embodiments, the human Vλ gene segment comprises Vλ1-51. In some embodiments, the human Vλ gene segment comprises Vλ2-14. In some embodiments, the human Jλ gene segment comprises Jλ2. In some embodiments, the human Vλ gene segment is Vλ1-51 and the human Jλ gene segment is Jλ2. In some embodiments, the human Vλ gene segment is Vλ2-14 and the human Jλ gene segment is Jλ2.

The present disclosure provides, among other things, a genetically modified mouse, mouse cell or mouse tissue whose germline genome comprises an engineered endogenous immunoglobulin κ light chain locus comprising a single rearranged human immunoglobulin λ light chain variable region operably linked to a mouse Cλ1 gene segment, wherein the single rearranged human immunoglobulin λ light chain variable region comprises a human Vλ1-51 gene segment and a human Jλ2 gene segment, wherein all immunoglobulin λ light chains expressed by B cells of the genetically modified mouse include human immunoglobulin λ light chain variable domains expressed from the single rearranged human immunoglobulin λ light chain variable region or a somatically hypermutated version thereof.

The present disclosure provides, among other things, a genetically modified mouse, mouse cell or mouse tissue whose germline genome comprises an engineered endogenous immunoglobulin κ light chain locus comprising a single rearranged human immunoglobulin λ light chain variable region operably linked to a mouse Cλ1 gene segment, wherein the single rearranged human immunoglobulin λ light chain variable region comprises a human Vλ2-14 gene segment and a human Jλ2 gene segment, wherein all immunoglobulin λ light chains expressed by B cells of the genetically modified mouse include human immunoglobulin λ light chain variable domains expressed from the single rearranged human immunoglobulin λ light chain variable region or a somatically hypermutated version thereof.

In some embodiments, provided non-human animals (e.g., rodents, e.g. rats or mice) are characterized by expression of antibodies from endogenous immunoglobulin κ light chain loci in the germline genome of said non-human animals, which antibodies contain (1) human Vλ domains and (2) non-human Cλ domains. In some embodiments, provided non-human animals are characterized by an improved usage of human Vλ regions from engineered immunoglobulin κ light chain loci (e.g., but not limited to, about 2-fold) including a limited human λ light chain variable region repertoire as compared to one or more reference engineered non-human animals.

In some embodiments, a non-human animal (e.g., rodent, e.g., rat or mouse), non-human (e.g., rodent, e.g., rat or mouse) cell or non-human (e.g., rodent, e.g., rat or mouse) tissue is provided, the germline genome of which comprises an endogenous immunoglobulin κ light chain locus comprising: (a) a single rearranged human immunoglobulin λ light chain variable region, and (b) a Cλ gene, wherein (a) is operably linked to (b), and wherein the non-human animal lacks a non-human animal Cκ gene at the endogenous immunoglobulin κ light chain locus.

In some embodiments, a non-human animal (e.g., rodent, e.g., rat or mouse), non-human (e.g., rodent, e.g., rat or mouse) cell or non-human (e.g., rodent, e.g., rat or mouse) tissue is provided, the genome of which comprises an endogenous immunoglobulin κ light chain locus comprising insertion of a single rearranged human immunoglobulin λ light chain variable region and a Cλ gene, which single rearranged human immunoglobulin λ light chain variable region is operably linked to said Cλ gene, and which Cλ gene is inserted in the place of a non-human Cκ gene at the endogenous immunoglobulin κ light chain locus. In many embodiments of a non-human animal, non-human cell or non-human tissue, a Cλ gene inserted in the place of a non-human Cκ gene at an endogenous immunoglobulin κ light chain locus is a non-human or human Cλ gene. In some embodiments, a non-human Cλ gene is or comprises a mammalian Cλ gene selected from the group consisting of a primate, goat, sheep, pig, dog, cow, or rodent (e.g., rat or mouse) Cλ gene.

In some embodiments, a non-human Cλ gene is or comprises a rodent Cλ gene.

In some embodiments, a rodent Cλ gene is or comprises a mouse Cλ gene. In some embodiments, a mouse Cλ gene comprises a sequence that is at least 80%, at least 85%, at least 90%, at least 95%, or at least 98% identical to a mouse Cλ gene selected from the group consisting of a mouse Cλ1, mouse Cλ2 and a mouse Cλ3. In some embodiments, a mouse Cλ gene comprises a sequence that is substantially identical or identical to a mouse Cλ gene selected from the group consisting of a mouse Cλ1, mouse Cλ2 and a mouse Cλ3. In some embodiments, a mouse Cλ1 gene is or comprises SEQ ID NO: 1. In some certain embodiments, a mouse Cλ2 gene is or comprises SEQ ID NO:2. In some certain embodiments, a mouse Cλ3 gene is or comprises SEQ ID NO:3. In some certain embodiments, a mouse Cλ gene comprises a sequence that is identical to a mouse Cλ1 gene.

In some embodiments, a mouse Cλ gene comprises a sequence that is 80% to 100%, 85% to 100%, 90% to 100%, 95% to 100%, or 98% to 100% identical to a mouse Cλ gene selected from the group consisting of a mouse Cλ1, mouse Cλ2 and a mouse Cλ3. In some embodiments, a mouse Cλ gene comprises a sequence that is 80% to 98%, 80% to 95%, 80% to 90%, or 80% to 85% identical to a mouse Cλ gene selected from the group consisting of a mouse Cλ1, mouse Cλ2 and a mouse Cλ3. In some embodiments, a mouse Cλ gene comprises a sequence that is 85% to 98%, 90% to 95%, or 88% to 93% identical to a mouse Cλ gene selected from the group consisting of a mouse Cλ1, mouse Cλ2 and a mouse Cλ3.

In some embodiments, a rodent Cλ gene is or comprises a rat Cλ gene. In some embodiments, a rat Cλ gene comprises a sequence that is at least 80%, at least 85%, at least 90%, at least 95%, or at least 98% identical to a rat Cλ gene selected from the group consisting of a rat Cλ1, rat Cλ2, rat Cλ3 and a rat Cλ4 gene. In some embodiments, a rat Cλ gene comprises a sequence that is substantially identical or identical to a rat Cλ gene selected from the group consisting of a rat Cλ1, rat Cλ2, rat Cλ3 and a rat Cλ4 gene. In some certain embodiments, a rat Cλ1 gene is or comprises SEQ ID NO:7. In some certain embodiments, a rat Cλ2 gene is or comprises SEQ ID NO:8. In some certain embodiments, a rat Cλ3 gene is or comprises SEQ ID NO:9. In some certain embodiments, a rat Cλ4 gene is or comprises SEQ ID NO: 10.

In some embodiments, a rat Cλ gene comprises a sequence that is 80% to 100%, 85% to 100%, 90% to 100%, 95% to 100%, or 98% to 100% identical to a rat Cλ gene selected from the group consisting of a rat Cλ1, rat Cλ2, rat Cλ3 and a rat Cλ4 gene. In some embodiments, a rat Cλ gene comprises a sequence that is 80% to 98%, 80% to 95%, 80% to 90%, or 80% to 85% identical to a rat Cλ gene selected from the group consisting of a rat Cλ1, rat Cλ2, rat Cλ3 and a rat Cλ4 gene. In some embodiments, a rat Cλ gene comprises a sequence that is 85% to 98%, 90% to 95%, or 88% to 93%, identical to a rat Cλ gene selected from the group consisting of a rat Cλ1, rat Cλ2, rat Cλ3 and a rat Cλ4 gene.

In some embodiments, a human Cλ gene comprises a sequence that is at least 80%, at least 85%, at least 90%, at least 95%, or at least 98% identical to a human Cλ gene selected from the group consisting of a human Cλ1, human Cλ2, human Cλ3, human Cλ6 and a human Cλ7 gene. In some embodiments, a human Cλ gene comprises a sequence that is substantially identical or identical to a human Cλ gene selected from the group consisting of a human Cλ, human Cλ2, human Cλ3, human Cλ6 and a human Cλ7 gene. In some embodiments, a human Cλ gene comprises a sequence that is identical to a human Cλ gene selected from the group consisting of a human Cλ1, human Cλ2, human Cλ3, human Cλ6 and a human Cλ7 gene. In some certain embodiments, a human Cλ1 gene is or comprises SEQ ID NO: 15. In some certain embodiments, a human Cλ2 gene is or comprises SEQ ID NO: 16. In some certain embodiments, a human Cλ3 gene is or comprises SEQ ID NO: 17. In some certain embodiments, a human Cλ6 gene is or comprises SEQ ID NO: 18. In some certain embodiments, a human Cλ7 gene is or comprises SEQ ID NO:18. In some certain embodiments, a human Cλ gene is or comprises a human Cλ2 gene.

In some embodiments, a human Cλ gene comprises a sequence that is 80% to 100%, 85% to 100%, 90% to 100%, 95% to 100%, or 98% to 100% identical to a human Cλ gene selected from the group consisting of a human Cλ1, human Cλ2, human Cλ3, human Cλ6 and a human Cλ7 gene. In some embodiments, a human Cλ gene comprises a sequence that is 80% to 98%, 80% to 95%, 80% to 90%, or 80% to 85% identical to a human Cλ gene selected from the group consisting of a human Cλ1, human Cλ2, human Cλ3, human Cλ6 and a human Cλ7 gene. In some embodiments, a human Cλ gene comprises a sequence that is 85% to 98%, 90% to 95%, or 88% to 93%, identical to a human Cλ gene selected from the group consisting of a human Cλ1, human Cλ2, human Cλ3, human Cλ6 and a human Cλ7 gene.

In some embodiments of a provided non-human animal (e.g., rodent, e.g., rat or mouse), non-human (e.g., rodent, e.g., rat or mouse) cell or non-human (e.g., rodent, e.g., rat or mouse) tissue, the germline genome of said non-human animal, non-human cell or non-human tissue further comprises an endogenous immunoglobulin heavy chain locus modified to comprise one or more human V_{H} gene segments, one or more human D_{H} gene segments, and one or more human J_{H} gene segments, which human V_{H}, D_{H} and J_{H} gene segments are operably linked to a non-human immunoglobulin heavy chain constant region at the endogenous immunoglobulin heavy chain locus (see, e.g., Macdonald, L.E., et al., "Precise and in situ genetic humanization of 6 Mb of mouse immunoglobulin genes," Proc. Natl. Acad. Sci. U.S.A, 111(14):5147-5152 (April 8, 2014), U.S. Patent Nos. 6,596,541, 8,642,835, 8,697,940 and 8,791,323, each of which is incorporated herein by reference in its entirety).

In some embodiments, insertion of one or more human V_{H} gene segments, one or more human D_{H} gene segments and one or more human J_{H} gene segments are in place of or replace, in whole or in part, non-human V_{H}, D_{H} and J_{H} gene segments (e.g., positionally replace or substitute coding sequences of non-human V_{H}, D_{H} and J_{H} gene segments with coding sequences of human V_{H}, D_{H} and J_{H} gene segments). In some embodiments, a non-human immunoglobulin heavy chain constant region is or comprises an endogenous non-human immunoglobulin heavy chain constant region. In many embodiments, a non-human immunoglobulin heavy chain constant region (e.g., endogenous) includes one or more non-human immunoglobulin heavy chain constant region genes or gene segments (e.g., IgM, IgD, IgG, IgE, IgA, etc.). In some certain embodiments, insertion includes human non-coding DNA that naturally appears between human V_{H}, D_{H} and J_{H} gene segments, and combinations thereof. In some certain embodiments, an immunoglobulin heavy chain locus as described herein comprises insertion of the human V_{H} gene segments V_{H}3-74, V_{H}3-73, V_{H}3-72, V_{H}2-70, V_{H}1-69, V_{H}3-66, V_{H}3-64, V_{H}4-61, V_{H}4-59, V_{H}1-58, V_{H}3-53, V_{H}5-51, V_{H}3-49, V_{H}3-48, V_{H}1-46, V_{H}1-45, V_{H}3-43, V_{H}4-39, V_{H}4-34, V_{H}3-33, V_{H}4-31, V_{H}3-30, V_{H}4-28, V_{H}2-26, V_{H}1-24, V_{H}3-23, V_{H}3-21, V_{H}3-20, V_{H}1-18, V_{H}3-15, V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2, V_{H}6-1, or any combination thereof, the human D_{H} gene segments D_{H}1-1, D_{H}2-2, D_{H}3-3, D_{H}4-4, D_{H}5-5, D_{H}6-6, D_{H}1-7, D_{H}2-8, D_{H}3-9, D_{H}3-10, D_{H}5-12, D_{H}6-13, D_{H}2-15, D_{H}3-16, D_{H}4-17, D_{H}6-19, D_{H}1-20, D_{H}2-21, D_{H}3-22, D_{H}6-25, D_{H}1-26, D_{H}7-27, or any combination thereof, and the human J_{H} gene segments J_{H}1, J_{H}2, J_{H}3, J_{H}4, J_{H}5, J_{H}6, or any combination thereof. In some certain embodiments, insertion includes human non-coding DNA that naturally appears adjacent to a human V_{H}3-74, V_{H}3-73, V_{H}3-72, V_{H}2-70, V_{H}1-69, V_{H}3-66, V_{H}3-64, V_{H}4-61, V_{H}4-59, V_{H}1-58, V_{H}3-53, V_{H}5-51, V_{H}3-49, V_{H}3-48, V_{H}1-46, V_{H}1-45, V_{H}3-43, V_{H}4-39, V_{H}4-34, V_{H}3-33, V_{H}4-31, V_{H}3-30, V_{H}4-28, V_{H}2-26, V_{H}1-24, V_{H}3-23, V_{H}3-21, V_{H}3-20, V_{H}1-18, V_{H}3-15, V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2, or V_{H}6-1 in an endogenous heavy chain locus, human non-coding DNA that naturally appears adjacent to a human D_{H}1-1, D_{H}2-2, D_{H}3-3, D_{H}4-4, D_{H}5-5, D_{H}6-6, D_{H}1-7, D_{H}2-8, D_{H}3-9, D_{H}3-10, D_{H}5-12, D_{H}6-13, D_{H}2-15, D_{H}3-16, D_{H}4-17, D_{H}6-19, D_{H}1-20, D_{H}2-21, D_{H}3-22, D_{H}6-25, D_{H}1-26, or D_{H}7-27, and human non-coding DNA that naturally appears adjacent to a human J_{H}1, J_{H}2, J_{H}3, J_{H}4, J_{H}5, or J_{H}6 in an endogenous heavy chain locus.

In some embodiments, a non-human animal (e.g., rodent, e.g., rat or mouse), non-human (e.g., rodent, e.g., rat or mouse) cell or non-human (e.g., rodent, e.g., rat or mouse) tissue described herein includes an Adam6 gene in its genome (e.g., its germline genome), which encodes an ADAM6 polypeptide, functional ortholog, functional homolog, or functional fragment thereof (see, e.g., U.S. Patent Nos. 8,642,835 and 8,697,940, each of which is incorporated herein by reference in its entirety). In some embodiments, a non-human animal (e.g., rodent, e.g., rat or mouse), non-human (e.g., rodent, e.g., rat or mouse) cell or non-human (e.g., rodent, e.g., rat or mouse) tissue described herein includes a rodent (e.g., a mouse or rat) Adam6 gene in its genome (e.g., its germline genome), which encodes a rodent (e.g., a mouse or rat) ADAM6 polypeptide, functional ortholog, functional homolog, or functional fragment thereof (see, e.g., U.S. Patent Nos. 8,642,835 and 8,697,940, each of which is incorporated herein by reference in its entirety). In some embodiments, an ADAM6 polypeptide, functional ortholog, functional homolog, or functional fragment thereof is expressed from an Adam6 gene. In some embodiments, an Adam6 gene in a genetically modified non-human animal as described herein does not originate from that specific non-human animal (e.g., a mouse that includes a rat Adam6 gene or a mouse Adam6 gene obtained from another strain of mouse). In some embodiments, a non-human animal described herein includes an ectopic Adam6 gene. An "ectopic" Adam6 gene, as used herein, refers to an Adam6 gene that is in a different context than the Adam6 gene appears in a wild-type non-human animal. For example, the Adam6 gene could be located on a different chromosome, located at a different locus, or positioned adjacent to different sequences. An exemplary ectopic Adam6 gene is a mouse Adam6 gene located within human immunoglobulin sequences (e.g., human heavy chain variable region gene segments). In some embodiments, a non-human animal described herein includes an inserted or integrated Adam6 gene.

In some embodiments, a non-human animal, non-human cell or non-human tissue described herein includes an insertion of one or more nucleotide sequences encoding one or more non-human Adam6 polypeptides, functional orthologs, functional homologs, or functional fragments thereof in its genome (e.g., its germline genome).

In some embodiments, a non-human animal (e.g., rodent, e.g., rat or mouse), non-human (e.g., rodent, e.g., rat or mouse) cell or non-human (e.g., rodent, e.g., rat or mouse) tissue described herein includes one or more nucleotide sequences encoding one or more non-human ADAM6 polypeptides, functional orthologs, functional homologs, or functional fragments thereof in its genome (e.g., its germline genome). In some embodiments, a non-human animal (e.g., rodent, e.g., rat or mouse), non-human (e.g., rodent, e.g., rat or mouse) cell or non-human (e.g., rodent, e.g., rat or mouse) tissuedescribed herein includes a mouse Adam6a gene and/or a mouse Adam6b gene in its genome (e.g. its germline genome). In some embodiments, a non-human animal, non-human cell or non-human tissue described herein includes one or more nucleotide sequences encoding a mouse ADAM6a, functional ortholog, functional homolog, or functional fragment thereof, and/or a mouse ADAM6b, functional ortholog, functional homolog, or functional fragment thereof.

In some embodiments, one or more nucleotide sequences encoding one or more non-human ADAM6 polypeptides, functional orthologs, functional homologs, or functional fragments thereof are inserted and/or are located on the same chromosome as the endogenous immunoglobulin heavy chain locus. In some embodiments, one or more nucleotide sequences encoding one or more non-human ADAM6 polypeptides, functional orthologs, functional homologs, or functional fragments thereof are inserted and/or are located in a position so that the one or more nucleotide sequences encoding one or more non-human ADAM6 polypeptides, functional orthologs, functional homologs, or functional fragments thereof are contiguous with human immunoglobulin heavy chain variable region gene segments. In some embodiments, one or more nucleotide sequences encoding one or more non-human ADAM6 polypeptides, functional orthologs, functional homologs, or functional fragments thereof are inserted and/or are located in a position so that the one or more nucleotide sequences encoding one or more non-human ADAM6 polypeptides, functional orthologs, functional homologs, or functional fragments thereof are adjacent to human immunoglobulin heavy chain variable region gene segments. In some embodiments, one or more nucleotide sequences encoding one or more non-human ADAM6 polypeptides, functional orthologs, functional homologs, or functional fragments thereof are inserted and/or are located in a position so that the one or more nucleotide sequences encoding one or more non-human ADAM6 polypeptides, functional orthologs, functional homologs, or functional fragments thereof are located in between human immunoglobulin heavy chain variable region gene segments. In some embodiments, one or more nucleotide sequences encoding one or more non-human ADAM6 polypeptides, functional orthologs, functional homologs, or functional fragments thereof are inserted and/or are located between a first and a second human V_{H} gene segment. In some embodiments, a first human V_{H} gene segment is human V_{H}1-2 and a second human V_{H} gene segment is human V_{H}6-1. In some embodiments, one or more nucleotide sequences encoding one or more non-human ADAM6 polypeptides, functional orthologs, functional homologs, or functional fragments thereof are inserted and/or are located in the place of a human Adam6 pseudogene. In some embodiments, one or more nucleotide sequences encoding one or more non-human ADAM6 polypeptides, functional orthologs, functional homologs, or functional fragments thereof are inserted between a human V_{H} gene segment and a human D_{H} gene segment.

In some embodiments, a non-human animal (e.g., rodent, e.g., rat or mouse), non-human (e.g., rodent, e.g., rat or mouse) cell or non-human (e.g., rodent, e.g., rat or mouse) tissue described herein includes an Adam6 gene that restores or enhances ADAM6 activity. In some embodiments, the Adam6 gene restores ADAM6 activity to the level of a comparable non-human animal that includes a functional, endogenous Adam6 gene. In some embodiments, the Adam6 gene enhances ADAM6 activity to a level that is at least 2 times, at least 3 times, at least 4 times, at least 5 times, at least 6 times, at least 7 times, at least 8 times, at least 9 times, or at least 10 times the ADAM6 activity of a comparable non-human animal that does not include a functional Adam6 gene.

In some embodiments, a non-human animal (e.g., rodent, e.g., rat or mouse), non-human (e.g., rodent, e.g., rat or mouse) cell or non-human (e.g., rodent, e.g., rat or mouse) tissue described herein includes an Adam6 gene that restores or enhances fertility when expressed in a male non-human animal. In some embodiments, the Adam6 gene restores fertility in a male non-human animal to a level of a comparable non-human animal that includes a functional, endogenous Adam6 gene. In some embodiments, the Adam6 gene restores fertility in a male non-human animal so that the number of pups produced by mating the male non-human animal is at least 70%, at least 80%, at least 90%, at least 95% the number of pups produced from a comparable mating of a comparable, male non-human animal that does not include a functional Adam6 gene. In some embodiments, the Adam6 gene enhances fertility in a male non-human animal so that number of pups produced by the mating of the male non-human animal include at least 2 times, at least 3 times, at least 4 times, at least 5 times, at least 6 times, at least 7 times, at least 8 times, at least 9 times, or at least 10 times the number of pups produced from a comparable mating of a comparable, male non-human animal that does not include a functional Adam6 gene.

In some embodiments, a non-human immunoglobulin heavy chain locus as described herein lacks at least one endogenous non-human Adam6 gene. In some embodiments, the lack of the at least one endogenous non-human Adam6 gene reduces ADAM6 activity and/or fertility in a male rodent (e.g., a mouse or rat) that lacks an endogenous non-human Adam6 gene. In some embodiments, a non-human immunoglobulin heavy chain locus as described herein includes a disruption of at least one endogenous non-human Adam6 gene. In some embodiments, the disruption of at least one endogenous non-human Adam6 gene reduces ADAM6 activity and/or fertility in a male rodent (e.g., a mouse or rat) that lacks an endogenous non-human Adam6 gene.

In some embodiments of a non-human animal (e.g., rodent, e.g., rat or mouse), non-human (e.g., rodent, e.g., rat or mouse) cell or non-human (e.g., rodent, e.g., rat or mouse) tissue as described herein, the non-human animal, non-human cell or non-human tissue is homozygous or heterozygous for an engineered endogenous immunoglobulin heavy chain locus comprising human heavy chain variable region gene segments, as described herein.

In some embodiments of a non-human animal (e.g., rodent, e.g., rat or mouse), non-human (e.g., rodent, e.g., rat or mouse) cell or non-human (e.g., rodent, e.g., rat or mouse) tissue as described herein, the non-human animal, non-human cell or non-human tissue is homozygous or heterozygous for an engineered endogenous immunoglobulin κ light chain locus, comprising human light chain variable gene segments (human variable λ light chain gene segments) as described herein.

In some embodiments, a non-human animal (e.g., a rodent, e.g., a rat or a mouse), non-human (e.g., rodent, e.g., rat or mouse) cell or non-human (e.g., rodent, e.g., rat or mouse) tissue as described herein, comprises a first engineered endogenous immunoglobulin κ light chain locus allele comprising a single rearranged human immunoglobulin λ light chain variable region operably linked to a rodent Cλ gene segment, wherein the single rearranged human immunoglobulin λ light chain variable region comprises a human Vλ gene segment and a human Jλ gene segment. In some embodiments, the non-human animal, non-human cell or non-human tissue comprises a second engineered endogenous immunoglobulin κ light chain locus allele comprising a single rearranged human immunoglobulin κ light chain variable region operably linked to a rodent Cκ gene segment, wherein the single rearranged human immunoglobulin κ light chain variable region comprises a human Vκ gene segment and a human Jκ gene segment. In some embodiments, such a non-human animal or non-human tissue can express a λ light chain from the first engineered endogenous immunoglobulin κ light chain locus allele and a κ light chain from the second engineered endogenous immunoglobulin κ light chain locus allele. In some embodiments, the single rearranged human immunoglobulin κ light chain variable region comprises Vκ3-20 or Vκ1-39 and the single rearranged human immunoglobulin λ light chain variable region comprises Vλ1-51 or Vλ2-14. In one embodiment, the single rearranged human immunoglobulin κ light chain variable region is Vκ3-20/Jκ1 or Vκ1-39/Jκ5, and the single rearranged human immunoglobulin λ light chain variable region is Vλ1-51/Jλ2 or Vλ2-14/Jλ2.

In some embodiments of a provided non-human animal (e.g., rodent, e.g., rat or mouse), non-human (e.g., rodent, e.g., rat or mouse) cell or non-human (e.g., rodent, e.g., rat or mouse) tissue, the endogenous immunoglobulin λ light chain locus is deleted in whole or in part. In some embodiments of a provided non-human animal, non-human cell or non-human tissue, the endogenous immunoglobulin λ light chain locus is functionally silenced or otherwise non-functional (e.g., by gene targeting). In some certain embodiments of a provided non-human animal, non-human cell or non-human tissue, the non-human animal, non-human cell or non-human tissue is homozygous for a functionally silenced or otherwise non-functional endogenous immunoglobulin λ light chain locus as described herein.

In some embodiments, a non-human animal (e.g., rodent, e.g., rat or mouse), non-human (e.g., rodent, e.g., rat or mouse) cell or non-human (e.g., rodent, e.g., rat or mouse) tissue as described herein does not detectably express endogenous immunoglobulin λ light chains. In some embodiments, a non-human animal, non-human cell or non-human tissue as described herein does not detectably express endogenous immunoglobulin κ light chains. In some embodiments, a non-human animal, non-human cell or non-human tissue as described herein does not detectably express endogenous immunoglobulin λ light chains or endogenous immunoglobulin κ light chains.

In some embodiments, a non-human animal (e.g., rodent, e.g., rat or mouse), non-human (e.g., rodent, e.g., rat or mouse) cell or non-human (e.g., rodent, e.g., rat or mouse) tissue as described herein does not detectably express endogenous immunoglobulin heavy chains. In some embodiments, a non-human animal, non-human cell or non-human tissue as described herein does not detectably express endogenous immunoglobulin λ light chains, endogenous immunoglobulin κ light chains, and endogenous immunoglobulin heavy chains.

In some embodiments, a non-human animal, non-human cell or non-human tissue as described herein has a genome further comprising a nucleic acid sequence encoding an exogenous terminal deoxynucleotidyltransferase (TdT) operably linked to a transcriptional control element. (See, e.g., WO 2017/210586 and U.S. Publication No. 2017/0347633, each of which is incorporated herein by reference in its entirety).

In some embodiments, a transcriptional control element includes a RAG1 transcriptional control element, a RAG2 transcriptional control element, an immunoglobulin heavy chain transcriptional control element, an immunoglobulin κ light chain transcriptional control element, an immunoglobulin λ light chain transcriptional control element, or any combination thereof.

In some embodiments, a nucleic acid sequence encoding an exogenous TdT is located at an immunoglobulin κ light chain locus, an immunoglobulin λ light chain locus, an immunoglobulin heavy chain locus, a RAG1 locus, or a RAG2 locus.

In some embodiments, the TdT is a human TdT. In some embodiments, the TdT is a short isoform of TdT (TdTS).

In some embodiments, a single rearranged human immunoglobulin λ light chain variable region is introduced into an endogenous immunoglobulin κ light chain locus in manner that maintains the integrity of non-human immunoglobulin κ light chain enhancer regions (or enhancer sequences) near the insertion point (e.g., a non-human immunoglobulin κ intronic enhancer and/or a non-human immunoglobulin κ 3' enhancer). Thus, such non-human animals have wild-type immunoglobulin κ light chain enhancer regions (or enhancer sequences) operably linked to human and non-human immunoglobulin λ light chain sequences (e.g., human Vλ and Jλ gene segments, and a non-human Cλ or Cκ) or operably linked to human immunoglobulin λ light chain sequences (e.g., human Vλ and Jλ gene segments, and a human Cλ or Cκ).

In some embodiments, a non-human immunoglobulin κ light chain locus that is altered, displaced, disrupted, deleted, replaced or engineered with one or more human immunoglobulin λ light chain sequences as described herein is a murine immunoglobulin κ light chain locus. In some embodiments, one or more human immunoglobulin λ light chain sequences as described herein are inserted into one copy (i.e., allele) of a non-human immunoglobulin κ light chain locus of the two copies of said non-human immunoglobulin κ light chain locus, giving rise to a non-human animal that is heterozygous with respect to the human immunoglobulin κ light chain sequence. In some embodiments, a non-human animal is provided that is homozygous for an immunoglobulin κ light chain locus that includes one or more human immunoglobulin λ light chain sequences as described herein.

In some embodiments, one or more endogenous non-human immunoglobulin λ light chain sequences (or portions thereof) of an endogenous non-human immunoglobulin λ light chain locus are not deleted. In some embodiments, one or more endogenous non-human immunoglobulin λ light chain sequences (or portions thereof) of an endogenous non-human immunoglobulin λ light chain locus are deleted. In some embodiments, one or more endogenous non-human immunoglobulin λ light chain sequences (e.g., V, J and/or C or any combination thereof) of an endogenous non-human immunoglobulin λ light chain locus is altered, displaced, disrupted, deleted or replaced so that said non-human immunoglobulin λ light chain locus is functionally silenced. In some embodiments, one or more endogenous non-human immunoglobulin λ light chain sequences (e.g., V, J and/or C or any combination thereof) of an endogenous non-human immunoglobulin λ light chain locus is altered, displaced, disrupted, deleted or replaced with a targeting vector so that said non-human immunoglobulin λ light chain locus is functionally inactivated (i.e., unable to produce a functional light chain of an antibody that is expressed and/or detectable in the antibody repertoire of a non-human animal as described herein). Guidance for inactivation of an endogenous non-human immunoglobulin λ light chain locus is provided in, e.g., U.S. Patent No. 9,006,511 (see, e.g., Figure 2), which is incorporated herein by reference in its entirety.

An engineered immunoglobulin κ light chain locus or transgene (e.g., including a limited human λ light chain variable region repertoire as described herein) or its expression product can be detected using a variety of methods including, for example, PCR, Southern blot, restriction fragment length polymorphism (RFLP), a gain or loss of allele assay, Western blot, FACS analysis, etc. In some embodiments, a non-human animal, non-human cell or non-human tissue as described herein is heterozygous with respect to an engineered immunoglobulin κ light chain locus as described herein. In some embodiments, a non-human animal, non-human cell or non-human tissue as described herein is hemizygous with respect to an engineered immunoglobulin κ light chain locus as described herein. In some embodiments, a non-human animal, non-human cell or non-human tissue as described herein contains one or more copies of an engineered immunoglobulin κ light chain locus or transgene as described herein. In some embodiments, a non-human animal, non-human cell or non-human tissue as described herein contains an engineered endogenous immunoglobulin κ light chain locus as depicted in the Drawing.

The present disclosure recognizes that a non-human animal (e.g., rodent, e.g., rat or mouse), non-human (e.g., rodent, e.g., rat or mouse) cell or non-human (e.g., rodent, e.g., rat or mouse) tissue as described herein will utilize human heavy chain, and λ light chain variable region gene segments included in its genome in its antibody selection and generation mechanisms (e.g., recombination and somatic hypermutation). As such, in various embodiments, human immunoglobulin human heavy chain and λ light chain variable domains generated by non-human animals, non-human cells or non-human tissues described herein are encoded by the human heavy and λ light chain variable region gene segments included in their genome, respectively, or somatically hypermutated variants thereof.

In some embodiments, a non-human animal (e.g., rodent, e.g., rat or mouse) is provided whose genome comprises an engineered immunoglobulin κ light chain locus, where the non-human animal includes a B cell that includes a human heavy variable region sequence and/or a human λ light chain variable region sequence, that is somatically hypermutated. In some embodiments, a human heavy chain variable region sequence and/or a human λ light chain present in a B cell of a non-human animal (e.g., rodent, e.g., rat or mouse) of the present disclosure has 1, 2, 3, 4, 5, or more somatic hypermutations. Those skilled in the art are aware of methods for identifying source gene segments in a mature antibody sequence. For example, various tools are available to aid in this analysis, such as, for example, DNAPLOT, IMGT/V-QUEST, JOINSOLVER, SoDA, and Ab-origin.

The present disclosure provides, among other things, cells and tissues from non-human animals (e.g., rodents, e.g., rats, mice) described herein. In some embodiments, provided are splenocytes (and/or other lymphoid tissue) from a non-human animal as described herein. In some embodiments, provided is a B cell from a non-human animal as described herein. In some embodiments, provided is a pro-B cell from a non-human animal as described herein. In some embodiments, provided is a pre-B cell from a non-human animal as described herein. In some embodiments, provided is an immature B cell from a non-human animal as described herein. In some embodiments, provided is a mature naïve B cell from a non-human animal as described herein. In some embodiments, provided is an activated B cell from a non-human animal as described herein. In some embodiments, provided is a memory B cell from a non-human animal as described herein. In some embodiments, provided is a B lineage lymphocyte from a non-human animal as described herein. In some embodiments, provided is plasma or a plasma cell from a non-human animal as described herein. In some embodiments, provided is a stem cell from a non-human animal as described herein. In some embodiments, a stem cell is an embryonic stem cell. In some embodiments, provided is a germ cell from a non-human animal as described herein. In some embodiments, a germ cell is an oocyte. In some embodiments, a germ cell is a sperm cell. In some embodiments, a sperm cell from a non-human animal as described herein expresses one or more ADAM6 polypeptides, functional orthologs, functional homologs, or functional fragments thereof. In some embodiments, any cell or tissue from a non-human animal as described herein may be isolated. In some embodiments, provided is an isolated cell and/or an isolated tissue from a non-human animal as described herein. In some embodiments, a hybridoma is provided, wherein the hybridoma is made with a B cell of a non-human animal as described herein. In some embodiments, a hybridoma is made with a B cell of a non-human animal that has been immunized with an antigen of interest. In some embodiments, a hybridoma is made with a B cell of a non-human animal that expresses an antibody that binds (e.g., specifically binds) to an epitope on an antigen of interest.

Any non-human animals (e.g., rodents, e.g., rats or mice) as described herein may be immunized with one or more antigens of interest under conditions and for a time sufficient that the non-human animal develops an immune response to said one or more antigens of interest. Those skilled in the art are aware of methods for immunizing non-human animals. An exemplary, non-limiting method for immunizing non-human animals can be found in US Patent No. 7,582, 298, which is incorporated herein by reference in its entirety.

The present disclosure provides, among other things, immunized non-human animals (e.g., rodents, e.g., rats or mice) as described herein, and cells and tissues isolated from the same. In some embodiments, a non-human animal described herein produces a population of B cells in response to immunization with an antigen that includes one or more epitopes. In some embodiments, a non-human animal produces a population of B cells that express antibodies that bind (e.g., specifically bind) to one or more epitopes of antigen of interest. In some embodiments, antibodies expressed by a population of B cells produced in response to an antigen include a heavy chain having a human heavy chain variable domain encoded by a human heavy chain variable region sequence and/or a lambda light chain having a human lambda light chain variable domain encoded by a human lambda light chain variable region sequence as described herein. In some embodiments, antibodies expressed by a population of B cells produced in response to an antigen include (i) a heavy chain having a human heavy chain variable domain encoded by a human heavy chain variable region sequence, and (ii) a lambda light chain having a human lambda light chain variable domain encoded by a human lambda light chain variable region sequence as described herein.

In some embodiments, a non-human animal (e.g., rodents, e.g., rats or mice) produces a population of B cells that express antibodies that bind to one or more epitopes of antigen of interest, where antibodies expressed by the population of B cells produced in response to an antigen include: (i) a heavy chain having a human heavy chain variable domain encoded by a human heavy chain variable region sequence, and (ii) a lambda light chain having a human lambda light chain variable domain encoded by a human lambda light chain variable region sequence as described herein. In some embodiments, a human heavy chain variable region sequence and/or a human λ light chain variable region sequence as described herein is somatically hypermutated. In some embodiments, at least about 5%, 10%, 15%, 20%, 25%, 30%, 35%, 40%, 45%, 50%, 55%, 60%, 65%, 70%, 75%, 80%, 85%, 90% of the B cells in a population of B cells produced in response to an antigen include a human heavy chain variable region sequence and/or a human λ light chain variable region sequence that is somatically hypermutated.

### Specific Exemplary Embodiments - Immunoglobulin κ Light Chain Loci

In some embodiments, provided non-human animals (e.g., rodents, e.g., rats or mice) comprise an engineered endogenous immunoglobulin κ light chain locus comprising a single rearranged human immunoglobulin λ light chain variable region and inserted upstream of, and operably linked to, a non-human or human Cλ gene segment, which non-human or human Cλ gene segment is inserted in the place of a non-human Cκ gene. As described herein, such engineered endogenous immunoglobulin κ light chain locus further includes non-human immunoglobulin κ light chain enhancer regions (or enhancer sequences). In some embodiments, an engineered endogenous immunoglobulin κ light chain locus (or allele) comprises a single rearranged human immunoglobulin λ light chain variable region that includes a human Vλ gene segment that appears in cluster A of a human immunoglobulin λ light chain locus. In some embodiments, an engineered endogenous immunoglobulin κ light chain locus (or allele) comprises a single rearranged human immunoglobulin λ light chain variable region that includes a human Vλ gene segment that appears in cluster B of a human immunoglobulin λ light chain locus. In some embodiments, an engineered endogenous immunoglobulin κ light chain locus (or allele) comprises a single rearranged human immunoglobulin λ light chain variable region that includes a human Vλ gene segment that appears in cluster C of a human immunoglobulin λ light chain locus. In some embodiments, an engineered endogenous immunoglobulin κ light chain locus (or allele) comprises a single rearranged human immunoglobulin λ light chain variable region that includes a human Vλ gene segment selected from the group consisting of: Vλ4-69, Vλ8-61, Vλ4-60, Vλ6-57, Vλ10-54, Vλ5-52, Vλ1-51, Vλ9-49, Vλ1-47, Vλ7-46, Vλ5-45, Vλ1-44, Vλ7-43, Vλ1-40, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, an engineered κ light chain locus (or allele) comprises a single rearranged human immunoglobulin λ light chain variable region that includes a human Vλ gene segment selected from the group consisting of: Vλ5-52, Vλ1-51, Vλ9-49, Vλ1-47, Vλ7-46, Vλ5-45, Vλ1-44, Vλ7-43, Vλ1-40, Vλ5-39, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, an engineered κ light chain locus (or allele) comprises a single rearranged human immunoglobulin λ light chain variable region that includes a human Vλ gene segment selected from the group consisting of: Vλ5-52, Vλ1-51, Vλ9-49, Vλ1-47, Vλ7-46, Vλ5-45, Vλ1-44, Vλ7-43, Vλ1-40, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1. In some embodiments, an engineered κ light chain locus (or allele) comprises a single rearranged human immunoglobulin λ light chain variable region that includes a human Vλ gene segment selected from the group consisting of: Vλ1-51, Vλ5-45, Vλ1-44, Vλ1-40, Vλ3-21, and Vλ2-14. In some embodiments, an engineered κ light chain locus (or allele) comprises a single rearranged human immunoglobulin λ light chain variable region that includes a human Vλ gene segment selected from the group consisting of: Vλ1-51, Vλ1-40, and Vλ2-14. In some embodiments, an engineered κ light chain locus (or allele) comprises a single rearranged human immunoglobulin λ light chain variable region that includes a human Vλ gene segment selected from Vλ1-51 or Vλ2-14. In some embodiments, an engineered immunoglobulin κ light chain locus (or allele) comprises a single rearranged human immunoglobulin λ light chain variable region that includes a human Jλ gene segment selected from the group consisting of: Jλ1, Jλ2, Jλ3, Jλ6, and Jλ7. In some embodiments, an engineered immunoglobulin κ light chain locus (or allele) comprises a single rearranged human immunoglobulin λ light chain variable region that includes a human Jλ gene segment selected from the group consisting of: Jλ1, Jλ2, Jλ3, and Jλ7. In some embodiments, an engineered immunoglobulin κ light chain locus (or allele) comprises a single rearranged human immunoglobulin λ light chain variable region that includes a human Jλ2 gene segment.

The present disclosure recognizes that a non-human animal (e.g., rodent, e.g., rat or mouse) as described herein will utilize human λ light chain variable region gene segments included in its genome in its antibody selection and generation mechanisms (e.g., recombination and somatic hypermutation). As such, in various embodiments, human immunoglobulin λ light chain variable domains generated by non-human animals described herein are encoded by the human λ light chain variable region gene segments included in their genome or somatically hypermutated variants thereof.

In some embodiments, a non-human animal (e.g., rodent, e.g., rat or mouse) is provided whose genome comprises an engineered endogenous immunoglobulin κ light chain locus, where the non-human animal includes a B cell that includes a human heavy variable region sequence, and/or a human λ light chain variable region sequence that is somatically hypermutated. In some embodiments, a human heavy variable region sequence, and/or a human λ light chain variable region sequence present in a B cell of a non-human animal (e.g., rodent, e.g., rat or mouse) of the present disclosure has 1, 2, 3, 4, 5, or more somatic hypermutations. Those skilled in the art are aware of methods for identifying source gene segments in a mature antibody sequence. For example, various tools are available to aid in this analysis, such as, for example, DNAPLOT, IMGT/V-QUEST, JOINSOLVER, SoDA, and Ab-origin.

In many embodiments, an engineered endogenous immunoglobulin κ light chain locus (or allele) contains non-human immunoglobulin κ light chain enhancer regions (or enhancer sequences) that appear in a wild-type immunoglobulin κ light chain locus (or allele). In some embodiments, an engineered endogenous immunoglobulin κ light chain locus (or allele) contains non-human immunoglobulin κ light chain enhancer regions (or enhancer sequences) that appear in a wild-type immunoglobulin κ light chain locus (or allele) of a different species (e.g., a different rodent species).

In some embodiments, a non-human animal (e.g., rodent, e.g., rat or mouse) as described herein comprises, in its germline genome, a limited human λ light chain variable region repertoire (e.g., a single rearranged human immunoglobulin λ light chain variable region) operably linked to one or more non-human immunoglobulin κ light chain enhancers (i.e., enhancer sequences or enhancer regions). In some certain embodiments, said limited human λ light chain variable region repertoire (e.g., a single rearranged human immunoglobulin λ light chain variable region) is operably linked to a murine immunoglobulin κ light chain intronic enhancer region (Igκ Ei or Eiκ). In some certain embodiments, said limited human λ light chain variable region repertoire (e.g., a single rearranged human immunoglobulin λ light chain variable region) is operably linked to a murine immunoglobulin κ light chain 3' enhancer region (Igκ 3'E or 3'Eκ). In some certain embodiments, said limited human λ light chain variable region repertoire (e.g., a single rearranged human immunoglobulin λ light chain variable region) is operably linked to a murine Eiκ and operably linked to a murine 3'Eκ.

In some embodiments, a non-human Cλ gene of an engineered endogenous immunoglobulin κ light chain locus (or allele) is a rodent Cλ gene such as, for example, a mouse Cλ gene or a rat Cλ gene. In some certain embodiments, a non-human Cλ gene of an engineered immunoglobulin κ light chain locus (or allele) is or comprises a mouse Cλ gene from a genetic background that includes a 129 strain, a BALB/c strain, a C57BL/6 strain, a mixed 129xC57BL/6 strain or combinations thereof.

In some embodiments, a non-human Cλ gene of an engineered immunoglobulin κ light chain locus (or allele) as described herein comprises a sequence that is at least 80%, at least 85%, at least 90%, at least 95%, or at least 98% identical to SEQ ID NO:1 (mouse Cλ1), SEQ ID NO:2 (mouse Cλ2) or SEQ ID NO:3 (mouse Cλ3). In some embodiments, a non-human Cλ gene of an engineered immunoglobulin κ light chain locus (or allele) as described herein comprises a sequence that is substantially identical or identical to SEQ ID NO:1 (mouse Cλ1), SEQ ID NO:2 (mouse Cλ2) or SEQ ID NO:3 (mouse Cλ3). In some embodiments, a non-human Cλ gene of an engineered immunoglobulin κ light chain locus (or allele) as described herein is or comprises the sequence of a mouse Cλ1 gene.

In some embodiments, a non-human Cλ domain encoded by a sequence positioned at an engineered immunoglobulin κ light chain locus (or allele) as described herein comprises a sequence that is at least 80%, at least 85%, at least 90%, at least 95%, or at least 98% identical to SEQ ID NO:4 (mouse Cλ1), SEQ ID NO:5 (mouse Cλ2) or SEQ ID NO:6 (mouse Cλ3). In some embodiments, a non-human Cλ domain encoded by a sequence positioned at an engineered immunoglobulin κ light chain locus (or allele) as described herein comprises a sequence that is substantially identical or identical to SEQ ID NO:4 (mouse Cλ1), SEQ ID NO:5 (mouse Cλ2) or SEQ ID NO:6 (mouse Cλ3). In some embodiments, a non-human Cλ gene encoded by a sequence positioned at an engineered immunoglobulin κ light chain locus (or allele) as described herein is or comprises a mouse Cλ1 domain polypeptide.

In some embodiments, a non-human Cλ gene of an engineered immunoglobulin κ light chain locus (or allele) as described herein comprises a sequence that is at least 80%, at least 85%, at least 90%, at least 95%, or at least 98% identical to SEQ ID NO:7 (rat Cλ1), SEQ ID NO:8 (rat Cλ2), SEQ ID NO:9 (rat Cλ3) or SEQ ID NO: 10 (rat Cλ4). In some certain embodiments, a non-human Cλ gene of an engineered immunoglobulin κ light chain locus (or allele) as described herein comprises a sequence that is substantially identical or identical to SEQ ID NO:7 (rat Cλ1), SEQ ID NO:8 (rat Cλ2), SEQ ID NO:9 (rat Cλ3) or SEQ ID NO: 10 (rat Cλ4). In some certain embodiments, a non-human Cλ gene of an engineered immunoglobulin κ light chain locus (or allele) as described herein is or comprises the sequence of a rat Cλ1 gene.

In some embodiments, a non-human Cλ domain encoded by a sequence positioned at an engineered immunoglobulin κ light chain locus (or allele) as described herein comprises a sequence that is at least 80%, at least 85%, at least 90%, at least 95%, or at least 98% identical to SEQ ID NO: 11 (rat Cλ1), SEQ ID NO: 12 (rat Cλ2), SEQ ID NO: 13 (rat Cλ3) or SEQ ID NO: 14 (rat Cλ4). In some embodiments, a non-human Cλ domain encoded by a sequence positioned at an engineered immunoglobulin κ light chain locus (or allele) as described herein comprises a sequence that is substantially identical or identical to SEQ ID NO:11 (rat Cλ1), SEQ ID NO: 12 (rat Cλ2), SEQ ID NO: 13 (rat Cλ3) or SEQ ID NO: 14 (rat Cλ4). In some embodiments, a non-human Cλ domain encoded by a sequence positioned at an engineered immunoglobulin κ light chain locus (or allele) as described herein is or comprises a rat Cλ1 domain polypeptide.

In some embodiments, a human Cλ gene of an engineered immunoglobulin κ light chain locus (or allele) includes a human Cλ gene such as, for example, a human Cλ1 gene, a human Cλ2 gene, a human Cλ3 gene, a human Cλ6 gene or a human Cλ7 gene. In some certain embodiments, a human Cλ gene of an engineered immunoglobulin κ light chain locus (or allele) is or comprises a human Cλ2 gene.

In some embodiments, a human Cλ gene of an engineered immunoglobulin κ light chain locus (or allele) as described herein comprises a sequence that is at least 80%, at least 85%, at least 90%, at least 95%, or at least 98% identical to SEQ ID NO: 15 (human Cλ1), SEQ ID NO:16 (human Cλ2), SEQ ID NO: 17 (human Cλ3), SEQ ID NO:18 (human Cλ6) or SEQ ID NO: 19 (human Cλ7). In some embodiments, a human Cλ gene of an engineered immunoglobulin κ light chain locus (or allele) as described herein comprises a sequence that is substantially identical or identical to SEQ ID NO:15 (human Cλ1), SEQ ID NO:16 (human Cλ2), SEQ ID NO:17 (human Cλ3), SEQ ID NO:18 (human Cλ6) or SEQ ID NO:19 (human Cλ7). In some embodiments, a human Cλ gene of an engineered immunoglobulin κ light chain locus (or allele) as described herein is or comprises the sequence of a human Cλ2 gene.

In some embodiments, a human Cλ domain encoded by a sequence positioned at an engineered immunoglobulin κ light chain locus (or allele) as described herein comprises a sequence that is at least 80%, at least 85%, at least 90%, at least 95%, or at least 98% identical to SEQ ID NO:20 (human Cλ1), SEQ ID NO:21 (human Cλ2), SEQ ID NO:22 (human Cλ3), SEQ ID NO:23 (human Cλ6) or SEQ ID NO:24 (human Cλ7). In some embodiments, a human Cλ domain encoded by a sequence positioned at an engineered immunoglobulin κ light chain locus (or allele) as described herein comprises a sequence that is substantially identical or identical to SEQ ID NO:20 (human Cλ1), SEQ ID NO:21 (human Cλ2), SEQ ID NO:22 (human Cλ3), SEQ ID NO:23 (human Cλ6) or SEQ ID NO:24 (human Cλ7). In some embodiments, a human Cλ domain encoded by a sequence positioned at an engineered immunoglobulin κ light chain locus (or allele) as described herein is or comprises a human Cλ2 domain polypeptide.

### Specific Exemplary Embodiments - Immunoglobulin Heavy Chain Loci

In some embodiments, provided non-human animals (e.g., rodents, e.g., rats or mice) comprise an engineered endogenous immunoglobulin κ light chain locus including a limited human immunoglobulin λ light chain variable region repertoire (e.g., a single rearranged human immunoglobulin λ light chain variable region) as described herein and further comprise engineered immunoglobulin heavy chain loci (or alleles) characterized by the presence of a plurality of human V_{H}, D_{H} and J_{H} gene segments arranged in germline configuration and operably linked to non-human (e.g., rodent, e.g., rat or mouse) immunoglobulin heavy chain constant region genes, enhancers and regulatory regions. In some embodiments, an engineered immunoglobulin heavy chain locus (or allele) as described herein comprises one or more human V_{H} gene segments, one or more human D_{H} gene segments and one or more human J_{H} gene segments operably linked to a non-human immunoglobulin heavy chain constant region. In some certain embodiments, an engineered immunoglobulin heavy chain locus (or allele) comprises at least human V_{H} gene segment V_{H}3-74, V_{H}3-73, V_{H}3-72, V_{H}2-70, V_{H}1-69, V_{H}3-66, V_{H}3-64, V_{H}4-61, V_{H}4-59, V_{H}1-58, V_{H}3-53, V_{H}5-51, V_{H}3-49, V_{H}3-48, V_{H}1-46, V_{H}1-45, V_{H}3-43, V_{H}4-39, V_{H}4-34, V_{H}3-33, V_{H}4-31, V_{H}3-30, V_{H}4-28, V_{H}2-26, V_{H}1-24, V_{H}3-23, V_{H}3-21, V_{H}3-20, V_{H}1-18, V_{H}3-15, V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2, V_{H}6-1, or any combination thereof. In some certain embodiments, an engineered immunoglobulin heavy chain locus (or allele) comprises at least human D_{H} gene segment D_{H}1-1, D_{H}2-2, D_{H}3-3, D_{H}4-4, D_{H}5-5, D_{H}6-6, D_{H}1-7, D_{H}2-8, D_{H}3-9, D_{H}3-10, D_{H}5-12, D_{H}6-13, D_{H}2-15, D_{H}3-16, D_{H}4-17, D_{H}6-19, D_{H}1-20, D_{H}2-21, D_{H}3-22, D_{H}6-25, D_{H}1-26, D_{H}7-27, or any combination thereof. In some certain embodiments, an engineered immunoglobulin heavy chain locus (or allele) comprises at least human J_{H} gene segment J_{H}1, J_{H}2, J_{H}3, J_{H}4, J_{H}5, J_{H}6, or any combination thereof.

The present disclosure recognizes that a non-human animal (e.g., rodent, e.g., rat or mouse) as described herein will utilize human heavy chain variable region gene segments comprised in its genome in its antibody selection and generation mechanisms (e.g., recombination and somatic hypermutation). As such, in various embodiments, human immunoglobulin heavy chain variable domains generated by non-human animals described herein are encoded by the human heavy chain variable region gene segments included in their genome or somatically hypermutated variants thereof.

In some embodiments, a non-human animal (e.g., rodent, e.g., rat or mouse) includes a B cell that includes a human heavy variable region sequence, and/or a human λ light chain variable region sequence that are somatically hypermutated. In some embodiments, a human heavy variable region sequence, and/or a human λ light chain variable region sequence present in a B cell of a non-human animal (e.g., rodent, e.g., rat or mouse) of the present disclosure have 1, 2, 3, 4, 5, or more somatic hypermutations. Those skilled in the art are aware of methods for identifying source gene segments in a mature antibody sequence. For example, various tools are available to aid in this analysis, such as, for example, DNAPLOT, IMGT/V-QUEST, JOINSOLVER, SoDA, and Ab-origin.

In some embodiments, a non-human immunoglobulin heavy chain constant region includes one or more non-human immunoglobulin heavy chain constant region genes such as, for example, immunoglobulin M (IgM), immunoglobulin D (IgD), immunoglobulin G (IgG), immunoglobulin E (IgE) and immunoglobulin A (IgA). In some certain embodiments, a non-human immunoglobulin heavy chain constant region includes a rodent IgM, rodent IgD, rodent IgG3, rodent IgG1, rodent IgG2b, rodent IgG2a, rodent IgE and rodent IgA constant region genes. In some embodiments, said human V_{H}, D_{H} and J_{H} gene segments are operably linked to one or more non-human immunoglobulin heavy chain enhancers (i.e., enhancer sequences or enhancer regions). In some embodiments, said human V_{H}, D_{H} and J_{H} gene segments are operably linked to one or more non-human immunoglobulin heavy chain regulatory regions (or regulatory sequences). In some embodiments, said human V_{H}, D_{H} and J_{H} gene segments are operably linked to one or more non-human immunoglobulin heavy chain enhancers (or enhancer sequence) and one or more non-human immunoglobulin heavy chain regulatory regions (or regulatory sequence).

In some embodiments, an engineered immunoglobulin heavy chain locus as described herein does not contain an endogenous Adam6 gene. In some embodiments, an engineered immunoglobulin heavy chain locus as described herein does not contain an endogenous Adam6 gene (or Adam6-encoding sequence) in the same germline genomic position as found in a germline genome of a wild-type non-human animal of the same species. In some embodiments, an engineered immunoglobulin heavy chain locus as described herein does not contain a human Adam6 pseudogene. In some embodiments, an engineered immunoglobulin heavy chain locus as described herein comprises insertion of at least one nucleotide sequence that encodes one or more non-human (e.g., rodent) Adam6 polypeptides, functional orthologs, functional homologs, or functional fragments thereof. In some embodiments, said insertion may be outside of an engineered immunoglobulin heavy chain locus as described herein (e.g., but not limited to, upstream of a 5' most V_{H} gene segment), within an engineered immunoglobulin heavy chain locus or elsewhere in the germline genome of a non-human animal (e.g., but not limited to, a randomly introduced non-human Adam6-encoding sequence), cell or tissue.

**In** various embodiments, a provided non-human animal (e.g., rodent, e.g., rat or mouse) as described herein does not detectably express, in whole or in part, an endogenous non-human V_{H} region in an antibody molecule. **In** various embodiments, a provided non-human animal as described herein does not contain (or lacks, or contains a deletion of) one or more nucleotide sequences that encode, in whole or in part, an endogenous non-human V_{H} region (e.g., V_{H}, D_{H} and/or J_{H}) in an antibody molecule. **In** various embodiments, a provided non-human animal as described herein has a germline genome that includes a deletion of endogenous non-human V_{H}, D_{H} and J_{H} gene segments, in whole or in part. **In** various embodiments, a provided non-human animal is fertile.

Guidance for the creation of targeting vectors, non-human (e.g., rodent, e.g., rat or mouse) cells and animals harboring such engineered immunoglobulin heavy chain loci (or alleles) can be found in Macdonald (2014), U.S. Patent Nos. 6,596,541, 8,642,835, 8,697,940 and 8,791,323, each of which is incorporated herein by reference in its entirety. Persons skilled in the art are aware of a variety of technologies, known in the art, for accomplishing such genetic engineering and/or manipulation of non-human (e.g., mammalian) genomes or for otherwise preparing, providing, or manufacturing such sequences for introducing into the germline genome of non-human animals.

### Specific Exemplary Embodiments - Combinations of Immunoglobulin Loci

In some embodiments, a non-human animal (e.g., rodent, e.g., rat or mouse) as provided herein comprises in its germline genome an engineered endogenous immunoglobulin κ light chain locus including a limited human immunoglobulin λ light chain variable region repertoire (e.g., a single rearranged human immunoglobulin λ light chain variable region) as described herein and further comprises one or more additional immunoglobulin loci including human immunoglobulin gene segments or other human or humanized genes (e.g., a human gene encoding TdT) (e.g., via cross-breeding or multiple gene targeting strategies). Such a non-human animal may be prepared as described above, or using methods known in the art, to achieve a desired engineered genotype depending on the intended use of the non-human animal. Additional human immunoglobulin gene segments at other immunoglobulin loci or other human or humanized genes (e.g., a human gene encoding TdT) may be introduced through the further alteration of the genome of cells (e.g., embryonic stem cells) having the genetic modifications as described above or through breeding techniques known in the art with other genetically modified strains as desired.

In some embodiments, a non-human animal (e.g., rodent, e.g., rat or mouse) as provided herein comprises in its germline genome an engineered endogenous immunoglobulin κ light chain locus including a limited human immunoglobulin λ light chain variable region repertoire (e.g., a single rearranged human immunoglobulin λ light chain variable region) as described herein and further comprises in it germline genome an engineered endogenous immunoglobulin heavy chain locus comprising one or more human V_{H} gene segments, one or more human D gene segments, and one or more human J_{H} gene segments operably linked to one or more non-human animal immunoglobulin heavy chain constant region genes. In some embodiments, a non-human animal is heterozygous or homozygous for an engineered endogenous immunoglobulin κ light chain locus including a limited human immunoglobulin λ light chain variable region repertoire (e.g., a single rearranged human immunoglobulin λ light chain variable region) as described herein. In some embodiments, a non-human animal is heterozygous or homozygous for an engineered endogenous immunoglobulin heavy chain locus as described herein. In some embodiments, a non-human animal is homozygous for an engineered endogenous immunoglobulin κ light chain locus including a limited human immunoglobulin λ light chain variable region repertoire (e.g., a single rearranged human immunoglobulin λ light chain variable region) as described herein and is homozygous for an engineered endogenous immunoglobulin heavy chain locus as described herein.

In some embodiments, a non-human animal (e.g., rodent, e.g., rat or mouse) as provided herein comprises in its germline genome an engineered endogenous immunoglobulin κ light chain locus including a limited human immunoglobulin λ light chain variable region repertoire (e.g., a single rearranged human immunoglobulin λ light chain variable region) as described herein, an engineered endogenous immunoglobulin heavy chain locus comprising one or more human V_{H} gene segments, one or more human D gene segments, and one or more human J_{H} gene segments operably linked to one or more non-human animal immunoglobulin heavy chain constant region genes, and further comprises a functionally inactivated (e.g., deleted in whole or in part, or otherwise rendered non-functional) endogenous immunoglobulin λ light chain locus. In some embodiments, a non-human animal is heterozygous or homozygous for an engineered endogenous immunoglobulin κ light chain locus including a limited human immunoglobulin λ light chain variable region repertoire (e.g., a single rearranged human immunoglobulin λ light chain variable region) as described herein. **In** some embodiments, a non-human animal is heterozygous or homozygous for an engineered endogenous immunoglobulin heavy chain locus as described herein. **In** some embodiments, a non-human animal is heterozygous or homozygous for a functionally inactivated (e.g., deleted in whole or in part, or otherwise rendered non-functional) endogenous immunoglobulin λ light chain locus. **In** some embodiments, a non-human animal is homozygous for an engineered endogenous immunoglobulin κ light chain locus including a limited human immunoglobulin λ light chain variable region repertoire (e.g., a single rearranged human immunoglobulin λ light chain variable region) as described herein, homozygous for an engineered endogenous immunoglobulin heavy chain locus as described herein, and homozygous for a functionally inactivated (e.g., deleted in whole or in part, or otherwise rendered non-functional) endogenous immunoglobulin λ light chain locus.

In some embodiments, a germline genome of a genetically modified non-human animal, which is a rodent (e.g., a mouse or a rat), comprises an engineered endogenous immunoglobulin κ locus comprising two alleles. In some embodiments, a first allele comprises a limited human λ light chain variable region repertoire and a second allele comprises a limited human κ light chain variable region repertoire. In some embodiments, a germline genome of a genetically modified rodent (e.g., a mouse or a rat), a rodent (e.g., mouse or rat) cell or a rodent (e.g., a mouse or a rat) tissue, as described herein, comprises a first engineered endogenous immunoglobulin κ light chain locus allele comprising a single rearranged human immunoglobulin λ light chain variable region operably linked to a rodent (e.g., mouse or rat) Cλ gene segment, wherein the single rearranged human immunoglobulin λ light chain variable region comprises a human Vλ gene segment and a human Jλ gene segment. In some embodiments, a genetically modified rodent (e.g., mouse or rat), and thus a rodent (e.g., mouse or rat) cell or a rodent (e.g., a mouse or a rat) tissue, as described herein, comprises a second engineered endogenous immunoglobulin κ light chain locus allele comprising a single rearranged human immunoglobulin κ light chain variable region operably linked to a rodent (e.g., mouse or rat) Cκ gene segment, wherein the single rearranged human immunoglobulin κ light chain variable region comprises a human Vκ gene segment and a human Jκ gene segment. In some embodiments, such a rodent (e.g., mouse or rat) tissue can express a λ light chain from the first engineered endogenous immunoglobulin κ light chain locus allele and a κ light chain from the second engineered endogenous immunoglobulin κ light chain locus allele. In some embodiments, the single rearranged human immunoglobulin κ light chain variable region comprises Vκ3-20 or Vκ1-39 and the single rearranged human immunoglobulin λ light chain variable region comprises Vλ1-51 or Vλ2-14. In one embodiment, the single rearranged human immunoglobulin κ light chain variable region is Vκ3-20/Jκ1, and the single rearranged human immunoglobulin λ light chain variable region is Vλ1-51/Jλ2 or Vλ2-14/Jλ2.

In some embodiments, provided non-human animals (e.g., rodents, e.g., rats or mice) have a germline genome comprising (a) a homozygous or heterozygous immunoglobulin heavy chain locus comprising human V_{H}, D_{H} and J_{H} gene segments operably linked to one or more endogenous non-human immunoglobulin heavy chain constant regions such that the non-human animal expresses an immunoglobulin heavy chain that comprises a human V_{H} domain sequence fused with a non-human C_{H} domain sequence; (b) an immunoglobulin κ light chain locus comprising a single rearranged human immunoglobulin λ light chain operably linked to a non-human (e.g., rodent) immunoglobulin Cλ gene segment such that the non-human animal expresses an immunoglobulin light chain that comprises a human Vλ domain sequence fused with a non-human Cλ domain sequence.

In some embodiments, provided non-human animals (e.g., rodents, e.g., rats or mice) have a germline genome comprising (a) a homozygous or heterozygous immunoglobulin heavy chain locus comprising human V_{H}, D_{H} and J_{H} gene segments operably linked to one or more endogenous non-human immunoglobulin heavy chain constant regions such that the non-human animal expresses an immunoglobulin heavy chain that comprises a human V_{H} domain sequence fused with a non-human C_{H} domain sequence; (b) an immunoglobulin κ light chain locus comprising a single rearranged human immunoglobulin λ light chain operably linked to a non-human (e.g., rodent) immunoglobulin Cλ gene segment such that the non-human animal expresses an immunoglobulin light chain that comprises a human Vλ domain sequence fused with a non-human Cλ domain sequence; and (c) a homozygous or heterozygous functionally inactivated or deleted, in whole or in part, endogenous immunoglobulin λ light chain locus.

For example, as described herein, non-human animals comprising an engineered endogenous immunoglobulin κ light chain locus as described herein may further comprise (e.g., via cross-breeding or multiple gene targeting strategies) one or more modifications as described in U.S. Patent Nos. 8,642,835, 8,697,940, 9,006,511, 9,035,128, 9,066,502, 9,150,662 and 9,163,092; each of which is incorporated by reference in its entirety.

### Nucleic Acid Constructs

Typically, a polynucleotide molecule containing human immunoglobulin λ light chain sequences (e.g., a single rearranged human λ light chain variable region, or one or two unrearranged Vλ gene segments with at least one unrearranged Jλ gene segment), or portion(s) thereof, is linked with (e.g., is inserted into) a vector, preferably a DNA vector, in order to replicate the polynucleotide molecule in a host cell.

Human immunoglobulin λ light chain sequences can be cloned directly from known sequences or sources (e.g., libraries) or synthesized from germline sequences designed *in silico* based on published sequences available from GenBank or other publically available databases (e.g., IMGT). Alternatively, bacterial artificial chromosome (BAC) libraries can provide immunoglobulin DNA sequences of interest (e.g., human Vλ and Jλ sequences and combinations thereof). BAC libraries can contain an insert size of 100-150kb and are capable of harboring inserts as large as 300kb (Shizuya, et al., 1992, Proc. Natl. Acad. Sci., USA 89:8794-8797; Swiatek, et al., 1993, Genes and Development 7:2071-2084; Kim, et al., 1996, Genomics 34 213-218; incorporated herein by reference in their entireties). For example, a human BAC library harboring average insert sizes of 164-196kb has been described (Osoegawa, K. et al., 2001, Genome Res. 11(3):483-96; Osoegawa, K. et al., 1998, Genomics 52:1-8, Article No. GE985423, each of which is incorporated herein by reference in its entirety). Human and non-human animal genomic BAC libraries have been constructed and are commercially available (e.g., ThermoFisher). Genomic BAC libraries can also serve as a source of immunoglobulin DNA sequences as well as transcriptional control regions.

Alternatively, immunoglobulin DNA sequences may be isolated, cloned and/or transferred from yeast artificial chromosomes (YACs). For example, the nucleotide sequence of the human immunoglobulin λ light chain locus has been determined (see, e.g., Dunham, I. et al., 1999, Nature 402:489-95, which is incorporated herein by reference in its entirety). Further, YACs have previously been employed to assemble a human immunoglobulin λ light chain locus transgene (see, e.g., Popov, A.V. et al., 1996, Gene 177:195-201; Popov, A.V. et al., 1999, J. Exp. Med. 189(10):1611-19, each of which is incorporated herein by reference in its entirety). An entire immunoglobulin λ light chain locus (human or non-human) can be cloned and contained within several YACs. Regardless of the sequences included, if multiple YACs are employed and contain regions of overlapping similarity, they can be recombined within yeast host strains to produce a single construct representing the entire locus or desired portions of the locus (e.g., a region targeted with a targeting vector). YAC arms can be additionally modified with mammalian selection cassettes by retrofitting to assist in introducing the constructs into embryonic stems cells or embryos by methods known in the art and/or described herein.

DNA and amino acid sequences of human immunoglobulin λ light chain gene segments for use in constructing an engineered immunoglobulin κ light chain locus as described herein may be obtained from published databases (e.g., GenBank, IMGT, etc.) and/or published antibody sequences.

In some certain embodiments, nucleic acid constructs containing human immunoglobulin λ light chain gene segments (e.g., a single rearranged human λ light chain variable region, or one or two unrearranged Vλ gene segments with at least one unrearranged Jλ gene segment) are operably linked to a human or non-human (e.g., rodent, e.g., rat or mouse) immunoglobulin λ or immunoglobulin κ light chain constant region (Cλ or Cκ, respectively) gene. In some certain embodiments, nucleic acid constructs containing human immunoglobulin λ light chain gene segments (e.g., a single rearranged human λ light chain variable region, or one or two unrearranged Vλ gene segments with at least one unrearranged Jλ gene segment) are operably linked to one or more non-human (e.g., rodent, e.g., rat or mouse) immunoglobulin κ or immunoglobulin λ light chain enhancer regions (or enhancer sequences). In some embodiments, nucleic acid constructs containing human immunoglobulin λ light chain gene segments (e.g., a single rearranged human λ light chain variable region, or one or two unrearranged Vλ gene segments with at least one unrearranged Jλ gene segment) are operably linked to a non-human (e.g., rodent, e.g., rat or mouse) or human Cλ region gene and non-human immunoglobulin κ light chain enhancer regions (or enhancer sequences).

In some embodiments, nucleic acid constructs containing unrearranged human Vλ and Jλ sequences further comprise intergenic DNA that is of human and/or murine origin. In some embodiments, intergenic DNA is or comprises non-coding murine immunoglobulin κ light chain sequence, non-coding human immunoglobulin κ light chain sequence, non-coding murine immunoglobulin λ light chain sequence, non-coding human immunoglobulin λ light chain sequence, or combinations thereof.

Nucleic acid constructs can be prepared using methods known in the art. For example, a nucleic acid construct can be prepared as part of a larger plasmid. Such preparation allows the cloning and selection of the correct constructions in an efficient manner as is known in the art. Nucleic acid constructs containing human immunoglobulin λ light chain sequences, in whole or in part, as described herein can be located between restriction sites on the plasmid so that they can be isolated from the remaining plasmid sequences for incorporation into a desired non-human animal (e.g., rodent, e.g., rat or mouse).

Various methods employed in preparation of nucleic acid constructs (e.g., plasmids) and transformation of host organisms are known in the art. For other suitable expression systems for both prokaryotic and eukaryotic cells, as well as general recombinant procedures, see Principles of Gene Manipulation: An Introduction to Genetic Manipulation, 5th Ed., ed. By Old, R.W. and S.B. Primrose, Blackwell Science, Inc., 1994 and Molecular Cloning: A Laboratory Manual, 2nd Ed., ed. by Sambrook, J. et al., Cold Spring Harbor Laboratory Press: 1989, each of which is incorporated herein by reference in its entirety.

### Targeting Vectors

Targeting vectors can be employed to introduce a nucleic acid construct into a target genomic locus. Targeting vectors can comprise a nucleic acid construct and homology arms that flank said nucleic acid construct; those skilled in the art will be aware of a variety of options and features generally applicable to the design, structure, and/or use of targeting vectors. For example, targeting vectors can be in linear form or in circular form, and they can be single-stranded or double-stranded. Targeting vectors can be deoxyribonucleic acid (DNA) or ribonucleic acid (RNA). For ease of reference, homology arms are referred to herein as 5' and 3' (i.e., upstream and downstream) homology arms. This terminology relates to the relative position of the homology arms to a nucleic acid construct within a targeting vector. 5' and 3' homology arms correspond to regions within a targeted locus or to a region within another targeting vector, which are referred to herein as "5' target sequence" and "3' target sequence," respectively. In some embodiments, homology arms can also function as a 5' or a 3' target sequence.

In some embodiments, methods described herein employ two, three or more targeting vectors that are capable of recombining with each other. In various embodiments, targeting vectors are large targeting vectors (LTVEC), as described elsewhere herein. In some embodiments, first, second, and third targeting vectors each comprise a 5' and a 3' homology arm. The 3' homology arm of the first targeting vector comprises a sequence that overlaps with the 5' homology arm of the second targeting vector (i.e., overlapping sequences), which allows for homologous recombination between first and second LTVECs.

In the case of double targeting methods, a 5' homology arm of a first targeting vector and a 3' homology arm of a second targeting vector can be similar to corresponding segments within a target genomic locus (i.e., a target sequence), which can promote homologous recombination of the first and the second targeting vectors with corresponding genomic segments and modify the target genomic locus.

In the case of triple targeting methods, a 3' homology arm of a second targeting vector can comprise a sequence that overlaps with a 5' homology arm of a third targeting vector (i.e., overlapping sequences), which can allow for homologous recombination between the second and the third LTVEC. The 5' homology arm of the first targeting vector and the 3' homology arm of the third targeting vector are similar to corresponding segments within the target genomic locus (i.e., the target sequence), which can promote homologous recombination of the first and the third targeting vectors with the corresponding genomic segments and modify the target genomic locus.

A homology arm and a target sequence or two homology arms "correspond" or are "corresponding" to one another when the two regions share a sufficient level of sequence identity to one another so that they can act as substrates for a homologous recombination reaction. The sequence identity between a given target sequence and the corresponding homology arm found on a targeting vector (i.e., overlapping sequence) or between two homology arms can be any degree of sequence identity that allows for homologous recombination to occur. To give but one example, an amount of sequence identity shared by a homology arm of a targeting vector (or a fragment thereof) and a target sequence of another targeting vector or a target sequence of a target genomic locus (or a fragment thereof) can be, e.g., but not limited to, at least 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity, such that the sequences undergo homologous recombination.

Moreover, a corresponding region of similarity (e.g., identity) between a homology arm and a corresponding target sequence can be of any length that is sufficient to promote homologous recombination at the target genomic locus. For example, a given homology arm and/or corresponding target sequence can comprise corresponding regions of similarity that are, e.g., but not limited to, about 5-10kb, 5-15kb, 5-20kb, 5-25kb, 5-30kb, 5-35kb, 5-40kb, 5-45kb, 5-50kb, 5-55kb, 5-60kb, 5-65kb, 5-70kb, 5-75kb, 5-80kb, 5-85kb, 5-90kb, 5-95kb, 5-100kb, 100-200kb, or 200-300kb in length (such as described elsewhere herein) such that a homology arm has sufficient similarity to undergo homologous recombination with a corresponding target sequence(s) within a target genomic locus of the cell or within another targeting vector. In some embodiments, a given homology arm and/or corresponding target sequence comprise corresponding regions of similarity that are, e.g., but not limited to, about 10-100kb, 15-100kb, 20-100kb, 25-100kb, 30-100kb, 35-100kb, 40-100kb, 45-100kb, 50-100kb, 55-100kb, 60-100kb, 65-100kb, 70-100kb, 75-100kb, 80-100kb, 85-100kb, 90-100kb, or 95-100kb in length (such as described elsewhere herein) such that a homology arm has sufficient similarity to undergo homologous recombination with a corresponding target sequence(s) within a target genomic locus of the cell or within another targeting vector.

Overlapping sequences of a 3' homology arm of a first targeting vector and a 5' homology arm of a second targeting vector or of a 3' homology arm of a second targeting vector and a 5' homology arm of a third targeting vector can be of any length that is sufficient to promote homologous recombination between said targeting vectors. For example, a given overlapping sequence of a homology arm can comprise corresponding overlapping regions that are about 1-5kb, 5-10kb, 5-15kb, 5-20kb, 5-25kb, 5-30kb, 5-35kb, 5-40kb, 5-45kb, 5-50kb, 5-55kb, 5-60kb, 5-65kb, 5-70kb, 5-75kb, 5-80kb, 5-85kb, 5-90kb, 5-95kb, 5-100kb, 100-200kb, or 200-300kb in length such that an overlapping sequence of a homology arm has sufficient similarity to undergo homologous recombination with a corresponding overlapping sequence within another targeting vector. In some embodiments, a given overlapping sequence of a homology arm comprises an overlapping region that is about 1-100kb, 5-100kb, 10-100kb, 15-100kb, 20-100kb, 25-100kb, 30-100kb, 35-100kb, 40-100kb, 45-100kb, 50-100kb, 55-100kb, 60-100kb, 65-100kb, 70-100kb, 75-100kb, 80-100kb, 85-100kb, 90-100kb, or 95-100kb in length such that an overlapping sequence of a homology arm has sufficient similarity to undergo homologous recombination with a corresponding overlapping sequence within another targeting vector. In some embodiments, an overlapping sequence is from 1-5kb, inclusive. In some embodiments, an overlapping sequence is from about 1kb to about 70kb, inclusive. In some embodiments, an overlapping sequence is from about 10kb to about 70kb, inclusive. In some embodiments, an overlapping sequence is from about 10kb to about 50kb, inclusive. In some embodiments, an overlapping sequence is at least 10kb. In some embodiments, an overlapping sequence is at least 20kb. For example, an overlapping sequence can be from about 1kb to about 5kb, inclusive, from about 5kb to about 10kb, inclusive, from about 10kb to about 15kb, inclusive, from about 15kb to about 20kb, inclusive, from about 20kb to about 25kb, inclusive, from about 25kb to about 30 kb, inclusive, from about 30kb to about 35kb, inclusive, from about 35kb to about 40kb, inclusive, from about 40kb to about 45kb, inclusive, from about 45kb to about 50kb, inclusive, from about 50kb to about 60kb, inclusive, from about 60kb to about 70kb, inclusive, from about 70kb to about 80kb, inclusive, from about 80kb to about 90kb, inclusive, from about 90kb to about 100kb, inclusive, from about 100kb to about 120kb, inclusive, from about 120kb to about 140kb, inclusive, from about 140kb to about 160kb, inclusive, from about 160kb to about 180kb, inclusive, from about 180kb to about 200kb, inclusive, from about 200kb to about 220kb, inclusive, from about 220kb to about 240kb, inclusive, from about 240kb to about 260kb, inclusive, from about 260kb to about 280kb, inclusive, or about 280kb to about 300 kb, inclusive. To give but one example, an overlapping sequence can be from about 20kb to about 60kb, inclusive. Alternatively, an overlapping sequence can be at least 1kb, at least 5kb, at least 10kb, at least 15kb, at least 20kb, at least 25kb, at least 30kb, at least 35kb, at least 40kb, at least 45kb, at least 50kb, at least 60kb, at least 70kb, at least 80kb, at least 90kb, at least 100kb, at least 120kb, at least 140kb, at least 160kb, at least 180kb, at least 200kb, at least 220kb, at least 240kb, at least 260kb, at least 280kb, or at least 300kb. In some embodiments, an overlapping sequence can be at most 400kb, at most 350kb, at most 300kb, at most 280kb, at most 260kb, at most 240kb, at most 220kb, at most 200kb, at most 180kb, at most 160kb, at most 140kb, at most 120kb, at most 100kb, at most 90kb, at most 80 kb, at most 70kb, at most 60 kb or at most 50kb.

Homology arms can, in some embodiments, correspond to a locus that is native to a cell (e.g., a targeted locus), or alternatively they can correspond to a region of a heterologous or exogenous segment of DNA that was integrated into the genome of the cell, including, for example, transgenes, expression cassettes, or heterologous or exogenous regions of DNA. In some embodiments, homology arms can, in some embodiments, correspond to a region on a targeting vector in a cell. In some embodiments, homology arms of a targeting vector may correspond to a region of a yeast artificial chromosome (YAC), a bacterial artificial chromosome (BAC), a human artificial chromosome, or any other engineered region contained in an appropriate host cell. Still further, homology arms of a targeting vector may correspond to or be derived from a region of a BAC library, a cosmid library, or a P1 phage library. In some certain embodiments, homology arms of a targeting vector correspond to a locus that is native, heterologous, or exogenous to a prokaryote, a yeast, a bird (e.g., chicken), a non-human mammal, a rodent, a human, a rat, a mouse, a hamster a rabbit, a pig, a bovine, a deer, a sheep, a goat, a cat, a dog, a ferret, a primate (e.g., marmoset, rhesus monkey), a domesticated mammal, an agricultural mammal, or any other organism of interest. In some embodiments, homology arms correspond to a locus of the cell that shows limited susceptibility to targeting using a conventional method or that has shown relatively low levels of successful integration at a targeted site, and/or significant levels of off-target integration, in the absence of a nick or double-strand break induced by a nuclease agent (e.g., a Cas protein). In some embodiments, homology arms are designed to include engineered DNA.

In some embodiments, 5' and 3' homology arms of a targeting vector(s) correspond to a targeted genome. Alternatively, homology arms correspond to a related genome. For example, a targeted genome is a mouse genome of a first strain, and targeting arms correspond to a mouse genome of a second strain, wherein the first strain and the second strain are different. In certain embodiments, homology arms correspond to the genome of the same animal or are from the genome of the same strain, e.g., the targeted genome is a mouse genome of a first strain, and the targeting arms correspond to a mouse genome from the same mouse or from the same strain.

A homology arm of a targeting vector can be of any length that is sufficient to promote a homologous recombination event with a corresponding target sequence, including, for example, 1-5kb, inclusive, 5-10kb, inclusive, 5-15kb, inclusive, 5-20kb, inclusive, 5-25kb, inclusive, 5-30kb, inclusive, 5-35kb, inclusive, 5-40kb, inclusive, 5-45kb, inclusive, 5-50kb, inclusive, 5-55kb, inclusive, 5-60kb, inclusive, 5-65kb, inclusive, 5-70kb, inclusive, 5-75kb, inclusive, 5-80kb, inclusive, 5-85kb, inclusive, 5-90kb, inclusive, 5-95kb, inclusive, 5-100kb, inclusive, 100-200kb, inclusive, or 200-300kb, inclusive, in length. In some embodiments, a homology arm of a targeting vector has a length that is sufficient to promote a homologous recombination event with a corresponding target sequence that is 1-100kb, inclusive, 5-100kb, inclusive, 10-100kb, inclusive, 15-100kb, inclusive, 20-100kb, inclusive, 25-100kb, inclusive, 30-100kb, inclusive, 35-100kb, inclusive, 40-100kb, inclusive, 45-100kb, inclusive, 50-100kb, inclusive, 55-100kb, inclusive, 60-100kb, inclusive, 65-100kb, inclusive, 70-100kb, inclusive, 75-100kb, inclusive, 80-100kb, inclusive, 85-100kb, inclusive, 90-100kb, inclusive, or 95-100kb, inclusive, in length. As described herein, large targeting vectors can employ targeting arms of greater length.

Nuclease agents (e.g., CRISPR/Cas systems) can be employed in combination with targeting vectors to facilitate the modification of a target locus (e.g., modification of an immunoglobulin κ light chain locus, or modification of a previously modified or engineered immunoglobulin κ light chain locus). Such nuclease agents may promote homologous recombination between a targeting vector and a target locus. When nuclease agents are employed in combination with a targeting vector, the targeting vector can comprise 5' and 3' homology arms corresponding to 5' and 3' target sequences located in sufficient proximity to a nuclease cleavage site so as to promote the occurrence of a homologous recombination event between target sequences and homology arms upon a nick or double-strand break at the nuclease cleavage site. The term "nuclease cleavage site" includes a DNA sequence at which a nick or double-strand break is created by a nuclease agent (e.g., a Cas9 cleavage site). Target sequences within a targeted locus that correspond to 5' and 3' homology arms of a targeting vector are "located in sufficient proximity" to a nuclease cleavage site if the distance is such as to promote the occurrence of a homologous recombination event between 5' and 3' target sequences and homology arms upon a nick or double-strand break at the recognition site. Thus, in certain embodiments, target sequences corresponding to 5' and/or 3' homology arms of a targeting vector are within at least one nucleotide of a given recognition site or are within at least 10 nucleotides to about 14kb of a given recognition site. In some embodiments, a nuclease cleavage site is immediately adjacent to at least one or both of the target sequences.

The spatial relationship of target sequences that correspond to homology arms of a targeting vector and a nuclease cleavage site can vary. For example, target sequences can be located 5' to a nuclease cleavage site, target sequences can be located 3' to a recognition site, or target sequences can flank a nuclease cleavage site.

Combined use of a targeting vector (including, for example, a large targeting vector) with a nuclease agent can result in an increased targeting efficiency compared to use of a targeting vector alone. For example, when a targeting vector is used in conjunction with a nuclease agent, targeting efficiency of a targeting vector can be increased by at least two-fold, at least three-fold, at least four-fold, at least five-fold, at least six-fold, at least seven-fold, at least eight-fold, at least nine-fold, at least ten-fold or within a range formed from these integers, such as 2-10-fold when compared to use of a targeting vector alone.

Some targeting vectors are "large targeting vectors" or "LTVECs," which includes targeting vectors that comprise homology arms that correspond to and are derived from nucleic acid sequences larger than those typically used by other approaches intended to perform homologous recombination in cells. A LTVEC can be, for example, at least 10kb in length, or the sum total of a 5' homology arm and a 3' homology arm can be, for example, at least 10kb. LTVECs also include targeting vectors comprising nucleic acid constructs larger than those typically used by other approaches intended to perform homologous recombination in cells. For example, LTVECs make possible the modification of large loci that cannot be accommodated by traditional plasmid-based targeting vectors because of their size limitations. For example, a targeted locus can be (i.e., 5' and 3' homology arms can correspond to) a locus of a cell that is not targetable using a conventional method or that can be targeted only incorrectly or only with significantly low efficiency in the absence of a nick or double-strand break induced by a nuclease agent (e.g., a Cas protein).

In some embodiments, methods described herein may employ two or three LTVECs that are capable of recombining with each other and with a target genomic locus in a three-way or a four-way recombination event. Such methods make possible the modification of large loci that cannot be achieved using a single LTVEC.

Examples of LTVECs include vectors derived from a bacterial artificial chromosome (BAC), a human artificial chromosome, or a yeast artificial chromosome (YAC). LTVECs can be in linear form or in circular form. Examples of LTVECs and methods for making them are described, e.g., in Macdonald (2014), U.S. Patent Nos. 6,586,251, 6,596,541 and No. 7,105,348; and International Patent Application Publication No. WO 2002/036789, each of which is incorporated herein by reference in their entireties.

### Methods of making provided non-human animals

Compositions and methods for making non-human animals (e.g., rodents, e.g., rats or mice) whose germline genome comprises an engineered immunoglobulin κ light chain locus that includes one or more human immunoglobulin λ light chain sequences (e.g., human Vλ and Jλ gene segments) in place of non-human immunoglobulin κ light chain sequences, including human immunoglobulin λ light chain encoding sequences that include specific polymorphic forms of human Vλ and Jλ segments (e.g., specific V and/or J alleles or variants) are provided, including compositions and methods for making non-human animals that express antibodies comprising immunoglobulin λ light chains that contain human variable regions and non-human or human constant regions, assembled from an immunoglobulin κ light chain locus that contains a single rearranged human immunoglobulin λ light chain variable region operably linked to a non-human or human immunoglobulin λ light chain constant region gene, which non-human or human immunoglobulin λ light chain constant region gene is located in place of a non-human immunoglobulin κ light chain constant region gene that normally appears in a wild-type non-human immunoglobulin κ light chain locus. In some embodiments, compositions and methods for making non-human animals that express such antibodies under the control of an endogenous immunoglobulin κ enhancer(s) and/or an endogenous immunoglobulin κ regulatory sequence(s) are also provided. In some embodiments, compositions and methods for making non-human animals that express such antibodies under the control of a heterologous immunoglobulin κ enhancer(s) and/or a heterologous immunoglobulin κ regulatory sequence(s) are also provided.

Methods described herein include inserting a single rearranged human immunoglobulin λ light chain variable region encoding a human immunoglobulin λ light chain variable domain upstream of a non-human or human immunoglobulin λ light chain constant region gene (e.g., a rodent, e.g., a murine such as rat or mouse) or human Cλ region gene), which non-human or human immunoglobulin λ light chain constant region gene is located in the place of a non-human immunoglobulin κ light chain constant region gene that normally appears in a wild-type non-human immunoglobulin κ light chain locus, so that an antibody is expressed, which antibody is characterized by the presence of a light chain that contains a human λ light chain variable domain and a non-human Cλ domain (e.g., a rodent (e.g., a murine such as rat or mouse) Cλ domain) or by the presence of a light chain that contains human λ light chain variable and human Cλ domain, and is expressed both on the surface of B cells and in the blood serum of a non-human animal.

In some embodiments, methods include insertion of genetic material that contains a single rearranged human immunoglobulin λ light chain variable region into an immunoglobulin κ light chain locus (e.g., a wild-type, modified or engineered immunoglobulin κ light chain locus of a non-human animal (e.g., rodent, e.g. rat or mouse)). In some embodiments, methods include insertion of genetic material that contains a single rearranged human immunoglobulin λ light chain variable region into an immunoglobulin κ light chain locus of a modified or engineered strain of non-human animal (e.g., rodent, e.g., rat or mouse).

In some embodiments, methods include multiple insertions in a single ES cell clone. In some embodiments, methods include sequential insertions made in a successive ES cell clones. In some embodiments, methods include a single insertion made in an engineered ES cell clone.

In some embodiments, methods include DNA insertion(s) upstream of a non-human (e.g., rodent, e.g., rat or mouse) Cλ1 gene (or human Cλ2 gene) so that said DNA insertion(s) is operably linked to said non-human (e.g., rodent, e.g., rat or mouse) Cλ1 gene (or human Cλ2 gene), which DNA insertion(s) comprise one or two human Vλ gene segments selected from the group consisting of: Vλ4-69, Vλ8-61, Vλ4-60, Vλ6-57, Vλ10-54, Vλ5-52, Vλ1-51, Vλ9-49, Vλ1-47, Vλ7-46, Vλ5-45, Vλ1-44, Vλ7-43, Vλ1-40, Vλ5-39, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1, and one or more human Jλ gene segments selected from the group consisting of: Jλ1, Jλ2, Jλ3, Jλ6, and Jλ7, and which non-human (e.g., rodent, e.g., rat or mouse) Cλ1 gene (or human Cλ2 gene) is located in the place of a non-human (e.g., rodent, e.g., rat or mouse) Cκ gene of an endogenous immunoglobulin κ light chain locus. In some embodiments, methods include DNA insertion(s) upstream of a non-human (e.g., rodent, e.g., rat or mouse) Cλ1 gene (or human Cλ2 gene) so that said DNA insertion(s) is operably linked to said non-human (e.g., rodent, e.g., rat or mouse) Cλ1 gene (or human Cλ2 gene), which DNA insertion(s) comprise a single rearranged human λ light chain variable region that includes a human Vλ gene segments selected from the group consisting of: Vλ4-69, Vλ8-61, Vλ4-60, Vλ6-57, Vλ10-54, Vλ5-52, Vλ1-51, Vλ9-49, Vλ1-47, Vλ7-46, Vλ5-45, Vλ1-44, Vλ7-43, Vλ1-40, Vλ5-39, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3, and Vλ3-1, and a human Jλ gene segments selected from the group consisting of: Jλ1, Jλ2, Jλ3, Jλ6, and Jλ7, and which non-human (e.g., rodent, e.g., rat or mouse) Cλ1 gene (or human Cλ2 gene) is located in the place of a non-human (e.g., rodent, e.g., rat or mouse) Cκ gene of an endogenous immunoglobulin κ light chain locus.

Where appropriate, a human immunoglobulin λ light chain sequence (i.e., a sequence containing human Vλ and Jλ gene segments) encoding a human immunoglobulin λ light chain variable domain may separately be modified to include codons that are optimized for expression in a non-human animal (e.g., see U.S. Patent Nos. 5,670,356 and 5,874,304, each of which is incorporated herein by reference). Codon optimized sequences are engineered sequences, and preferably encode the identical polypeptide (or a biologically active fragment of a full-length polypeptide which has substantially the same activity as the full-length polypeptide) encoded by the non-codon optimized parent polynucleotide. In some embodiments, a human immunoglobulin λ light chain sequence encoding a human immunoglobulin λ light chain variable domain may separately include an altered sequence to optimize codon usage for a particular cell type (e.g., a rodent cell, e.g., rat or mouse cell). For example, the codons of each nucleotide sequence to be inserted into the genome of a non-human animal as described herein (e.g., a rodent, e.g., rat or mouse) may be optimized for expression in a cell of the non-human animal. Such a sequence may be described as a codon-optimized sequence.

Insertion of nucleotide sequences encoding human immunoglobulin λ light chain variable domains employs a minimal modification of the germline genome of a non-human animal as described herein and results in expression of antibodies comprising light chains having human Vλ domains, which human immunoglobulin λ light chain variable domains are expressed from endogenous engineered immunoglobulin κ light chain loci. Methods for generating engineered non-human animals (e.g., rodents, e.g., rats or mice), including knockouts and knock-ins, are known in the art (see, e.g., Gene Targeting: A Practical Approach, Joyner, ed., Oxford University Press, Inc., 2000; incorporated herein by reference in its entirety). For example, generation of genetically engineered rodents may optionally involve disruption of the genetic loci of one or more endogenous rodent genes (or gene segments) and introduction of one or more heterologous genes (or gene segments or nucleotide sequences) into the rodent genome, in some embodiments, at the same location as an endogenous rodent gene (or gene segments). In some embodiments, nucleotide sequences encoding human Vλ domains are introduced upstream of a non-human (e.g., rodent, e.g., rat or mouse) or human immunoglobulin λ light chain constant region gene of a randomly inserted engineered light chain transgene in the germline genome of a rodent. In some embodiments, nucleotide sequences encoding human Vλ domains are introduced upstream of a non-human (e.g., rodent, e.g., rat or mouse) or human immunoglobulin λ light chain constant region gene of an endogenous immunoglobulin κ light chain locus in the germline genome of a rodent; in some certain embodiments, an endogenous immunoglobulin κ light chain locus is altered, modified, or engineered to contain human immunoglobulin λ gene segments (e.g., human V and J) operably linked to a mouse Cλ1 gene or operably linked to a human Cλ2 gene.

Schematic illustrations (not to scale) of exemplary methods for constructing an engineered immunoglobulin κ light chain locus as described herein are provided in Figures 1-6. In particular, Figures 1-6 set forth exemplary strategies for construction of an engineered immunoglobulin κ light chain locus characterized by insertion of nucleotide sequences containing a single rearranged human immunoglobulin λ light chain variable region, as well as corresponding targeting vectors. A targeting vector can be linearized and electroporated into rodent embryonic stem (ES) cells to create a rodent whose germline genome comprises the engineered immunoglobulin κ light chain locus. As described in the examples section below, rodent ES cells employed in electroporation of the targeting vector contained an engineered immunoglobulin κ light chain locus as previously described in U.S. Patent No. 10,143,186; incorporated herein by reference in their entireties. Positive rodent ES cell clones are confirmed using screening methods known in the art. Any remaining selection cassette may be deleted as desired via recombinase-mediated deletion.

Alternatively, a human Cλ gene may be employed in a targeting vector instead of a mouse Cλ gene. A targeting vector can be constructed in a similar manner to the above (or in the Examples below) except that a sequence encoding a human Cλ gene (e.g., Cλ2) can be engineered into the targeting vector. Using such an approach allows developing human antibody therapeutics as DNA encoding the variable and constant regions of light chains may be isolated together, thereby eliminating any subsequent cloning step linking to a human light chain constant region for the preparation of fully-human antibodies.

Targeting vectors for constructing an engineered immunoglobulin κ light chain locus as described herein may be incorporated into the germline genome of a non-human cell (e.g., a rodent (e.g., rat or mouse) embryonic stem cell). In some embodiments, targeting vectors as described herein are incorporated into a wild-type immunoglobulin κ light chain locus in the germline genome of a non-human (e.g., rodent, e.g., rat or mouse) cell that further contains human V_{H}, D_{H} and J_{H} genomic DNA (e.g., containing a plurality of human V_{H}, D_{H} and J_{H} gene segments) operably linked with one or more immunoglobulin heavy chain constant region genes (e.g., see Macdonald (2014), U.S. Patent Nos. 6,596,541, 8,642,835, 8,697,940 and 8,791,323, each of which is incorporated herein by reference in its entirety). In some embodiments, targeting vectors as described herein are incorporated into a modified or engineered immunoglobulin κ light chain locus in the germline genome of a non-human cell that further contains human V_{H}, D_{H} and J_{H} genomic DNA (e.g., containing a plurality of human V_{H}, D_{H} and J_{H} gene segments) operably linked with one or more immunoglobulin heavy chain constant region genes (e.g., see Macdonald (2014), U.S. Patent Nos. 6,596,541, 8,642,835, 8,697,940, 8,791,323, 9,006,511, 9,012,717, 9,029,628, 9,035,128, 9,066,502, 9,150,662 and 9,163,092, each of which is incorporated herein by reference in its entirety).

A targeting vector is introduced into non-human (e.g., rodent, e.g., mouse or rat) embryonic stem cells by electroporation so that the sequence contained in the targeting vector results in the capacity of a non-human (e.g., rodent, e.g., rat or mouse) cell or non-human animal (e.g., a rodent, e.g., rat or mouse) that expresses antibodies having light chains that include human immunoglobulin λ light chain variable domains and non-human or human light chain constant (Cλ or Cκ) domains, and which light chains are expressed from an engineered endogenous immunoglobulin κ light chain locus. As described herein, a genetically engineered non-human animal is generated where an engineered immunoglobulin κ light chain locus has been created in the germline genome of the non-human animal (e.g., an endogenous immunoglobulin κ light chain locus containing a human immunoglobulin λ light chain sequence (i.e., a a rearranged human λ light chain variable region) operably linked to a rodent or human Cλ gene in the place of an endogenous rodent Cκ gene). Antibodies are expressed on the surface of non-human animal B cells and in the serum of said non-human animal, which antibodies are characterized by light chains having human Vλ domains and non-human or human Cλ domains. When an endogenous immunoglobulin κ light chain locus in the germline genome of a non-human animal as described herein is not targeted by the targeting vector, an engineered immunoglobulin κ light chain transgene is preferably inserted at a location other than that of an endogenous non-human animal immunoglobulin κ light chain locus (e.g., randomly inserted transgene).

Creation of an engineered immunoglobulin κ light chain locus in a non-human animal as described above provides an engineered non-human animal strain that produces antibodies that include immunoglobulin λ light chains expressed from such an engineered immunoglobulin κ light chain locus having a human Vλ domain and a non-human (e.g., rodent, e.g., rat or mouse) or human Cλ domain. Leveraged with the presence of an engineered immunoglobulin heavy chain locus that includes a plurality of human V_{H}, D_{H} and J_{H} gene segments operably linked to immunoglobulin heavy chain constant region genes, an engineered non-human animal strain that produces antibodies and antibody components for the development of human antibody-based therapeutics is created. Thus, a single engineered non-human animal strain is realized that has the capacity to provide an alternative *in vivo* system for exploiting human Vλ domains for the development of new antibody-based medicines to treat human disease.

In some embodiments, a method of making a genetically modified non-human animal (e.g., rodent, e.g., rat or mouse) comprises introducing a single rearranged human immunoglobulin λ light chain variable region comprising a human Vλ gene segment and a human Jλ gene segment into an engineered endogenous immunoglobulin κ light chain locus in the genome of a non-human (e.g., rodent, e.g., rat or mouse) embryonic stem (ES) cell. In some embodiments, a method of making a genetically modified non-human animal comprises introducing a non-human (e.g., rodent, e.g., rat or mouse) Cλ gene segment into the engineered endogenous immunoglobulin κ light chain locus in the genome of the non-human ES cell. In some embodiments, a method of making a genetically modified non-human animal (e.g., rodent, e.g., rat or mouse) comprises generating a non-human animal using a non-human (e.g., rodent, e.g., rat or mouse) ES cell as described above.

In some embodiments, a method of making a genetically modified non-human animal (e.g., rodent, e.g., rat or mouse) comprises introducing a single rearranged human immunoglobulin λ light chain variable region comprising a human Vλ gene segment and a human Jλ gene segment into an engineered endogenous immunoglobulin κ light chain locus in the genome of a non-human ES cell. In some embodiments, a method of making a genetically modified non-human animal comprises introducing a non-human Cλ gene segment into an engineered endogenous immunoglobulin κ light chain locus in the genome of a non-human ES cell.

In some embodiments, a method of making a genetically modified non-human animal (e.g., rodent, e.g., rat or mouse) comprises the steps of: (a) introducing a single rearranged human immunoglobulin λ light chain variable region comprising a human Vλ gene segment and a human Jλ gene segment into an engineered endogenous immunoglobulin κ light chain locus in the genome of a non-human ES cell; and (b) introducing a non-human Cλ gene segment into the engineered endogenous immunoglobulin κ light chain locus in the genome of the non-human ES cell.

In some embodiments, a method of making a genetically modified non-human animal (e.g., rodent, e.g., rat or mouse) comprises the steps of: (a) introducing a single rearranged human immunoglobulin λ light chain variable region comprising a human Vλ gene segment and a human Jλ gene segment into an engineered endogenous immunoglobulin κ light chain locus in the genome of a non-human embryonic stem (ES) cell; (b) introducing a non-human Cλ gene segment into the engineered endogenous immunoglobulin κ light chain locus in the genome of the non-human ES cell; and (c) generating a non-human animal using the non-human ES cell generated in steps (a) and (b).

In some embodiments, a method of making a genetically modified non-human animal (e.g., rodent, e.g., rat or mouse) comprises introducing one or more unrearranged human V_{H} gene segments, one or more unrearranged human D_{H} gene segments, and one or more unrearranged human J_{H} gene segments operably linked to one or more endogenous immunoglobulin heavy chain constant region genes into an engineered endogenous immunoglobulin heavy chain locus in the genome of the non-human ES cell.

In some embodiments, a method of making a genetically modified non-human animal (e.g., rodent, e.g., rat or mouse) further comprises the step of: (d) introducing one or more unrearranged human V_{H} gene segments, one or more unrearranged human D_{H} gene segments, and one or more unrearranged human J_{H} gene segments operably linked to one or more endogenous immunoglobulin heavy chain constant region genes into an engineered endogenous immunoglobulin heavy chain locus in the genome of the non-human ES cell.

In some embodiments, a method of making a genetically modified non-human animal (e.g., rodent, e.g., rat or mouse) comprises engineering an endogenous immunoglobulin κ light chain locus in the germline genome of the non-human animal to comprise a single rearranged human immunoglobulin λ light chain variable region operably linked to a non-human Cλ gene segment, wherein the single rearranged human immunoglobulin λ light chain variable region comprises a human Vλ gene segment and a human Jλ gene segment, so that all immunoglobulin λ light chains expressed by B cells of the genetically modified non-human animal include human immunoglobulin λ light chain variable domains expressed from the single rearranged human immunoglobulin λ light chain variable region or a somatically hypermutated version thereof.

In some embodiments, a method of making a genetically modified non-human animal (e.g., rodent, e.g., rat or mouse) comprises engineering an endogenous immunoglobulin heavy chain locus in the germline genome of the non-human animal (e.g., rodent, e.g., rat or mouse) to comprise one or more unrearranged human V_{H} gene segments, one or more unrearranged human D_{H} gene segments, and one or more unrearranged human J_{H} gene segments operably linked to one or more non-human (e.g., rodent, e.g., rat or mouse) immunoglobulin heavy chain constant region genes, so that all heavy chains expressed by B cells of the genetically modified non-human animal (e.g., rodent, e.g., rat or mouse) include human immunoglobulin heavy chain variable domains and non-human (e.g., rodent, e.g., rat or mouse) immunoglobulin heavy chain constant domains.

In some embodiments of a method of making a non-human animal (e.g., rodent, e.g., rat or mouse), a DNA fragment is introduced into a non-human embryonic stem cell whose germline genome comprises one or more engineered immunoglobulin loci (e.g., immunoglobulin heavy chain, immunoglobulin κ light chain, immunoglobulin λ light chain, and combinations thereof). In some certain embodiments, engineered immunoglobulin loci are endogenous engineered immunoglobulin loci.

In some embodiments of a method of making a non-human animal (e.g., rodent, e.g., rat or mouse), a DNA fragment comprises an engineered sequence that includes immunoglobulin λ light chain sequence and/or immunoglobulin κ light chain sequence. In some embodiments of a method of making a non-human animal, a DNA fragment comprises an engineered sequence that includes immunoglobulin λ light chain sequence and/or immunoglobulin κ light chain sequence and with a non-immunoglobulin sequence (e.g., a recombination signal sequence, a resistance gene, and combinations thereof).

In some embodiments of a method of making a non-human animal (e.g., rodent, e.g., rat or mouse), a DNA fragment is introduced into a non-human embryonic stem cell whose genome comprises an endogenous immunoglobulin heavy chain locus comprising insertion of one or more human V_{H} gene segments, one or more human D_{H} gene segments and one or more human J_{H} gene segments, which human V_{H}, D_{H} and J_{H} gene segments are operably linked to a non-human immunoglobulin heavy chain constant region. In some embodiments, an ES cell further comprises a nucleotide sequence encoding one or more rodent ADAM6 polypeptides, functional orthologs, functional homologs, or functional fragments thereof.

In some embodiments of a method of making a non-human animal (e.g., rodent, e.g., rat or mouse), a DNA fragment is introduced into a non-human embryonic stem cell whose germline genome comprises an endogenous immunoglobulin κ light chain locus comprising insertion of a single rearranged human λ light chain variable region, which human Vλ and Jλ gene segments are operably linked to a non-human immunoglobulin κ or λ light chain constant region gene.

In some embodiments of a method of making a non-human animal (e.g., rodent, e.g., rat or mouse), modifying the germline genome of a non-human animal so that it comprises an engineered immunoglobulin κ light chain locus comprising a limited human λ light chain variable region repertoire (e.g., a single rearranged human immunoglobulin λ light chain variable region) is carried out in a non-human embryonic stem cell whose genome comprises an endogenous immunoglobulin heavy chain locus comprising insertion of one or more human V_{H} gene segments, one or more human D_{H} gene segments and one or more human J_{H} gene segments, which human V_{H}, D_{H} and J_{H} gene segments are operably linked to a non-human immunoglobulin heavy chain constant region.

In some embodiments of a method of making a non-human animal (e.g., rodent, e.g., rat or mouse), modifying the germline genome of a non-human animal so that it comprises an engineered immunoglobulin κ light chain locus comprising a limited human λ light chain variable region repertoire is carried out in a non-human embryonic stem cell whose germline genome comprises an endogenous immunoglobulin κ light chain locus comprising insertion of one or more human Vλ and one or more human Jλ gene segments, which human Vλ and Jλ gene segments are operably linked to a non-human immunoglobulin κ light chain constant region gene. In some embodiments of a method of making a non-human animal, modifying the germline genome of a non-human animal so that it comprises an engineered immunoglobulin κ light chain locus including a limited human λ light chain variable region repertoire is carried out in a non-human embryonic stem cell whose germline genome comprises an endogenous immunoglobulin κ light chain locus comprising insertion of one or more human Vλ and one or more human Jλ gene segments, and a human immunoglobulin κ light chain sequence positioned, placed or located between said one or more human Vλ gene segments and said one or more human Jλ gene segments, which human Vλ and Jλ gene segments are operably linked to a non-human immunoglobulin κ light chain constant region gene.

In some embodiments of a method of making a non-human animal (e.g., rodent, e.g., rat or mouse), insertion of one or more human V_{H} gene segments, one or more human D_{H} gene segments and one or more human J_{H} gene segments includes human non-coding DNA that naturally appears adjacent to the human V_{H} gene segments, human non-coding DNA that naturally appears adjacent to the human D_{H} gene segments and human non-coding DNA that naturally appears adjacent to the human J_{H} gene segments in an endogenous human immunoglobulin locus.

In some embodiments, a non-human animal (e.g., rodent, e.g., rat or mouse) made, generated, produced, obtained or obtainable from a method as described herein is provided.

In some embodiments, the genome of a non-human animal (e.g., rodent, e.g., rat or mouse) as described herein further comprises one or more human immunoglobulin heavy variable regions as described in Macdonald (2014), U.S. Patent Nos. 6,596,541, 8,642,835, 8,697,940 and 8,791,323, each of which is incorporated herein by reference in its entirety. Alternatively, an engineered immunoglobulin κ light chain locus including a limited human λ light chain variable region repertoire as described herein can be engineered into an embryonic stem cell of a different modified strain such as, e.g., a VELOCIMMUNE^{®} strain (see, e.g., Macdonald (2014), U.S. Patent Nos. 6,596,541 and/or 8,642,835; incorporated herein by reference in their entireties). Homozygosity of the engineered immunoglobulin κ light chain locus including a limited human λ light chain variable region repertoire as described herein can subsequently be achieved by breeding. Alternatively, in the case of a randomly inserted engineered immunoglobulin κ light chain transgene (described above), non-human animal strains can be selected based on, among other things, expression of human Vλ domains from the transgene. In some embodiments, a VELOCIMMUNE^{®} mouse can be a VELOCIMMUNE^{®} 1 (VI-1) mouse, which includes eighteen human V_{H} gene segments, all of the human D_{H} gene segments, and all of the human J_{H} gene segments. A VI-1 mouse can also include sixteen human Vκ gene segments and all of the human Jκ gene segments. In some embodiments, a VELOCIMMUNE^{®} mouse can be a VELOCIMMUNE^{®} 2 (VI-2) mouse, which includes thirty-nine human V_{H} gene segments, all of the human D_{H} gene segments, and all of the human J_{H} gene segments. A VI-2 mouse can also include human thirty Vκ gene segments and all of the human Jκ gene segments. In some embodiments, a VELOCIMMUNE^{®} mouse can be a VELOCIMMUNE^{®} 3 (VI-3) mouse, which includes eighty human V_{H} gene segments, all of the human D_{H} gene segments, and all of the human J_{H} gene segments. A VI-3 mouse can also include human forty Vκ gene segments and all of the human Jκ gene segments.

Alternatively, and/or additionally, in some embodiments, the germline genome of a non-human animal (e.g., rodent, e.g., rat or mouse) as described herein further comprises a deleted, inactivated, functionally silenced or otherwise non-functional endogenous immunoglobulin λ light chain locus. Genetic modifications to delete or render non-functional a gene or genetic locus may be achieved using methods described herein and/or methods known in the art.

A genetically engineered founder non-human animal (e.g., rodent, e.g., rat or mouse) can be identified based upon the presence of an engineered immunoglobulin κ light chain locus including a limited human λ light chain variable region repertoire as described herein in its germline genome and/or expression of antibodies having a human λ light chain variable domain and a non-human or human Cλ or Cκ domain in tissues or cells of the non-human animal. A genetically engineered founder non-human animal can then be used to breed additional non-human animals carrying the engineered immunoglobulin κ light chain locus including a limited human λ light chain variable region repertoire as described herein, thereby creating a cohort of non-human animals each carrying one or more copies of an engineered immunoglobulin κ light chain locus including a limited human λ light chain variable region repertoire. Moreover, genetically engineered non-human animals carrying an engineered immunoglobulin κ light chain locus including a limited human λ light chain variable region repertoire as described herein can further be bred to other genetically engineered non-human animals carrying other transgenes (e.g., human immunoglobulin genes) or engineered immunoglobulin loci as desired.

Genetically engineered non-human animals (e.g., rodents, e.g., rats or mice) may also be produced to contain selected systems that allow for regulated, directed, inducible and/or cell-type specific expression of a transgene or integrated sequence(s). For example, non-human animals as described herein may be engineered to contain one or more sequences encoding a human λ light chain variable domain of an antibody that is/are conditionally expressed (e.g., reviewed in Rajewski, K. et al., 1996, J. Clin. Invest. 98(3):600-3, incorporated herein by reference in its entirety). Exemplary systems include the Cre//oxP recombinase system of bacteriophage P1 (see, e.g., Lakso, M. et al., 1992, Proc. Natl. Acad. Sci. U.S.A. 89:6232-6, incorporated herein by reference in its entirety) and the FLP/Frt recombinase system of S. cerevisiae (O'Gorman, S. et al, 1991, Science 251:1351-5, incorporated herein by reference in its entirety). Such animals can be provided through the construction of "double" genetically engineered animals, e.g., by mating two genetically engineered animals, one containing a transgene comprising a selected modification (e.g., an engineered immunoglobulin κ light chain locus including a limited human λ light chain variable region repertoire as described herein) and the other containing a transgene encoding a recombinase (e.g., a Cre recombinase).

Non-human animals (e.g., rodents, e.g., rats or mice) as described herein may be prepared as described above, or using methods known in the art, to comprise additional human, humanized or otherwise engineered genes, oftentimes depending on the intended use of the non-human animal. Genetic material of such human, humanized or otherwise engineered genes may be introduced through the further alteration of the genome of cells (e.g., embryonic stem cells) having the genetic modifications or alterations as described above or through breeding techniques known in the art with other genetically modified or engineered strains as desired. In some embodiments, non-human animals as described herein are prepared to further comprise human heavy chain gene segments (see e.g., Murphy, A.J. et al., (2014) Proc. Natl. Acad. Sci. U.S.A. 111(14):5153-5158; Macdonald (2014), U.S. Patent Nos. 6,596,541, 8,642,835, 8,697,940 and 8,791,323; U.S. Patent No: 8,791,323; and U.S. Patent Application Publication No. 2013/0096287 A1; each of which is incorporated herein by reference in its entirety).

In some embodiments, non-human animals (e.g., rodents, e.g., rats or mice) as described herein may be prepared by introducing a targeting vector described herein into a cell from a modified or engineered strain. For example, a targeting vector as described herein may be introduced into a VELOCIMMUNE^{®} mouse. VELOCIMMUNE^{®} mice express antibodies that have fully human variable domains and mouse constant domains. In another example, a targeting vector as described herein may be introduced into an engineered mouse as described in any one of U.S. Patent Nos. 9,006,511, 9,012,717, 9,029,628, 9,035,128, 9,066,502, 9,150,662 and 9,163,092, incorporated herein by reference in their entireties. In some embodiments, non-human animals as described herein are prepared to further comprise human immunoglobulin genes (variable and/or constant region genes). In some embodiments, non-human animals as described herein comprise an engineered immunoglobulin κ light chain locus including a limited human λ light chain variable region repertoire as described herein and genetic material from a heterologous species (e.g., humans), wherein the genetic material encodes, in whole or in part, one or more human heavy chain variable regions.

For example, as described herein, non-human animals (e.g., rodents, e.g., rats or mice) comprising an engineered immunoglobulin κ light chain locus including a limited human λ light chain variable region repertoire as described herein may further comprise (e.g., via cross-breeding or multiple gene targeting strategies) one or more modifications as described in Murphy, A.J. et al., (2014) Proc. Natl. Acad. Sci. U.S.A. 111(14):5153-8; Macdonald (2014); U.S. Patent Nos. 6,596,541, 8,642,835, 8,697,940 and 8,791,323; all of which are incorporated herein by reference in their entireties. In some embodiments, a non-human animal comprising an engineered immunoglobulin κ light chain locus including a limited human λ light chain variable region repertoire as described herein is crossed to a non-human animal comprising a humanized immunoglobulin heavy chain variable region locus (see, e.g., Macdonald (2014), U.S. Patent Nos. 6,596,541, 8,642,835, 8,697,940 and/or 8,791,323; incorporated herein by reference in their entireties). In some embodiments, a non-human animal comprising an engineered immunoglobulin κ light chain locus including a limited human λ light chain variable region repertoire as described herein is crossed to a non-human animal comprising a humanized immunoglobulin heavy chain variable region locus (see, e.g., Macdonald (2014), U.S. Patent Nos. 6,596,541, 8,642,835, 8,697,940 and/or 8,791,323; incorporated herein by reference) and an inactivated endogenous immunoglobulin λ light chain locus (see, e.g., U.S. Patent Nos. 9,006,511, 9,012,717, 9,029,628, 9,035,128, 9,066,502, 9,150,662 and 9,163,092, incorporated herein by reference in their entireties).

Although embodiments describing the construction of an engineered immunoglobulin κ light chain locus in a mouse (i.e., a mouse with an engineered immunoglobulin κ light chain locus characterized by the presence of a limited human λ light chain variable region repertoire operably linked with a mouse or human Cλ or Cκ gene so that antibodies containing human λ light chain variable domains and mouse or human Cλ or Cκ domains are expressed) are extensively discussed herein, other non-human animals that comprise an engineered immunoglobulin κ light chain locus are also provided. Such non-human animals include any of those which can be genetically modified to express antibodies as described herein, including, e.g., mammals, e.g., mouse, rat, rabbit, pig, bovine (e.g., cow, bull, buffalo), deer, sheep, goat, chicken, cat, dog, ferret, primate (e.g., marmoset, rhesus monkey), etc. For example, for those non-human animals for which suitable genetically modifiable ES cells are not readily available, other methods are employed to make a non-human animal comprising the genetic modification. Such methods include, e.g., modifying a non-ES cell genome (e.g., a fibroblast or an induced pluripotent cell) and employing somatic cell nuclear transfer (SCNT) to transfer the genetically modified genome to a suitable cell, e.g., an enucleated oocyte, and gestating the modified cell (e.g., the modified oocyte) in a non-human animal under suitable conditions to form an embryo.

Methods for modifying the germline genome of a non-human animal (e.g., a pig, cow, rodent, chicken, etc. genome) include, e.g., employing a zinc finger nuclease (ZFN), a transcription activator-like effector nuclease (TALEN), or a Cas protein (i.e., a CRISPR/Cas system) to include an engineered immunoglobulin κ light chain locus including a limited human λ light chain variable region repertoire as described herein. Guidance for methods for modifying the germline genome of a non-human animal can be found in, e.g., U.S. Patent No. 9,738,897, and U.S. Patent Application Publication Nos. US 2016/0145646 (published May 26, 2016) and US 2016/0177339 (published June 23, 2016); each of which is incorporated herein by reference in its entirety.

In some embodiments, a non-human animal as described herein is a mammal. In some embodiments, a non-human animal as described herein is a small mammal, e.g., of the superfamily Dipodoidea or Muroidea. In some embodiments, a genetically modified animal as described herein is a rodent. In some embodiments, a rodent as described herein is selected from a mouse, a rat, and a hamster. In some embodiments, a rodent as described herein is selected from the superfamily Muroidea. In some embodiments, a genetically modified animal as described herein is from a family selected from Calomyscidae (e.g., mouse-like hamsters), Cricetidae (e.g., hamster, New World rats and mice, voles), Muridae (true mice and rats, gerbils, spiny mice, crested rats), Nesomyidae (climbing mice, rock mice, white-tailed rats, Malagasy rats and mice), Platacanthomyidae (e.g., spiny dormice), and Spalacidae (e.g., mole rates, bamboo rats, and zokors). In some certain embodiments, a genetically modified rodent as described herein is selected from a true mouse or rat (family Muridae), a gerbil, a spiny mouse, and a crested rat. In some certain embodiments, a genetically modified mouse as described herein is from a member of the family Muridae. In some embodiment, a non-human animal as described herein is a rodent. In some certain embodiments, a rodent as described herein is selected from a mouse and a rat. In some embodiments, a non-human animal as described herein is a mouse.

In some embodiments, a non-human animal as described herein is a rodent that is a mouse of a C57BL strain selected from C57BL/A, C57BL/An, C57BL/GrFa, C57BL/KaLwN, C57BL/6, C57BL/6J, C57BL/6ByJ, C57BL/6NJ, C57BL/10, C57BL/10ScSn, C57BL/10Cr, and C57BL/Ola. In some certain embodiments, a mouse as described herein is a 129-strain selected from the group consisting of a strain that is 129P1, 129P2, 129P3, 129X1, 129S1 (e.g., 129S1/SV, 129S1/SvIm), 129S2, 129S4, 129S5, 129S9/SvEvH, 129/SvJae, 129S6 (129/SvEvTac), 129S7, 129S8, 129T1, 129T2 (see, e.g., Festing et al., 1999, Mammalian Genome 10:836; Auerbach, W. et al., 2000, Biotechniques 29(5):1024-1028, 1030, 1032, each of which is incorporated herein by reference in its entirety). In some certain embodiments, a genetically modified mouse as described herein is a mix of an aforementioned 129 strain and an aforementioned C57BL/6 strain. In some certain embodiments, a mouse as described herein is a mix of aforementioned 129 strains, or a mix of aforementioned BL/6 strains. In some certain embodiments, a 129 strain of the mix as described herein is a 129S6 (129/SvEvTac) strain. In some embodiments, a mouse as described herein is a BALB strain, e.g., BALB/c strain. In some embodiments, a mouse as described herein is a mix of a BALB strain and another aforementioned strain.

In some embodiments, a non-human animal as described herein is a rat. In some certain embodiments, a rat as described herein is selected from a Wistar rat, an LEA strain, a Sprague Dawley strain, a Fischer strain, F344, F6, and Dark Agouti. In some certain embodiments, a rat strain as described herein is a mix of two or more strains selected from the group consisting of Wistar, LEA, Sprague Dawley, Fischer, F344, F6, and Dark Agouti.

A rat pluripotent and/or totipotent cell can be from any rat strain, including, for example, an ACI rat strain (an inbred strain originally derived from August and Copenhagen strains), a Dark Agouti (DA) rat strain, a Wistar rat strain, a LEA rat strain, a Sprague Dawley (SD) rat strain, or a Fischer rat strain such as Fisher F344 or Fisher F6. Rat pluripotent and/or totipotent cells can also be obtained from a strain derived from a mix of two or more strains recited above. For example, the rat pluripotent and/or totipotent cell can be from a DA strain or an ACI strain. The ACI rat strain is characterized as having black agouti, with white belly and feet and an RT1av1 haplotype. Such strains are available from a variety of sources including Harlan Laboratories. An example of a rat ES cell line from an ACI rat is an ACI.G1 rat ES cell. The DA rat strain is characterized as having an agouti coat and an RT1av1 haplotype. Such rats are available from a variety of sources including Charles River and Harlan Laboratories. Examples of a rat ES cell line from a DA rat are the DA.2B rat ES cell line and the DA.2C rat ES cell line. In some embodiments, the rat pluripotent and/or totipotent cells are from an inbred rat strain (see, e.g., U.S. Patent Application Publication No. 2014-0235933 A1, published August 21, 2014, incorporated herein by reference in its entirety). Guidance for making modifications in a rat genome (e.g., in a rat ES cell) using methods and/or constructs as described herein can be found in, e.g., in U.S. Patent Application Publication Nos. 2014-0310828 and 2017-0204430; both of which are incorporated herein by reference in their entireties.

### Methods of using provided non-human animals, cells or tissues

Non-human animals (e.g., rodents, e.g., rats or mice), non-human (e.g., rodent, e.g., rat or mouse) cells and non-human (e.g., rodent, e.g., rat or mouse) tissues as described herein can be used as a platform for the development of antibodies. In particular, non-human animals, non-human cells and non-human tissues as described herein represent a particularly advantageous platform for the generation and identification of human heavy chain variable domains that pair with human λ light chain variable domains expressed from a limited human λ light chain repertoire, and antibodies that include such human heavy chain variable domains. Such human heavy chain variable domains can be used, e.g., in the production of bispecific antigen-binding proteins.

Accordingly, the present disclosure provides that non-human animals (e.g., rodents, e.g., rats or mice), non-human (e.g., rodent, e.g., rat or mouse) cells and non-human (e.g., rodent, e.g., rat or mouse) tissues described herein can be used in methods of making antibodies. Antibodies made in accordance with the present disclosure can include, for example, human antibodies, chimeric antibodies, reverse chimeric antibodies, fragments of any of these antibodies, or combinations thereof.

In some embodiments, non-human animals (e.g., rodents, e.g., rats or mice), non-human (e.g., rodent, e.g., rat or mouse) cells and non-human (e.g., rodent, e.g., rat or mouse) tissues as described herein may be employed for making a human antibody (e.g., a fully human antibody), which human antibody comprises variable domains derived from nucleic acid sequences encoded by genetic material of a cell of a non-human animal as described herein. In some embodiments, a non-human animal (e.g., genetically modified rodent, e.g., genetically modified rat or mouse) as described herein is immunized with an antigen of interest under conditions and for a time sufficient that the non-human animal develops an immune response to said antigen of interest. Antibodies and/or antibody sequences (i.e., sequences that encode for part of an antibody, e.g., a variable region sequence) are isolated and/or identified from the immunized non-human animal (or one or more cells, for example, one or more B cells) and characterized using various assays measuring, for example, affinity, specificity, epitope mapping, ability for blocking ligand-receptor interaction, inhibition receptor activation, etc. In various embodiments, antibodies produced by non-human animals, non-human cells and non-human tissues as described herein comprise one or more human variable regions that are derived from one or more human variable region nucleotide sequences isolated from the non-human animal, non-human cell or non-human tissue. In some embodiments, anti-drug antibodies (e.g., anti-idiotype antibody) may be raised in non-human animals, non-human cells and non-human tissues as described herein. In various embodiments, antibodies produced by non-human animals, non-human cells and non-human tissues as described herein are reverse chimeric antibodies that include a human light chain variable domain and a non-human (e.g., rodent, e.g., rat or mouse) light chain constant domain and/or a human heavy chain variable domain and a non-human (e.g. rodent, e.g., rat or mouse) heavy chain constant domain.

In various embodiments, antibodies produced by non-human animals (e.g., rodents, e.g., rats or mice), non-human (e.g., rodent, e.g., rat or mouse) cells and non-human (e.g., rodent, e.g., rat or mouse) tissues include heavy and light chains having a human variable domain and a non-human constant domain. In some embodiments, antibodies produced by non-human animals, non-human cells and non-human tissues as described herein are reverse chimeric antibodies that include a human light chain variable domain and a non-human (e.g. rodent) light chain constant domain. In some embodiments, antibodies produced by non-human animals, non-human cells and non-human tissues as described herein are reverse chimeric antibodies that include a human heavy chain variable domain and a non-human (e.g. rodent, e.g., rat or mouse) heavy chain constant domain.

In some embodiments, provided methods include immunizing a non-human animal (e.g., rodent, e.g., rat or mouse) as described herein with an antigen of interest. In some embodiments, provided methods include identifying a lymphocyte (e.g., a clonally selected lymphocyte) from said non-human animal, where the lymphocyte expresses an antibody that binds (e.g., specifically binds) the antigen of interest. In some embodiments, a lymphocyte is a B cell. In some embodiments, a human heavy chain variable region sequence and/or a human lambda light chain variable region sequence is obtained from the lymphocyte (e.g., B cell) and/or identified (e.g., genotyped, e.g., sequenced). In some embodiments, an amino acid sequence of a human heavy chain variable domain and/or a human lambda light chain variable domain is obtained from the lymphocyte (e.g., B cell) and/or identified (e.g., sequenced). In some embodiments, a human heavy chain variable region sequence and/or a human lambda light chain variable region sequence from a B cell of a non-human animal is expressed in a host cell. In some embodiments, a variant of a human heavy chain variable region sequence and/or a human lambda light chain variable region sequence from a B cell of a non-human animal is expressed in a host cell. In some embodiments, a variant includes one or more mutations. In some embodiments, one or more mutations can improve a pharmacokinetic and/or a pharmacodynamic property of an antibody including a variant. In some embodiments, one or more mutations can improve the specificity, the affinity, and/or the immunogenicity of an antibody including a variant.

In some embodiments, methods of making a human antibody include identifying a nucleotide sequence encoding a human immunoglobulin heavy chain variable domain and/or a human immunoglobulin light chain variable domain from a non-human animal (e.g., rodent, e.g., rat or mouse), non-human (e.g., rodent, e.g., rat or mouse) cell or non-human (e.g., rodent, e.g., rat or mouse) tissue described herein; and (i) joining or ligating the nucleotide sequence encoding the human immunoglobulin heavy chain variable domain to a nucleotide sequence encoding a human immunoglobulin heavy chain constant domain, thereby forming a human immunoglobulin heavy chain sequence encoding a fully human immunoglobulin heavy chain, and/or (ii) joining or ligating the nucleotide sequence encoding the human immunoglobulin λ light chain variable domain to a nucleotide sequence encoding a human immunoglobulin λ light chain constant domain, thereby forming a human immunoglobulin λ light chain sequence encoding a fully human immunoglobulin λ light chain. In certain embodiments, a human immunoglobulin heavy chain sequence, and a human immunoglobulin λ light chain sequence are expressed in a cell (e.g., a host cell, a mammalian cell) so that fully human immunoglobulin heavy chains and fully human immunoglobulin λ light chains are expressed and form human antibodies. In some embodiments, human antibodies are isolated from the cell or culture media including the cell.

In some embodiments, non-human animals (e.g., rodents, e.g., rats or mice) as described herein may be employed for methods of making a bispecific antibody, the method comprising: (a) contacting a first genetically modified non-human animal as described herein, comprising a single rearranged human Ig λ light chain variable region operably linked to non-human (e.g., rodent, e.g., rat or mouse) constant region, with a first epitope of a first antigen, (b) contacting a second genetically modified non-human animal as described herein, comprising a single rearranged human Ig λ light chain variable region operably linked to non-human (e.g., rodent, e.g., rat or mouse) constant region (e.g., the same single rearranged human Ig λ light chain variable region as present in the first non-human animal), with a second epitope of a second antigen, (c) isolating a B cell that expresses a first antibody specific for the first epitope of the first antigen from the first genetically modified non-human animal and determining a first human immunoglobulin heavy chain variable domain of the first antibody; (d) isolating a B cell that expresses a second antibody specific for the second epitope of the second antigen from the second genetically modified non-human animal and determining a second human immunoglobulin heavy chain variable domain of the second antibody; (e) operably linking a nucleotide sequence encoding the first human immunoglobulin heavy chain variable domain to a nucleotide sequence encoding a first human immunoglobulin constant domain to produce a first nucleotide sequence encoding a first human heavy chain; (f) operably linking a nucleotide sequence encoding the second human immunoglobulin heavy chain variable domain to a nucleotide sequence encoding a second human immunoglobulin constant domain to produce a second nucleotide sequence encoding a second human heavy chain; (g) expressing in a mammalian cell: (i) the first nucleotide sequence; (ii) the second nucleotide sequence; and (iii) a third nucleotide sequence comprising the single rearranged human immunoglobulin λ light chain variable region or a somatically hypermutated version thereof operably linked to a human immunoglobulin λ light chain constant region.

In some embodiments, non-human (e.g., rodent, e.g., rat or mouse) cells as described here may be employed for methods of making a bispecific antibody, the method comprising: (a) expressing in a non-human cell: (i) a first nucleotide sequence comprising a first human immunoglobulin heavy chain variable region operably linked to a first human immunoglobulin constant region; (ii) a second nucleotide sequence comprising a second human immunoglobulin heavy chain variable region operably linked to a second human immunoglobulin constant region; and (iii) a third nucleotide sequence comprising human immunoglobulin λ light chain variable region operably linked to a human immunoglobulin λ light chain constant region; wherein the first human immunoglobulin heavy chain variable region encodes a first human heavy chain variable domain identified in a first genetically modified non-human animal that had been immunized with a first epitope of a first antigen, and the second human immunoglobulin heavy chain variable region encodes a second human heavy chain variable domain identified in a second genetically modified non-human animal that had been immunized with a second epitope of a second antigen, wherein the first and second genetically modified non-human animals are each a genetically modified non-human animal as described herein, comprising a single rearranged human Ig λ light chain variable region operably linked to non-human (e.g., rodent, e.g., rat or mouse) constant region; and wherein the human immunoglobulin λ light chain variable region of the third nucleotide is the single rearranged human immunoglobulin λ light chain variable region or a somatically hypermutated version thereof. Amino acids or nucleic acids identified are used to make antibodies or antigen binding proteins containing variable domains identified herein.

Non-human animals (e.g., rodents, e.g., rats or mice), non-human (e.g., rodent, e.g., rat or mouse) cells and non-human (e.g., rodent, e.g., rat or mouse) tissues as described herein may be employed for identifying a nucleotide or nucleic acid sequence encoding a human variable domain generated by a non-human animal, non-human cell or non-human tissue described herein, e.g., as part of an antibody against an epitope or antigen.

Non-human animals (e.g., rodents, e.g., rats or mice), non-human (e.g., rodent, e.g., rat or mouse) cells and non-human (e.g., rodent, e.g., rat or mouse) tissues as described herein may be employed for identifying an amino acid sequence of a human variable domain generated by a non-human animal, non-human cell or non-human tissue described herein, e.g., as part of an antibody against an epitope or antigen.

Non-human animals (e.g., rodents, e.g., rats or mice), non-human (e.g., rodent, e.g., rat or mouse) cells and non-human (e.g., rodent, e.g., rat or mouse) tissues as described herein provide an improved *in vivo* system and source of biological materials (e.g., cells, nucleotides, polypeptides, protein complexes) for producing human antibodies that are useful for a variety of assays. In various embodiments, non-human animals, non-human cells and non-human tissues as described herein are used to develop therapeutics that target a polypeptide of interest (e.g., a transmembrane or secreted polypeptide) and/or modulate one or more activities associated with said polypeptide of interest and/or modulate interactions of said polypeptide of interest with other binding partners (e.g., a ligand or receptor polypeptide). For example, in various embodiments, non-human animals, non-human cells and non-human tissues as described herein are used to develop therapeutics that target one or more receptor polypeptides, modulate receptor polypeptide activity and/or modulate receptor polypeptide interactions with other binding partners. In various embodiments, non-human animals, non-human cells and non-human tissues as described herein are used to identify, screen and/or develop candidate therapeutics (e.g., antibodies, scFvs, etc.) that bind one or more polypeptides of interest. In various embodiments, non-human animals, non-human cells and non-human tissues as described herein are used to screen and develop candidate therapeutics (e.g., antibodies, scFvs, etc.) that block activity of one or more polypeptides of interest or that block the activity of one or more receptor polypeptides of interest. In various embodiments, non-human animals, non-human cells and non-human tissues as described herein are used to determine the binding profile of antagonists and/or agonists of one or more polypeptides of interest. In some embodiments, non-human animals, non-human cells and non-human tissues as described herein are used to determine the epitope or epitopes of one or more candidate therapeutic antibodies that bind one or more polypeptides of interest.

In various embodiments, non-human animals (e.g., rodents, e.g., rats or mice), non-human (e.g., rodent, e.g., rat or mouse) cells and non-human (e.g., rodent, e.g., rat or mouse) tissues as described herein are used to determine the pharmacokinetic profiles of one or more human antibody candidates. In various embodiments, one or more non-human animals, non-human cells and non-human tissues as described herein and one or more control or reference non-human animals, non-human cells and non-human tissues are each exposed to one or more human antibody candidates at various doses (e.g., 0.1 mg/kg, 0.2 mg/kg, 0.3 mg/kg, 0.4 mg/kg, 0.5 mg/kg, 1 mg/kg, 2 mg/kg, 3 mg/kg, 4 mg/kg, 5 mg/mg, 7.5 mg/kg, 10 mg/kg, 15 mg/kg, 20 mg/kg, 25 mg/kg, 30 mg/kg, 40 mg/kg, or 50 mg/kg or more). Candidate therapeutic antibodies may be dosed to non-human animals as described herein via any desired route of administration including parenteral and non-parenteral routes of administration. Parenteral routes include, e.g., intravenous, intraarterial, intraportal, intramuscular, subcutaneous, intraperitoneal, intraspinal, intrathecal, intracerebroventricular, intracranial, intrapleural or other routes of injection. Non-parenteral routes include, e.g., oral, nasal, transdermal, pulmonary, rectal, buccal, vaginal, ocular. Administration may also be by continuous infusion, local administration, sustained release from implants (gels, membranes or the like), and/or intravenous injection. Blood is isolated from non-human animals (humanized and control) at various time points (e.g., 0 hr, 6 hr, 1 day, 2 days, 3 days, 4 days, 5 days, 6 days, 7 days, 8 days, 9 days, 10 days, 11 days, or up to 30 or more days). Various assays may be performed to determine the pharmacokinetic profiles of administered candidate therapeutic antibodies using samples obtained from non-human animals, non-human cells and non-human tissues as described herein including, but not limited to, total IgG, anti-therapeutic antibody response, agglutination, etc.

In various embodiments, non-human animals (e.g., rodents, e.g., rats or mice), non-human (e.g., rodent, e.g., rat or mouse) cells and non-human (e.g., rodent, e.g., rat or mouse) tissues as described herein are used to measure the therapeutic effect of blocking or modulating the activity of a polypeptide of interest and the effect on gene expression as a result of cellular changes or, in the context of a receptor polypeptide, the density of a receptor polypeptide on the surface of cells in the non-human animal, non-human cell or non-human tissue. In various embodiments, a non-human animal as described herein (or cells isolated therefrom), non-human cells or non-human tissues are exposed to a candidate therapeutic that binds a polypeptide of interest and, after a subsequent period of time, analyzed for effects on specific cellular processes that are associated with said polypeptide of interest, for example, ligand-receptor interactions or signal transduction.

Non-human animals (e.g., rodents, e.g., rats or mice) as described herein express human antibody variable regions, thus cells, cell lines, and cell cultures can be generated to serve as a source of human antibody variable regions for use in binding and functional assays, e.g., to assay for binding or function of an antagonist or agonist, particularly where the antagonist or agonist is specific for a human antigen of interest or specific for an epitope that functions in ligand-receptor interaction (binding). In various embodiments, epitopes bound by candidate therapeutic antibodies or fragments (e.g., a Fab fragment, a monovalent fragment consisting of the VH, VL, CH1 and CL domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VH and VL domains of a single arm of an antibody, (v) a dAb fragment (Ward et al., (1989) Nature 341 :544-546), which comprises a single variable domain; and (vi) an isolated complementarity determining region (CDR)) can be determined using cells isolated from non-human animals as described herein.

Cells from provided non-human animals (e.g., rodents, e.g., rats or mice) can be isolated and used on an ad hoc basis, or can be maintained in culture for many generations. In various embodiments, cells from a provided non-human animal are immortalized (e.g., via use of a virus) and maintained in culture indefinitely (e.g., in serial cultures).

In some embodiments, a non-human (e.g., rodent, e.g., rat or mouse) cell is a non-human lymphocyte. In some embodiments, a non-human cell is selected from a B cell, dendritic cell, macrophage, monocyte and a T cell. In some embodiments, a non-human cell is an immature B cell, a mature naive B cell, an activated B cell, a memory B cell, and/or a plasma cell.

In some embodiments, a non-human (e.g., rodent, e.g., rat or mouse) cell is a non-human embryonic stem (ES) cell. In some embodiments, a non-human ES cell is a rodent ES cell. In some certain embodiments, a rodent ES cell is a mouse ES cell and is from a 129 strain, C57BL strain, BALB/c or a mixture thereof. In some certain embodiments, a rodent embryonic stem cell is a mouse embryonic stem cell and is a mixture of 129 and C57BL strains. In some certain embodiments, a rodent embryonic stem cell is a mouse embryonic stem cell and is a mixture of 129, C57BL and BALB/c strains.

In some embodiments, use of a non-human (e.g., rodent, e.g., rat or mouse) ES cell as described herein to make a non-human animal is provided. In some certain embodiments, a non-human ES cell is a mouse ES cell and is used to make a mouse comprising engineered immunoglobulin κ light chain locus including a limited human λ light chain variable region repertoire as described herein. In some certain embodiments, a non-human ES cell is a rat ES cell and is used to make a rat comprising engineered immunoglobulin κ light chain locus including a limited human λ light chain variable region repertoire as described herein.

In some embodiments, a non-human (e.g., rodent, e.g., rat or mouse) tissue is selected from adipose, bladder, brain, breast, bone marrow, eye, heart, intestine, kidney, liver, lung, lymph node, muscle, pancreas, plasma, serum, skin, spleen, stomach, thymus, testis, ovum, and a combination thereof.

In some embodiments, an immortalized cell made, generated, produced or obtained from an isolated non-human cell or tissue as described herein is provided.

In some embodiments, a non-human (e.g., rodent, e.g., rat or mouse) embryo made, generated, produced, or obtained from a non-human ES cell as described herein is provided. In some certain embodiments, a non-human embryo is a rodent embryo; in some embodiments, a mouse embryo; in some embodiments, a rat embryo.

Non-human animals (e.g, rodents, e.g., rats or mice) as described herein provide an *in vivo* system for the generation of variants of human antibody variable regions that binds a polypeptide of interest (e.g., human Vλ domain variants). Such variants include human antibody variable regions having a desired functionality, specificity, low cross-reactivity to a common epitope shared by two or more variants of a polypeptide of interest. In some embodiments, non-human animals as described herein are employed to generate panels of human antibody variable regions that contain a series of variant variable regions that are screened for a desired or improved functionality.

Non-human animals (e.g, rodents, e.g., rats or mice) as described herein provide an *in vivo* system for generating human antibody variable region libraries (e.g., a human Vλ domain library). Such libraries provide a source for heavy and/or light chain variable region sequences that may be grafted onto different Fc regions based on a desired effector function, used as a source for affinity maturation of the variable region sequence using techniques known in the art (e.g., site-directed mutagenesis, error-prone PCR, etc.) and/or used as a source of antibody components for the generation of antibody-based therapeutic molecules such as, for example, chimeric antigen receptors (i.e., a molecule engineered using antibody components, e.g., an scFv), multi-specific binding agents (e.g., bi-specific binding agents) and fusion proteins (e.g., single domain antibodies, scFvs, etc.).

In some embodiments, a method of producing an antibody in a non-human (e.g., rodent, e.g., rat or mouse) animal is provided, the method comprising the steps of (a) immunizing a non-human animal (e.g., rodent, e.g., rat or mouse) as described herein with an antigen of interest; (b) maintaining the non-human animal (e.g., rodent, e.g., rat or mouse) under conditions sufficient that the non-human animal produces an immune response (e.g., expresses antibodies) to the antigen of interest; and (c) recovering an antibody from the non-human animal (e.g., rodent, e.g., rat or mouse), or a cell (e.g., B cell) of the non-human animal (e.g., rodent, e.g., rat or mouse), that binds the antigen of interest.

In some embodiments of a method of producing an antibody in a non-human animal, a non-human cell is a B cell. In some embodiments of a method of producing an antibody in a non-human animal, a non-human cell is a hybridoma.

In some embodiments, an antibody prepared by a method is provided, comprising the steps of: (a) providing a non-human animal as described herein; (b) immunizing the non-human animal with an antigen of interest; (c) maintaining the non-human animal under conditions sufficient that the non-human animal produces an immune response to the antigen of interest; and (d) recovering an antibody from the non-human animal, or a non-human cell, that binds the antigen of interest, wherein the antibody of (d) includes human V_{H} and Vλ domains.

In some embodiments of an antibody prepared by a method, a human heavy chain variable domain is encoded by a rearranged human heavy chain variable region comprising a human V_{H}3-74, V_{H}3-73, V_{H}3-72, V_{H}2-70, V_{H}1-69, V_{H}3-66, V_{H}3-64, V_{H}4-61, V_{H}4-59, V_{H}1-58, V_{H}3-53, V_{H}5-51, V_{H}3-49, V_{H}3-48, V_{H}1-46, V_{H}1-45, V_{H}3-43, V_{H}4-39, V_{H}4-34, V_{H}3-33, V_{H}4-31, V_{H}3-30, V_{H}4-28, V_{H}2-26, V_{H}1-24, V_{H}3-23, V_{H}3-21, V_{H}3-20, V_{H}1-18, V_{H}3-15, V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2 V_{H}6-1, or somatically hypermutated variant thereof.

In some embodiments of an antibody prepared by a method, a human λ light chain variable domain, obtained from a non-human animal comprising a limited human immunoglobulin λ light chain variable region repertoire as described herein, is encoded by a rearranged human λ light chain variable region comprising a human Vλ4-69, Vλ8-61, Vλ4-60, Vλ6-57, Vλ10-54, Vλ5-52, Vλ1-51, Vλ9-49, Vλ1-47, Vλ7-46, Vλ5-45, Vλ1-44, Vλ7-43, Vλ1-40, Vλ5-39, Vλ5-37, Vλ1-36, Vλ3-27, Vλ3-25, Vλ2-23, Vλ3-22, Vλ3-21, Vλ3-19, Vλ2-18, Vλ3-16, Vλ2-14, Vλ3-12, Vλ2-11, Vλ3-10, Vλ3-9, Vλ2-8, Vλ4-3 Vλ3-1, or somatically hypermutated variant thereof.

In some embodiments, a non-human animal (e.g., rodent, e.g., rat or mouse), non-human (e.g., rodent, e.g., rat or mouse) cell or non-human (e.g., rodent, e.g., rat or mouse) tissue as described herein is provided for use in the manufacture and/or development of a drug (e.g., an antibody or fragment thereof) for therapy or diagnosis.

In some embodiments, a non-human animal (e.g., rodent, e.g., rat or mouse), non-human (e.g., rodent, e.g., rat or mouse) cell or non-human (e.g., rodent, e.g., rat or mouse) tissue as described herein is provided for use in the manufacture of a medicament for the treatment, prevention or amelioration of a disease, disorder or condition.

In some embodiments, use of a non-human animal (e.g., rodent, e.g., rat or mouse), non-human (e.g., rodent, e.g., rat or mouse) cell or non-human (e.g., rodent, e.g., rat or mouse) tissue as described herein in the manufacture and/or development of a drug or vaccine for use in medicine, such as use as a medicament, is provided.

In some embodiments, use of a non-human animal (e.g., rodent, e.g., rat or mouse), non-human (e.g., rodent, e.g., rat or mouse) cell, or non-human (e.g., rodent, e.g., rat or mouse) tissue as described herein in the manufacture and/or development of an antibody or fragment thereof is provided.

Non-human animals (e.g., rodents, e.g., rats or mice) as described herein provide an *in vivo* system for the analysis and testing of a drug or vaccine. In various embodiments, a candidate drug or vaccine may be delivered to one or more non-human animals as described herein, followed by monitoring of the non-human animals to determine one or more of the immune response to the drug or vaccine, the safety profile of the drug or vaccine, or the effect on a disease or condition and/or one or more symptoms of a disease or condition. Exemplary methods used to determine the safety profile include measurements of toxicity, optimal dose concentration, antibody (i.e., anti-drug) response, efficacy of the drug or vaccine and possible risk factors. Such drugs or vaccines may be improved and/or developed in such non-human animals.

Vaccine efficacy may be determined in a number of ways. Briefly, non-human animals (e.g., rodents, e.g., rats or mice) as described herein are vaccinated using methods known in the art and then challenged with a vaccine or a vaccine is administered to already-infected non-human animals. The response of a non-human animal(s) to a vaccine may be measured by monitoring of, and/or performing one or more assays on, the non-human animal(s) (or cells isolated therefrom) to determine the efficacy of the vaccine. The response of a non-human animal(s) to the vaccine is then compared with control animals, using one or more measures known in the art and/or described herein.

Vaccine efficacy may further be determined by viral neutralization assays. Briefly, non-human animals (e.g., rodents, e.g., rats or mice) as described herein are immunized and serum is collected on various days post-immunization. Serial dilutions of serum are preincubated with a virus during which time antibodies in the serum that are specific for the virus will bind to it. The virus/serum mixture is then added to permissive cells to determine infectivity by a plaque assay or microneutralization assay. If antibodies in the serum neutralize the virus, there are fewer plaques or lower relative luciferase units compared to a control group.

Non-human animals (e.g., rodents, e.g., rats or mice) as described herein produce human antibody variable regions and, therefore, provide an *in vivo* system for the production of human antibodies for use in diagnostic applications (e.g., immunology, serology, microbiology, cellular pathology, etc.). In various embodiments, non-human animals as described herein may be used to produce human antibody variable regions that bind relevant antigenic sites for identification of cellular changes such as, for example, expression of specific cell surface markers indicative of pathological changes. Such antibodies can be conjugated to various chemical entities (e.g., a radioactive tracer) and be employed in various *in vivo* and/or *in vitro* assays as desired.

Non-human animals (e.g., rodents, e.g., rats or mice) as described herein provide an improved *in vivo* system for development and selection of human antibodies for use in oncology and/or infectious diseases. In various embodiments, non-human animals as described herein and control non-human animals (e.g., having a genetic modification that is different than as described herein or no genetic modification, i.e., wild-type) may be implanted with a tumor (or tumor cells) or infected with a virus (e.g., influenza, HIV, HCV, HPV, etc.). Following implantation of infection, non-human animals may be administered a candidate therapeutic. The tumor or virus may be allowed sufficient time to be established in one or more locations within the non-human animals prior to administration of a candidate therapeutic. Alternatively, and/or additionally, the immune response may be monitored in such non-human animals so as to characterize and select potential human antibodies that may be developed as a therapeutic.

### Antigen-binding Proteins Binding More Than One Epitope

The compositions and methods described herein can be used to make binding proteins that bind more than one epitope, e.g., bispecific antibodies. Advantages of the invention include the ability to select suitably high binding (e.g., affinity matured) heavy chain immunoglobulin chains each of which will associate with a single light chain.

Synthesis and expression of bispecific binding proteins has been problematic, in part due to issues associated with identifying a suitable light chain that can associate and express with two different heavy chains, and in part due to isolation issues. The methods and compositions described herein allow for a genetically modified non-human animal (e.g., rodent, e.g., rat or mouse) to select, through otherwise natural processes, a suitable light chain including a human λ light chain variable domain that can associate and express with more than one heavy chain, including heavy chains that are somatically hypermutated (e.g., affinity matured). Human Vλ and V_{H} sequences from suitable B cells of immunized mice as described herein that express affinity matured antibodies having reverse chimeric heavy chains (i.e., human variable domain and non-human (e.g., rodent, e.g., rat or mouse) constant domain) can be identified and cloned in frame in an expression vector with a suitable human constant region gene sequence (e.g., a human IgG1). Two such constructs can be prepared, wherein each construct encodes a human heavy chain variable domain that binds a different epitope. A rearranged human λ light chain variable region (e.g., a human Vλ1-51/Jλ2 or a human Vλ2-14/Jλ2), including gene segments having a germline sequence or a somatically hypermutated version thereof (e.g., as identified from a B cell), can be fused in frame to a suitable human constant region gene (e.g., a human λ constant gene). These three fully-human heavy and light constructs can be placed in a suitable cell for expression. The cell will express two major species: a homodimeric heavy chain with the identical light chain, and a heterodimeric heavy chain with the identical light chain. To allow for a facile separation of these major species, one of the heavy chains is modified to omit a Protein A-binding determinant, resulting in a differential affinity of a homodimeric binding protein from a heterodimeric binding protein. Compositions and methods that address this issue are described in U.S. Patent Nos. 8,586,713; 9,309,326; and 9,982,013, each of which are hereby incorporated by reference.

In some embodiments, an antigen-binding protein as described herein is provided, where human Vλ and V_{H} sequences are derived from mice described herein that have been immunized with an antigen comprising an epitope of interest or antigen of interest.

In some embodiments, an antigen-binding protein is provided that comprises a first and a second polypeptide, a first polypeptide comprising, from N-terminal to C-terminal, a first antigen-binding domain that selectively binds a first epitope, followed by a constant domain that comprises a first C_{H}3 domain of a human IgG selected from IgG1, IgG2, and IgG4; and, a second polypeptide comprising, from N-terminal to C-terminal, a second antigen-binding domain that selectively binds a second epitope, followed by a constant domain that comprises a second C_{H}3 domain of a human IgG selected from IgG1, IgG2, and IgG4, wherein the second C_{H}3 domain comprises a modification that reduces or eliminates binding of the second C_{H}3 domain to protein A.

In some embodiments, an antigen-binding protein is provided that comprises a first heavy chain and a second heavy chain. In some embodiments, a first heavy chain comprises, from N-terminal to C-terminal, a first human heavy chain variable domain that (with a human λ light chain variable domain) selectively binds a first epitope, followed by a constant domain that comprises a first C_{H}3 domain of a human IgG selected from IgG1, IgG2, and IgG4. In some embodiments, a second heavy chain comprises, from N-terminal to C-terminal, a second human heavy chain variable domain that (with the same human λ light chain variable domain) selectively binds a second epitope (which can be on the same or a different antigen than a first epitope), followed by a constant domain that comprises a second C_{H}3 domain of a human IgG selected from IgG1, IgG2, and IgG4, wherein the second C_{H}3 domain comprises a modification that reduces or eliminates binding of the second C_{H}3 domain to protein A.

In some embodiments, a C_{H}3 domain comprises a modification that reduces or eliminates binding of the C_{H}3 domain to protein A. In some embodiments, a modification that reduces or eliminates binding of a C_{H}3 domain to protein A comprises an H95R modification (by IMGT exon numbering; H435R by EU numbering). In some embodiments, a modification that reduces or eliminates binding of a C_{H}3 domain to protein A comprises a Y96F modification (IMGT; Y436F by EU). In some embodiments, a modification that reduces or eliminates binding of a C_{H}3 domain to protein A comprises an H95R modification (by IMGT exon numbering; H435R by EU numbering) and a Y96F modification (IMGT; Y436F by EU).

In some embodiments, a C_{H}3 domain is an IgG1 domain comprising an H95R modification (by IMGT exon numbering; H435R by EU numbering), a Y96F modification (IMGT; Y436F by EU), or both, and further comprises a D16E modification, a L18M modification, a N44S modification, a K52N modification, a V57M modification, a V82I modification, or a combination thereof (IMGT; D356E, L358M, N384S, K392N, V397M, V422I, or combination thereof by EU).

In some embodiments, a C_{H}3 domain is an IgG2 domain comprising an H95R modification (by IMGT exon numbering; H435R by EU numbering), a Y96F modification (IMGT; Y436F by EU), or both, and further comprises a N44S modification, a K52N modification, a V82I modification, or a combination thereof (IMGT; N384S, K392N, V422I, or combination thereof by EU).

In some embodiments, a C_{H}3 domain is an IgG4 domain comprising an H95R modification (by IMGT exon numbering; H435R by EU numbering), a Y96F modification (IMGT; Y436F by EU), or both, and further comprises a Q15R modification, an N44S modification, a K52N modification, a V57M modification, a R69K modification, a E79Q modification, and a V82I modification (IMGT; Q355R, N384S, K392N, V397M, R409K, E419Q, V422I, or combination thereof by EU).

One method for making an antigen-binding protein that binds more than one epitope is to immunize a first non-human animal (e.g., rodent, e.g., rat or mouse) with a limited human immunoglobulin λ light chain variable region repertoire, as described herein, with a first antigen that comprises a first epitope of interest, where the non-human animal (e.g., rodent, e.g., rat or mouse) includes one or more unrearranged human V_{H} gene segments, one or more unrearranged human D gene segments, and one or more unrearranged human J_{H} gene segments operably linked to a non-human immunoglobulin heavy chain constant region. When immunized, such a non-human animal will make a reverse chimeric antibody, comprising a human λ light chain variable domain expressed from the limited human immunoglobulin λ light chain variable region repertoire (e.g., a single human rearranged λ light chain variable region). A B cell can be identified that encodes a human immunoglobulin heavy chain variable domain that binds the epitope of interest. In some embodiments, a nucleotide sequence encoding the human immunoglobulin heavy chain variable domain can be retrieved (e.g., by PCR) and cloned into an expression construct in frame with a suitable human immunoglobulin heavy chain constant domain. In some embodiments, this process can be repeated to identify a second human immunoglobulin heavy chain variable domain that binds a second epitope of a second antigen, and a second human immunoglobulin heavy chain variable domain can be retrieved and cloned into an expression vector in frame to a second suitable immunoglobulin constant region. In some embodiments, a nucleotide sequence encoding a human immunoglobulin λ light chain variable domain can be retrieved (e.g., by PCR) and cloned into an expression construct in frame with a suitable human immunoglobulin λ light chain constant region. In some embodiments, a first and second immunoglobulin human heavy chain constant domain can be the same or different isotype. In some embodiments, one of the immunoglobulin human heavy chain constant domains (but not the other) can be modified as described herein or in U.S. Patent Nos. 8,586,713; 9,309,326; and 9,982,013, each of which are hereby incorporated by reference. In some embodiments, an antigen-binding protein can be expressed in a suitable cell and isolated based on its differential affinity for Protein A as compared to a homodimeric antigen-binding protein, e.g., as described in U.S. Patent Nos. 8,586,713; 9,309,326; and 9,982,013, each of which are hereby incorporated by reference. In some embodiments, a first and second antigen can be the same and a first and second epitopes can be different. In some embodiments, a first and second antigen are different. In some embodiments, a bispecific antigen-binding protein binds an antigen with higher affinity than a monospecific antigen-binding protein that binds the same antigen. In some embodiments, a bispecific antigen-binding protein binds a first epitope and a second epitope with different affinities.

In some embodiments, a method for making a bispecific antigen-binding protein is provided, comprising identifying a first affinity-matured (e.g., comprising one or more somatic hypermutations) human heavy chain variable domain from a non-human animal (e.g., rodent, e.g., rat or mouse) as described herein, which includes a single human rearranged λ light chain variable region at an endogenous κ light chain locus, and one or more unrearranged human V_{H} gene segements, one or more unrearranged human D_{H} gene segments, and one or more unrearranged human J_{H} gene segments operably linked to a non-human immunoglobulin heavy chain constant region. In some embodiments, a method for making a bispecific antigen-binding protein comprises identifying a second affinity-matured (e.g., comprising one or more somatic hypermutations) heavy chain variable domain from a non-human animal (e.g., rodent, e.g., rat or mouse) as described herein, which includes a single human rearranged λ light chain variable region at an endogenous κ light chain locus (e.g. the same single human rearranged λ light chain variable region as in the non-human animal used to generate the first variable domain), and one or more unrearranged human V_{H} gene segements, one or more unrearranged human D_{H} gene segments, and one or more unrearranged human J_{H} gene segments operably linked to a non-human immunoglobulin heavy chain constant region. In some embodiments, a nucleotide sequence encoding a first affinity- matured human heavy chain variable domain is cloned in frame with a human heavy chain constant region lacking a Protein A-determinant modification, as described in U.S. Patent Nos. 8,586,713; 9,309,326; and 9,982,013, each of which are hereby incorporated by reference, to form a first nucleotide sequence encoding a fully human heavy chain. In some embodiments, a nucleotide sequence encoding a second affinity- matured human heavy chain variable domain is cloned in frame with a human heavy chain constant region comprising a Protein A-determinant as described in U.S. Patent Nos. 8,586,713; 9,309,326; and 9,982,013, each of which are hereby incorporated by reference, to form a second fully human heavy chain. In some embodiments, a nucleotide encoding a first fully human heavy chain, as described in this paragraph, and a nucleotide encoding a second fully human heavy chain, as described in this paragraph, are introduced into a host cell (e.g., a mammalian cell). In some embodiments, a nucleotide encoding a first fully human heavy chain, as described in this paragraph, is included on a first expression vector. In some embodiments, a nucleotide encoding a second fully human heavy chain, as described in this paragraph, is included on a second expression vector. In some instances, the first and second expression vectors are the same or different. In some embodiments, a method of making a bispecfic antibody comprises expressing a nucleotide encoding a first fully human heavy chain, as described in this paragraph, a nucleotide encoding a second fully human heavy chain, as described in this paragraph, and a nucleotide encoding a human λ light chain comprising a human λ light chain variable domain in a host cell so that a bispecific antibody is formed, where the human λ light chain variable domain has a sequence produced by a non-human animal as described herein having a limited human λ light chain variable region repertoire (e.g., a single human rearranged λ light chain variable region). In other embodiments, a human λ light chain variable domain has a sequence derived from the same human V/J rearrangement as present in a non-human animal (e.g., the same single human rearranged λ light chain variable region as present in a non-human animal). In some embodiments, a method of making a bispecific antibody comprises obtaining a bispecific antibody, e.g., from the host cell or its culture medium.

In some embodiments, host cells can be cells of prokaryotes and eukaryotes (single-cell or multiple-cell), bacterial cells (e.g., strains of Escherichia coli, Bacillus spp., Streptomyces spp., etc.), mycobacteria cells, fungal cells, yeast cells (e.g., Saccharomyces cerevisiae, Schizosaccharomyces pombe, Pichia pastoris, Pichia methanolica, etc.), plant cells, insect cells (e.g., SF-9, SF-21, baculovirus-infected insect cells, Trichoplusia ni, etc.), non-human animal cells, human cells, or cell fusions such as, for example, hybridomas or quadromas. In some embodiments, a cell is a human, monkey, ape, hamster, rat, or mouse cell. In some embodiments, a cell is eukaryotic and is selected from the following cells: CHO (e.g., CHO K1, DXB-11 CHO, Veggie-CHO), COS (e.g., COS-7), retinal cell, Vero, CV1, kidney (e.g., HEK293, 293 EBNA, MSR 293, MDCK, HaK, BHK), HeLa, HepG2, WI38, MRC 5, Colo205, HB 8065, HL-60, (e.g., BHK21), Jurkat, Daudi, A431 (epidermal), CV-1, U937, 3T3, L cell, C127 cell, SP2/0, NS-0, MMT 060562, Sertoli cell, BRL 3A cell, HT1080 cell, myeloma cell, tumor cell, and a cell line derived from an aforementioned cell. In some embodiments, a cell comprises one or more viral genes, e.g., a retinal cell that expresses a viral gene (e.g., a PER.C6^{®} cell).

### Pharmaceutical Compositions

In some embodiments, an antigen-binding protein, a nucleic acid encoding an antigen-binding protein, or a therapeutically relevant portion thereof produced by a non-human animal disclosed herein or derived from an antibody, a nucleic acid, or a therapeutically relevant portion thereof produced by a non-human animal (e.g., rodent, e.g., rat or mouse) disclosed herein can be administered to a subject (e.g., a human subject). In some embodiments, a pharmaceutical composition includes an antibody produced by a non-human animal disclosed herein. In some embodiments, a pharmaceutical composition can include a buffer, a diluent, an excipient, or any combination thereof. In some embodiments, a composition, if desired, can also contain one or more additional therapeutically active substances.

Although the descriptions of pharmaceutical compositions provided herein are principally directed to pharmaceutical compositions that are suitable for ethical administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with routine, if any, experimentation.

For example, a pharmaceutical composition provided herein may be in a sterile injectable form (e.g., a form that is suitable for subcutaneous injection or intravenous infusion). For example, in some embodiments, a pharmaceutical composition is provided in a liquid dosage form that is suitable for injection. In some embodiments, a pharmaceutical composition is provided as powders (e.g., lyophilized and/or sterilized), optionally under vacuum, which can be reconstituted with an aqueous diluent (e.g., water, buffer, salt solution, etc.) prior to injection. In some embodiments, a pharmaceutical compositionsis diluted and/or reconstituted in water, sodium chloride solution, sodium acetate solution, benzyl alcohol solution, phosphate buffered saline, etc. In some embodiments, a powder should be mixed gently with the aqueous diluent (e.g., not shaken).

Formulations of the pharmaceutical compositions described herein may be prepared by any method known or hereafter developed in the art of pharmacology. In general, such preparatory methods include the step of bringing the active ingredient into association with a diluent or another excipient and/or one or more other accessory ingredients, and then, if necessary and/or desirable, shaping and/or packaging the product into a desired single- or multidose unit.

In some embodiments, a pharmaceutical composition including an antibody produced by a non-human animal (e.g., rodent, e.g., rat or mouse) disclosed herein can be included in a container for storage or administration, for example, a vial, a syringe (e.g., an IV syringe), or a bag (e.g., an IV bag). A pharmaceutical composition in accordance with the present disclosure may be prepared, packaged, and/or sold in bulk, as a single unit dose, and/or as a plurality of single unit doses. As used herein, a "unit dose" is discrete amount of the pharmaceutical composition comprising a predetermined amount of the active ingredient. The amount of the active ingredient is generally equal to the dosage of the active ingredient that would be administered to a subject and/or a convenient fraction of such a dosage such as, for example, one-half or one-third of such a dosage.

Relative amounts of the active ingredient, a pharmaceutically acceptable excipient, and/or any additional ingredients in a pharmaceutical composition in accordance with the disclosure will vary, depending upon the identity, size, and/or condition of the subject treated and further depending upon the route by which the composition is to be administered. By way of example, a composition may comprise between 0.1% and 100% (w/w) active ingredient.

A pharmaceutical composition may additionally comprise a pharmaceutically acceptable excipient, which, as used herein, includes any and all solvents, dispersion media, diluents, or other liquid vehicles, dispersion or suspension aids, surface active agents, isotonic agents, thickening or emulsifying agents, preservatives, solid binders, lubricants and the like, as suited to the particular dosage form desired. Remington's The Science and Practice of Pharmacy, 21st Edition, A. R. Gennaro (Lippincott, Williams & Wilkins, Baltimore, MD, 2006) discloses various excipients used in formulating pharmaceutical compositions and known techniques for the preparation thereof. Except insofar as any conventional excipient medium is incompatible with a substance or its derivatives, such as by producing any undesirable biological effect or otherwise interacting in a deleterious manner with any other component(s) of a pharmaceutical composition, its use is contemplated to be within the scope of this disclosure.

In some embodiments, a pharmaceutically acceptable excipient is at least 95%, at least 96%, at least 97%, at least 98%, at least 99%, or 100% pure. In some embodiments, an excipient is approved for use in humans and for veterinary use. In some embodiments, an excipient is approved by the United States Food and Drug Administration. In some embodiments, an excipient is pharmaceutical grade. In some embodiments, an excipient meets the standards of the United States Pharmacopoeia (USP), the European Pharmacopoeia (EP), the British Pharmacopoeia, and/or the International Pharmacopoeia.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Such excipients may optionally be included in pharmaceutical formulations. Excipients such as cocoa butter and suppository waxes, coloring agents, coating agents, sweetening, flavoring, and/or perfuming agents can be present in the composition, according to the judgment of the formulator.

In some embodiments, a provided pharmaceutical composition comprises one or more pharmaceutically acceptable excipients (e.g., preservative, inert diluent, dispersing agent, surface active agent and/or emulsifier, buffering agent, etc.). In some embodiments, a pharmaceutical composition comprises one or more preservatives. In some embodiments, a pharmaceutical composition comprises no preservative.

In some embodiments, a pharmaceutical composition is provided in a form that can be refrigerated and/or frozen. In some embodiments, a pharmaceutical composition is provided in a form that cannot be refrigerated and/or frozen. In some embodiments, reconstituted solutions and/or liquid dosage forms may be stored for a certain period of time after reconstitution (e.g., 2 hours, 12 hours, 24 hours, 2 days, 5 days, 7 days, 10 days, 2 weeks, a month, two months, or longer). In some embodiments, storage of antibody compositions for longer than the specified time results in antibody degradation.

Liquid dosage forms and/or reconstituted solutions may comprise particulate matter and/or discoloration prior to administration. In some embodiments, a solution should not be used if discolored or cloudy and/or if particulate matter remains after filtration.

General considerations in the formulation and/or manufacture of pharmaceutical agents may be found, for example, in Remington: The Science and Practice of Pharmacy 21st ed., Lippincott Williams & Wilkins, 2005.

### Kits

The present disclosure further provides a pack or kit comprising one or more containers filled with at least non-human cell, protein (single or complex (e.g., an antibody or fragment thereof)), DNA fragment, targeting vector, or any combination thereof, as described herein. Kits may be used in any applicable method (e.g., a research method). Optionally associated with such container(s) can be a notice in the form prescribed by a governmental agency regulating the manufacture, use or sale of pharmaceuticals or biological products, which notice reflects (a) approval by the agency of manufacture, use or sale for human administration, (b) directions for use, and/or (c) a contract that governs the transfer of materials and/or biological products (e.g., a non-human animal or non-human cell as described herein) between two or more entities and combinations thereof.

In some embodiments, a kit comprising a non-human cell, non-human tissue, immortalized cell, non-human ES cell, or non-human embryo as described herein is provided. In some embodiments, a kit comprising an amino acid (e.g., an antibody or fragment thereof) from a non-human animal, non-human cell, non-human tissue, immortalized cell, non-human ES cell, or non-human embryo as described herein is provided. In some embodiments, a kit comprising a nucleic acid (e.g., a nucleic acid encoding an antibody or fragment thereof) from a non-human animal, non-human cell, non-human tissue, immortalized cell, non-human ES cell, or non-human embryo as described herein is provided. In some embodiments, a kit comprising a sequence (amino acid and/or nucleic acid sequence) identified from a non-human animal, non-human cell, non-human tissue, immortalized cell, non-human ES cell, or non-human embryo as described herein is provided.

In some embodiments, a kit as described herein for use in the manufacture and/or development of a drug (e.g., an antibody or fragment thereof) for therapy or diagnosis is provided.

In some embodiments, a kit as described herein for use in the manufacture and/or development of a drug (e.g., an antibody or fragment thereof) for the treatment, prevention or amelioration of a disease, disorder or condition is provided.

Other features of certain embodiments will become apparent in the course of the following descriptions of exemplary embodiments, which are given for illustration and are not intended to be limiting thereof.

### EXAMPLES

The following examples are provided so as to describe to the skilled artisan how to make and use methods and compositions described herein; and are not intended to limit the scope of the present disclosure. Unless indicated otherwise, temperature is indicated in Celsius and pressure is at or near atmospheric.

In general, targeting vectors carrying a rearranged human λ light chain variable region were made using VELOCIGENE^{®} technology (see, e.g., US Pat. No. 6,586,251 and Valenzuela et al. (2003) High-throughput engineering of the mouse genome coupled with high-resolution expression analysis, Nature Biotech. 21(6): 652-659, which are each incorporated by reference herein). Mouse genomic Bacterial Artificial Chromosome (BAC) clones were modified to generate genomic constructs that contain a single rearranged human lambda light chain variable region. The constructs were inserted into an endogenous κ light chain locus that was previously modified to delete the endogenous Vκ and Jκ gene segments.

### Example 1 - Generation of a Targeting Vector Including a Rearranged Human Vλ1-51/Jλ2 and a Mouse Cκ

A rearranged human Vλ1-51/Jλ2 was made using standard molecular biology techniques recognized in the art. The human Vλ1-51 gene segment and human Jλ2 gene segment included in the rearranged human Vλ1-51/Jλ2 had a human Vλ1-51 germline sequence and a human Jλ2 germline sequence, respectively.

A DNA fragment was made by de novo DNA synthesis (Blue Heron Biotech). The fragment contained a AscI site, 2kb of the human Vλ1-51 promoter region, a human Vλ1-51 5' untranslated region (UTR), a coding sequence of exon 1 of a human Vλ1-51 gene segment (which includes a sequence encoding a leader peptide), intron 1 of a human Vλ1-51 gene segment, a rearranged human Vλ1-51/Jλ2 variable sequence including a second exon of a human Vλ1-51 gene segment and a Jλ2 gene segment, the first 412 bp of the human Jκ5-Cκ intron (including the Jκ5 splice donor site (SD)), and a PI-SceI site (SEQ ID NO: 31; see also Figure 1A and Figures 9A-C).

The fragment described in the paragraph above was ligated into a previously described targeting vector to produce Targeting Vector A in Figure 1A. The previously described targeting vector contained an ~23kb 5' mouse homology arm derived from BAC CT7-302g12, a Frt-Ub-Neo-Frt cassette, an AscI site, a rearranged human Vκ3-20/Jκ1 variable region, a PI-SceI site, and an ~75kb 3' mouse homology arm derived from BAC CT7-254m04 (see, e.g., Figure 14B of U.S. Patent No. 9,334,334, and Figure 2 of U.S. Patent No. 10,143,186, each of which is incorporated herein by reference). The mouse homology arms were identical to those previously described by Macdonald et al. (2014) Proc. Natl. Acad. Sci. USA, 111:5147-52, which is incorporated herein by reference. The 5' arm contained ~23kb of genomic sequence upstream of the endogenous kappa locus, and the 3' arm contained ~2.4kb of the mouse Jκ-Cκ intron, the mouse Cκ exon, and ~72 kb of genomic sequence downstream of the mouse κ locus. See Figure 1A.

Targeting Vector A contained, from 5' to 3', an ~23 kb 5' mouse homology arm derived from BAC CT7-302g12, a Frt-Ub-Neo-Frt cassette, an AscI site, 2kb of the human Vλ1-51 promoter region, a human Vλ1-51 5' untranslated region (UTR), a coding sequence of exon 1 of a human Vλ1-51 gene segment (which includes a sequence encoding a leader peptide), intron 1 of a human Vλ1-51 gene segment, a rearranged human Vλ1-51/Jλ2 variable sequence including a second exon of a human Vλ1-51 gene segment and a Jλ2 gene segment, the first 412bp of the human Jκ5-Cκ intron (including the Jκ5 splice donor site (SD)), a PI-SceI site, and an ~75kb 3' mouse homology arm derived from BAC CT7-254m04, which includes ~2.4kb of the mouse Jκ-Cκ intron, the mouse Cκ exon, and ~72 kb of genomic sequence downstream of the mouse κ locus.

### Example 2 - Generation of ES Cells Including a Rearranged Human Vλ1-51/Jλ2 and a Mouse Cκ

Targeting Vector A, as described in Example 1, is electroporated into mouse ES cells in which the endogenous Vκ and Jκ gene segments have been replaced with a rearranged human Vκ3-20/Jκ1, and in which the immunoglobulin heavy chain locus comprises unrearranged human V_{H}, D, and J_{H} gene segments operably linked to mouse heavy chain constant region genes. The immunoglobulin heavy chain locus of the mouse ES cells also includes functional mouse Adam6a and Adam6b genes (see US Patent No. 10,130,081, which incorporated herein by reference). Selection for clones that have undergone homologous recombination yields modified ES cells for generating chimeric mice that express antibodies from a rearranged human Vλ1-51/Jλ2 and a mouse Cκ.

Positive ES cell clones are confirmed by TAQMAN^{™} screening using primers and probes specific for the rearranged human Vλ1-51/Jλ2 inserted into the endogenous κ locus (see Valenzuela et al., above, which is incorporated herein by reference). Approximate positions of various probes that can be used for detection are shown in Figure 3 by circled dash marks. Primers and probes used in the assay are listed among those in Table A below.

**TABLE A - Primers and Probes Used on Modification of Allele Assay**

| **Name** | **Forward Primer** | **Probe** | **Reverse Primer** |
|---|---|---|---|
| *Lambda Universal Light Chain Primers and Probes* | | | |
| hTU_ 1-51 | GGGACGAGGCCGATTAT TACTG (SEQ ID NO: 41) | CGGAACATGGATAGCA GCCTGA  (SEQ ID NO: 42) | CCTCCGCCGAATACCA CA  (SEQ ID NO: 43) |
| hTU2 _1-51 | GCATCACCGGACTCCAG ACT  (SEQ ID NO: 44) | CGAGGCCGATTATTACT GCGGAACA (SEQ ID NO: 45) | AGGACGGTCAGCTTGG TC (SEQ ID NO: 46) |
| hTU_ 2-14 | CAGGACTCGGACAATC TTCATC  (SEQ ID NO: 47) | ATGGCCTGGGCTCTGCT GCTC  (SEQ ID NO: 48) | TGTGCCCTGAGTGAGG AG (SEQ ID NO: 49) |
| hTU2 _2-14 | AGCGCAGAAGGCAGGAC TC (SEQ ID NO: 50) | ACAATCTTCATCATGGC CTGGGCTC (SEQ ID NO: 51) | CGTCACCTGTGCCCTGA G (SEQ ID NO: 52) |
| hTU3 _1-51pro | TGTTGCCCAAGCTGGAG TG (SEQ ID NO: 53) | TGGCATGATCTCGGCTC ACTGC  (SEQ ID NO: 54) | GAGGCTGAGGCAGGAG AA  (SEQ ID NO: 55) |
| mIgK C-4 | GGCCACTCACAAGACAT CAAC  (SEQ ID NO: 56) | TCACCCATTGTCAAGAG CTTCAACA (SEQ ID NO: 57) | CGTCTCAGGACCTTTGT CTCTAAC  (SEQ ID NO: 58) |
| mIgK LC1-2 | TCCTTGTTACTTCATACC ATCCTCT  (SEQ ID NO: 59) | TTCCTTCCTCAGGCCAG CCC (SEQ ID NO: 60) | AGGGTGACTGATGGCG AAGACT  (SEQ ID NO: 61) |

| *Parental Primers and Probes* | | | |
|---|---|---|---|
| 1635h 2 | TCCAGGCACCCTGTCTTT G  (SEQ ID NO: 62) | AAAGAGCCACCCTCTCC TGCAGGG (SEQ ID NO: 63) | AAGTAGCTGCTGCTAA CACTCTGACT (SEQ ID NO: 64) |
| ULCm 1 | AGGTGAGGGTACAGATA AGTGTTATGAG (SEQ ID NO: 65) | CCATTATGATGCTCCAT GCCTCTCTGTTC (SEQ ID NO: 66) | TGACAAATGCCCTAAT TATAGTGATCA (SEQ ID NO: 67) |
| Neo | GGTGGAGAGGCTATTCG GC  (SEQ ID NO: 68) | TGGGCACAACAGACAAT CGGCTG (SEQ ID NO: 69) | GAACACGGCGGCATCA G  (SEQ ID NO: 70) |
| Hyg | TGCGGCCGATCTTAGCC (SEQ ID NO: 71) | ACGAGCGGGTTCGGCCC ATTC  (SEQ ID NO: 72) | TTGACCGATTCCTTGCG G (SEQ ID NO: 73) |

ES cells bearing an engineered endogenous κ light chain locus including a rearranged human Vλ1-51/Jλ2 (see Figure 3) arere transfected with a construct that expresses FLP in order to remove the FRTed neomycin cassette introduced by the targeting construct. Optionally, the neomycin cassette is removed by breeding to mice that express FLP recombinase (e.g., US 6,774,279, which is incorporated by reference in its entirety). Optionally, the neomycin cassette is retained in the mice.

### Example 3 - Generation of Mice Expressing Light Chains Derived from a Rearranged Human Vλ1-51/Jλ2 and a Mouse Cκ

Targeted ES cells described in Example 2 above are used as donor ES cells and introduced into an 8-cell stage mouse embryo by the VELOCIMOUSE^{®} method (see, e.g., US Pat. No. 7,294,754 and Poueymirou et al. (2007) F0 generation mice that are essentially fully derived from the donor gene-targeted ES cells allowing immediate phenotypic analyses, Nature Biotech. 25(1): 91-99, each of which are incorporated herein by reference). VELOCIMICE^{®} bearing an engineered endogenous κ light chain locus including a rearranged human Vλ1-51/Jλ2 linked to a mouse Cκ are identified by genotyping using a modification of allele assay (Valenzuela et al., above, which is incorporated herein by reference) that detects the presence of the rearranged human Vλ1-51/Jλ2. Mice bearing engineered endogenous κ light chain locus including a rearranged human Vλ1-51/Jλ2 linked to a mouse Cx at one locus allele may bear engineered endogenous κ light chain locus comprising a rearranged human Vκ3-20/Jκ1 linked to a mouse mouse Cκ at the second locus allele, and contain engineered immunoglobulin heavy chain locus comprising unrearranged human V_{H}, D, and J_{H} gene segments operably linked to mouse heavy chain constant region genes and functional mouse Adam6a and Adam6b genes.

Mice are bred to homozygosity for the engineered endogenous κ light chain locus including a rearranged human Vλ1-51/Jλ2 and a mouse Cκ, and homozygosity for engineered immunoglobulin heavy chain locus comprising unrearranged human V_{H}, D, and J_{H} gene segments operably linked to mouse heavy chain constant region genes and functional mouse Adam6a and Adam6b genes. In one method, this can be accomplished by first breeding the mouse comprising engineered endogenous κ light chain locus including a rearranged human Vλ1-51/Jλ2 linked to a mouse Cx at one light chain locus allele, engineered endogenous κ light chain locus comprising a rearranged human Vκ3-20/Jκ1 linked to a mouse Cx at the second light chain locus allele, and an engineered immunoglobulin heavy chain locus comprising unrearranged human V_{H}, D, and J_{H} gene segments operably linked to mouse heavy chain constant region genes and functional mouse Adam6a and Adam6b genes (at both heavy chain alleles) to mice comprising a knockout (KO) of endogenous heavy and light chain kappa loci, and optionally also a KO of endogenous lambda loci, as described further below. Alternatively, mice can be bred to mice comprising a KO of endogenous light chain kappa loci, optionally a KO of endogenous lambda loci, and homozygous for an engineered immunoglobulin heavy chain locus comprising unrearranged human V_{H}, D, and J_{H} gene segments operably linked to mouse heavy chain constant region genes and functional mouse Adam6a and Adam6b genes. Resultant mice are bred to homozygosity at immunoglobulin loci, e.g., modified and/or endogenous immunoglobulin loci, e.g., humanized lambda light chain and heavy chain loci.

Pups are genotyped and pups heterozygous or homozygous for the engineered endogenous κ light chain locus including a rearranged human Vλ1-51/Jλ2 and a mouse Cκ are selected and expression of human λ light chain variable domains from the rearranged human Vλ1-51/Jλ2 or somatically hypermutated variants thereof is assessed and characterized. B cell surface expression of human Vλ1-51/Jλ2 in mice comprising engineered light chain described herein is detected using FACS analysis.

### Example 4 - Generation of a Targeting Vector Including a Rearranged Human Vλ1-51/Jλ2 and a Mouse Cλ1

To replace the mouse Cκ in Targeting Vector A with a mouse Cλ1, an additional cloning step was performed (see Figure 1B). Targeting Vector A was cut *in vitro* with two Cas9:gRNA complexes: the first cutting upstream of the intronic kappa enhancer (Eᵢ), and the second cutting downstream of the Cκ. Gibson assembly was then performed to replace the deleted region with a 4.9kb synthetic fragment containing a 45bp overlap with the 3' end of the Cas9-cut targeting vector, Eᵢ, the mouse Cλ1, a loxP-Ub-Hyg-loxP cassette, and an 80bp overlap with the 5' end of the Cas9-cut targeting vector. The synthetic fragment was obtained from a restriction fragment of plasmid pE of Figure 1A of PCT/US2018/063841, which is incorporated herein by reference. The resulting targeting vector, Targeting Vector B, is shown in Figure 1B.

Targeting Vector B is identical to Targeting Vector A, except that the mouse Cκ was replaced with a mouse Cλ1 and a loxP-Ub-Hyg-loxP cassette was inserted downstream of the Cκ polyA signal.

### Example 5 - Generation of ES Cells Including a Rearranged Human Vλ1-51/Jλ2 and a Mouse Cλ1

Targeting Vector B, as described in Example 4, was electroporated into mouse ES cells in which the endogenous Vκ and Jκ gene segments have been replaced with a rearranged human Vκ3-20/Jκ1, and in which the immunoglobulin heavy chain locus comprised unrearranged human V_{H}, D, and J_{H} gene segments operably linked to mouse heavy chain constant region genes. The immunoglobulin heavy chain locus of the mouse ES cells also included functional mouse Adam6a and Adam6b genes (see US Patent No. 10,130,081, which incorporated herein by reference). Selection for clones that have undergone homologous recombination yielded modified ES cells for generating chimeric mice that express antibodies from a rearranged human Vλ1-51/Jλ2 and a mouse Cλ1.

Positive ES cell clones were confirmed by TAQMAN^{™} screening using primers and probes specific for the rearranged human Vλ1-51/Jλ2 and mouse Cλ1 inserted into the endogenous κ locus (see Valenzuela et al., above, which is incorporated herein by reference). Approximate positions of various probes used for detection are shown in Figure 4 by circled dash marks. Primers and probes used in the assay are listed among those in Table A above.

ES cells bearing an engineered endogenous κ light chain locus including a rearranged human Vλ1-51/Jλ2 and a mouse Cλ1 (see Figure 4) were transfected with constructs that express FLP and/or CRE in order to remove the FRTed neomycin or the Floxed hygromycin cassettes. Optionally, the neomycin cassette may be removed by breeding to mice that express FLP recombinase (e.g., US 6,774,279, which is incorporated by reference in its entirety), and/or the hygromycin cassette may be removed by breeding to mice that express CRE recombinase. Optionally, the neomycin and/or the hygromycin cassettes are retained in the mice.

### Example 6 - Generation of Mice Expressing Light Chains Derived from a Rearranged Human Vλ1-51/Jλ2 and a Mouse Cλ1

Targeted ES cells described in Example 5 above were used as donor ES cells and introduced into an 8-cell stage mouse embryo by the VELOCIMOUSE^{®} method (see, e.g., US Pat. No. 7,294,754 and Poueymirou et al. (2007) F0 generation mice that are essentially fully derived from the donor gene-targeted ES cells allowing immediate phenotypic analyses, Nature Biotech. 25(1): 91-99, each of which are incorporated herein by reference). VELOCIMICE^{®} bearing an engineered endogenous κ light chain locus including a rearranged human Vλ1-51/Jλ2 and a mouse Cλ1 were identified by genotyping using a modification of allele assay (see Valenzuela et al., above, which is incorporated herein by reference) that detects the presence of the rearranged human Vλ1-51/Jλ2 or mouse Cλ1. Mice bearing engineered endogenous κ light chain locus including a rearranged human Vλ1-51/Jλ2 linked to a mouse Cλ1 at one locus allele may bear engineered endogenous κ light chain locus comprising a rearranged human Vκ3-20/Jκ1 linked to a mouse mouse Cκ at the second locus allele, and contain engineered immunoglobulin heavy chain locus comprising unrearranged human V_{H}, D, and J_{H} gene segments operably linked to mouse heavy chain constant region genes and functional mouse Adam6a and Adam6b genes.

Mice are bred to homozygosity for the modified κ light chain locus that includes the rearranged human Vλ1-51/Jλ2 and mouse Cλ1, and homozygosity for engineered immunoglobulin heavy chain locus comprising unrearranged human V_{H}, D, and J_{H} gene segments operably linked to mouse heavy chain constant region genes and functional mouse Adam6a and Adam6b genes. In one method, this can be accomplished by first breeding the mouse comprising engineered endogenous κ light chain locus including a rearranged human Vλ1-51/Jλ2 linked to a mouse Cλ1 at one light chain locus allele, engineered endogenous κ light chain locus comprising a rearranged human Vκ3-20/Jκ1 linked to a mouse Cx at the second light chain locus allele, and an engineered immunoglobulin heavy chain locus comprising unrearranged human V_{H}, D, and J_{H} gene segments operably linked to mouse heavy chain constant region genes and functional mouse Adam6a and Adam6b genes (at both heavy chain alleles) to mice comprising a knockout (KO) of endogenous heavy and light chain kappa loci, and optionally also a KO of endogenous lambda loci, as described further below. Alternatively, mice can be bred to mice comprising a KO of endogenous light chain kappa loci, optionally a KO of endogenous lambda loci, and homozygous for an engineered immunoglobulin heavy chain locus comprising unrearranged human V_{H}, D, and J_{H} gene segments operably linked to mouse heavy chain constant region genes and functional mouse Adam6a and Adam6b genes. Resultant mice are bred to homozygosity at immunoglobulin loci, e.g., modified and/or endogenous immunoglobulin loci, e.g., humanized lambda light chain and heavy chain loci.

Pups are genotyped and pups heterozygous or homozygous for the an engineered endogenous κ light chain locus including a rearranged human Vλ1-51/Jλ2 and a mouse Cλ1 are selected and expression of human λ light chain variable domains from the rearranged human Vλ1-51/Jλ2 or somatically hypermutated variants thereof is assessed and characterized. B cell surface expression of human Vλ1-51/Jλ2 in mice comprising engineered light chain described herein was detected using FACS analysis.

### Example 7 - Generation of a Targeting Vector Including a Rearranged Human Vλ2-14/Jλ2 and a Mouse Cκ

A rearranged human Vλ2-14/Jλ2 was made using standard molecular biology techniques recognized in the art. The human Vλ2-14 gene segment and human Jλ2 gene segment included in the rearranged human Vλ2-14/Jλ2 had a human Vλ2-14 germline sequence and a human Jλ2 germline sequence, respectively.

A DNA fragment was made by de novo DNA synthesis (Blue Heron Biotech). The fragment contained a AscI site, 2kb of the human Vλ1-51 promoter region, a human Vλ1-51 5' untranslated region (UTR), a coding sequence of exon 1 of a human Vλ2-14 gene segment (which includes a sequence encoding a leader peptide), intron 1 of a human Vλ2-14 gene segment, a rearranged human Vλ2-14/Jλ2 variable sequence including a second exon of a human Vλ2-14 gene segment and a Jλ2 gene segment, the first 412 bp of the human Jκ5-Cκ intron (including the Jκ5 splice donor site (SD)), and a PI-SceI site (SEQ ID NO: 36; see also Figure 2A and Figures 13A-C).

The fragment described in the paragraph above was ligated into a previously described targeting vector to produce Targeting Vector C in Figure 2A. The previously described targeting vector contained an ~23kb 5' mouse homology arm derived from BAC CT7-302g12, a Frt-Ub-Neo-Frt cassette, an AscI site, a rearranged human Vκ3-20/Jκ1 variable region, a PI-SceI site, and an ~75kb 3' mouse homology arm derived from BAC CT7-254m04 (see, e.g., Figure 14B of U.S. Patent No. 9,334,334, and Figure 2 of U.S. Patent No. 10,143,186, each of which is incorporated herein by reference). The mouse homology arms were identical to those previously described by Macdonald et al. (2014) Proc. Natl. Acad. Sci. USA, 111:5147-52, which is incorporated herein by reference. The 5' arm contained ~23kb of genomic sequence upstream of the endogenous kappa locus, and the 3' arm contained ~2.4kb of the mouse Jκ-Cκ intron, the mouse Cκ exon, and ~72 kb of genomic sequence downstream of the mouse κ locus. See Figure 2A.

Targeting Vector C contains, from 5' to 3', an ~23 kb 5' mouse homology arm derived from BAC CT7-302g12, a Frt-Ub-Neo-Frt cassette, an AscI site, 2kb of the human Vλ1-51 promoter region, a human Vλ1-51 5' UTR, a coding sequence of exon 1 of Vλ2-14 gene segment (which includes a sequence encoding a leader peptide), intron 1 of a human Vλ2-14 gene segment, a rearranged human Vλ2-14/Jλ2 variable sequence including a second exon of a human Vλ2-14 gene segment and a Jλ2 gene segment, the first 412bp of the human Jκ5-Cκ intron (including the Jκ5 splice site), a PI-SceI site, and an ~75kb 3' mouse homology arm derived from BAC CT7-254m04, which includes ~2.4kb of the mouse Jκ-Cκ intron, the mouse Cκ exon, and ~72 kb of genomic sequence downstream of the mouse κ locus.

### Example 8 - Generation of ES Cells Including a Rearranged Human Vλ2-14/Jλ2 and a Mouse Cκ

Targeting Vector C is electroporated into mouse ES cells in which the endogenous Vκ and Jκ gene segments have been replaced with a rearranged human Vκ3-20/Jκ1, and in which the immunoglobulin heavy chain locus comprises unrearranged human V_{H}, D, and J_{H} gene segments operably linked to mouse heavy chain constant region genes. The immunoglobulin heavy chain locus of the mouse ES cells also includes functional mouse Adam6a and Adam6b genes (see US Patent No. 10,130,081, which incorporated herein by reference). Selection for clones that have undergone homologous recombination yields modified ES cells for generating chimeric mice that express antibodies from a rearranged human Vλ2-14/Jλ2 and a mouse Cκ.

Positive ES cell clones are confirmed by TAQMAN^{™} screening using primers and probes specific for the rearranged human Vλ2-14/Jλ2 inserted into the endogenous κ locus (see Valenzuela et al., above, which is incorporated herein by reference). Approximate positions of various probes used for detection are shown in Figure 5 by circled dash marks. Primers and probes used in the assay are listed among those in Table A above.

ES cells bearing an engineered endogenous κ light chain locus including a rearranged human Vλ2-14/Jλ2 (see Figure 5) are transfected with a construct that expresses FLP in order to remove the FRTed neomycin cassette introduced by the targeting construct. Optionally, the neomycin cassette is removed by breeding to mice that express FLP recombinase *(e.g.,* US 6,774,279, which is incorporated by reference in its entirety). Optionally, the neomycin cassette is retained in the mice.

### Example 9 - Generation of Mice Expressing Light Chains Derived from a Rearranged Human Vλ2-14/Jλ2 and a Mouse Cκ

Targeted ES cells described in Example 8 above are used as donor ES cells and introduced into an 8-cell stage mouse embryo by the VELOCIMOUSE^{®} method (see, e.g., US Pat. No. 7,294,754 and Poueymirou et al. (2007) F0 generation mice that are essentially fully derived from the donor gene-targeted ES cells allowing immediate phenotypic analyses, Nature Biotech. 25(1): 91-99, each of which are incorporated herein by reference). VELOCIMICE^{®} bearing an engineered endogenous κ light chain locus including a rearranged human Vλ2-14/Jλ2 and a mouse Cκ are identified by genotyping using a modification of allele assay (Valenzuela et al., above, which is incorporated herein by reference) that detects the presence of the rearranged human Vλ2-14/Jλ2. Mice bearing engineered endogenous κ light chain locus including a rearranged human Vλ2-14/Jλ2 linked to a mouse Cκ at one locus allele may bear engineered endogenous κ light chain locus comprising a rearranged human Vκ3-20/Jκ1 linked to a mouse mouse Cκ at the second locus allele, and contain engineered immunoglobulin heavy chain locus comprising unrearranged human V_{H}, D, and J_{H} gene segments operably linked to mouse heavy chain constant region genes and functional mouse Adam6a and Adam6b genes.

Mice are bred to homozygosity for the engineered endogenous κ light chain locus including a rearranged human Vλ2-14/Jλ2 and a mouse Cκ, and homozygosity for engineered immunoglobulin heavy chain locus comprising unrearranged human V_{H}, D, and J_{H} gene segments operably linked to mouse heavy chain constant region genes and functional mouse Adam6a and Adam6b genes. In one method, this can be accomplished by first breeding the mouse comprising engineered endogenous κ light chain locus including a rearranged human Vλ2-14/Jλ2 and a mouse Cκ at one light chain locus allele, engineered endogenous κ light chain locus comprising a rearranged human Vκ3-20/Jκ1 linked to a mouse Cx at the second light chain locus allele, and an engineered immunoglobulin heavy chain locus comprising unrearranged human V_{H}, D, and J_{H} gene segments operably linked to mouse heavy chain constant region genes and functional mouse Adam6a and Adam6b genes (at both heavy chain alleles) to mice comprising a knockout (KO) of endogenous heavy and light chain kappa loci, and optionally also a KO of endogenous lambda loci, as described further below. Alternatively, mice can be bred to mice comprising a KO of endogenous light chain kappa loci, optionally a KO of endogenous lambda loci, and homozygous for an engineered immunoglobulin heavy chain locus comprising unrearranged human V_{H}, D, and J_{H} gene segments operably linked to mouse heavy chain constant region genes and functional mouse Adam6a and Adam6b genes. Resultant mice are bred to homozygosity at immunoglobulin loci, e.g., modified and/or endogenous immunoglobulin loci, e.g., humanized lambda light chain and heavy chain loci.

Pups are genotyped and pups heterozygous or homozygous for the engineered endogenous κ light chain locus including a rearranged human Vλ2-14/Jλ2 and a mouse Cκ are selected and expression of human λ light chain variable domains from the rearranged human Vλ2-14/Jλ2 or somatically hypermutated variants thereof is assessed and characterized. B cell surface expression of human Vλ2-14/Jλ2 in mice comprising engineered light chain described herein is detected using FACS analysis.

### Example 10 - Generation of a Targeting Vector Including a Rearranged Human Vλ2-14/Jλ2 and a Mouse Cλ1

To replace the mouse Cκ in Targeting Vector C with a mouse Cλ1, an additional cloning step was performed (see Figure 2B). Targeting Vector C was cut *in vitro* with two Cas9:gRNA complexes: the first cutting upstream of the intronic kappa enhancer (Eᵢ), and the second cutting downstream of the Cκ. Gibson assembly was then performed to replace the deleted region with a 4.9kb synthetic fragment containing a 45bp overlap with the 3' end of the Cas9-cut targeting vector, Eᵢ, the mouse Cλ1, a loxP-Ub-Hyg-loxP cassette, and an 80bp overlap with the 5' end of the Cas9-cut targeting vector. The synthetic fragment was obtained from a restriction fragment of plasmid pE of Figure 1A of PCT/US2018/063841, which is incorporated herein by reference. The resulting targeting vector, Targeting Vector D, is shown in Figure 2B.

Targeting Vector D is identical to Targeting Vector C, except that the mouse Cκ was replaced with a mouse Cλ1 and a loxP-Ub-Hyg-loxP cassette was inserted downstream of the Cκ polyA signal.

### Example 11 - Generation of ES Cells Including a Rearranged Human Vλ2-14/Jλ2 and a Mouse Cλ1

Targeting Vector D is electroporated into mouse ES cells in which the endogenous Vκ and Jκ gene segments have been replaced with a rearranged human Vκ3-20/Jκ1, and in which the immunoglobulin heavy chain locus comprises unrearranged human V_{H}, D, and J_{H} gene segments operably linked to mouse heavy chain constant region genes. The immunoglobulin heavy chain locus of the mouse ES cells also includes functional mouse Adam6a and Adam6b genes (see US Patent No. 10,130,081, which incorporated herein by reference). Selection for clones that have undergone homologous recombination yields modified ES cells for generating chimeric mice that express antibodies from a rearranged human Vλ2-14/Jλ2 and a mouse Cλ1.

Positive ES cell clones are confirmed by TAQMAN^{™} screening using primers and probes specific for the rearranged human Vλ2-14/Jλ2 and mouse Cλ1 inserted into the endogenous κ locus (see Valenzuela et al., above, which is incorporated herein by reference). Approximate positions of various probes used for detection are shown in Figure 6 by circled dash marks. Primers and probes used in the assay are listed among those in Table A above.

ES cells bearing an engineered endogenous κ light chain locus including a rearranged human Vλ2-14/Jλ2 and a mouse Cλ1 (see Figure 6) are transfected with constructs that express FLP and/or CRE in order to remove the FRTed neomycin or the Floxed hygromycin cassettes. Optionally, the neomycin cassette is removed by breeding to mice that express FLP recombinase (e.g., US 6,774,279, which is incorporated by reference in its entirety), and/or the hygromycin cassette is removed by breeding to mice that express CRE recombinase. Optionally, the neomycin and/or the hygromycin cassettes are retained in the mice.

### Example 12 - Generation of Mice Expressing Light Chains Derived from a Rearranged Human Vλ2-14/Jλ2 and a Mouse Cλ1

Targeted ES cells described in Example 11 above are used as donor ES cells and introduced into an 8-cell stage mouse embryo by the VELOCIMOUSE^{®} method (see, e.g., US Pat. No. 7,294,754 and Poueymirou et al. (2007) F0 generation mice that are essentially fully derived from the donor gene-targeted ES cells allowing immediate phenotypic analyses, Nature Biotech. 25(1): 91-99, each of which are incorporated herein by reference). VELOCIMICE^{®} bearing an engineered endogenous κ light chain locus including a rearranged human Vλ2-14/Jλ2 and a mouse Cλ1 are identified by genotyping using a modification of allele assay (see Valenzuela et al., above, which is incorporated herein by reference) that detects the presence of the rearranged human Vλ2-14/Jλ2 or mouse Cλ1. Mice bearing engineered endogenous κ light chain locus including a rearranged human Vλ2-14/Jλ2 linked to a mouse Cλ1 at one locus allele may bear engineered endogenous κ light chain locus comprising a rearranged human Vκ3-20/Jκ1 linked to a mouse mouse Cκ at the second locus allele, and contain engineered immunoglobulin heavy chain locus comprising unrearranged human V_{H}, D, and J_{H} gene segments operably linked to mouse heavy chain constant region genes and functional mouse Adam6a and Adam6b genes.

Mice are bred to homozygosity for the modified κ light chain locus that includes the rearranged human Vλ2-14/Jλ2 and mouse Cλ1, and homozygosity for engineered immunoglobulin heavy chain locus comprising unrearranged human V_{H}, D, and J_{H} gene segments operably linked to mouse heavy chain constant region genes and functional mouse Adam6a and Adam6b genes. In one method, this can be accomplished by first breeding the mouse comprising engineered endogenous κ light chain locus including a rearranged human Vλ2-14/Jλ2 and a mouse Cλ1 at one light chain locus allele, engineered endogenous κ light chain locus comprising a rearranged human Vκ3-20/Jκ1 linked to a mouse Cx at the second light chain locus allele, and an engineered immunoglobulin heavy chain locus comprising unrearranged human V_{H}, D, and J_{H} gene segments operably linked to mouse heavy chain constant region genes and functional mouse Adam6a and Adam6b genes (at both heavy chain alleles) to mice comprising a knockout (KO) of endogenous heavy and light chain kappa loci, and optionally also a KO of endogenous lambda loci, as described further below. Alternatively, mice can be bred to mice comprising a KO of endogenous light chain kappa loci, optionally a KO of endogenous lambda loci, and homozygous for an engineered immunoglobulin heavy chain locus comprising unrearranged human V_{H}, D, and J_{H} gene segments operably linked to mouse heavy chain constant region genes and functional mouse Adam6a and Adam6b genes. Resultant mice are bred to homozygosity at immunoglobulin loci, e.g., modified and/or endogenous immunoglobulin loci, e.g., humanized lambda light chain and heavy chain loci.

Pups are genotyped and pups heterozygous or homozygous for the an engineered endogenous κ light chain locus including a rearranged human Vλ2-14/Jλ2 and a mouse Cλ1 are selected and expression of human λ light chain variable domains from the rearranged human Vλ2-14/Jλ2 or somatically hypermutated variants thereof is assessed and characterized. B cell surface expression of human Vλ12-14/Jλ2 in mice comprising engineered light chain described herein is detected using FACS analysis.

### Example 13 - Breeding of Mice

This Example describes several genetically modified mouse strains that can be bred to any one of the genetically modified mice described herein to create multiple genetically modified mouse strains harboring multiple genetically modified immunoglobulin loci.

Many various breeding schemes are envisaged that result in mice homozygous at the engineered light and heavy chain loci described herein, such that the mice express light chains with rearranged human Igλ variable domain and heavy chains with rearranged human heavy chain variable domains. In one such scheme, genetically modified mice described in Examples 3, 6, 9, and 12 above, which are heterozygous for rearranged human Vλ1-51/Jλ2 or rearranged human Vλ2-14/Jλ2 light chain on one light chain locus allele (at the endogenous kappa locus) and rearranged human Vκ3-20/Jκ1 at a second light chain locus allele (at the endogenous kappa locus), and homozygous for engineered immunoglobulin heavy chain locus comprising unrearranged human V_{H}, D, and J_{H} gene segments described above, are bred to mice homozygous for deletion of endogenous mouse Igκ, Igλ and Ig heavy chain loci. The resultant progeny expressing the rearranged λ light chain variable domain is bred to each other to obtain mice homozygous for rearranged human Vλ1-51/Jλ2 or rearranged human Vλ2-14/Jλ2 light chain and homozygous for engineered immunoglobulin heavy chain locus comprising unrearranged human V_{H}, D, and J_{H} gene segments (and also comprising functional mouse Adam6a and Adam6b genes as described above). Breeding is performed by standard techniques recognized in the art and, alternatively, by a commercial breeder (e.g., The Jackson Laboratory).

**Endogenous Igκ Knockout (Igκ KO).** For example, mice comprising a rearranged human lambda light chain variable region and human heavy chain variable region described above may be bred to mice comprising a deletion of all or a part of endogenous Igκ locus that renders the endogenous Igκ light chain locus incapable of expressing an endogenous Igκ light chain. Such exemplary mice are described in Macdonald et al. (2014) Proc. Natl. Acad. Sci. USA, 111:5147-52, which is incorporated herein by reference.

**Endogenous Igλ Knockout (Igλ KO).** To optimize the usage of the limited human λ light chain variable region repertoire, mice comprising a rearranged human lambda light chain variable region and human heavy chain variable region described above are bred to mice containing a deletion of all or part of the endogenous Igλ light chain locus that renders the endogenous Igλ light chain locus incapable of expressing an endogenous λ light chain (see, e.g., Example 1 of U.S. Patent No, 9,066,502, incorporated herein by reference). In this manner, the progeny obtained will express, as their only λ light chain, light chains having variable domains expressed from the limited human λ light chain variable region repertoire (e.g., a rearranged human Vλ/Jλ as described in Examples 3, 6, 9 and 12). Mouse strains bearing limited human λ light chain variable region repertoire and a deletion of all or part of the endogenous λ light chain locus are screened for presence of the limited human λ light chain variable region repertoire and absence of endogenous mouse λ light chains.

**Endogenous Ig heavy chain knock-out (IgH KO).** For example, mice comprising a rearranged human lambda light chain variable region and unrearranged human heavy chain variable region described above may be bred to mice comprising a deletion of all or a part of endogenous Ig heavy chain locus that renders the endogenous Ig heavy chain locus incapable of expressing an endogenous Ig heavy chain. Such exemplary mice are described in Example 7 of U.S. Patent No. 10,457,960, incorporated herein by reference.

Thus, as described above, mice comprising a rearranged human lambda light chain variable region and human heavy chain variable region as described above may be bred to mice comprising a deletion of all or a part of endogenous Igλ, Igκ, and/or Ig heavy chain loci. Subsequent progeny is bred to homozygosity at immunoglobulin loci, e.g., modified and/or endogenous immunoglobulin loci, e.g., humanized lambda light chain and heavy chain loci.

### Example 14 - Heavy Chain Gene Usage and Somatic Hypermutation Frequencies of Lambda Light Chains

Heavy chain gene usage from mice comprising a rearranged human Vλ1-51/Jλ2 or rearranged human Vλ2-14/Jλ2 light chain variable region and a replacement of the endogenous heavy chain variable region with human heavy chain variable region described herein above may be determined via several methods known in the art. In one such method, Next Generation Sequencing techniques are used.

Splenocytes or bone marrow harvested from mice comprising a universal rearranged human Vλ1-51/Jλ2 or rearranged human Vλ2-14/Jλ2 light chain mice and a replacement of the endogenous heavy chain variable region with human heavy chain variable region described herein above are analyzed for heavy chain variable region segment usage of their antibodies. Bone marrow is collected from the femurs by flushing the femurs with 1x phosphate buffered saline (PBS, Gibco) containing 2.5% fetal bovine serum (FBS), and bone marrow cells are FACS sorted for different B cell subtypes if necessary. Single cell suspensions are prepared from mouse spleens. Red blood cells from spleen and bone marrow preparation are lysed with ACK lysis buffer (Gibco). Splenic B cells are positively enriched from total splenocytes by magnetic cell sorting using anti-CD19 (mouse, a marker for B cells) magnetic beads and MACS^{®} columns (Miltenyi Biotech). Total RNA is isolated from bone marrow and purified splenic B cells using an RNeasy Plus RNA isolation kit (Qiagen) according to manufacturer's instructions.

Reverse transcription is performed to generate human heavy chain cDNA containing IgM or IgG constant region sequence, using a SMARTer^{™} RACE cDNA Amplification Kit (Clontech) and an IgM or IgG specific primer. During reverse transcription, a DNA sequence, which is a reverse complement of the template switching (TS) primer, is attached to the 3' end of newly synthesized cDNAs. Purified cDNAs are amplified by two rounds of semi-nested PCR to generate a plurality of cDNAs encoding the total IgM or IgG variable domain complement expressed by cell from which mRNA was obtained, followed by a third round of PCR to attach sequencing primers and indexes. Human variable domain cDNAs are size selected using Pippin Prep (SAGE Science) and quantified by qPCR using a KAPA Library Quantification Kit (KAPA Biosystems) before loading samples onto a MiSeq sequencer (Illumina) for sequencing.

For bioinformatic analysis, Raw Illumina sequences are de-muliplexed and filtered based on quality, length and match to corresponding IgM or IgG constant region gene primer. Overlapping paired-end reads are merged and analyzed using custom in-house pipeline. The pipeline uses local installation of IgBLAST (NCBI, v2.2.25+) to align rearranged heavy chain sequences to human germline heavy chain V, D, and J gene segment database, and denotes productive and non-productive joinings along with the presence of stop codons. CDR3 sequences and expected non-template nucleotides are extracted using boundaries as defined in International Immunogenetics Information System (IMGT).

The above method allows determination of various human V, D, and J segment usage as well as frequency of specific human VDJ rearrangement in universal rearranged human light chain mice described herein.

Similar Next Generation Sequencing techniques can be used to determine the presence and frequency of somatic hypermutations in the lambda light chain of mice comprising universal rearranged human Vλ1-51/Jλ2 or Vλ2-14/Jλ2 light chain variable region. For example, for mice comprising universal rearranged human Vλ1-51/Jλ2 or Vλ2-14/Jλ2 light chain variable region linked to a mouse Cλ (e.g., Cλ1) constant region, reverse transcription PCR described above is performed to generate cDNA containing immunoglobulin λ constant region (e.g., Cλ1) gene sequence using immunoglobulin Cλ (e.g., Cλ1) specific primers, and the bioinformatic analysis is conducted to align rearranged light chain sequences to human germline Vλ and Jλ gene segment database. For universal rearranged human Vλ1-51/Jλ2 or Vλ2-14/Jλ2 light chain variable region linked to a mouse Cκ constant region, mouse Cκ specific primers are used instead. Presence and frequency of somatic hypermutations in the lambda light chain is quantified.

### Example 15 - Generation of Human Antibodies from Mice

After breeding mice that comprise a limited human λ light chain variable region repertoire (e.g., a rearranged human Vλ/Jλ as described in Examples 3, 6, 9 and 12) to various desired strains containing modifications and deletions of other endogenous Ig loci (as described, e.g., in Example 13), selected mice can be immunized with an antigen of interest.

Generally, mice comprising a limited human λ light chain variable region repertoire (mice that comprise in their germline genomes human heavy chain variable region gene segments and a replacement of the endogenous mouse κ light chain locus with either the engineered Vλ1-51/Jλ2 human light chain region or the engineered Vλ2-14/Jλ2 human light chain region (described above), with or without removal of endogenous λ locus), are immunized with an antigen of interest. Bleeds are collected and the antibody immune response is monitored by a standard antigen-specific ELISA immunoassay.

When a desired immune response is achieved, in one method, splenocytes (and/or other lymphatic tissue) are harvested and fused with mouse myeloma cells to preserve their viability and form immortal hybridoma cell lines. Generated hybridoma cell lines are screened (e.g., by an ELISA assay) and selected to identify hybridoma cell lines that produce antigen-specific antibodies. Hybridomas may be further characterized for relative binding affinity and isotype as desired. Using this technique, and the immunogen described above, several antigen-specific chimeric antibodies (i.e., antibodies possessing human variable domains and mouseconstant domains) are obtained.

Alternatively, in another method, DNA encoding antigen-specific chimeric antibodies produced by B cells of the engineered mice described and/or exemplified herein, and/or the variable domains of λ light and/or heavy chains thereof, may be isolated directly from antigen-specific B cells. For example, high affinity chimeric antibodies having a human variable region and a mouse constant region may be isolated and characterized, and particular antibodies (and/or B cells that produce them) of interest are defined. To give but a few examples, assessed characteristics of such antibodies, and/or variable and/or constant regions thereof, may be or include one or more of affinity, selectivity, identity of epitope, etc. Antigen-specific antibodies are isolated directly from antigen-positive B cells (from immunized mice) without fusion to myeloma cells, as described in, e.g., U.S. Patent No. 7,582,298, specifically incorporated herein by reference in its entirety.

DNA encoding the variable regions of heavy chain and λ light chains may be isolated or otherwise prepared, and may be linked to human heavy chain (e.g., of a desired isotype) and human λ light chain constant regions, respectively, for the preparation of fully-human antibodies. The isolated heavy and λ light chain variable regions may be somatically mutated. This adds additional diversity to the antigen-specific repertoire. Fully-human antibodies (and/or heavy or λ light chains thereof) may be produced in a cell, typically a mammalian cell such as a CHO cell. Fully human antibodies may then be characterized for relative binding affinity and/or neutralizing activity of the antigen of interest.

### Example 16 - Binding Affinity of Bispecific Antibodies

Fully human bispecific antibodies are constructed from cloned human heavy chain variable regions of selected monospecific antibodies specific to the antigen of interest described above using standard recombinant DNA techniques known in the art. Briefly, two human heavy chains comprising human heavy chain variable regions from two selected monospecific antibodies obtained from mice comprising either the engineered human Vλ1-51/Jλ2 light chain or engineered human Vλ2-14/Jλ2 light chain region are expressed in a suitable cell line together with the human light chain comprising the same λ variable region as present in the mouse, or a somatically hypermutated version thereof; and bispecific antibody comprising desired characteristics (e.g., desired affinity for two antigens) is obtained.

Binding of bispecific or parental monospecific antibodies specific to the antigen of interest to all or a portion of the antigen is determined using a real-time surface plasmon resonance biosensor assay on a BIACORE^{™} 2000 instrument (GE Healthcare).

### CERTAIN EMBODIMENTS

Embodiment 1. A genetically modified rodent, whose germline genome comprises:
   an engineered endogenous immunoglobulin κ light chain locus comprising a single rearranged human immunoglobulin λ light chain variable region operably linked to a rodent Cλ gene segment, wherein the single rearranged human immunoglobulin λ light chain variable region comprises a human Vλ gene segment and a human Jλ gene segment,
   wherein all immunoglobulin λ light chains expressed by B cells of the genetically modified rodent include human immunoglobulin λ light chain variable domains expressed from the single rearranged human immunoglobulin λ light chain variable region or a somatically hypermutated version thereof.
Embodiment 2. The genetically modified rodent of embodiment 1, wherein the germline genome of the genetically modified rodent is homozygous for the engineered endogenous immunoglobulin κ light chain locus.
Embodiment 3. The genetically modified rodent of embodiment 1, wherein the germline genome of the genetically modified rodent is heterozygous for the engineered endogenous immunoglobulin κ light chain locus.
Embodiment 4. The genetically modified rodent of any of embodiments 1-3, whose germline genome further comprises:
   an engineered endogenous immunoglobulin heavy chain locus comprising one or more unrearranged human V_{H} gene segments, one or more unrearranged human D_{H} gene segments, and one or more unrearranged human J_{H} gene segments operably linked to one or more rodent immunoglobulin heavy chain constant region genes,
   wherein all heavy chains expressed by B cells of the genetically modified rodent include human immunoglobulin heavy chain variable domains and rodent immunoglobulin heavy chain constant domains.
Embodiment 5. The genetically modified rodent of embodiment 4, wherein the germline genome of the genetically modified rodent is homozygous for the engineered endogenous immunoglobulin heavy chain locus.
Embodiment 6. The genetically modified rodent of any one of embodiments 1-5, wherein the genetically modified rodent lacks a rodent Cκ gene at the engineered endogenous immunoglobulin κ light chain locus.
Embodiment 7. The genetically modified rodent of any one of embodiments 1-6, wherein the human Vλ gene segment is selected from a group consisting of: Vλ1-51, Vλ5-45, Vλ1-44, Vλ1-40, Vλ3-21, and Vλ2-14.
Embodiment 8. The genetically modified rodent of any one of embodiments 1-7, wherein the human Jλ gene segment is selected from a group consisting of: Jλ1, Jλ2, Jλ3, Jλ6, and Jλ7.
Embodiment 9. The genetically modified rodent of any one of embodiments 4-8, wherein the one or more unrearranged human V_{H} gene segments, one or more unrearranged human D_{H} gene segments, and one or more unrearranged human J_{H} gene segments are in place of one or more endogenous V_{H} gene segments, one or more endogenous D_{H} gene segments, one or more endogenous J_{H} gene segments, or a combination thereof.
Embodiment 10. The genetically modified rodent of any one of embodiments 4-9, wherein the one or more unrearranged human V_{H} gene segments, one or more unrearranged human D_{H} gene segments, and one or more unrearranged human J_{H} gene segments replace one or more endogenous V_{H} gene segments, one or more endogenous D_{H} gene segments, and one or more endogenous J_{H} gene segments, respectively.
Embodiment 11. The genetically modified rodent of embodiment 9 or 10, wherein the one or more rodent immunoglobulin heavy chain constant region genes are one or more endogenous rodent immunoglobulin heavy chain constant region genes.
Embodiment 12. The genetically modified rodent of any one of embodiments 4-11, wherein:
   (i) the one or more unrearranged human V_{H} gene segments comprise V_{H}3-74, V_{H}3-73, V_{H}3-72, V_{H}2-70, V_{H}1-69, V_{H}3-66, V_{H}3-64, V_{H}4-61, V_{H}4-59, V_{H}1-58, V_{H}3-53, V_{H}5-51, V_{H}3-49, V_{H}3-48, V_{H}1-46, V_{H}1-45, V_{H}3-43, V_{H}4-39, V_{H}4-34, V_{H}3-33, V_{H}4-31, V_{H}3-30, V_{H}4-28, V_{H}2-26, V_{H}1-24, V_{H}3-23, V_{H}3-21, V_{H}3-20, V_{H}1-18, V_{H}3-15, V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2, V_{H}6-1, or any combination thereof,
   (ii) the one or more unrearranged human D_{H} gene segments comprise D_{H}1-1, D_{H}2-2, D_{H}3-3, D_{H}4-4, D_{H}5-5, D_{H}6-6, D_{H}1-7, D_{H}2-8, D_{H}3-9, D_{H}3-10, D_{H}5-12, D_{H}6-13, D_{H}2-15, D_{H}3-16, D_{H}4-17, D_{H}6-19, D_{H}1-20, D_{H}2-21, D_{H}3-22, D_{H}6-25, D_{H}1-26, D_{H}7-27, or any combination thereof, and
   (iii) the one or more unrearranged human J_{H} gene segments comprise J_{H}1, J_{H}2, J_{H}3, J_{H}4, J_{H}5, J_{H}6, or any combination thereof.
Embodiment 13. The genetically modified rodent of any one of embodiments 4-12, wherein the engineered endogenous immunoglobulin heavy chain locus lacks a functional endogenous rodent Adam6 gene.
Embodiment 14. The genetically modified rodent of any one of embodiments 4-13, wherein the germline genome of the genetically modified rodent comprises one or more nucleotide sequences encoding one or more rodent ADAM6 polypeptides, functional orthologs, functional homologs, or functional fragments thereof.
Embodiment 15. The genetically modified rodent of embodiment 14, wherein the one or more rodent ADAM6 polypeptides, functional orthologs, functional homologs, or functional fragments thereof are expressed by the genetically modified rodent.
Embodiment 16. The genetically modified rodent of embodiment 14 or 15, wherein the one or more nucleotide sequences encoding one or more rodent ADAM6 polypeptides, functional orthologs, functional homologs, or functional fragments thereof are included on the same chromosome as the engineered endogenous immunoglobulin heavy chain locus.
Embodiment 17. The genetically modified rodent of any one of embodiments 14-16, wherein the engineered endogenous immunoglobulin heavy chain locus comprises the one or more nucleotide sequences encoding one or more rodent ADAM6 polypeptides, functional orthologs, functional homologs, or functional fragments thereof.
Embodiment 18. The genetically modified rodent of any one of embodiments 14-17, wherein the one or more nucleotide sequences encoding one or more rodent ADAM6 polypeptides, functional orthologs, functional homologs, or functional fragments thereof are in place of a human Adam6 pseudogene.
Embodiment 19. The genetically modified rodent of any one of embodiments 14-18, wherein the one or more nucleotide sequences encoding one or more rodent ADAM6 polypeptides, functional orthologs, functional homologs, or functional fragments thereof replace a human Adam6 pseudogene.
Embodiment 20. The genetically modified rodent of any one of embodiments 14-19, wherein the one or more human V_{H} gene segments comprise a first and a second human V_{H} gene segment, and the one or more nucleotide sequences encoding one or more rodent ADAM6 polypeptides, functional orthologs, functional homologs, or functional fragments thereof are between the first human V_{H} gene segment and the second human V_{H} gene segment.
Embodiment 21. The genetically modified rodent of embodiment 20, wherein the first human V_{H} gene segment is V_{H}1-2 and the second human V_{H} gene segment is V_{H}6-1.
Embodiment 22. The genetically modified rodent of any one of embodiments 14-17, wherein the one or more nucleotide sequences encoding one or more rodent ADAM6 polypeptides, functional orthologs, functional homologs, or functional fragments thereof are between a human V_{H} gene segment and a human D_{H} gene segment.
Embodiment 23. The genetically modified rodent of any one of embodiments 1-22, wherein the rodent Cλ gene has a sequence that is at least 80% identical to: (i) a mouse Cλ1, (ii) a mouse Cλ2, or (iii) a mouse Cλ3 gene.
Embodiment 24. The genetically modified rodent of any one of embodiments 1-23, wherein the rodent Cλ gene comprises a mouse Cλ gene.
Embodiment 25. The genetically modified rodent of any one of embodiments 1-23, wherein the rodent Cλ gene comprises a mouse Cλ1 gene.
Embodiment 26. The genetically modified rodent of embodiments 1-22, wherein the rodent Cλ gene has a sequence that is at least 80% identical to: (i) a rat Cλ1, (ii) a rat Cλ2, (iii) a rat Cλ3 or (iv) a rat Cλ4 gene.
Embodiment 27. The genetically modified rodent of any one of embodiments 1-22, wherein the rodent Cλ gene comprises a rat Cλ gene.
Embodiment 28. The genetically modified rodent of any one of embodiments 1-27, wherein the single rearranged human immunoglobulin λ light chain variable region is in place of one or more rodent Vκ gene segments, one or more rodent Jκ gene segments, or any combination thereof.
Embodiment 29. The genetically modified rodent of any one of embodiments 1-28, wherein the single rearranged human immunoglobulin λ light chain variable region replaces one or more rodent Vκ gene segments, one or more rodent Jκ gene segments, or any combination thereof.
Embodiment 30. The genetically modified rodent of any one of embodiments 1-29, further comprising an inactivated endogenous immunoglobulin λ light chain locus.
Embodiment 31. The genetically modified rodent of any one of embodiments 1-30, wherein the endogenous Vλ gene segments, the endogenous Jλ gene segments, and the endogenous Cλ genes are deleted in whole or in part.
Embodiment 32. The genetically modified rodent of any one of embodiments 1-31, wherein the rodent does not detectably express endogenous immunoglobulin κ light chain variable domains.
Embodiment 33. The genetically modified rodent of any one of embodiments 1-32, wherein all immunoglobulin light chains expressed by B cells of the genetically modified rodent include human immunoglobulin λ light chain variable domains expressed from the single rearranged human immunoglobulin λ light chain variable region or a somatically hypermutated version thereof.
Embodiment 34. The genetically modified rodent of any one of embodiments 1-33, wherein the rodent is a rat or a mouse.
Embodiment 35. A breeding colony of genetically modified rodents comprising a first genetically modified rodent, a second genetically modified rodent, and a third genetically modified rodent, wherein the third genetically modified rodent is the progeny of the first genetically modified rodent and the second genetically modified rodent, and wherein the first, second, and third genetically modified rodent each comprise in their germline genomes:
   an engineered endogenous immunoglobulin κ light chain locus comprising a single rearranged human immunoglobulin λ light chain variable region operably linked to a rodent Cλ gene segment, wherein the single rearranged human immunoglobulin λ light chain variable region comprises a human Vλ gene segment and a human Jλ gene segment,
   wherein all immunoglobulin λ light chains expressed by B cells of the first, second, and third genetically modified rodent include human immunoglobulin λ light chain variable domains expressed from the single rearranged human immunoglobulin λ light chain variable region or a somatically hypermutated version thereof.
Embodiment 36. The breeding colony of embodiment 35, wherein the germline genomes of the first, second, and third genetically modified rodents are homozygous for the engineered endogenous immunoglobulin heavy chain locus.
Embodiment 37. The breeding colony of embodiment 35 or 36, wherein the human Vλ gene segment in the engineered endogenous immunoglobulin κ light chain locus of each of the first, second, and third genetically modified rodents is selected from a group consisting of: Vλ1-51, Vλ5-45, Vλ1-44, Vλ1-40, Vλ3-21, and Vλ2-14.
Embodiment 38. The breeding colony of any one of embodiments 35-37, wherein the human Jλ gene segment in the engineered endogenous immunoglobulin κ light chain locus of each of the first, second, and third genetically modified rodents is selected from a group consisting of: Jλ1, Jλ2, Jλ3, Jλ6, and Jλ7.
Embodiment 39. The breeding colony of any one of embodiments 35-38, wherein the first, second, and third genetically modified rodent each comprise in their germline genomes:
   an engineered endogenous immunoglobulin heavy chain locus comprising one or more unrearranged human V_{H} gene segments, one or more unrearranged human D_{H} gene segments, and one or more unrearranged human J_{H} gene segments operably linked to one or more endogenous immunoglobulin heavy chain constant region genes,
   wherein all heavy chains expressed by B cells of the genetically modified rodent include a human immunoglobulin heavy chain variable domains and a rodent immunoglobulin heavy chain constant domains.
Embodiment 40. The breeding colony of embodiment 39, wherein the germline genomes of the first, second, and third genetically modified rodents are homozygous for the engineered endogenous immunoglobulin heavy chain locus.
Embodiment 41. A rodent embryo whose genome comprises an engineered endogenous immunoglobulin κ light chain locus comprising a single rearranged human immunoglobulin λ light chain variable region operably linked to a rodent Cλ gene segment, wherein the single rearranged human immunoglobulin λ light chain variable region comprises a human Vλ gene segment and a human Jλ gene segment.
Embodiment 42. The rodent embryo of embodiment 41, wherein the genome of the rodent embryo is homozygous for the engineered endogenous immunoglobulin κ light chain locus.
Embodiment 43. The rodent embryo of embodiment 41 or 42, wherein the human Vλ gene segment is selected from a group consisting of: Vλ1-51, Vλ5-45, Vλ1-44, Vλ1-40, Vλ3-21, and Vλ2-14.
Embodiment 44. The rodent embryo of any one of embodiments 41-43, wherein the human Jλ gene segment is selected from a group consisting of: Jλ1, Jλ2, Jλ3, Jλ6, and Jλ7.
Embodiment 45. The rodent embryo of any one of embodiments 41-44, further comprising an engineered endogenous immunoglobulin heavy chain locus comprising one or more unrearranged human V_{H} gene segments, one or more unrearranged human D_{H} gene segments, and one or more unrearranged human J_{H} gene segments operably linked to one or more endogenous immunoglobulin heavy chain constant region genes.
Embodiment 46. The rodent embryo of embodiment 45, wherein the genome of the rodent embryo is homozygous for the engineered endogenous immunoglobulin heavy chain locus.
Embodiment 47. A B cell of the genetically modified rodent of any one of embodiments 1-34, comprising:
   the single rearranged human immunoglobulin λ light chain variable region of the engineered endogenous κ light chain locus or a somatically hypermutated version thereof.
Embodiment 48. A B cell of the genetically modified rodent of any one of embodiments 4-34, comprising:
   the single rearranged human immunoglobulin λ light chain variable region of the engineered endogenous κ light chain locus or somatically hypermutated version thereof; and
   a rearranged human immunoglobulin heavy chain variable region derived from a human V_{H} gene segment of the one or more unrearranged human V_{H} gene segments, a human D_{H} gene segment of the one or more unrearranged human D_{H} gene segments, and a human J_{H} gene segment of the one or more unrearranged human J_{H} gene segments in the engineered endogenous heavy chain locus.
Embodiment 49. A hybridoma generated from the B cell of embodiment 47 or 48.
Embodiment 50. A population of B cells of a single genetically modified rodent that comprises in its germline genome:
   (a) an engineered endogenous immunoglobulin κ light chain locus comprising a single rearranged human immunoglobulin λ light chain variable region operably linked to a rodent Cλ gene segment, wherein the single rearranged human immunoglobulin λ light chain variable region comprises a human Vλ gene segment operably linked to a human Jλ gene segment, and
   (b) an engineered endogenous immunoglobulin heavy chain locus comprising one or more unrearranged human V_{H} gene segments, one or more unrearranged human D_{H} gene segments, and one or more unrearranged human J_{H} gene segments operably linked to one or more endogenous immunoglobulin heavy chain constant region genes,
   wherein all antibodies expressed by the population of B cells include:
   (i) human immunoglobulin λ light chain variable domains expressed from the single rearranged human immunoglobulin λ light chain variable region or a somatically hypermutated version thereof, and
   (ii) a plurality of human immunoglobulin heavy chain variable domains expressed from at least two different rearranged human immunoglobulin heavy chain variable regions or somatically hypermutated version thereof.
Embodiment 51. A stem cell comprising an engineered endogenous immunoglobulin κ light chain locus comprising a single rearranged human immunoglobulin λ light chain variable region operably linked to a rodent Cλ gene segment, wherein the single rearranged human immunoglobulin λ light chain variable region comprises a human Vλ gene segment and a human Jλ gene segment.
Embodiment 52. The stem cell of embodiment 51, wherein the genome of the stem cell is homozygous for the engineered endogenous immunoglobulin κ light chain locus.
Embodiment 53. The stem cell of embodiment 51 or 52, wherein the human Vλ gene segment is selected from a group consisting of: Vλ1-51, Vλ5-45, Vλ1-44, Vλ1-40, Vλ3-21, and Vλ2-14.
Embodiment 54. The stem cell of any one of embodiments 51-53, wherein the human Jλ gene segment is selected from a group consisting of: Jλ1, Jλ2, Jλ3, Jλ6, and Jλ7.
Embodiment 55. The stem cell of any one of embodiments 51-54, further comprising an engineered endogenous immunoglobulin heavy chain locus comprising one or more unrearranged human V_{H} gene segments, one or more unrearranged human D_{H} gene segments, and one or more unrearranged human J_{H} gene segments operably linked to one or more endogenous immunoglobulin heavy chain constant region genes.
Embodiment 56. A mammalian cell expressing an antibody, wherein the antibody comprises a heavy chain comprising a human immunoglobulin heavy chain variable domain and a light chain comprising a human immunoglobulin λ light chain variable domain, wherein the human immunoglobulin heavy chain variable domain, the human immunoglobulin λ light chain variable domain, or both were identified from a genetically modified rodent of any one of embodiments 4-34.
Embodiment 57. An antibody prepared by a method comprising the steps of:
   (a) exposing a genetically modified rodent of any one of embodiments 1-34 to an antigen of interest;
   (b) maintaining the genetically modified rodent under conditions sufficient for the genetically modified rodent to produce an immune response to the antigen of interest; and
   (c) recovering from the genetically modified rodent:
      (i) an antibody that binds the antigen of interest,
      (ii) a nucleotide that encodes a human light or heavy chain variable domain, a light chain, or a heavy chain of an antibody that binds the antigen of interest, or
      (iii) a cell that expresses an antibody that binds the antigen of interest,
   wherein an antibody of (c) includes human heavy chain variable and human λ light chain variable domains.
Embodiment 58. A method of making an antibody, the method comprising:
   (a) exposing a genetically modified rodent of any one of embodiments 1-34 to an antigen;
   (b) allowing the genetically modified rodent to develop an immune response to the antigen; and
   (c) isolating an antibody specific to the antigen, a B cell expressing an antibody specific to the antigen, or one or more nucleotide sequences encoding an antibody specific to the antigen from the genetically modified rodent.
Embodiment 59. A method of making an antibody comprising the steps of:
   (a) expressing in a mammalian cell said antibody comprising two human immunoglobulin λ light chains and two human immunoglobulin heavy chains, wherein each human immunoglobulin λ light chain includes a human immunoglobulin λ light chain variable domain and each human immunoglobulin heavy chain includes a human immunoglobulin heavy chain variable domain, wherein the amino acid sequence of at least one of the human immunoglobulin heavy chain variable domains, at least one of the λ light chain variable domains, or a combination thereof was identified in a genetically modified rodent of any one of embodiments 1-34; and
   (b) obtaining the antibody.
Embodiment 60. The method of embodiment 58 or 59, wherein the antibody is a bispecific antibody.
Embodiment 61. A method of making a bispecific antibody, the method comprising:
   (a) contacting a first genetically modified rodent according to any one of embodiments 1-35 with a first epitope of a first antigen,
   (b) contacting a second genetically modified rodent according to any one of embodiments 1-35 with a second epitope of a second antigen,
   (c) isolating a B cell that expresses a first antibody specific for the first epitope of the first antigen from the first genetically modified rodent and determining a first human immunoglobulin heavy chain variable domain of the first antibody;
   (d) isolating a B cell that expresses a second antibody specific for the second epitope of the second antigen from the second genetically modified rodent and determining a second human immunoglobulin heavy chain variable domain of the second antibody;
   (e) operably linking a nucleotide sequence encoding the first human immunoglobulin heavy chain variable domain to a nucleotide sequence encoding a first human immunoglobulin constant domain to produce a first nucleotide sequence encoding a first human heavy chain;
   (f) operably linking a nucleotide sequence encoding the second human immunoglobulin heavy chain variable domain to a nucleotide sequence encoding a second human immunoglobulin constant domain to produce a second nucleotide sequence encoding a second human heavy chain;
   (g) expressing in a mammalian cell:
      (i) the first nucleotide sequence;
      (ii) the second nucleotide sequence; and
      (iii) a third nucleotide sequence comprising the single rearranged human immunoglobulin λ light chain variable region or a somatically hypermutated version thereof operably linked to a human immunoglobulin λ light chain constant region.
Embodiment 62. A method of making a bispecific antibody, the method comprising:
   (a) expressing in a mammalian cell:
      (i) a first nucleotide sequence comprising a first human immunoglobulin heavy chain variable region operably linked to a first human immunoglobulin constant region;
      (ii) a second nucleotide sequence comprising a second human immunoglobulin heavy chain variable region operably linked to a second human immunoglobulin constant region; and
      (iii) a third nucleotide sequence comprising human immunoglobulin λ light chain variable region operably linked to a human immunoglobulin λ light chain constant region;

   wherein the first human immunoglobulin heavy chain variable region encodes a first human heavy chain variable domain identified from a first antibody in a first genetically modified rodent that had been immunized with a first epitope of a first antigen, wherein the first antibody specifically binds the first epitope of the first antigen; and
   wherein the second human immunoglobulin heavy chain variable region encodes a second human heavy chain variable domain identified from a second antibody in a second genetically modified rodent that had been immunized with a second epitope of a second antigen, wherein the second antibody specifically binds the second epitope of the second antigen;
   wherein the first and second genetically modified rodents are each a genetically modified rodent according to any one of embodiments 4-34; and
   wherein the human immunoglobulin λ light chain variable region of the third nucleotide is the single rearranged human immunoglobulin λ light chain variable region or a somatically hypermutated version thereof.
Embodiment 63. The method of embodiment 59 or 62, wherein the first and second genetically modified rodents are the same genetically modified rodent.
Embodiment 64. The method of embodiment 59 or 62, wherein the first and second genetically modified rodents are different genetically modified rodents.
Embodiment 65. The method of any one of embodiments 59-64, wherein the first and second antigens are the same antigen, and the first and second epitopes are different epitopes.
Embodiment 66. The method of any one of embodiments 59-64, wherein the first and second antigens are different antigens.
Embodiment 67. A method for making a human immunoglobulin heavy chain, the method comprising the steps of:
   (a) exposing a genetically modified rodent of any one of embodiments 4-34 to an antigen of interest;
   (b) obtaining a human immunoglobulin heavy chain variable domain sequence of an antibody that specifically binds the antigen and that was generated by the genetically modified rodent; and
   (c) operably linking the human immunoglobulin heavy variable domain sequence to a human immunoglobulin heavy chain constant domain sequence to form a human immunoglobulin heavy chain.
Embodiment 68. A human immunoglobulin heavy chain generated by the method of embodiment 67.
Embodiment 69. A method for making a human immunoglobulin heavy chain variable domain, the method comprising the steps of:
   (a) exposing a genetically modified rodent of any one of embodiment 4-34 to antigen of interest; and
   (b) obtaining a human immunoglobulin heavy chain variable domain sequence of an antibody that specifically binds the antigen and that was generated by the genetically modified rodent.
Embodiment 70. A human immunoglobulin heavy chain variable domain generated by the method of embodiment 69.
Embodiment 71. A method of making a collection of human immunoglobulin heavy chain variable domains, comprising
   (a) exposing a genetically modified rodent of any one of embodiments 4-35 to an antigen of interest, and
   (b) isolating the collection of human immunoglobulin heavy chain variable domains from the genetically modified rodent,

   wherein the collection of human immunoglobulin heavy chain variable domains each bind to a human immunoglobulin λ light chain variable domain expressed from the single rearranged human immunoglobulin λ light chain variable region or a somatically hypermutated version thereof, and
   wherein the human λ light chain variable domain paired with any one of the human immunoglobulin heavy chain variable domains in the collection binds the antigen.
Embodiment 72. A method for making a human immunoglobulin λ light chain, the method comprising the steps of:
   (a) exposing a genetically modified rodent of any one of embodiments 1-34 to an antigen of interest;
   (b) obtaining a human immunoglobulin λ light chain variable domain sequence of an antibody that specifically binds the antigen and that was generated by the genetically modified rodent; and
   (c) operably linking the human immunoglobulin λ light variable domain sequence to a human immunoglobulin λ light chain constant domain sequence to form a human immunoglobulin λ light chain.
Embodiment 73. A human immunoglobulin λ light chain generated by the method of embodiment 72.
Embodiment 74. A method for generating a human immunoglobulin λ light chain variable domain, the method comprising the steps of:
   (a) exposing a genetically modified rodent of any one of embodiments 1-35 to an antigen of interest; and
   (b) obtaining a human immunoglobulin λ light chain variable domain sequence of an antibody that specifically binds the antigen and that was generated by the genetically modified rodent.
Embodiment 75. A human immunoglobulin λ light chain variable domain generated by the method of embodiment 74.
Embodiment 76. A method for making a nucleotide sequence encoding a human immunoglobulin heavy chain, the method comprising the steps of:
   (a) exposing a genetically modified rodent of any one of embodiments 4-34 to an antigen of interest;
   (b) obtaining a human immunoglobulin heavy chain variable region encoding a human immunoglobulin heavy chain variable domain sequence of an antibody that specifically binds the antigen and that was generated by the genetically modified rodent; and
   (c) operably linking the human immunoglobulin heavy variable region to a human immunoglobulin heavy chain constant region sequence to form a nucleotide sequence encoding a human immunoglobulin heavy chain.
Embodiment 77. A nucleotide sequence encoding a human immunoglobulin heavy chain generated by the method of embodiment 76.
Embodiment 78. A method for making a nucleotide sequence comprising a human immunoglobulin heavy chain variable region, the method comprising the steps of:
   (a) exposing a genetically modified rodent of any one of embodiments 4-34 to an antigen of interest; and
   (b) obtaining a human immunoglobulin heavy chain variable region encoding a human immunoglobulin heavy chain variable domain sequence of an antibody that specifically binds the antigen and that was generated by the genetically modified rodent.
Embodiment 79. A nucleotide sequence comprising a human immunoglobulin heavy chain variable region generated by the method of embodiment 78.
Embodiment 80. A method for making a nucleotide sequence encoding a human immunoglobulin λ light chain, the method comprising the steps of:
   (a) exposing a genetically modified rodent of any one of embodiments 1-34 to an antigen of interest;
   (b) obtaining a human immunoglobulin λ light chain variable region encoding a human immunoglobulin λ light chain variable domain sequence of an antibody that specifically binds the antigen and that was generated by the genetically modified rodent; and
   (c) operably linking the human immunoglobulin λ light variable region to a human immunoglobulin λ light chain constant region sequence to form a nucleotide sequence encoding a human immunoglobulin λ light chain.
Embodiment 81. A nucleotide sequence encoding a human immunoglobulin λ light chain generated by the method of embodiment 80.
Embodiment 82. A method for making a nucleotide sequence comprising a human immunoglobulin λ light chain variable region, the method comprising the steps of:
   (a) exposing a genetically modified rodent of any one of embodiments 1-34 to an antigen of interest; and
   (b) obtaining a human immunoglobulin λ light chain variable region encoding a human immunoglobulin λ light chain variable domain sequence of an antibody that specifically binds the antigen and that was generated by the genetically modified rodent.
Embodiment 83. A nucleotide sequence comprising a human immunoglobulin λ light chain variable region generated by the method of embodiment 82.
Embodiment 84. A targeting vector comprising:
   (i) a 5' homology arm comprising a nucleotide sequence corresponding to a 5' target sequence in an endogenous rodent κ light chain locus;
   (ii) a single rearranged human immunoglobulin λ light chain variable region comprising a Vλ gene segment and a Jλ gene segment;
   (iii) a rodent Cλ gene segment; and
   (iv) a 3' homology arm comprising a nucleotide sequence corresponding to a 3' target sequence in the endogenous rodent κ light chain locus.
Embodiment 85. The targeting vector of embodiment 84, wherein the human Vλ gene segment is selected from a group consisting of: Vλ1-51, Vλ5-45, Vλ1-44, Vλ1-40, Vλ3-21, and Vλ2-14.
Embodiment 86. The targeting vector of embodiment 84 or 85, wherein the human Jλ gene segment is selected from a group consisting of: Jλ1, Jλ2, Jλ3, Jλ6, and Jλ7.
Embodiment 87. A method of making a genetically modified rodent comprising the steps of:
   (a) introducing a single rearranged human immunoglobulin λ light chain variable region comprising a human Vλ gene segment and a human Jλ gene segment into an engineered endogenous immunoglobulin κ light chain locus in the genome of a rodent ES cell; and
   (b) generating a rodent using the rodent ES cell generated in step (a).
Embodiment 88. A method of making a genetically modified rodent comprising the steps of:
   (a) introducing a single rearranged human immunoglobulin λ light chain variable region operably linked to a rodent Cλ gene segment into an engineered endogenous immunoglobulin κ light chain locus in the genome of a rodent ES cell, wherein the single rearranged human immunoglobulin λ light chain variable region comprises a human Vλ gene segment and a human Jλ gene segment; and
   (b) generating a rodent using the rodent ES cell generated in step (a).
Embodiment 89. The method of embodiment 87 or 88, wherein the genome of the rodent ES cell comprises one or more unrearranged human V_{H} gene segments, one or more unrearranged human D_{H} gene segments, and one or more unrearranged human J_{H} gene segments operably linked to one or more endogenous immunoglobulin heavy chain constant region genes at an engineered endogenous immunoglobulin heavy chain locus.
Embodiment 90. A method of making a genetically modified rodent ES cell comprising the steps of:
   introducing a single rearranged human immunoglobulin λ light chain variable region comprising a human Vλ gene segment and a human Jλ gene segment into an engineered endogenous immunoglobulin κ light chain locus in the genome of a rodent ES cell.
Embodiment 91. A method of making a genetically modified rodent ES cell comprising the steps of:
   introducing a single rearranged human immunoglobulin λ light chain variable region operably linked to a rodent Cλ gene segment into an engineered endogenous immunoglobulin κ light chain locus in the genome of a rodent ES cell, wherein the single rearranged human immunoglobulin λ light chain variable region comprises a human Vλ gene segment and a human Jλ gene segment.
Embodiment 92. The method of embodiment 90 or 91, wherein the genome of the rodent ES cell comprises:
   one or more unrearranged human V_{H} gene segments, one or more unrearranged human D_{H} gene segments, and one or more unrearranged human J_{H} gene segments operably linked to one or
   more endogenous immunoglobulin heavy chain constant region genes at an engineered endogenous immunoglobulin heavy chain locus.
Embodiment 93. A method of making a genetically modified rodent, comprising the step of:
   (a) engineering an endogenous immunoglobulin κ light chain locus in the germline genome of the rodent to comprise a single rearranged human immunoglobulin λ light chain variable region operably linked to a rodent Cλ gene segment, wherein the single rearranged human immunoglobulin λ light chain variable region comprises a human Vλ gene segment and a human Jλ gene segment,
   so that all immunoglobulin λ light chains expressed by B cells of the genetically modified rodent include human immunoglobulin λ light chain variable domains expressed from the single rearranged human immunoglobulin λ light chain variable region or a somatically hypermutated version thereof.
Embodiment 94. The method of embodiment 93, wherein the method further comprises the step of:
   (b) engineering an endogenous immunoglobulin heavy chain locus in the germline genome of the rodent to comprise one or more unrearranged human V_{H} gene segments, one or more unrearranged human D_{H} gene segments, and one or more unrearranged human J_{H} gene segments operably linked to one or more rodent immunoglobulin heavy chain constant region genes,
   so that all heavy chains expressed by B cells of the genetically modified rodent include a human immunoglobulin heavy chain variable domains and a rodent immunoglobulin heavy chain constant domains.
Embodiment 95. The method of embodiment 93 or 94, wherein step (a) and/or step (b) are carried out in a rodent ES cell.
Embodiment 96. The method of any one of embodiment 93-95, wherein the endogenous immunoglobulin κ light chain locus lacks a rodent Cx gene.
Embodiment 97. The method of any one of embodiments 93-96, wherein the human Vλ gene segment is selected from a group consisting of: Vλ1-51, Vλ5-45, Vλ1-44, Vλ1-40, Vλ3-21, and Vλ2-14.
Embodiment 98. The method of any one of embodiments 93-97, wherein the human Jλ gene segment is selected from a group consisting of: Jλ1, Jλ2, Jλ3, Jλ6, and Jλ7.
Embodiment 99. The method of any one of embodiments 94-98, wherein the one or more unrearranged human V_{H} gene segments, one or more unrearranged human D_{H} gene segments, and one or more unrearranged human J_{H} gene segments are in place of one or more endogenous V_{H} gene segments, one or more endogneous D_{H} gene segments, one or more endogenous J_{H} gene segments, or a combination thereof.
Embodiment 100. The method of any one of embodiments 94-99, wherein the one or more unrearranged human V_{H} gene segments, one or more unrearranged human D_{H} gene segments, and one or more unrearranged human J_{H} gene segments replace one or more endogenous V_{H} gene segments, one or more endogenous D_{H} gene segments, and one or more endogenous J_{H} gene segments, respectively.
Embodiment 101. The method of any one of embodiments 94-100, wherein the one or more rodent immunoglobulin heavy chain constant region genes are one or more endogenous rodent immunoglobulin heavy chain constant region genes.
Embodiment 102. The method of any one of embodiments 94-101, wherein:
   (i) the one or more unrearranged human V_{H} gene segments comprise V_{H}3-74, V_{H}3-73, V_{H}3-72, V_{H}2-70, V_{H}1-69, V_{H}3-66, V_{H}3-64, V_{H}4-61, V_{H}4-59, V_{H}1-58, V_{H}3-53, V_{H}5-51, V_{H}3-49, V_{H}3-48, V_{H}1-46, V_{H}1-45, V_{H}3-43, V_{H}4-39, V_{H}4-34, V_{H}3-33, V_{H}4-31, V_{H}3-30, V_{H}4-28, V_{H}2-26, V_{H}1-24, V_{H}3-23, V_{H}3-21, V_{H}3-20, V_{H}1-18, V_{H}3-15, V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2, V_{H}6-1, or any combination thereof,
   (ii) the one or more unrearranged human D_{H} gene segments comprise D_{H}1-1, D_{H}2-2, D_{H}3-3, D_{H}4-4, D_{H}5-5, D_{H}6-6, D_{H}1-7, D_{H}2-8, D_{H}3-9, D_{H}3-10, D_{H}5-12, D_{H}6-13, D_{H}2-15, D_{H}3-16, D_{H}4-17, D_{H}6-19, D_{H}1-20, D_{H}2-21, D_{H}3-22, D_{H}6-25, D_{H}1-26, D_{H}7-27, or any combination thereof, and
   (iii) the one or more unrearranged human J_{H} gene segments comprise J_{H}1, J_{H}2, J_{H}3, J_{H}4, J_{H}5, J_{H}6, or any combination thereof.
Embodiment 103. The method of any one of embodiments 94-102, wherein the endogenous immunoglobulin heavy chain locus lacks a functional endogenous rodent Adam6 gene.
Embodiment 104. The method of any one of embodiments 94-103, wherein the germline genome of the genetically modified rodent comprises one or more nucleotide sequences encoding one or more rodent ADAM6 polypeptides, functional orthologs, functional homologs, or functional fragments thereof.
Embodiment 105. The method of embodiment 104, wherein the one or more rodent ADAM6 polypeptides, functional orthologs, functional homologs, or functional fragments thereof are expressed by the genetically modified rodent.
Embodiment 106. The method of embodiment 104 or 105, wherein the one or more nucleotide sequences encoding one or more rodent ADAM6 polypeptides, functional orthologs, functional homologs, or functional fragments thereof are included on the same chromosome as the engineered endogenous immunoglobulin heavy chain locus.
Embodiment 107. The method of any one of embodiments 104-106, wherein the engineered endogenous immunoglobulin heavy chain locus comprises the one or more nucleotide sequences encoding one or more rodent ADAM6 polypeptides, functional orthologs, functional homologs, or functional fragments thereof.
Embodiment 108. The method of any one of embodiments 104-107, wherein the one or more nucleotide sequences encoding one or more rodent ADAM6 polypeptides, functional orthologs, functional homologs, or functional fragments thereof are in place of a human Adam6 pseudogene.
Embodiment 109. The method of any one of embodiments 104-108, wherein the one or more nucleotide sequences encoding one or more rodent ADAM6 polypeptides, functional orthologs, functional homologs, or functional fragments thereof replace a human Adam6 pseudogene.
Embodiment 110. The method of any one of embodiments 104-109, wherein the one or more human V_{H} gene segments comprise a first and a second human V_{H} gene segment, and the one or more nucleotide sequences encoding one or more rodent ADAM6 polypeptides, functional orthologs, functional homologs, or functional fragments thereof are between the first human V_{H} gene segment and the second human V_{H} gene segment.
Embodiment 111. The method of embodiment 110, wherein the first human V_{H} gene segment is V_{H}1-2 and the second human V_{H} gene segment is V_{H}6-1.
Embodiment 112. The method of any one of embodiments 104-111, wherein the one or more nucleotide sequences encoding one or more rodent ADAM6 polypeptides, functional orthologs, functional homologs, or functional fragments thereof are between a human V_{H} gene segment and a human D_{H} gene segment.
Embodiment 113. The method of any one of embodiments 93-112, wherein the rodent Cλ gene has a sequence that is at least 80% identical to: (i) a mouse Cλ1, (ii) mouse Cλ2, or (iii) a mouse Cλ3 gene.
Embodiment 114. The method of any one of embodiments 93-113, wherein the rodent Cλ gene comprises a mouse Cλ gene.
Embodiment 115. The method of any one of embodiments 93-114, wherein the rodent Cλ gene comprises a mouse Cλ1 gene.
Embodiment 116. The method of any one of embodiments 93-112, wherein the rodent Cλ gene has a sequence that is at least 80% identical to: (i) a rat Cλ1, (ii) a rat Cλ2, (iii) a rat Cλ3, or (iv) a rat Cλ4 gene.
Embodiment 117. The method of any one of embodiments 93-112, wherein the rodent Cλ gene comprises a rat Cλ gene.
Embodiment 118. The method of any one of embodiments 93-117, wherein the single rearranged human immunoglobulin λ light chain variable region is in place of one or more rodent Vκ gene segments, one or more rodent Jκ gene segments, or any combination thereof.
Embodiment 119. The method of any one of embodiments 93-118, wherein the single rearranged human immunoglobulin λ light chain variable region replaces one or more rodent Vκ gene segments, one or more rodent Jκ gene segments, or any combination thereof.
Embodiment 120. The method of any one of embodiments 93-119, wherein the germline genome of the rodent further comprises an inactivated endogenous immunoglobulin λ light chain locus.
Embodiment 121. The method of any one of embodiments 93-120, wherein the endogenous Vλ gene segments, the endogenous Jλ gene segments, and the endogenous Cλ genes are deleted in whole or in part.
Embodiment 122. The method of any one of embodiments 93-121, wherein the rodent is a rat or a mouse.

### EQUIVALENTS

It is to be appreciated by those skilled in the art that various alterations, modifications, and improvements to the present disclosure will readily occur to those skilled in the art. Such alterations, modifications, and improvements are intended to be part of the present disclosure, and are intended to be within the spirit and scope of the invention. Accordingly, the foregoing description and drawing are by way of example only and any invention described in the present disclosure if further described in detail by the claims that follow.

Those skilled in the art will appreciate typical standards of deviation or error attributable to values obtained in assays or other processes as described herein. The publications, websites and other reference materials referenced herein to describe the background of the invention and to provide additional detail regarding its practice are hereby incorporated by reference in their entireties.

### ASPECTS

1. A genetically modified rodent, whose germline genome comprises:
   an engineered endogenous immunoglobulin κ light chain locus comprising a single rearranged human immunoglobulin λ light chain variable region operably linked to a rodent Cλ gene segment, wherein the single rearranged human immunoglobulin λ light chain variable region comprises a human Vλ gene segment and a human Jλ gene segment,
   wherein all immunoglobulin λ light chains expressed by B cells of the genetically modified rodent include human immunoglobulin λ light chain variable domains expressed from the single rearranged human immunoglobulin λ light chain variable region or a somatically hypermutated version thereof.
2. The genetically modified rodent of aspect 1, wherein the germline genome of the genetically modified rodent is homozygous for the engineered endogenous immunoglobulin κ light chain locus.
3. The genetically modified rodent of aspect 1, wherein the germline genome of the genetically modified rodent is heterozygous for the engineered endogenous immunoglobulin κ light chain locus.
4. The genetically modified rodent of any of aspects 1-3, whose germline genome further comprises:
   an engineered endogenous immunoglobulin heavy chain locus comprising one or more unrearranged human V_{H} gene segments, one or more unrearranged human D_{H} gene segments, and one or more unrearranged human J_{H} gene segments operably linked to one or more rodent immunoglobulin heavy chain constant region genes,
   wherein all heavy chains expressed by B cells of the genetically modified rodent include human immunoglobulin heavy chain variable domains and rodent immunoglobulin heavy chain constant domains.
5. The genetically modified rodent of aspect 4, wherein the germline genome of the genetically modified rodent is homozygous for the engineered endogenous immunoglobulin heavy chain locus.
6. The genetically modified rodent of any one of aspects 1-5, wherein the genetically modified rodent lacks a rodent Cκ gene at the engineered endogenous immunoglobulin κ light chain locus.
7. The genetically modified rodent of any one of aspects 1-6, wherein the human Vλ gene segment comprises Vλ1-51, Vλ5-45, Vλ1-44, Vλ1-40, Vλ3-21, or Vλ2-14.
8. The genetically modified rodent of any one of aspects 1-7, wherein the human Vλ gene segment comprises Vλ1-51.
9. The genetically modified rodent of any one of aspects 1-7, wherein the human Vλ gene segment comprises Vλ2-14.
10. The genetically modified rodent of any one of aspects 1-9, wherein the human Jλ gene segment comprises Jλ1, Jλ2, Jλ3, Jλ6, or Jλ7.
11. The genetically modified rodent of any one of aspects 1-10, wherein the human Jλ gene segment comprises Jλ2.
12. The genetically modified rodent of any one of aspects 4-11, wherein the one or more unrearranged human V_{H} gene segments, one or more unrearranged human D_{H} gene segments, and one or more unrearranged human J_{H} gene segments are in place of one or more endogenous V_{H} gene segments, one or more endogenous D_{H} gene segments, one or more endogenous J_{H} gene segments, or a combination thereof.
13. The genetically modified rodent of any one of aspects 4-12, wherein the one or more unrearranged human V_{H} gene segments, one or more unrearranged human D_{H} gene segments, and one or more unrearranged human J_{H} gene segments replace one or more endogenous V_{H} gene segments, one or more endogenous D_{H} gene segments, and one or more endogenous J_{H} gene segments, respectively.
14. The genetically modified rodent of aspect 12 or 13, wherein the one or more rodent immunoglobulin heavy chain constant region genes are one or more endogenous rodent immunoglobulin heavy chain constant region genes.
15. The genetically modified rodent of any one of aspects 4-14, wherein:
   (i) the one or more unrearranged human V_{H} gene segments comprise V_{H}3-74, V_{H}3-73, V_{H}3-72, V_{H}2-70, V_{H}1-69, V_{H}3-66, V_{H}3-64, V_{H}4-61, V_{H}4-59, V_{H}1-58, V_{H}3-53, V_{H}5-51, V_{H}3-49, V_{H}3-48, V_{H}1-46, V_{H}1-45, V_{H}3-43, V_{H}4-39, V_{H}4-34, V_{H}3-33, V_{H}4-31, V_{H}3-30, V_{H}4-28, V_{H}2-26, V_{H}1-24, V_{H}3-23, V_{H}3-21, V_{H}3-20, V_{H}1-18, V_{H}3-15, V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2, V_{H}6-1, or any combination thereof,
   (ii) the one or more unrearranged human D_{H} gene segments comprise D_{H}1-1, D_{H}2-2, D_{H}3-3, D_{H}4-4, D_{H}5-5, D_{H}6-6, D_{H}1-7, D_{H}2-8, D_{H}3-9, D_{H}3-10, D_{H}5-12, D_{H}6-13, D_{H}2-15, D_{H}3-16, D_{H}4-17, D_{H}6-19, D_{H}1-20, D_{H}2-21, D_{H}3-22, D_{H}6-25, D_{H}1-26, D_{H}7-27, or any combination thereof, and
   (iii) the one or more unrearranged human J_{H} gene segments comprise J_{H}1, J_{H}2, J_{H}3, J_{H}4, J_{H}5, J_{H}6, or any combination thereof.
16. The genetically modified rodent of any one of aspects 4-15, wherein the germline genome of the genetically modified rodent comprises one or more nucleotide sequences encoding one or more rodent ADAM6 polypeptides, functional orthologs, functional homologs, or functional fragments thereof.
17. The genetically modified rodent of any one of aspects 1-16, wherein the rodent Cλ gene has a sequence that is at least 80% identical to: (i) a mouse Cλ1, (ii) a mouse Cλ2, or (iii) a mouse Cλ3 gene.
18. The genetically modified rodent of any one of aspects 1-17, wherein the rodent Cλ gene comprises a mouse Cλ gene.
19. The genetically modified rodent of any one of aspects 1-17, wherein the rodent Cλ gene comprises a mouse Cλ1 gene.
20. The genetically modified rodent of any one of aspects 1-19, wherein the single rearranged human immunoglobulin λ light chain variable region is in place of one or more rodent Vκ gene segments, one or more rodent Jκ gene segments, or any combination thereof.
21. The genetically modified rodent of any one of aspects 1-20, further comprising an inactivated endogenous immunoglobulin λ light chain locus.
22. The genetically modified rodent of any one of aspects 1-21, wherein all immunoglobulin light chains expressed by B cells of the genetically modified rodent include human immunoglobulin λ light chain variable domains expressed from the single rearranged human immunoglobulin λ light chain variable region or a somatically hypermutated version thereof.
23. The genetically modified rodent of any one of aspects 1-22, wherein the engineered endogenous immunoglobulin κ light chain locus further comprises one or more endogenous enhancers at their endogenous location in the endogenous immunoglobulin κ light chain locus.
24. The genetically modified rodent of any one of aspects 1-23, wherein the rodent is a rat or a mouse.
25. A genetically modified mouse, whose germline genome comprises:
   an engineered endogenous immunoglobulin κ light chain locus comprising a single rearranged human immunoglobulin λ light chain variable region operably linked to a mouse Cλ1 gene segment, wherein the single rearranged human immunoglobulin λ light chain variable region comprises a human Vλ1-51 gene segment and a human Jλ2 gene segment,
   wherein all immunoglobulin λ light chains expressed by B cells of the genetically modified mouse include human immunoglobulin λ light chain variable domains expressed from the single rearranged human immunoglobulin λ light chain variable region or a somatically hypermutated version thereof.
26. A genetically modified mouse, whose germline genome comprises:
   an engineered endogenous immunoglobulin κ light chain locus comprising a single rearranged human immunoglobulin λ light chain variable region operably linked to a mouse Cλ1 gene segment, wherein the single rearranged human immunoglobulin λ light chain variable region comprises a human Vλ2-14 gene segment and a human Jλ2 gene segment,
   wherein all immunoglobulin λ light chains expressed by B cells of the genetically modified mouse include human immunoglobulin λ light chain variable domains expressed from the single rearranged human immunoglobulin λ light chain variable region or a somatically hypermutated version thereof.
27. A rodent embryo whose genome comprises an engineered endogenous immunoglobulin κ light chain locus comprising a single rearranged human immunoglobulin λ light chain variable region operably linked to a rodent Cλ gene segment, wherein the single rearranged human immunoglobulin λ light chain variable region comprises a human Vλ gene segment and a human Jλ gene segment.
28. A B cell of the genetically modified rodent or mouse of any one of aspects 1-26, comprising: the single rearranged human immunoglobulin λ light chain variable region of the engineered endogenous κ light chain locus or a somatically hypermutated version thereof.
29. A hybridoma generated from the B cell of aspect 28.
30. A population of B cells of a single genetically modified rodent, wherein the rodent comprises in its germline genome:
   (a) an engineered endogenous immunoglobulin κ light chain locus comprising a single rearranged human immunoglobulin λ light chain variable region operably linked to a rodent Cλ gene segment, wherein the single rearranged human immunoglobulin λ light chain variable region comprises a human Vλ gene segment operably linked to a human Jλ gene segment, and
   (b) an engineered endogenous immunoglobulin heavy chain locus comprising one or more unrearranged human V_{H} gene segments, one or more unrearranged human D_{H} gene segments, and one or more unrearranged human J_{H} gene segments operably linked to one or more endogenous immunoglobulin heavy chain constant region genes,
   wherein all antibodies expressed by the population of B cells include:
   (i) human immunoglobulin λ light chain variable domains expressed from the single rearranged human immunoglobulin λ light chain variable region or a somatically hypermutated version thereof, and
   (ii) a plurality of human immunoglobulin heavy chain variable domains expressed from at least two different rearranged human immunoglobulin heavy chain variable regions or somatically hypermutated version thereof.
31. An embryonic stem cell comprising an engineered endogenous immunoglobulin κ light chain locus comprising a single rearranged human immunoglobulin λ light chain variable region operably linked to a rodent Cλ gene segment, wherein the single rearranged human immunoglobulin λ light chain variable region comprises a human Vλ gene segment and a human Jλ gene segment.
32. A mammalian cell expressing an antibody, wherein the antibody comprises a heavy chain comprising a human immunoglobulin heavy chain variable domain and a light chain comprising a human immunoglobulin λ light chain variable domain, wherein the human immunoglobulin heavy chain variable domain, the human immunoglobulin λ light chain variable domain, or both were identified from a genetically modified rodent or mouse of any one of aspects 4-26.
33. A method of making an antibody, the method comprising:
   (a) exposing a genetically modified rodent or mouse of any one of aspects 1-26 to an antigen;
   (b) allowing the genetically modified rodent to develop an immune response to the antigen; and
   (c) isolating an antibody specific to the antigen, a B cell expressing an antibody specific to the antigen, or one or more nucleotide sequences encoding an antibody specific to the antigen from the genetically modified rodent.
34. A method of making an antibody comprising the steps of:
   (a) expressing in a mammalian cell said antibody comprising two human immunoglobulin λ light chains and two human immunoglobulin heavy chains, wherein each human immunoglobulin λ light chain includes a human immunoglobulin λ light chain variable domain and each human immunoglobulin heavy chain includes a human immunoglobulin heavy chain variable domain, wherein the amino acid sequence of at least one of the human immunoglobulin heavy chain variable domains, at least one of the λ light chain variable domains, or a combination thereof was identified in a genetically modified rodent or mouse of any one of aspects 4-26; and
   (b) obtaining the antibody.
35. A method of making a bispecific antibody, the method comprising:
   (a) contacting a first genetically modified rodent or mouse according to any one of aspects 4-26 with a first epitope of a first antigen,
   (b) contacting a second genetically modified rodent or mouse according to any one of aspects 4-26 with a second epitope of a second antigen,
   (c) isolating a B cell that expresses a first antibody specific for the first epitope of the first antigen from the first genetically modified rodent and determining a first human immunoglobulin heavy chain variable domain of the first antibody;
   (d) isolating a B cell that expresses a second antibody specific for the second epitope of the second antigen from the second genetically modified rodent and determining a second human immunoglobulin heavy chain variable domain of the second antibody;
   (e) operably linking a nucleotide sequence encoding the first human immunoglobulin heavy chain variable domain to a nucleotide sequence encoding a first human immunoglobulin constant domain to produce a first nucleotide sequence encoding a first human heavy chain;
   (f) operably linking a nucleotide sequence encoding the second human immunoglobulin heavy chain variable domain to a nucleotide sequence encoding a second human immunoglobulin constant domain to produce a second nucleotide sequence encoding a second human heavy chain;
   (g) expressing in a mammalian cell:
      (i) the first nucleotide sequence;
      (ii) the second nucleotide sequence; and
      (iii) a third nucleotide sequence comprising the single rearranged human immunoglobulin λ light chain variable region or a somatically hypermutated version thereof operably linked to a human immunoglobulin λ light chain constant region.
36. A method of making a bispecific antibody, the method comprising:
   (a) expressing in a mammalian cell:
      (i) a first nucleotide sequence comprising a first human immunoglobulin heavy chain variable region operably linked to a first human immunoglobulin constant region;
      (ii) a second nucleotide sequence comprising a second human immunoglobulin heavy chain variable region operably linked to a second human immunoglobulin constant region; and
      (iii) a third nucleotide sequence comprising human immunoglobulin λ light chain variable region operably linked to a human immunoglobulin λ light chain constant region;

   wherein the first human immunoglobulin heavy chain variable region encodes a first human heavy chain variable domain identified from a first antibody in a first genetically modified rodent that had been immunized with a first epitope of a first antigen, wherein the first antibody specifically binds the first epitope of the first antigen; and
   wherein the second human immunoglobulin heavy chain variable region encodes a second human heavy chain variable domain identified from a second antibody in a second genetically modified rodent that had been immunized with a second epitope of a second antigen, wherein the second antibody specifically binds the second epitope of the second antigen;
   wherein the first and second genetically modified rodents are each a genetically modified rodent or mouse according to any one of aspects 4-26; and
   wherein the human immunoglobulin λ light chain variable region of the third nucleotide is the single rearranged human immunoglobulin λ light chain variable region or a somatically hypermutated version thereof.
37. A method for making a human immunoglobulin heavy chain, the method comprising the steps of:
   (a) exposing a genetically modified rodent or mouse of any one of aspects 4-26 to an antigen of interest;
   (b) obtaining a human immunoglobulin heavy chain variable domain sequence of an antibody that specifically binds the antigen and that was generated by the genetically modified rodent; and
   (c) operably linking the human immunoglobulin heavy variable domain sequence to a human immunoglobulin heavy chain constant domain sequence to form a human immunoglobulin heavy chain.
38. A method for making a human immunoglobulin heavy chain variable domain, the method comprising the steps of:
   (a) exposing a genetically modified rodent or mouse of any one of aspects 4-26 to antigen of interest; and
   (b) obtaining a human immunoglobulin heavy chain variable domain sequence of an antibody that specifically binds the antigen and that was generated by the genetically modified rodent.
39. A method of making a collection of human immunoglobulin heavy chain variable domains, comprising
   (a) exposing a genetically modified rodent or mouse of any one of aspects 4-26 to an antigen of interest, and
   (b) isolating the collection of human immunoglobulin heavy chain variable domains from the genetically modified rodent,

   wherein the collection of human immunoglobulin heavy chain variable domains each bind to a human immunoglobulin λ light chain variable domain expressed from the single rearranged human immunoglobulin λ light chain variable region or a somatically hypermutated version thereof, and
   wherein the human λ light chain variable domain paired with any one of the human immunoglobulin heavy chain variable domains in the collection binds the antigen.
40. A method for making a human immunoglobulin λ light chain, the method comprising the steps of:
   (a) exposing a genetically modified rodent or mouse of any one of aspects 1-26 to an antigen of interest;
   (b) obtaining a human immunoglobulin λ light chain variable domain sequence of an antibody that specifically binds the antigen and that was generated by the genetically modified rodent; and
   (c) operably linking the human immunoglobulin λ light variable domain sequence to a human immunoglobulin λ light chain constant domain sequence to form a human immunoglobulin λ light chain.
41. A method for generating a human immunoglobulin λ light chain variable domain, the method comprising the steps of:
   (a) exposing a genetically modified rodent or mouse of any one of aspects 1-26 to an antigen of interest; and
   (b) obtaining a human immunoglobulin λ light chain variable domain sequence of an antibody that specifically binds the antigen and that was generated by the genetically modified rodent.
42. A method for making a nucleotide sequence encoding a human immunoglobulin heavy chain, the method comprising the steps of:
   (a) exposing a genetically modified rodent or mouse of any one of aspects 4-26 to an antigen of interest;
   (b) obtaining a human immunoglobulin heavy chain variable region encoding a human immunoglobulin heavy chain variable domain sequence of an antibody that specifically binds the antigen and that was generated by the genetically modified rodent; and
   (c) operably linking the human immunoglobulin heavy variable region to a human immunoglobulin heavy chain constant region sequence to form a nucleotide sequence encoding a human immunoglobulin heavy chain.
43. A method for making a nucleotide sequence comprising a human immunoglobulin heavy chain variable region, the method comprising the steps of:
   (a) exposing a genetically modified rodent or mouse of any one of aspects 4-26 to an antigen of interest; and
   (b) obtaining a human immunoglobulin heavy chain variable region encoding a human immunoglobulin heavy chain variable domain sequence of an antibody that specifically binds the antigen and that was generated by the genetically modified rodent.
44. A method for making a nucleotide sequence encoding a human immunoglobulin λ light chain, the method comprising the steps of:
   (a) exposing a genetically modified rodent or mouse of any one of aspects 1-26 to an antigen of interest;
   (b) obtaining a human immunoglobulin λ light chain variable region encoding a human immunoglobulin λ light chain variable domain sequence of an antibody that specifically binds the antigen and that was generated by the genetically modified rodent; and
   (c) operably linking the human immunoglobulin λ light variable region to a human immunoglobulin λ light chain constant region sequence to form a nucleotide sequence encoding a human immunoglobulin λ light chain.
45. A method for making a nucleotide sequence comprising a human immunoglobulin λ light chain variable region, the method comprising the steps of:
   (a) exposing a genetically modified rodent or mouse of any one of aspects 1-26 to an antigen of interest; and
   (b) obtaining a human immunoglobulin λ light chain variable region encoding a human immunoglobulin λ light chain variable domain sequence of an antibody that specifically binds the antigen and that was generated by the genetically modified rodent.
46. A targeting vector comprising:
   (i) a 5' homology arm comprising a nucleotide sequence corresponding to a 5' target sequence in an endogenous rodent κ light chain locus;
   (ii) a single rearranged human immunoglobulin λ light chain variable region comprising a Vλ gene segment and a Jλ gene segment;
   (iii) a rodent Cλ gene segment; and
   (iv) a 3' homology arm comprising a nucleotide sequence corresponding to a 3' target sequence in the endogenous rodent κ light chain locus.
47. A method of making a genetically modified rodent comprising the steps of:
   (a) introducing a single rearranged human immunoglobulin λ light chain variable region comprising a human Vλ gene segment and a human Jλ gene segment into an engineered endogenous immunoglobulin κ light chain locus in the genome of a rodent ES cell; and
   (b) generating a rodent using the rodent ES cell generated in step (a).
48. A method of making a genetically modified rodent comprising the steps of:
   (a) introducing a single rearranged human immunoglobulin λ light chain variable region operably linked to a rodent Cλ gene segment into an engineered endogenous immunoglobulin κ light chain locus in the genome of a rodent ES cell, wherein the single rearranged human immunoglobulin λ light chain variable region comprises a human Vλ gene segment and a human Jλ gene segment; and
   (b) generating a rodent using the rodent ES cell generated in step (a).
49. A method of making a genetically modified rodent ES cell comprising the steps of:
   introducing a single rearranged human immunoglobulin λ light chain variable region comprising a human Vλ gene segment and a human Jλ gene segment into an engineered endogenous immunoglobulin κ light chain locus in the genome of a rodent ES cell.
50. A method of making a genetically modified rodent ES cell comprising the steps of:
   introducing a single rearranged human immunoglobulin λ light chain variable region operably linked to a rodent Cλ gene segment into an engineered endogenous immunoglobulin κ light chain locus in the genome of a rodent ES cell, wherein the single rearranged human immunoglobulin λ light chain variable region comprises a human Vλ gene segment and a human Jλ gene segment.
51. A method of making a genetically modified rodent, comprising the step of:
   (a) engineering an endogenous immunoglobulin κ light chain locus in the germline genome of the rodent to comprise a single rearranged human immunoglobulin λ light chain variable region operably linked to a rodent Cλ gene segment, wherein the single rearranged human immunoglobulin λ light chain variable region comprises a human Vλ gene segment and a human Jλ gene segment,
   so that all immunoglobulin λ light chains expressed by B cells of the genetically modified rodent include human immunoglobulin λ light chain variable domains expressed from the single rearranged human immunoglobulin λ light chain variable region or a somatically hypermutated version thereof.
52. An *in vitro* method for generating a recombinant rodent cell, comprising modifying the genome of the recombinant rodent cell to comprise:
   an engineered endogenous immunoglobulin κ light chain locus comprising a single rearranged human immunoglobulin λ light chain variable region operably linked to a rodent Cλ gene segment, wherein the single rearranged human immunoglobulin λ light chain variable region comprises a human Vλ gene segment and a human Jλ gene segment.

## Claims

1. A method of making a bispecific antibody, the method comprising:
(a) contacting a first genetically modified rodent with a first epitope of a first antigen,
(b) contacting a second genetically modified rodent with a second epitope of a second antigen, wherein the first and second genetically modified rodents are each a genetically modified rodent whose germline genome comprises:
(i) an engineered endogenous immunoglobulin κ light chain locus comprising a single rearranged human immunoglobulin λ light chain variable region operably linked to a rodent Cλ gene segment, wherein the single rearranged human immunoglobulin λ light chain variable region comprises a human Vλ gene segment and a human Jλ gene segment, wherein the rodent Cλ gene segment is in place of an endogenous Cκ gene segment, and wherein the single rearranged human immunoglobulin λ light chain variable region is in place of one or more rodent Vκ gene segments, one or more rodent Jκ gene segments, or any combination thereof, and
(ii) an engineered endogenous immunoglobulin heavy chain locus comprising one or more unrearranged human V_{H} gene segments, one or more unrearranged human D_{H} gene segments, and one or more unrearranged human J_{H} gene segments operably linked to one or more rodent immunoglobulin heavy chain constant region genes,
wherein all immunoglobulin λ light chains expressed by B cells of the genetically modified rodent include human immunoglobulin λ light chain variable domains expressed from the single rearranged human immunoglobulin λ light chain variable region or a somatically hypermutated version thereof,
wherein all heavy chains expressed by B cells of the genetically modified rodent include human immunoglobulin heavy chain variable domains and rodent immunoglobulin heavy chain constant domains, and
wherein the genetically modified rodent is a rat or a mouse;
(c) isolating a B cell that expresses a first antibody specific for the first epitope of the first antigen from the first genetically modified rodent and determining a first human immunoglobulin heavy chain variable domain of the first antibody;
(d) isolating a B cell that expresses a second antibody specific for the second epitope of the second antigen from the second genetically modified rodent and determining a second human immunoglobulin heavy chain variable domain of the second antibody;
(e) operably linking a nucleotide sequence encoding the first human immunoglobulin heavy chain variable domain to a nucleotide sequence encoding a first human immunoglobulin constant domain to produce a first nucleotide sequence encoding a first human heavy chain;
(f) operably linking a nucleotide sequence encoding the second human immunoglobulin heavy chain variable domain to a nucleotide sequence encoding a second human immunoglobulin constant domain to produce a second nucleotide sequence encoding a second human heavy chain;
(g) expressing in a mammalian cell:
(i) the first nucleotide sequence;
(ii) the second nucleotide sequence; and
(iii) a third nucleotide sequence comprising the single rearranged human immunoglobulin λ light chain variable region or a somatically hypermutated version thereof operably linked to a human immunoglobulin λ light chain constant region.

2. A method of making a bispecific antibody, the method comprising:
expressing in a mammalian cell:
(i) a first nucleotide sequence comprising a first human immunoglobulin heavy chain variable region operably linked to a first human immunoglobulin constant region;
(ii) a second nucleotide sequence comprising a second human immunoglobulin heavy chain variable region operably linked to a second human immunoglobulin constant region; and
(iii) a third nucleotide sequence comprising a human immunoglobulin λ light chain variable region operably linked to a human immunoglobulin λ light chain constant region;
wherein the first human immunoglobulin heavy chain variable region encodes a first human heavy chain variable domain identified from a first antibody in a first genetically modified rodent that had been immunized with a first epitope of a first antigen, wherein the first antibody specifically binds the first epitope of the first antigen; and
wherein the second human immunoglobulin heavy chain variable region encodes a second human heavy chain variable domain identified from a second antibody in a second genetically modified rodent that had been immunized with a second epitope of a second antigen, wherein the second antibody specifically binds the second epitope of the second antigen;
wherein the first and second genetically modified rodents are each a genetically modified rodent whose germline genome comprises:
(i) an engineered endogenous immunoglobulin κ light chain locus comprising a single rearranged human immunoglobulin λ light chain variable region operably linked to a rodent Cλ gene segment, wherein the single rearranged human immunoglobulin λ light chain variable region comprises a human Vλ gene segment and a human Jλ gene segment, wherein the rodent Cλ gene segment is in place of an endogenous Cκ gene segment, and wherein the single rearranged human immunoglobulin λ light chain variable region is in place of one or more rodent Vκ gene segments, one or more rodent Jκ gene segments, or any combination thereof, and
(ii) an engineered endogenous immunoglobulin heavy chain locus comprising one or more unrearranged human V_{H} gene segments, one or more unrearranged human D_{H} gene segments, and one or more unrearranged human J_{H} gene segments operably linked to one or more rodent immunoglobulin heavy chain constant region genes,
wherein all immunoglobulin λ light chains expressed by B cells of the genetically modified rodent include human immunoglobulin λ light chain variable domains expressed from the single rearranged human immunoglobulin λ light chain variable region or a somatically hypermutated version thereof,
wherein all heavy chains expressed by B cells of the genetically modified rodent include human immunoglobulin heavy chain variable domains and rodent immunoglobulin heavy chain constant domains, and
wherein the genetically modified rodent is a rat or a mouse; and
wherein the human immunoglobulin λ light chain variable region of the third nucleotide is the single rearranged human immunoglobulin λ light chain variable region or a somatically hypermutated version thereof.

3. The method of claim 1 or 2, wherein:
(a) the germline genome of the genetically modified rodent is:
(i) homozygous for the engineered endogenous immunoglobulin κ light chain locus, or
(ii) heterozygous for the engineered endogenous immunoglobulin κ light chain locus; and/or
(b) the germline genome of the genetically modified rodent is homozygous for the engineered endogenous immunoglobulin heavy chain locus.

4. The method of any one of claims 1-3, wherein:
(a) the genetically modified rodent lacks a rodent Cκ gene at the engineered endogenous immunoglobulin κ light chain locus; and/or
(b) the human Vλ gene segment comprises Vλ1-51, Vλ5-45, Vλ1-44, Vλ1-40, Vλ3-21, or Vλ2-14; and/or
(c) the human Jλ gene segment comprises Jλ1, Jλ2, Jλ3, Jλ6, or Jλ7.

5. The method of any one of claims 1-4:
(a) wherein the one or more unrearranged human V_{H} gene segments, one or more unrearranged human D_{H} gene segments, and one or more unrearranged human J_{H} gene segments are in place of one or more endogenous V_{H} gene segments, one or more endogenous D_{H} gene segments, one or more endogenous J_{H} gene segments, or a combination thereof,
optionally wherein the one or more rodent immunoglobulin heavy chain constant region genes are one or more endogenous rodent immunoglobulin heavy chain constant region genes; and/or
(b) wherein the one or more unrearranged human V_{H} gene segments, one or more unrearranged human D_{H} gene segments, and one or more unrearranged human J_{H} gene segments replace one or more endogenous V_{H} gene segments, one or more endogenous D_{H} gene segments, and one or more endogenous J_{H} gene segments, respectively,
optionally wherein the one or more rodent immunoglobulin heavy chain constant region genes are one or more endogenous rodent immunoglobulin heavy chain constant region genes; and/or
(c) wherein:
(i) the one or more unrearranged human V_{H} gene segments comprise V_{H}3-74, V_{H}3-73, V_{H}3-72, V_{H}2-70, V_{H}1-69, V_{H}3-66, V_{H}3-64, V_{H}4-61, V_{H}4-59, V_{H}1-58, V_{H}3-53, V_{H}5-51, V_{H}3-49, V_{H}3-48, V_{H}1-46, V_{H}1-45, V_{H}3-43, V_{H}4-39, V_{H}4-34, V_{H}3-33, V_{H}4-31, V_{H}3-30, V_{H}4-28, V_{H}2-26, V_{H}1-24, V_{H}3-23, V_{H}3-21, V_{H}3-20, V_{H}1-18, V_{H}3-15, V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2, V_{H}6-1, or any combination thereof,
(ii) the one or more unrearranged human D_{H} gene segments comprise D_{H}1-1, D_{H}2-2, D_{H}3-3, D_{H}4-4, D_{H}5-5, D_{H}6-6, D_{H}1-7, D_{H}2-8, D_{H}3-9, D_{H}3-10, D_{H}5-12, D_{H}6-13, D_{H}2-15, D_{H}3-16, D_{H}4-17, D_{H}6-19, D_{H}1-20, D_{H}2-21, D_{H}3-22, D_{H}6-25, D_{H}1-26, D_{H}7-27, or any combination thereof, and
(iii) the one or more unrearranged human J_{H} gene segments comprise J_{H}1, J_{H}2, J_{H}3, J_{H}4, J_{H}5, J_{H}6, or any combination thereof; and/or
(d) wherein the germline genome of the genetically modified rodent comprises one or more nucleotide sequences encoding one or more rodent ADAM6 polypeptides, functional orthologs, functional homologs, or functional fragments thereof.

6. The method of any one of claims 1-5:
(a) wherein the rodent Cλ gene has a sequence that is at least 80% identical to: (i) a mouse Cλ1, (ii) a mouse Cλ2, or (iii) a mouse Cλ3 gene; and/or
(b) wherein the rodent Cλ gene comprises a mouse Cλ gene or a mouse Cλ1 gene; and/or
(c) further comprising an inactivated endogenous immunoglobulin λ light chain locus; and/or
(d) wherein the engineered endogenous immunoglobulin κ light chain locus further comprises one or more endogenous enhancers at their endogenous location in the endogenous immunoglobulin κ light chain locus.

7. The method of any one of claims 1-6, wherein the rodent is a mouse and the single rearranged human immunoglobulin λ light chain variable region is operably linked to a mouse Cλ1 gene segment, and wherein the single rearranged human immunoglobulin λ light chain variable region comprises a human Vλ1-51 gene segment and a human Jλ2 gene segment, or a human Vλ2-14 gene segment and a human Jλ2 gene segment.

8. The method of any one of claims 1-7, wherein the first and second genetically modified rodents are the same genetically modified rodent.

9. The method of any one of claims 1-7, wherein the first and second genetically modified rodents are different genetically modified rodents.

10. The method of any one of claims 1-9, wherein the first and second antigens are the same antigen, and the first and second epitopes are different epitopes.

11. The method of any one of claims 1-10, wherein the first and second antigens are different antigens.

12. A method for making a human immunoglobulin heavy chain, the method comprising the steps of:
(a) exposing a genetically modified rodent to an antigen of interest, wherein the germline genome of the genetically modified rodent comprises:
(i) an engineered endogenous immunoglobulin κ light chain locus comprising a single rearranged human immunoglobulin λ light chain variable region operably linked to a rodent Cλ gene segment, wherein the single rearranged human immunoglobulin λ light chain variable region comprises a human Vλ gene segment and a human Jλ gene segment, wherein the rodent Cλ gene segment is in place of an endogenous Cκ gene segment, and wherein the single rearranged human immunoglobulin λ light chain variable region is in place of one or more rodent Vκ gene segments, one or more rodent Jκ gene segments, or any combination thereof, and
(ii) an engineered endogenous immunoglobulin heavy chain locus comprising one or more unrearranged human V_{H} gene segments, one or more unrearranged human D_{H} gene segments, and one or more unrearranged human J_{H} gene segments operably linked to one or more rodent immunoglobulin heavy chain constant region genes,
wherein all immunoglobulin λ light chains expressed by B cells of the genetically modified rodent include human immunoglobulin λ light chain variable domains expressed from the single rearranged human immunoglobulin λ light chain variable region or a somatically hypermutated version thereof,
wherein all heavy chains expressed by B cells of the genetically modified rodent include human immunoglobulin heavy chain variable domains and rodent immunoglobulin heavy chain constant domains, and
wherein the genetically modified rodent is a rat or a mouse;
(b) obtaining a human immunoglobulin heavy chain variable domain sequence of an antibody that specifically binds the antigen and that was generated by the genetically modified rodent; and
(c) operably linking the human immunoglobulin heavy variable domain sequence to a human immunoglobulin heavy chain constant domain sequence to form a human immunoglobulin heavy chain.

13. A human immunoglobulin heavy chain generated by the method of claim 12.

14. A method for making a human immunoglobulin λ light chain, the method comprising the steps of:
(a) exposing a genetically modified rodent to an antigen of interest, wherein the germline genome of the genetically modified rodent comprises:
an engineered endogenous immunoglobulin κ light chain locus comprising a single rearranged human immunoglobulin λ light chain variable region operably linked to a rodent Cλ gene segment, wherein the single rearranged human immunoglobulin λ light chain variable region comprises a human Vλ gene segment and a human Jλ gene segment, wherein the rodent Cλ gene segment is in place of an endogenous Cκ gene segment, and wherein the single rearranged human immunoglobulin λ light chain variable region is in place of one or more rodent Vκ gene segments, one or more rodent Jκ gene segments, or any combination thereof,
wherein all immunoglobulin λ light chains expressed by B cells of the genetically modified rodent include human immunoglobulin λ light chain variable domains expressed from the single rearranged human immunoglobulin λ light chain variable region or a somatically hypermutated version thereof, and
wherein the genetically modified rodent is a rat or a mouse;
(b) obtaining a human immunoglobulin λ light chain variable domain sequence of an antibody that specifically binds the antigen and that was generated by the genetically modified rodent; and
(c) operably linking the human immunoglobulin λ light variable domain sequence to a human immunoglobulin λ light chain constant domain sequence to form a human immunoglobulin λ light chain.

15. A human immunoglobulin λ light chain generated by the method of claim 14.
